(19) Europäisches Patentamt
European Patent Office
Office européen des brevets

(11) EP 1 219 631 A1

(12) **EUROPEAN PATENT APPLICATION**
published in accordance with Art. 158(3) EPC

(43) Date of publication:
**03.07.2002 Bulletin 2002/27**

(21) Application number: **00954922.1**

(22) Date of filing: **23.08.2000**

(51) Int Cl.[7]: **C07J 1/00**, C07J 31/00, C07J 41/00, A61K 31/568

(86) International application number:
**PCT/JP00/05636**

(87) International publication number:
**WO 01/14406 (01.03.2001 Gazette 2001/09)**

(84) Designated Contracting States:
**AT BE CH CY DE DK ES FI FR GB GR IE IT LI LU MC NL PT SE**
Designated Extension States:
**AL LT LV MK RO SI**

(30) Priority: **23.08.1999 JP 27495699**
**22.10.1999 JP 33833499**
**30.06.2000 JP 2000237721**
**19.07.2000 JP 2000219800**

(71) Applicant: **CHUGAI SEIYAKU KABUSHIKI KAISHA**
**Tokyo, 115-8543 (JP)**

(72) Inventors:
- **KAIHO, Shin-Ichi Chugai Seiyaku KK Gotenba-shi Shizuoka 412-8513 (JP)**
- **OHIZUMI, Iwao, Chugai Seiyaku Kabushiki Kaisha Gotenba-shi Shizuoka 412-8513 (JP)**
- **TAMURA, Kunio, Chugai Seiyaku Kabushiki Kaisha Gotenba-shi Shizuoka 412-8513 (JP)**
- **KATO, Nobuaki, Chugai Seiyaku Kabushiki Kaisha Gotenba-shi Shizuoka 412-8513 (JP)**
- **YONEYA, Takaaki, Chugai Seiyaku Kabushiki Kaisha Gotenba-shi Shizuoka 412-8513 (JP)**
- **TACHIBANA, Kazutaka, Chugai Seiyaku KK Gotenba-shi Shizuoka 412-8513 (JP)**

(74) Representative: **VOSSIUS & PARTNER**
**Siebertstrasse 4**
**81675 München (DE)**

(54) **ANTIANDROGEN AGENTS**

(57) Compounds represented by the general formula (I), pharmaceutically acceptable salts thereof, or prodrugs of the compounds or their salts:

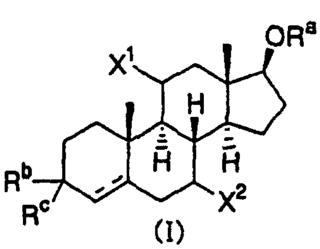

(I)

[wherein $X^1$ and $X^2$ represent independently a hydrogen atom or a group represented by the general formula (II)

$$-Ar-A-R^1 \qquad (II)$$

$R^a$ represents a hydrogen atom or a protective group of a hydroxyl group, $R^b$ and $R^c$, when taken together with the carbon atom in 3-position to which they are bound, represent an optionally protected -(C=O)-, and the dashed line in combination with the solid line represents the formation of a single bond or a double bond;

in addition, Ar represents a single bond or an aromatic hydrocarbon group, A represents a methylene group or -O-, $R^1$ represents an optionally substituted alkyl group, an optionally substituted alkenyl group or an optionally substituted alkynyl group;

provided that $X^1$ and $X^2$ are not a hydrogen atom at the same time], as well as substances that act as antagonist against but not as agonist for the androgen receptor, pharmaceutically acceptable salts thereof, or prodrugs of the substances or their salts are useful as antiandrogenic agents and may be used as preventives or therapeutics of a disease selected from prostate cancer, prostatomegaly, male pattern alopecia, sexual prematurity, acne vulgaris, seborrhea and hursutism.

EP 1 219 631 A1

**Description**

TECHNICAL FIELD

This invention relates to androstane derivatives having various substituents in 7- or 11-position, substances that act as antagonist against but not as agonist for the androgen receptor, and pharmaceuticals that contain said androstane derivatives and said substances.

BACKGROUND ART

It has become known to date that prostate cancer, prostatomegaly, male pattern alopecia, sexual prematurity, acne vulgaris, seborrhea and hursutism are closely associated with the male hormone, androgen. For example, it is known that prostate cancer and prostatomegaly are rare in castrated men and patients with gonad dysfunction.

Already used antiandrogenic agents, or agonists for the androgen receptor, include, for example, cyproterone acetate, chlormadinone acetate, flutamide and bicaltamide. Cyproterone acetate is known to suppress the progress of acne and the onset of baldness in the teens. Cyproterone acetate is also used in women for treatment of masculinization and alopecia. Flutamide and bicaltamide are used as therapeutics for prostatomegaly.

These antiandrogenic agents have exhibited marked efficacy in many cases including drug therapy of prostate cancer and comprise an important part of the effective therapeutics. However, one of the problems with these antiandrogenic agents is that even if they exhibit marked efficacy, recurrence is common in almost all cases after the lapse of two to five years; in other words, they are known to induce androgen tolerance.

It was recently reported that hydroxyflutamide, the active essence of flutamide, elevated the transcriptional activity of the androgen receptor at a concentration of 10 mol/L. Plasma levels of hyddroxyflutamide in prostate cancer patients under flutamide treatment are several mol/L which,
according to the report, is the level at which the agonist action is manifested (see J. Biol. Chem., vol. 270, 19998-20003, 1995). It was also reported that a two-week continuous administration of cyproterone acetate and chlormadinone acetate to castrated rats increased the prostate weight (Folia endocrinol., vol. 66, 597-606, 1990). As for flutamide and bicartamide, cases of side effects such as hepatoxicity have also been reported.

Speaking of the so-called pure antagonists which are substances that act as antagonist against but not as agonist for a nuclear receptor, namely, substances that can completely inhibit the action of the receptor, they have been known for the estrogen receptor (see, for example, WO98/25916, European Patent Publication No. 0138504, USP 4,659,516 and Cancer Res., 1991, 51, 3867). The molecular structures of the hormone-binding domains of nuclear receptors are being unravelled by X-ray crystallography and the like for RXR (retinoid-X receptor), RAR (retinoic acid receptor) and the like (see, for example, Nature, vol. 375, 377-382, 1995).

WO97/49709 discloses androgen receptor modifiers that are nonsteroidal four-ring compounds.

Steroid compounds having an aminocarbonylalkyl group in 7-position or an aminocarbonylalkynyl group in 17-position are known by being described in WO91/00732. These are androgen synthesis inhibitors and/or substances that act as antanosit against the androgen receptor and steroid compounds are disclosed that have a freely selectable double bond in 1(2) position, 4(5) position, 6(7) position, 9(10) position and/or 11(12) position in the general formula and the only specific compounds that are disclosed have a double bond in 4(5) position. One of the compounds that are mentioned as the most preferred is EM-101 which is a steroid compound having a 10-(N-butyl-N-methylaminocarbonyl)decyl group in 7α-position and a hydroxyl group in 17β-position. However, these compounds have problems such as inadequate antagonist action against the androgen receptor, strong toxicity, etc.

As a steroid compound having an aromatic ring or an alkyloxy group in 11-position, RU486 is described in WO95/17192 and known as an agent for dealing with multidrug tolerance.

DISCLOSURE OF INVENTION

An object of the this invention is to provide androstane derivatives having various substituents in 7- or 11-position, pharmaceutically acceptable salts thereof, or prodrugs of the derivatives or their salts.

Another object of this invention is to provide substances that act as antagonist against but not as agonist for the androgen receptor, pharmaceutically acceptable salts thereof, or prodrugs of the substances or their salts.

Still another object of this invention is to provide pharmaceuticals that contain said androstane derivatives and pharmaceuticals that contain said substances.

With a view to attaining these objects, the present inventor hypothesized that one of the causes of side effects, such as androgen tolerance and the increase in prostate weight, that are developed by heretofore known antagonists against the androgen receptor is the proliferation of androgen-responsive cells (e.g. prostate cells) due to the agonist action possessed by said antagonists, and anticipated that finding a pure antagonist against the androgen receptor,

namely, an antagonist that does not act as agonist for the androgen receptor, would lead to the finding of antiandrogenic agents that do not show any side effects such as the development of androgen tolerance and hepatoxicity after prolonged administration; the inventor then undertook the designing of said antagonist. To begin with, the androgen receptor was modelled from existing nuclear receptors such as RXR and RAR by the homology technique using software packages such as Homology (from MSI) and Look (from MAG). Second, it was found that if a pure antagonist against the androgen receptor was designed by using testosterone and/or dihydrotestosterone as a ligand and, with the resulting model of a complex between said ligand and the androgen receptor being utilized, by introducing into suitable positions those side chains which had suitable lengths and functional groups to form the interaction with the receptor, substances or compounds could be designed that could be anticipated to act as pure antagonist against the androgen receptor and/or antiandrogenic agents that had lesser side effects such as lower hepatoxicity; the present invention has been accomplished on the basis of this finding.

According to a first aspect of this invention, there are provided compounds represented by the general formula (I), pharmaceutically acceptable salts thereof, or prodrugs of the compounds or their salts:

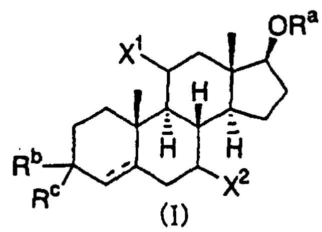

(I)

[wherein $X^1$ and $X^2$ represent independently a hydrogen atom or a group represented by the general formula (II)

$$-Ar-A-R^1 \qquad (II)$$

$R^a$ represents a hydrogen atom or a protective group of a hydroxyl group, $R^b$ and $R^c$, when taken together with the carbon atom in 3-position to which they are bound, represent an optionally protected -(C=O)-, and the dashed line in combination with the solid line represents the formation of a single bond or a double bond;

in addition, Ar represents a single bond or an aromatic hydrocarbon group, A represents a methylene group or -O-, $R^1$ represents an optionally substituted alkyl group, an optionally substituted alkenyl group or an optionally substituted alkynyl group;

provided that $X^1$ and $X^2$ are not a hydrogen atom at the same time].

According to a second aspect of this invention, there are provided substances that act as antagonist against but not as agonist for the androgen receptor, pharmaceutically acceptable salts thereof, or prodrugs of the substances or their salts.

According to a third aspect of this invention, there are provided pharmaceuticals that contain compounds represented by the general formula (I), as well as pharmaceuticals that contain substances that act as antagonist against but not as agonist for the androgen receptor.

## BEST MODE FOR CARRYING OUT THE INVENTION

In this specification, straight-chained or branched alkyl groups having 1 - 3 carbon atoms include methyl group, ethyl group, n-propyl group and i-propyl group.

Straight-chained or branched alkyl groups having 1 - 6 carbon atoms include, for example, methyl group, ethyl group, n-propyl group, i-propyl group, n-butyl group, s-butyl group, i-butyl group, t-butyl group, n-pentyl group, 3-methylbutyl group, 2-methylbutyl group, 1-methylbutyl group, 1-ethylpropyl group and n-hexyl group.

In this specification, ω position means the terminal position of a divalent group which is other than 1-position. For example, in hexane-1,6-diyl group, ω position is 6-position.

In this specification, the single bond means that the group of interest does not exist but that the groups adjacent both sides of said group directly form a single bond. For example, to say Ar is a single bond in the group represented by the general formula (II) shows that 7-position and/or 11-position of the steroid ring in the compound represented by the general formula (I) and A directly form a single bond.

In this specification, to say that the dashed line in combination with the solid line represents the formation of

a single bond or a double bond means, for example, that the bond between 4-position and 5-position of the steroid ring denoted by the dashed line is a single bond or a double bond. This is also true with compound (2) in process A to be described later and it is meant that the bond between 5-position and 6-position of the steroid ring denoted by the dashed line is a single bond or a double bond.

In the definition of the compounds represented by the general formula (I), $X^1$ and $X^2$ represent independently a hydrogen atom or a group represented by the general formula (II)

$$-Ar-A-R^1 \qquad (II)$$

(wherein, in addition, Ar represents a single bond or an aromatic hydrocarbon group, A represents a methylene group or -O-, $R^1$ represents an optionally substituted alkyl group, an optionally substituted alkenyl group or an optionally substituted alkynyl group); preferred are the case where $X^1$ is $-Ar-A-R^1$ (wherein Ar, A and $R^1$ have the same meanings as defined above) and $X^2$ is a hydrogen atom, and the case where $X^1$ is a hydrogen atom and $X^2$ is $-Ar-A-R^1$ (wherein Ar, A and $R^1$ have the same meanings as defined above). Further preferred are compounds in which the steric configuration of $X^1$ in 11-position of the steroid ring is β configuration and those in which the steric configuration of $X^2$ in 7-position is α configuration. Note that $X^1$ and $X^2$ are not a hydrogen atom at the same time.

While $R^a$ represents a hydrogen atom or a protective group of a hydroxyl group, a hydrogen atom is preferred. Protective groups of a hydroxyl group include acyl groups such as formyl group, acetyl group, propionyl group, butyryl group, isobutyryl group, valeryl group, isovaleryl group, pivaloyl group, caproyl group, trifluoroacetyl group and benzoyl group, alkoxycarbonyl groups such as methoxycarbonyl group, ethoxycarbonyl group, propoxycarbonyl group, isopropoxycarbonyl group, allyloxycarbonyl group, benzyloxycarbonyl group and phenoxycarbonyl group, substituted silyl groups such as trimethylsilyl group, triethylsilyl group, triisopropylsilyl group, dimethylisopropylsilyl group, diethylisopropylsilyl group, dimethyltexylsilyl group, t-butyldimethylsilyl group, t-butyldiphenylsilyl group, tribenzylsilyl group, tri-p-xylylsilyl group, triphenylsilyl group, diphenylmethylsilyl group and t-butylmethoxyphenylsilyl group, substituted methyl groups such as methoxymethyl group, methoxyethoxymethyl group, methylthiomethyl group, t-butylthiomethyl group, β-trichloroethyloxymethyl group, trimethylsilylethoxymethyl group, p-methoxybenzyloxymethyl group and p-chlorobenzyloxymethyl group, 2-oxacycloalkyl groups such as tetrahydrofurallyl and tetrahydropyranyl, and aralkyl groups such as benzyl group. Among these, substituted silyl groups such as trimethylsilyl group, triethylsilyl group, triisopropylsilyl group, dimethylisopropylsilyl group, diethylisopropylsilyl group, dimethyltexylsilyl group, t-butyldimethylsilyl group, t-butyldiphenylsilyl group, tribenzylsilyl group, tri-p-xylylsilyl group, triphenylsilyl group, diphenylmethylsilyl group and t-butylmethoxyphenylsilyl group, as well as substituted methyl groups such as methoxymethyl group, methoxyethoxymethyl group, methylthiomethyl group, t-butylthiomethyl group, β-trichloroethyloxymethyl group, trimethylsilylethoxymethyl group, p-methoxybenzyloxymethyl group and p-chlorobenzyloxymethyl group are preferred, and t-butyldimethylsilyl group and methoxymethyl group are particularly preferred.

$R^b$ and $R^c$, when taken together with the carbon atom in 3-position to which they are bound, represent an optionally protected -(C=O)- and they preferably represent -(C=O)-. Examples of protected -(C=O)- include noncyclic acetals or ketals such as dimethoxystyrene, bis(2,2,2-trichloroethyloxy)methylene, dibenzylmethylene, bis(2-nitrobenzyloxy)methylene, bis(acetyloxy)methylene, bis(methylthio)methylene, bis(ethylthio)methylene, bis(propylthio)methylene, bis(butylthio)methylene, bis(phenylthio)methylene, bis(benzylthio)methylene, bis(acetylthio) methylene, trimethylsilyloxymethylthiomethylene, trimethylsilyloxyethylthiomethylene, trimethylsilyloxyphenylthiomethylene, methyloxymethylthiomethylene, methyloxyphenylthiomethylene, methyloxy-2-(methylthio)ethylthiomethylene, bis(methylselenenyl)methylene and bis(phenylselenenyl)methylene, and cyclic acetals or ketals such as 1,3-dioxane, 5,5-dibromo-1,3-dioxane, 5-(2-pyridyl)-1,3-dioxane, 1,3-dioxolane, 4-bromomethyl-1,3-dioxolane, 4-(3-butenyl)-1,3-dioxolane, 4-phenyl-1,3-dioxolane, 4-(2-nitrophenyl)-1,3-dioxolane, 4,5-dimethoxymethyl-1,3-dioxolane, 1,5-dihydro-3H-2,4-benzodioxepin, 1,3-dithian, 1,3-dithiolan, 1,5-dihydro-3H-2,4-benzodithiepin and 1,3-oxathiolan; preferred are 1,3-dioxane, 1,3-dioxolane and 1,3-dithian, etc. and particularly preferred are 1,3-dioxolane, etc.

The dashed line denotes that in combination with the solid line, it forms a single bond or a double bond; in other words, a single bond and a double bond may be mentioned as the bond between 4-position and 5-position of the steroid ring; preferably, the formation of a single bond is meant. In the case where the dashed line forms a single bond in combination with the solid line, the hydrogen atom in 5-position of the steroid ring is preferably in α configuration.

In the group represented by the general formula (II), Ar represents a single bond or an aromatic hydrocarbon group.

If Ar is an aromatic hydrocarbon group, exemplary aromatic hydrocarbon rings include benzene ring, naphthalene ring, anthracene ring, naphthacene ring, pentacene ring, hexacene ring, phenanthrene ring, triphenylene ring, pyrene ring, chrysene ring, picene ring, perylene ring, pentaphene ring, coronene ring, heptaphene ring, pyranthrene ring and ovalene ring; the benzene ring is preferred. The aromatic hydrocarbon group as Ar means a group having

one bond each in two different positions in the above-mentioned aromatic hydrocarbon rings and the p-phenylene group is preferably mentioned.

A represents a methylene group or -O- and a methylene group is preferred.

If Ar is an aromatic hydrocarbon group, A is preferably -O-.

$R^1$ represents an optionally substituted alkyl group, an optionally substituted alkenyl group or an optionally substituted alkynyl group; preferably, $R^1$ is $R^{1a}$

[where $R^{1a}$ is the general formula (III)

$$-G-E-J-Y-L-Q-Z \qquad (III)$$

{wherein G represents an optionally substituted straight-chained or branched alkylene group having 1 - 30 carbon atoms, an optionally substituted straight-chained or branched alkenylene groups having 2 - 30 carbon atoms or an optionally substituted straight-chained or branched alkynylene group having 2 - 30 carbon atoms, E represents a single bond or -O-, J represents a single bond, an optionally substituted aromatic hydrocarbon group or an optionally substituted heterocyclic group, Y represents a single bond or -O-, L represents a single bond, a straight-chained or branched alkylene group having 1 - 10 carbon atoms, a straight-chained or branched alkenylene group having 2 - 10 carbon atoms or a straight-chained or branched alkynylene group having 2 - 10 carbon atoms, Q represents a single bond or one group selected from among the following formulae:

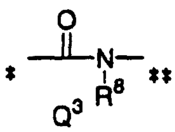
$Q^3$

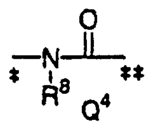
$Q^4$

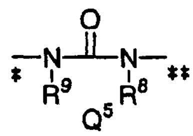
$Q^5$

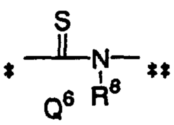
$Q^6$

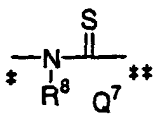
$Q^7$

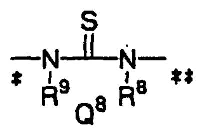
$Q^8$

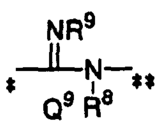
$Q^9$

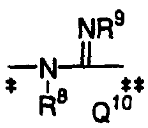
$Q^{10}$

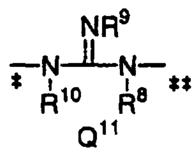
$Q^{11}$

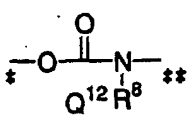
$Q^{12}$

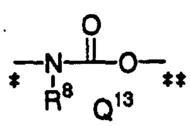
$Q^{13}$

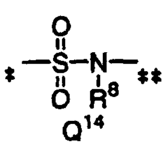
$Q^{14}$

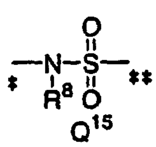
$Q^{15}$

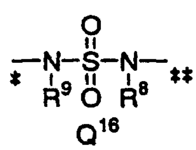
$Q^{16}$

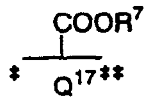
$Q^{17}$

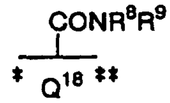
$Q^{18}$

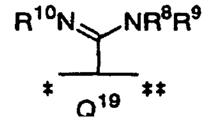
$Q^{19}$

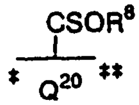
$Q^{20}$

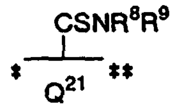
$Q^{21}$

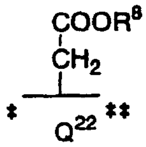
$Q^{22}$

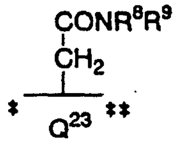
$Q^{23}$

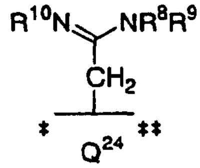
$Q^{24}$

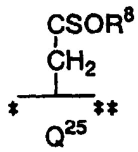
$Q^{25}$

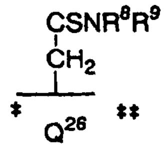
$Q^{26}$

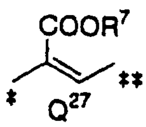
$Q^{27}$

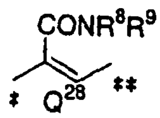
$Q^{28}$

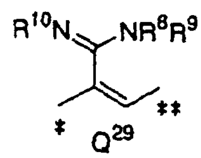
$Q^{29}$

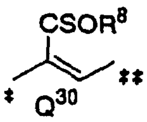
$Q^{30}$

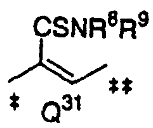
$Q^{31}$

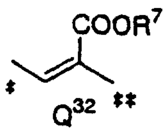
$Q^{32}$

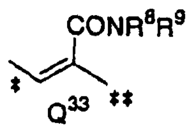
$Q^{33}$

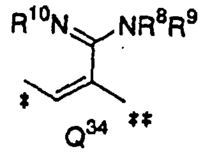
$Q^{34}$

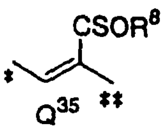
$Q^{35}$

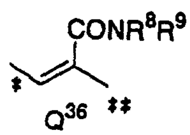
$Q^{36}$

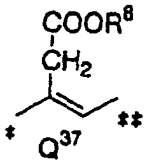
Q37

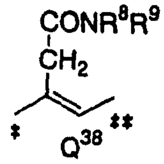
Q38

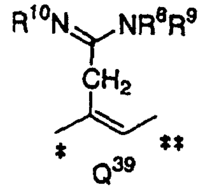
Q39

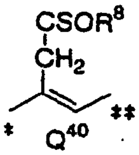
Q40

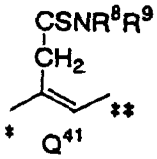
Q41

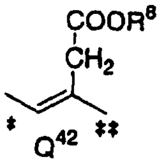
Q42

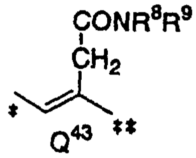
Q43

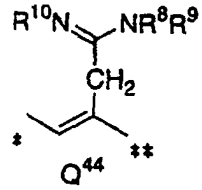
Q44

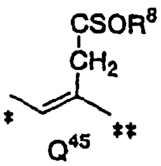
Q45

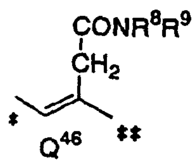
Q46

and

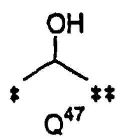
Q47

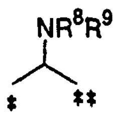
Q48

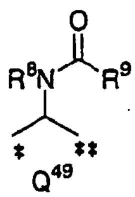
Q49

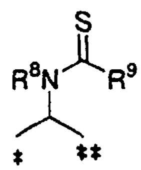
Q50

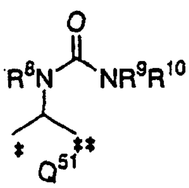
Q51

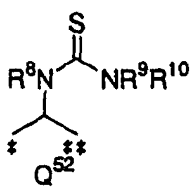
Q52

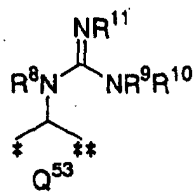
Q53

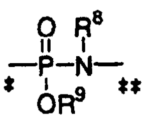
Q54

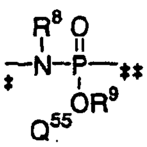
Q55

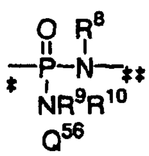
Q56

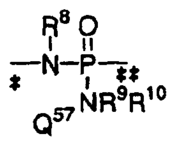
Q57

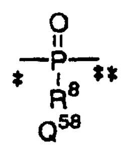
Q58

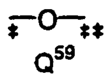
Q59
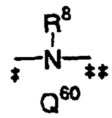
Q60
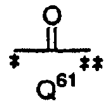
Q61
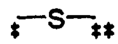
Q62
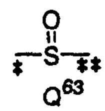
Q63
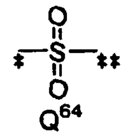
Q64

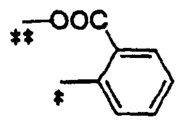
Q65
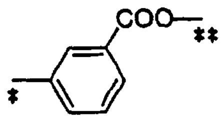
Q66
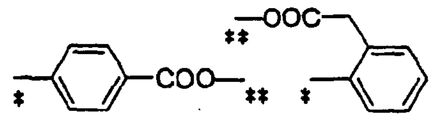
Q67 Q68

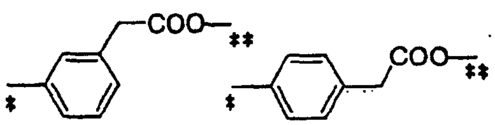
Q69 Q70
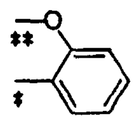
Q71
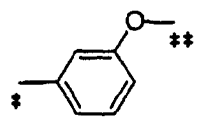
Q72

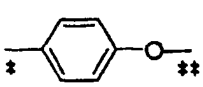
Q73
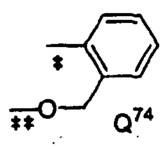
Q74
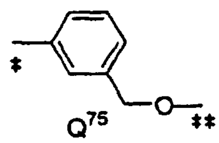
Q75
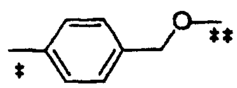
Q76

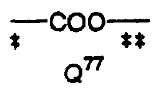
Q77
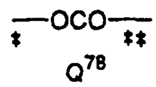
Q78

(where $R^7$ and $R^8$ represent independently a hydrogen atom or a straight-chained or branched lower alkyl group having 1 - 6 carbon atoms, $R^9$, $R^{10}$ and $R^{11}$ each independently represent a hydrogen atom or a straight-chained or branched lower alkyl group having 1 - 3 carbon atoms), Z represents a hydrogen atom, a straight-chained or branched alkyl group having 1 - 10 carbon atoms that may optionally be substituted by a halogen atom, a straight-chained or branched alkenyl group having 2 - 10 carbon atoms that may optionally be substituted by a halogen atom, a straight-chained or branched alkynyl group having 2 - 10 carbon atoms that may optionally be substituted by a halogen atom, $-O-R^d$ (where $R^d$ represents a hydrogen atom or a protective group of a hydroxyl group), or -COOH), provided that when Q is $Q^3$, the nitrogen atom and $R^8$ in $Q^3$ may combine with Z to form a heterocyclic group}].

Examples of the substituent in G which G represents an optionally substituted straight-chained or branched alkylene group having 1 - 30 carbon atoms, an optionally substituted straight-chained or branched alkenylene group having 2 - 30 carbon atoms or an optionally substituted straight-chained or branched alkynylene group having 2 - 30 carbon atoms include $-(CH_2)_m-COOR^{7a}$, $-(CH_2)_p-CONR^{8a}R^{9a}$, $-NR^{8b}R^{9b}$, hydroxyl group, oxo group, etc. Here, m and p represent independently 0 or 1, $R^{7a}$ represents a hydrogen atom or a straight-chained or branched alkyl group having 1 - 6 carbon atoms, $R^{8a}$, $R^{9a}$, $R^{8b}$ and $R^{9b}$ each independently represent a hydrogen atom or a straight-chained or branched alkyl group having 1 - 3 carbon atoms. The substituent is preferably absent or a hydroxyl group and its absence is particularly preferred. In the case where G is substituted, the number of substituents is from one to four, preferably one.

If G represents an optionally substituted straight-chained or branched alkylene group having 1 - 30 carbon atoms, exemplary straight-chained or branched alkylene groups having 1 - 30 carbon atoms include straight-chained alkylene groups such as methylene group, ethane-1,2-diyl group, propane-1,3-diyl group, butane-1,4-diyl group, pentane-1,5-diyl group, hexane-1,6-diyl group, heptane-1,7-diyl group, octane-1,8-diyl group, nonane-1,9-diyl group, decane-1,10-diyl group, undecane-1,11-diyl group, dodecane-1,12-diyl group, tridecane-1,13-diyl group, tetradecane-1,14-diyl group, pentadecane-1,15-diyl group, hexadecane-1,16-diyl group, heptadecane-1,17-diyl group, octadecane-1,18-diyl group, nonadecane-1,19-diyl group, icosane-1,20-diyl group, henicosane-1,21-diyl group, docosane-1,22-diyl group, tricosane-1,23-diyl group, tetracosane-1,24-diyl group, pentacosane-1,25-diyl group, hexacosane-1,26-diyl group, heptacosane-1,27-diyl group, octacosane-1,28-diyl group, nonacosane-1,29-diyl group and triacontane-1,30-diyl group;

as well as branched alkylene groups such as 2-methylpropane-1,3-diyl group, 2-methylbutane-1,4-diyl group, 3-methylbutane-1,4-diyl group, 2,3-dimethylbutane-1,4-diyl group, 2-methylpentane-1,5-diyl group, 3-methylpentane-1,5-diyl group, 4-methylpentane-1,5-diyl group, 2,3-dimethylpentane-1,5-diyl group, 2,4-dimethylpentane-1,5-diyl group, 3,3-dimethylpentane-1,5-diyl group, 3,4-dimethylpentane-1,5-diyl group, 2,3,4-trimethylpentane-1,5-diyl group, 3-ethylpentane-1,5-diyl group, 3-ethyl-2-methylpentane-1,5-diyl group, 3-ethyl-4-methylpentane-1,5-diyl group, 2,4-dimethyl-3-ethylpentane-1,5-diyl group, 2-methylhexane-1,6-diyl group, 3-methylhexane-1,6-diyl group, 4-methylhexane-1,6-diyl group, 5-methylhexane-1,6-diyl group, 2,3-dimethylhexane-1,6-diyl group, 2,4-dimethylhexane-1,6-diyl group, 2,5-dimethylhexane-1,6-diyl group, 3,3-dimethylhexane-1,6-diyl group, 3,4-dimethylhexane-1,6-diyl group, 3,5-dimethylhexane-1,6-diyl group, 4,4-dimethylhexane-1,6-diyl group, 4,5-dimethylhexane-1,6-diyl group, 2,3,3-trimethylhexane-1,6-diyl group, 2,3,4-trimethylhexane-1,6-diyl group, 2,3,5-trimethylhexane-1,6-diyl group, 2,4,4-trimethylhexane-1,6-diyl group, 2,4,5-trimethylhexane-1,6-diyl group, 3,3,4-trimethylhexane-1,6-diyl group, 3,3,5-trimethylhexane-1,6-diyl group, 3,4,5-trimethylhexane-1,6-diyl group, 4,4,5-trimethylhexane-1,6-diyl group, 2,3,4,5-tetramethylhexane-1,6-diyl group, 3-ethylhexane-1,6-diyl group, 4-ethylhexane-1,6-diyl group, 3-ethyl-2-methylhexane-1,6-diyl group, 3-ethyl-4-methylhexane-1,6-diyl group, 3-ethyl-5-methylhexane-1,6-diyl group, 4-ethyl-2-methylhexane-1,6-diyl group, 4-ethyl-3-methylhexane-1,6-diyl group, 4-ethyl-5-methylhexane-1,6-diyl group, 2,4-dimethyl-3-ethylhexane-1,6-diyl group, 2,5-dimethyl-3-ethylhexane-1,6-diyl group, 4,5-dimethyl-3-ethylhexane-1,6-diyl group, 2,3-dimethyl-4-ethylhexane-1,6-diyl group, 2,5-dimethyl-4-ethylhexane-1,6-diyl group, 3,5-dimethyl-4-ethylhexane-1,6-diyl group, 3,4-diethylhexane-1,6-diyl group;

2-methylheptane-1,7-diyl group, 3-methylheptane-1,7-diyl group, 4-methylheptane-1,7-diyl group, 5-methylheptane-1,7-diyl group, 6-methylheptane-1,7-diyl group, 2,3-dimethylheptane-1,7-diyl group, 2,4-dimethylheptane-1,7-diyl group, 2,5-dimethylheptane-1,7-diyl group, 2,6-dimethylheptane-1,7-diyl group, 3,3-dimethylheptane-1,7-diyl group, 3,4-dimethylheptane-1,7-diyl group, 3,5-dimethylheptane-1,7-diyl group, 3,6-dimethylheptane-1,7-diyl group, 4,4-dimethylheptane-1,7-diyl group, 4,5-dimethylheptane-1,7-diyl group, 4,6-dimethylheptane-1,7-diyl group, 5,5-dimethylheptane-1,7-diyl group, 5,6-dimethylheptane-1,7-diyl group, 2,3,3-trimethylheptane-1,7-diyl group, 2,3,4-trimethylheptane-1,7-diyl group, 2,3,5-trimethylheptane-1,7-diyl group, 2,3,6-trimethylheptane-1,7-diyl group, 2,4,4-trimethylheptane-1,7-diyl group, 2,4,5-trimethylheptane-1,7-diyl group, 2,4,6-trimethylheptane-1,7-diyl group, 2,5,5-trimethylheptane-1,7-diyl group, 2,5,6-trimethylheptane-1,7-diyl group, 3,3,4-trimethylheptane-1,7-diyl group, 3,3,5-trimethylheptane-1,7-diyl group, 3,3,6-trimethylheptane-1,7-diyl group, 3,4,4-trimethylheptane-1,7-diyl group, 3,4,5-trimethylheptane-1,7-diyl group, 3,4,6-trimethylheptane-1,7-diyl group, 3,5,5-trimethylheptane-1,7-diyl group, 3,5,6-trimethylheptane-1,7-diyl group, 4,4,5-trimethylheptane-1,7-diyl group, 4,4,6-trimethylheptane-1,7-diyl group, 4,5,5-trimethylheptane-1,7-diyl group, 4,5,6-trimethylheptane-1,7-diyl group, 3-ethylheptane-1,7-diyl group, 4-ethylheptane-1,7-diyl group, 5-ethylheptane-1,7-diyl group, 3-ethyl-2-methylheptane-1,7-diyl group, 3-ethyl-4-methylheptane-1,7-diyl group, 3-ethyl-5-methylheptane-1,7-diyl group, 3-ethyl-6-methylheptane-1,7-diyl group, 4-ethyl-2-methylheptane-1,7-diyl group, 4-ethyl-3-methylheptane-1,7-diyl group, 4-ethyl-4-methylheptane-1,7-diyl group, 4-ethyl-5-methylheptane-1,7-diyl group, 4-ethyl-6-methylheptane-1,7-diyl group, 5-ethyl-2-methylheptane-1,7-diyl group, 5-ethyl-3-methylheptane-1,7-diyl group, 5-ethyl-4-methylheptane-1,7-diyl group, 5-ethyl-5-methylheptane-1,7-diyl group, 5-ethyl-6-methylheptane-1,7-diyl group, 4-n-propylheptane-1,7-diyl group, 4-i-propylheptane-1,7-diyl group;

2-methyloctane-1,8-diyl group, 3-methyloctane-1,8-diyl group, 3-methyloctane-1,8-diyl group, 4-methyloctane-1,8-diyl group, 5-methyloctane-1,8-diyl group, 6-methyloctane-1,8-diyl group, 7-methyloctane-1,8-diyl group, 2,3-dimethyloctane-1,8-diyl group, 2,4-dimethyloctane-1,8-diyl group, 2,5-dimethyloctane-1,8-diyl group, 2,6-dimethyloctane-1,8-diyl group, 2,7-dimethyloctane-1,8-diyl group, 3,3-dimethyloctane-1,8-diyl group, 3,4-dimethyloctane-1,8-diyl group, 3,5-dimethyloctane-1,8-diyl group, 3,6-dimethyloctane-1,8-diyl group, 3,7-dimethyloctane-1,8-diyl group, 4,4-dimethyloctane-1,8-diyl group, 4,5-dimethyloctane-1,8-diyl group, 4,6-dimethyloctane-1,8-diyl group, 4,7-dimethyloctane-1,8-diyl group, 5,5-dimethyloctane-1,8-diyl group, 5,6-dimethyloctane-1,8-diyl group, 5,7-dimethyloctane-1,8-diyl group, 6,6-dimethyloctane-1,8-diyl group, 6,7-dimethyloctane-1,8-diyl group, 3-ethyloctane-1,8-diyl group, 4-ethyloctane-1,8-diyl group, 5-ethyloctane-1,8-diyl group, 6-ethyloctane-1,8-diyl group, 2-methylnonane-1,9-diyl group, 3-methylnonane-1,9-diyl group, 4-methylnonane-1,9-diyl group, 5-methylnonane-1,9-diyl group, 6-methylnonane-1,9-diyl group, 7-methylnonane-1,9-diyl group, 8-methylnonane-1,9-diyl group;

2-methyldecane-1,10-diyl group, 3-methyldecane-1,10-diyl group, 4-methyldecane-1,10-diyl group, 5-methyldecane-1,10-diyl group, 6-methyldecane-1,10-diyl group, 7-methyldecane-1,10-diyl group, 8-methyldecane-1,10-diyl group, 4-ethyldecane-1,10-diyl group, 5-ethyldecane-1,10-diyl group, 6-ethyldecane-1,10-diyl group, 7-ethyldecane-1,10-diyl group, 5-n-propyldecane-1,10-diyl group, 6-n-propyldecane-1,10-diyl group, 3-ethyl-2-methyldecane-1,10-diyl group, 4-ethyl-2-methyldecane-1,10-diyl group, 5-ethyl-2-methyldecane-1,10-diyl group, 6-ethyl-2-methyldecane-1,10-diyl group, 7-ethyl-2-methyldecane-1,10-diyl group, 3-ethyl-3-methyldecane-1,10-diyl group, 4-ethyl-3-methyldecane-1,10-diyl group, 5-ethyl-3-methyldecane-1,10-diyl group, 6-ethyl-3-methyldecane-1,10-diyl group, 7-ethyl-3-methyldecane-1,10-diyl group, 3-ethyl-4-methyldecane-1,10-diyl group, 4-ethyl-4-methyldecane-1,10-diyl group, 5-ethyl-4-methyldecane-1,10-diyl group, 6-ethyl-4-methyldecane-1,10-diyl group, 7-ethyl-4-methyldecane-1,10-diyl group, 3-ethyl-5-methyldecane-1,10-diyl group, 4-ethyl-5-methyldecane-1,10-diyl group, 5-ethyl-5-methyldecane-1,10-diyl group, 6-ethyl-5-methyldecane-1,10-diyl group, 7-ethyl-5-methyldecane-1,10-diyl group;

2-methylundecane-1,11-diyl group, 3-methylundecane-1,11-diyl group, 4-methylundecane-1,11-diyl group, 5-methylundecane-1,11-diyl group, 6-methylundecane-1,11-diyl group, 7-methylundecane-1,11-diyl group, 8-methylundecane-1,11-diyl group, 9-methylundecane-1,11-diyl group, 10-methylundecane-1,11-diyl group, 3-ethylundecane-1,11-diyl group, 4-ethylundecane-1,11-diyl group, 5-ethylundecane-1,11-diyl group, 6-ethylundecane-1,11-diyl group, 7-ethylundecane-1,11-diyl group, 8-ethylundecane-1,11-diyl group, 9-ethylundecane-1,11-diyl group;

2-methyldodecane-1,12-diyl group, 3-methyldodecane-1,12-diyl group, 4-methyldodecane-1,12-diyl group, 5-methyldodecane-1,12-diyl group, 6-methyldodecane-1,12-diyl group, 7-methyldodecane-1,12-diyl group, 8-methyldodecane-1,12-diyl group, 9-methyldodecane-1,12-diyl group, 10-methyldodecane-1,12-diyl group, 11-methyldodecane-1,12-diyl group;

3-ethyldodecane-1,12-diyl group, 4-ethyldodecane-1,12-diyl group, 5-ethyldodecane-1,12-diyl group, 6-ethyldodecane-1,12-diyl group, 7-ethyldodecane-1,12-diyl group, 8-ethyldodecane-1,12-diyl group, 9-ethyldodecane-1,12-diyl group, 10-ethyldodecane-1,12-diyl group;

2-methyltridecane-1,13-diyl group, 3-methyltridecane-1,13-diyl group, 4-methyltridecane-1,13-diyl group, 5-methyltridecane-1,13-diyl group, 6-methyltridecane-1,13-diyl group, 7-methyltridecane-1,13-diyl group, 8-methyltridecane-1,13-diyl group, 9-methyltridecane-1,13-diyl group, 10-methyltridecane-1,13-diyl group, 11-methyltridecane-1,13-diyl group, 12-methyltridecane-1,13-diyl group;

3-ethyltridecane-1,13-diyl group, 4-ethyltridecane-1,13-diyl group, 5-ethyltridecane-1,13-diyl group, 6-ethyltridecane-1,13-diyl group, 7-ethyltridecane-1,13-diyl group, 8-ethyltridecane-1,13-diyl group, 9-ethyltridecane-1,13-diyl group, 10-ethyltridecane-1,13-diyl group, 11-ethyltridecane-1,13-diyl group;

2-methyltetradecane-1,14-diyl group, 3-methyltetradecane-1,14-diyl group, 4-methyltetradecane-1,14-diyl group, 5-methyltetradecane-1,14-diyl group, 6-methyltetradecane-1,14-diyl group, 7-methyltetradecane-1,14-diyl group, 8-methyltetradecane-1,14-diyl group, 9-methyltetradecane-1,14-diyl group, 10-methyltetradecane-1,14-diyl group, 11-methyltetradecane-1,14-diyl group, 12-methyltetradecane-1,14-diyl group, 13-methyltetradecane-1,14-diyl group;

3-ethyltetradecane-1,14-diyl group, 4-ethyltetradecane-1,14-diyl group, 5-ethyltetradecane-1,14-diyl group, 6-ethyltetradecane-1,14-diyl group, 7-ethyltetradecane-1,14-diyl group, 8-ethyltetradecane-1,14-diyl group, 9-ethyltetradecane-1,14-diyl group, 10-ethyltetradecane-1,14-diyl group, 11-ethyltetradecane-1,14-diyl group, 12-ethyltetradecane-1,14-diyl group;

2-methylpentadecane-1,15-diyl group, 3-methylpentadecane-1,15-diyl group, 4-methylpentadecane-1,15-diyl group, 5-methylpentadecane-1,15-diyl group, 6-methylpentadecane-1,15-diyl group, 7-methylpentadecane-1,15-diyl group, 8-methylpentadecane-1,15-diyl group, 9-methylpentadecane-1,15-diyl group, 10-methylpentadecane-1,15-diyl group, 11-methylpentadecane-1,15-diyl group, 12-methylpentadecane-1,15-diyl group, 13-methylpentadecane-1,15-diyl group, 14-methylpentadecane-1,15-diyl group;

3-ethylpentadecane-1,15-diyl group, 4-ethylpentadecane-1,15-diyl group, 5-ethylpentadecane-1,15-diyl group, 6-ethylpentadecane-1,15-diyl group, 7-ethylpentadecane-1,15-diyl group, 8-ethylpentadecane-1,15-diyl group, 9-ethylpentadecane-1,15-diyl group, 10-ethylpentadecane-1,15-diyl group, 11-ethylpentadecane-1,15-diyl group, 12-ethylpentadecane-1,15-diyl group, 13-ethylpentadecane-1,15-diyl group;

2-methylhexadecane-1,16-diyl group, 3-methylhexadecane-1,16-diyl group, 4-methylhexadecane-1,16-diyl group, 5-methylhexadecane-1,16-diyl group, 6-methylhexadecane-1,16-diyl group, 7-methylhexadecane-1,16-diyl group, 8-methylhexadecane-1,16-diyl group, 9-methylhexadecane-1,16-diyl group, 10-methylhexadecane-1,16-diyl group, 11-methylhexadecane-1,16-diyl group, 12-methylhexadecane-1,16-diyl group, 13-methylhexadecane-1,16-diyl group, 14-methylhexadecane-1,16-diyl group, 15-methylhexadecane-1,16-diyl group;

3-ethylhexadecane-1,16-diyl group, 4-ethylhexadecane-1,16-diyl group, 5-ethylhexadecane-1,16-diyl group, 6-ethylhexadecane-1,16-diyl group, 7-ethylhexadecane-1,16-diyl group, 8-ethylhexadecane-1,16-diyl group, 9-ethylhexadecane-1,16-diyl group, 10-ethylhexadecane-1,16-diyl group, 11-ethylhexadecane-1,16-diyl group, 12-ethylhexadecane-1,16-diyl group, 13-ethylhexadecane-1,16-diyl group, 14-ethylhexadecane-1,16-diyl group;

2-methylheptadecane-1,17-diyl group, 3-methylheptadecane-1,17-diyl group, 4-methylheptadecane-1,17-diyl group, 5-methylheptadecane-1,17-diyl group, 6-methylheptadecane-1,17-diyl group, 7-methylheptadecane-1,17-diyl group, 8-methylheptadecane-1,17-diyl group, 9-methylheptadecane-1,17-diyl group, 10-methylheptadecane-1,17-diyl group, 11-methylheptadecane-1,17-diyl group, 12-methylheptadecane-1,17-diyl group, 13-methylheptadecane-1,17-diyl group, 14-methylheptadecane-1,17-diyl group, 15-methylheptadecane-1,17-diyl group, 16-methylheptadecane-1,17-diyl group;

3-ethylheptadecane-1,17-diyl group, 4-ethylheptadecane-1,17-diyl group, 5-ethylheptadecane-1,17-diyl group, 6-ethylheptadecane-1,17-diyl group, 7-ethylheptadecane-1,17-diyl group, 8-ethylheptadecane-1,17-diyl group,

9-ethylheptadecane-1,17-diyl group, 10-ethylheptadecane-1,17-diyl group, 11-ethylheptadecane-1,17-diyl group, 12-ethylheptadecane-1,17-diyl group, 13-ethylheptadecane-1,17-diyl group, 14-ethylheptadecane-1,17-diyl group, 15-ethylheptadecane-1,17-diyl group;

2-methyloctadecane-1,18-diyl group, 3-methyloctadecane-1,18-diyl group, 4-methyloctadecane-1,18-diyl group, 5-methyloctadecane-1,18-diyl group, 6-methyloctadecane-1,18-diyl group, 7-methyloctadecane-1,18-diyl group, 8-methyloctadecane-1,18-diyl group, 9-methyloctadecane-1,18-diyl group, 10-methyloctadecane-1,18-diyl group, 11-methyloctadecane-1,18-diyl group, 12-methyloctadecane-1,18-diyl group, 13-methyloctadecane-1,18-diyl group, 14-methyloctadecane-1,18-diyl group, 15-methyloctadecane-1,18-diyl group, 16-methyloctadecane-1,18-diyl group, 17-methyloctadecane-1,18-diyl group;

3-ethyloctadecane-1,18-diyl group, 4-ethyloctadecane-1,18-diyl group, 5-ethyloctadecane-1,18-diyl group, 6-ethyloctadecane-1,18-diyl group, 7-ethyloctadecane-1,18-diyl group, 8-ethyloctadecane-1,18-diyl group, 9-ethyloctadecane-1,18-diyl group, 10-ethyloctadecane-1,18-diyl group, 11-ethyloctadecane-1,18-diyl group, 12-ethyloctadecane-1,18-diyl group, 13-ethyloctadecane-1,18-diyl group, 14-ethyloctadecane-1,18-diyl group, 15-ethyloctadecane-1,18-diyl group, 16-ethyloctadecane-1,18-diyl group;

2-methylnonadecane-1,19-diyl group, 3-methylnonadecane-1,19-diyl group, 4-methylnonadecane-1,19-diyl group, 5-methylnonadecane-1,19-diyl group, 6-methylnonadecane-1,19-diyl group, 7-methylnonadecane-1,19-diyl group, 8-methylnonadecane-1,19-diyl group, 9-methylnonadecane-1,19-diyl group, 10-methylnonadecane-1,19-diyl group, 11-methylnonadecane-1,19-diyl group, 12-methylnonadecane-1,19-diyl group, 13-methylnonadecane-1,19-diyl group, 14-methylnonadecane-1,19-diyl group, 15-methylnonadecane-1,19-diyl group, 16-methylnonadecane-1,19-diyl group, 17-methylnonadecane-1,19-diyl group, 18-methylnonadecane-1,19-diyl group;

3-ethylnonadecane-1,19-diyl group, 4-ethylnonadecane-1,19-diyl group, 5-ethylnonadecane-1,19-diyl group, 6-ethylnonadecane-1,19-diyl group, 7-ethylnonadecane-1,19-diyl group, 8-ethylnonadecane-1,19-diyl group, 9-ethylnonadecane-1,19-diyl group, 10-ethylnonadecane-1,19-diyl group, 11-ethylnonadecane-1,19-diyl group, 12-ethylnonadecane-1,19-diyl group, 13-ethylnonadecane-1,19-diyl group, 14-ethylnonadecane-1,19-diyl group, 15-ethylnonadecane-1,19-diyl group, 16-ethylnonadecane-1,19-diyl group, 17-ethylnonadecane-1,19-diyl group;

2-methylicosane-1,20-diyl group, 3-methylicosane-1,20-diyl group, 4-methylicosane-1,20-diyl group, 5-methylicosane-1,20-diyl group, 6-methylicosane-1,20-diyl group, 7-methylicosane-1,20-diyl group, 8-methylicosane-1,20-diyl group, 9-methylicosane-1,20-diyl group, 10-methylicosane-1,20-diyl group, 11-methylicosane-1,20-diyl group, 12-methylicosane-1,20-diyl group, 13-methylicosane-1,20-diyl group, 14-methylicosane-1,20-diyl group, 15-methylicosane-1,20-diyl group, 16-methylicosane-1,20-diyl group, 17-methylicosane-1,20-diyl group, 18-methylicosane-1,20-diyl group, 19-methylicosane-1,20-diyl group;

3-ethylicosane-1,20-diyl group, 4-ethylicosane-1,20-diyl group, 5-ethylicosane-1,20-diyl group, 6-ethylicosane-1,20-diyl group, 7-ethylicosane-1,20-diyl group, 8-ethylicosane-1,20-diyl group, 9-ethylicosane-1,20-diyl group, 10-ethylicosane-1,20-diyl group, 11-ethylicosane-1,20-diyl group, 12-ethylicosane-1,20-diyl group, 13-ethylicosane-1,20-diyl group, 14-ethylicosane-1,20-diyl group, 15-ethylicosane-1,20-diyl group, 16-ethylicosane-1,20-diyl group, 17-ethylicosane-1,20-diyl group, 18-ethylicosane-1,20-diyl group;

2-methylhenicosane-1,21-diyl group, 3-methylhenicosane-1,21-diyl group, 4-methylhenicosane-1,21-diyl group, 5-methylhenicosane-1,21-diyl group, 6-methylhenicosane-1,21-diyl group, 7-methylhenicosane-1,21-diyl group, 8-methylhenicosane-1,21-diyl group, 9-methylhenicosane-1,21-diyl group, 10-methylhenicosane-1,21-diyl group, 11-methylhenicosane-1,21-diyl group, 12-methylhenicosane-1,21-diyl group, 13-methylhenicosane-1,21-diyl group, 14-methylhenicosane-1,21-diyl group, 15-methylhenicosane-1,21-diyl group, 16-methylhenicosane-1,21-diyl group, 17-methylhenicosane-1,21-diyl group, 18-methylhenicosane-1,21-diyl group, 19-methylhenicosane-1,21-diyl group, 20-methylhenicosane-1,21-diyl group;

3-ethylhenicosane-1,21-diyl group, 4-ethylhenicosane-1,21-diyl group, 5-ethylhenicosane-1,21-diyl group, 6-ethylhenicosane-1,21-diyl group, 7-ethylhenicosane-1,21-diyl group, 8-ethylhenicosane-1,21-diyl group, 9-ethylhenicosane-1,21-diyl group, 10-ethylhenicosane-1,21-diyl group, 11-ethylhenicosane-1,21-diyl group, 12-ethylhenicosane-1,21-diyl group, 13-ethylhenicosane-1,21-diyl group, 14-ethylhenicosane-1,21-diyl group, 15-ethylhenicosane-1,21-diyl group, 16-ethylhenicosane-1,21-diyl group, 17-ethylhenicosane-1,21-diyl group, 18-ethylhenicosane-1,21-diyl group, 19-ethylhenicosane-1,21-diyl group;

2-methyldocosane-1,22-diyl group, 3-methyldocosane-1,22-diyl group, 4-methyldocosane-1,22-diyl group, 5-methyldocosane-1,22-diyl group, 6-methyldocosane-1,22-diyl group, 7-methyldocosane-1,22-diyl group, 8-methyldocosane-1,22-diyl group, 9-methyldocosane-1,22-diyl group, 10-methyldocosane-1,22-diyl group, 11-methyldocosane-1,22-diyl group, 12-methyldocosane-1,22-diyl group, 13-methyldocosane-1,22-diyl group, 14-methyldocosane-1,22-diyl group, 15-methyldocosane-1,22-diyl group, 16-methyldocosane-1,22-diyl group, 17-methyldocosane-1,22-diyl group, 18-methyldocosane-1,22-diyl group, 19-methyldocosane-1,22-diyl group, 20-methyldocosane-1,22-diyl group, 21-methyldocosane-1,22-diyl group;

3-ethyldocosane-1,22-diyl group, 4-ethyldocosane-1,22-diyl group, 5-ethyldocosane-1,22-diyl group, 6-ethyldocosane-1,22-diyl group, 7-ethyldocosane-1,22-diyl group, 8-ethyldocosane-1,22-diyl group, 9-ethyldocosane-1,22-diyl

group, 10-ethyldocosane-1,22-diyl group, 11-ethyldocosane-1,22-diyl group, 12-ethyldocosane-1,22-diyl group, 13-ethyldocosane-1,22-diyl group, 14-ethyldocosane-1,22-diyl group, 15-ethyldocosane-1,22-diyl group, 16-ethyldocosane-1,22-diyl group, 17-ethyldocosane-1,22-diyl group, 18-ethyldocosane-1,22-diyl group, 19-ethyldocosane-1,22-diyl group, 20-ethyldocosane-1,22-diyl group;

2-methyltricosane-1,23-diyl group, 3-methyltricosane-1,23-diyl group, 4-methyltricosane-1,23-diyl group, 5-methyltricosane-1,23-diyl group, 6-methyltricosane-1,23-diyl group, 7-methyltricosane-1,23-diyl group, 8-methyltricosane-1,23-diyl group, 9-methyltricosane-1,23-diyl group, 10-methyltricosane-1,23-diyl group, 11-methyltricosane-1,23-diyl group, 12-methyltricosane-1,23-diyl group, 13-methyltricosane-1,23-diyl group, 14-methyltricosane-1,23-diyl group, 15-methyltricosane-1,23-diyl group, 16-methyltricosane-1,23-diyl group, 17-methyltricosane-1,23-diyl group, 18-methyltricosane-1,23-diyl group, 19-methyltricosane-1,23-diyl group, 20-methyltricosane-1,23-diyl group, 21-methyltricosane-1,23-diyl group, 22-methyltricosane-1,23-diyl group;

3-ethyltricosane-1,23-diyl group, 4-ethyltricosane-1,23-diyl group, 5-ethyltricosane-1,23-diyl group, 6-ethyltricosane-1,23-diyl group, 7-ethyltricosane-1,23-diyl group, 8-ethyltricosane-1,23-diyl group, 9-ethyltricosane-1,23-diyl group, 10-ethyltricosane-1,23-diyl group, 11-ethyltricosane-1,23-diyl group, 12-ethyltricosane-1,23-diyl group, 13-ethyltricosane-1,23-diyl group, 14-ethyltricosane-1,23-diyl group, 15-ethyltricosane-1,23-diyl group, 16-ethyltricosane-1,23-diyl group, 17-ethyltricosane-1,23-diyl group, 18-ethyltricosane-1,23-diyl group, 19-ethyltricosane-1,23-diyl group, 20-ethyltricosane-1,23-diyl group, 21-ethyltricosane-1,23-diyl group;

2-methyltetracosane-1,24-diyl group, 3-methyltetracosane-1,24-diyl group, 4-methyltetracosane-1,24-diyl group, 5-methyltetracosane-1,24-diyl group, 6-methyltetracosane-1,24-diyl group, 7-methyltetracosane-1,24-diyl group, 8-methyltetracosane-1,24-diyl group, 9-methyltetracosane-1,24-diyl group, 10-methyltetracosane-1,24-diyl group, 11-methyltetracosane-1,24-diyl group, 12-methyltetracosane-1,24-diyl group, 13-methyltetracosane-1,24-diyl group, 14-methyltetracosane-1,24-diyl group, 15-methyltetracosane-1,24-diyl group, 16-methyltetracosane-1,24-diyl group, 17-methyltetracosane-1,24-diyl group, 18-methyltetracosane-1,24-diyl group, 19-methyltetracosane-1,24-diyl group, 20-methyltetracosane-1,24-diyl group, 21-methyltetracosane-1,24-diyl group, 22-methyltetracosane-1,24-diyl group, 23-methyltetracosane-1,24-diyl group;

3-ethyltetracosane-1,24-diyl group, 4-ethyltetracosane-1,24-diyl group, 5-ethyltetracosane-1,24-diyl group, 6-ethyltetracosane-1,24-diyl group, 7-ethyltetracosane-1,24-diyl group, 8-ethyltetracosane-1,24-diyl group, 9-ethyltetracosane-1,24-diyl group, 10-ethyltetracosane-1,24-diyl group, 11-ethyltetracosane-1,24-diyl group, 12-ethyltetracosane-1,24-diyl group, 13-ethyltetracosane-1,24-diyl group, 14-ethyltetracosane-1,24-diyl group, 15-ethyltetracosane-1,24-diyl group, 16-ethyltetracosane-1,24-diyl group, 17-ethyltetracosane-1,24-diyl group, 18-ethyltetracosane-1,24-diyl group, 19-ethyltetracosane-1,24-diyl group, 20-ethyltetracosane-1,24-diyl group, 21-ethyltetracosane-1,24-diyl group, 22-ethyltetracosane-1,24-diyl group;

2-methylpentacosane-1,25-diyl group, 3-methylpentacosane-1,25-diyl group, 4-methylpentacosane-1,25-diyl group, 5-methylpentacosane-1,25-diyl group, 6-methylpentacosane-1,25-diyl group, 7-methylpentacosane-1,25-diyl group, 8-methylpentacosane-1,25-diyl group, 9-methylpentacosane-1,25-diyl group, 10-methylpentacosane-1,25-diyl group, 11-methylpentacosane-1,25-diyl group, 12-methylpentacosane-1,25-diyl group, 13-methylpentacosane-1,25-diyl group, 14-methylpentacosane-1,25-diyl group, 15-methylpentacosane-1,25-diyl group, 16-methylpentacosane-1,25-diyl group, 17-methylpentacosane-1,25-diyl group, 18-methylpentacosane-1,25-diyl group, 19-methylpentacosane-1,25-diyl group, 20-methylpentacosane-1,25-diyl group, 21-methylpentacosane-1,25-diyl group, 22-methylpentacosane-1,25-diyl group, 23-methylpentacosane-1,25-diyl group, 24-methylpentacosane-1,25-diyl group;

3-ethylpentacosane-1,25-diyl group, 4-ethylpentacosane-1,25-diyl group, 5-ethylpentacosane-1,25-diyl group, 6-ethylpentacosane-1,25-diyl group, 7-ethylpentacosane-1,25-diyl group, 8-ethylpentacosane-1,25-diyl group, 9-ethylpentacosane-1,25-diyl group, 10-ethylpentacosane-1,25-diyl group, 11-ethylpentacosane-1,25-diyl group, 12-ethylpentacosane-1,25-diyl group, 13-ethylpentacosane-1,25-diyl group, 14-ethylpentacosane-1,25-diyl group, 15-ethylpentacosane-1,25-diyl group, 16-ethylpentacosane-1,25-diyl group, 17-ethylpentacosane-1,25-diyl group, 18-ethylpentacosane-1,25-diyl group, 19-ethylpentacosane-1,25-diyl group, 20-ethylpentacosane-1,25-diyl group, 21-ethylpentacosane-1,25-diyl group, 22-ethylpentacosane-1,25-diyl group, 23-ethylpentacosane-1,25-diyl group;

2-methylhexacosane-1,26-diyl group, 3-methylhexacosane-1,26-diyl group, 4-methylhexacosane-1,26-diyl group, 5-methylhexacosane-1,26-diyl group, 6-methylhexacosane-1,26-diyl group, 7-methylhexacosane-1,26-diyl group, 8-methylhexacosane-1,26-diyl group, 9-methylhexacosane-1,26-diyl group, 10-methylhexacosane-1,26-diyl group, 11-methylhexacosane-1,26-diyl group, 12-methylhexacosane-1,26-diyl group, 13-methylhexacosane-1,26-diyl group, 14-methylhexacosane-1,26-diyl group, 15-methylhexacosane-1,26-diyl group, 16-methylhexacosane-1,26-diyl group, 17-methylhexacosane-1,26-diyl group, 18-methylhexacosane-1,26-diyl group, 19-methylhexacosane-1,26-diyl group, 20-methylhexacosane-1,26-diyl group, 21-methylhexacosane-1,26-diyl group, 22-methylhexacosane-1,26-diyl group, 23-methylhexacosane-1,26-diyl group, 24-methylhexacosane-1,26-diyl group, 25-methylhexacosane-1,26-diyl group;

3-ethylhexacosane-1,26-diyl group, 4-ethylhexacosane-1,26-diyl group, 5-ethylhexacosane-1,26-diyl group, 6-ethylhexacosane-1,26-diyl group, 7-ethylhexacosane-1,26-diyl group, 8-ethylhexacosane-1,26-diyl group, 9-ethylhexacosane-1,26-diyl group, 10-ethylhexacosane-1,26-diyl group, 11-ethylhexacosane-1,26-diyl group, 12-ethylhexacosane-1,26-diyl group, 13-ethylhexacosane-1,26-diyl group, 14-ethylhexacosane-1,26-diyl group, 15-ethylhexacosane-1,26-diyl group, 16-ethylhexacosane-1,26-diyl group, 17-ethylhexacosane-1,26-diyl group, 18-ethylhexacosane-1,26-diyl group, 19-ethylhexacosane-1,26-diyl group, 20-ethylhexacosane-1,26-diyl group, 21-ethylhexacosane-1,26-diyl group, 22-ethylhexacosane-1,26-diyl group, 23-ethylhexacosane-1,26-diyl group, 24-ethylhexacosane-1,26-diyl group;
2-methylheptacosane-1,27-diyl group, 3-methylheptacosane-1,27-diyl group, 4-methylheptacosane-1,27-diyl group, 5-methylheptacosane-1,27-diyl group, 6-methylheptacosane-1,27-diyl group, 7-methylheptacosane-1,27-diyl group, 8-methylheptacosane-1,27-diyl group, 9-methylheptacosane-1,27-diyl group, 10-methylheptacosane-1,27-diyl group, 11-methylheptacosane-1,27-diyl group, 12-methylheptacosane-1,27-diyl group, 13-methylheptacosane-1,27-diyl group, 14-methylheptacosane-1,27-diyl group, 15-methylheptacosane-1,27-diyl group, 16-methylheptacosane-1,27-diyl group, 17-methylheptacosane-1,27-diyl group, 18-methylheptacosane-1,27-diyl group, 19-methylheptacosane-1,27-diyl group, 20-methylheptacosane-1,27-diyl group, 21-methylheptacosane-1,27-diyl group, 22-methylheptacosane-1,27-diyl group, 23-methylheptacosane-1,27-diyl group, 24-methylheptacosane-1,27-diyl group, 25-methylheptacosane-1,27-diyl group, 26-methylheptacosane-1,27-diyl group;
3-ethylheptacosane-1,27-diyl group, 4-ethylheptacosane-1,27-diyl group, 5-ethylheptacosane-1,27-diyl group, 6-ethylheptacosane-1,27-diyl group, 7-ethylheptacosane-1,27-diyl group, 8-ethylheptacosane-1,27-diyl group, 9-ethylheptacosane-1,27-diyl group, 10-ethylheptacosane-1,27-diyl group, 11-ethylheptacosane-1,27-diyl group, 12-ethylheptacosane-1,27-diyl group, 13-ethylheptacosane-1,27-diyl group, 14-ethylheptacosane-1,27-diyl group, 15-ethylheptacosane-1,27-diyl group, 16-ethylheptacosane-1,27-diyl group, 17-ethylheptacosane-1,27-diyl group, 18-ethylheptacosane-1,27-diyl group, 19-ethylheptacosane-1,27-diyl group, 20-ethylheptacosane-1,27-diyl group, 21-ethylheptacosane-1,27-diyl group, 22-ethylheptacosane-1,27-diyl group, 23-ethylheptacosane-1,27-diyl group, 24-ethylheptacosane-1,27-diyl group, 25-ethylheptacosane-1,27-diyl group;
2-methyloctacosane-1,28-diyl group, 3-methyloctacosane-1,28-diyl group, 4-methyloctacosane-1,28-diyl group, 5-methyloctacosane-1,28-diyl group, 6-methyloctacosane-1,28-diyl group, 7-methyloctacosane-1,28-diyl group, 8-methyloctacosane-1,28-diyl group, 9-methyloctacosane-1,28-diyl group, 10-methyloctacosane-1,28-diyl group, 11-methyloctacosane-1,28-diyl group, 12-methyloctacosane-1,28-diyl group, 13-methyloctacosane-1,28-diyl group, 14-methyloctacosane-1,28-diyl group, 15-methyloctacosane-1,28-diyl group, 16-methyloctacosane-1,28-diyl group, 17-methyloctacosane-1,28-diyl group, 18-methyloctacosane-1,28-diyl group, 19-methyloctacosane-1,28-diyl group, 20-methyloctacosane-1,28-diyl group, 21-methyloctacosane-1,28-diyl group, 22-methyloctacosane-1,28-diyl group, 23-methyloctacosane-1,28-diyl group, 24-methyloctacosane-1,28-diyl group, 25-methyloctacosane-1,28-diyl group, 26-methyloctacosane-1,28-diyl group, 27-methyloctacosane-1,28-diyl group;
3-ethyloctacosane-1,28-diyl group, 4-ethyloctacosane-1,28-diyl group, 5-ethyloctacosane-1,28-diyl group, 6-ethyloctacosane-1,28-diyl group, 7-ethyloctacosane-1,28-diyl group, 8-ethyloctacosane-1,28-diyl group, 9-ethyloctacosane-1,28-diyl group, 10-ethyloctacosane-1,28-diyl group, 11-ethyloctacosane-1,28-diyl group, 12-ethyloctacosane-1,28-diyl group, 13-ethyloctacosane-1,28-diyl group, 14-ethyloctacosane-1,28-diyl group, 15-ethyloctacosane-1,28-diyl group, 16-ethyloctacosane-1,28-diyl group, 17-ethyloctacosane-1,28-diyl group, 18-ethyloctacosane-1,28-diyl group, 19-ethyloctacosane-1,28-diyl group, 20-ethyloctacosane-1,28-diyl group, 21-ethyloctacosane-1,28-diyl group, 22-ethyloctacosane-1,28-diyl group, 23-ethyloctacosane-1,28-diyl group, 24-ethyloctacosane-1,28-diyl group, 25-ethyloctacosane-1,28-diyl group, 26-ethyloctacosane-1,28-diyl group;
2-methylnonacosane-1,29-diyl group, 3-methylnonacosane-1,29-diyl group, 4-methylnonacosane-1,29-diyl group, 5-methylnonacosane-1,29-diyl group, 6-methylnonacosane-1,29-diyl group, 7-methylnonacosane-1,29-diyl group, 8-methylnonacosane-1,29-diyl group, 9-methylnonacosane-1,29-diyl group, 10-methylnonacosane-1,29-diyl group, 11-methylnonacosane-1,29-diyl group, 12-methylnonacosane-1,29-diyl group, 13-methylnonacosane-1,29-diyl group, 14-methylnonacosane-1,29-diyl group, 15-methylnonacosane-1,29-diyl group, 16-methylnonacosane-1,29-diyl group, 17-methylnonacosane-1,29-diyl group, 18-methylnonacosane-1,29-diyl group, 19-methylnonacosane-1,29-diyl group, 20-methylnonacosane-1,29-diyl group, 21-methylnonacosane-1,29-diyl group, 22-methylnonacosane-1,29-diyl group, 23-methylnonacosane-1,29-diyl group, 24-methylnonacosane-1,29-diyl group, 25-methylnonacosane-1,29-diyl group, 26-methylnonacosane-1,29-diyl group, 27-methylnonacosane-1,29-diyl group and 28-methylnonacosane-1,29-diyl group.

If G represents an optionally substituted straight-chained or branched alkenylene group having 2 - 30 carbon atoms, exemplary straight-chained or branched alkenylene groups having 2 - 30 carbon atoms include straight-chained alkenylene groups such as ethylene-1,2-diyl group, 1-propene-1,3-diyl group, 2-propene-1,3-diyl group, 1-butene-1,4-diyl group, 2-butene-1,4-diyl group, 3-butene-1,4-diyl group, 1,3-butadiene-1,4-diyl group, 2-pentene-1,5-diyl group, 3-pentene-1,5-diyl group, 2,4-pentadiene-1,5-diyl group, 2-hexene-1,6-diyl group, 3-hexene-1,6-diyl group, 4-hexene-1,6-diyl group, 2,4-hexadiene-1,6-diyl group, 2-heptene-1,7-diyl group, 3-heptene-1,7-diyl group, 4-heptene-1,7-diyl group, 5-heptene-1,7-diyl group, 2,4-heptadiene-1,7-diyl group, 2,5-heptadiene-1,7-diyl group, 3,5-heptadiene-1,7-diyl group, 2-octene-1,8-diyl group, 3-octene-1,8-diyl group, 4-octene-1,8-diyl group, 5-octene-1,8-diyl group, 6-octene-1,8-diyl group, 2,4-octadiene-1,8-diyl group, 2,5-octadiene-1,8-diyl group, 2,6-octadiene-1,8-diyl group,

2,4,6-octatriene-1,8-diyl group, 2-nonene-1,9-diyl group, 3-nonene-1,9-diyl group, 4-nonene-1,9-diyl group, 5-nonene-1,9-diyl group, 6-nonene-1,9-diyl group, 7-nonene-1,9-diyl group, 2-decene-1,10-diyl group, 3-decene-1,10-diyl group, 4-decene-1,10-diyl group, 5-decene-1,10-diyl group, 6-decene-1,10-diyl group, 7-decene-1,10-diyl group, 8-decene-1,10-diyl group;
2-undecene-1,11-diyl group, 3-undecene-1,11-diyl group, 4-undecene-1,11-diyl group, 5-undecene-1,11-diyl group, 6-undecene-1,11-diyl group, 7-undecene-1,11-diyl group, 8-undecene-1,11-diyl group, 9-undecene-1,11-diyl group;
2-dodecene-1,11-diyl group, 3-dodecene-1,11-diyl group, 4-dodecene-1,11-diyl group, 5-dodecene-1,11-diyl group, 6-dodecene-1,11-diyl group, 7-dodecene-1,11-diyl group, 8-dodecene-1,11-diyl group, 9-dodecene-1,11-diyl group, 10-dodecene-1,11-diyl group;
2-tridecene-1,13-diyl group, 3-tridecene-1,13-diyl group, 4-tridecene-1,13-diyl group, 5-tridecene-1,13-diyl group, 6-tridecene-1,13-diyl group, 7-tridecene-1,13-diyl group, 8-tridecene-1,13-diyl group, 9-tridecene-1,13-diyl group, 10-tridecene-1,13-diyl group, 11-tridecene-1,13-diyl group;
2-tetradecene-1,14-diyl group, 3-tetradecene-1,14-diyl group, 4-tetradecene-1,14-diyl group, 5-tetradecene-1,14-diyl group, 6-tetradecene-1,14-diyl group, 7-tetradecene-1,14-diyl group, 8-tetradecene-1,14-diyl group, 9-tetradecene-1,14-diyl group, 10-tetradecene-1,14-diyl group, 11-tetradecene-1,14-diyl group, 12-tetradecene-1,14-diyl group;
2-pentadecene-1,15-diyl group, 3-pentadecene-1,15-diyl group, 4-pentadecene-1,15-diyl group, 5-pentadecene-1,15-diyl group, 6-pentadecene-1,15-diyl group, 7-pentadecene-1,15-diyl group, 8-pentadecene-1,15-diyl group, 9-pentadecene-1,15-diyl group, 10-pentadecene-1,15-diyl group, 11-pentadecene-1,15-diyl group, 12-pentadecene-1,15-diyl group, 13-pentadecene-1,15-diyl group;
2-hexadecene-1,16-diyl group, 3-hexadecene-1,16-diyl group, 4-hexadecene-1,16-diyl group, 5-hexadecene-1,16-diyl group, 6-hexadecene-1,16-diyl group, 7-hexadecene-1,16-diyl group, 8-hexadecene-1,16-diyl group, 9-hexadecene-1,16-diyl group, 10-hexadecene-1,16-diyl group, 11-hexadecene-1,16-diyl group, 12-hexadecene-1,16-diyl group, 13-hexadecene-1,16-diyl group, 14-hexadecene-1,16-diyl group;
2-heptadecene-1,17-diyl group, 3-heptadecene-1,17-diyl group, 4-heptadecene-1,17-diyl group, 5-heptadecene-1,17-diyl group, 6-heptadecene-1,17-diyl group, 7-heptadecene-1,17-diyl group, 8-heptadecene-1,17-diyl group, 9-heptadecene-1,17-diyl group, 10-heptadecene-1,17-diyl group, 11-heptadecene-1,17-diyl group, 12-heptadecene-1,17-diyl group, 13-heptadecene-1,17-diyl group, 14-heptadecene-1,17-diyl group, 15-heptadecene-1,17-diyl group;
2-octadecene-1,18-diyl group, 3-octadecene-1,18-diyl group, 4-octadecene-1,18-diyl group, 5-octadecene-1,18-diyl group, 6-octadecene-1,18-diyl group, 7-octadecene-1,18-diyl group, 8-octadecene-1,18-diyl group, 9-octadecene-1,18-diyl group, 10-octadecene-1,18-diyl group, 11-octadecene-1,18-diyl group, 12-octadecene-1,18-diyl group, 13-octadecene-1,18-diyl group, 14-octadecene-1,18-diyl group, 15-octadecene-1,18-diyl group, 16-octadecene-1,18-diyl group;
2-nonadecene-1,19-diyl group, 3-nonadecene-1,19-diyl group, 4-nonadecene-1,19-diyl group, 5-nonadecene-1,19-diyl group, 6-nonadecene-1,19-diyl group, 7-nonadecene-1,19-diyl group, 8-nonadecene-1,19-diyl group, 9-nonadecene-1,19-diyl group, 10-nonadecene-1,19-diyl group, 11-nonadecene-1,19-diyl group, 12-nonadecene-1,19-diyl group, 13-nonadecene-1,19-diyl group, 14-nonadecene-1,19-diyl group, 15-nonadecene-1,19-diyl group, 16-nonadecene-1,19-diyl group, 17-nonadecene-1,19-diyl group; 2-icosene-1,20-diyl group, 3-icosene-1,20-diyl group, 4-icosene-1,20-diyl group, 5-icosene-1,20-diyl group, 6-icosene-1,20-diyl group, 7-icosene-1,20-diyl group, 8-icosene-1,20-diyl group, 9-icosene-1,20-diyl group, 10-icosene-1,20-diyl group, 11-icosene-1,20-diyl group, 12-icosene-1,20-diyl group, 13-icosene-1,20-diyl group, 14-icosene-1,20-diyl group, 15-icosene-1,20-diyl group, 16-icosene-1,20-diyl group, 17-icosene-1,20-diyl group, 18-icosene-1,20-diyl group; 2-henicosene-1,21-diyl group, 3-henicosene-1,21-diyl group, 4-henicosene-1,21-diyl group, 5-henicosene-1,21-diyl group, 6-henicosene-1,21-diyl group, 7-henicosene-1,21-diyl group, 8-henicosene-1,21-diyl group, 9-henicosene-1,21-diyl group, 10-henicosene-1,21-diyl group, 11-henicosene-1,21-diyl group, 12-henicosene-1,21-diyl group, 13-henicosene-1,21-diyl group, 14-henicosene-1,21-diyl group, 15-henicosene-1,21-diyl group, 16-henicosene-1,21-diyl group, 17-henicosene-1,21-diyl group, 18-henicosene-1,21-diyl group, 19-henicosene-1,21-diyl group;
2-docosene-1,22-diyl group, 3-docosene-1,22-diyl group, 4-docosene-1,22-diyl group, 5-docosene-1,22-diyl group, 6-docosene-1,22-diyl group, 7-docosene-1,22-diyl group, 8-docosene-1,22-diyl group, 9-docosene-1,22-diyl group, 10-docosene-1,22-diyl group, 11-docosene-1,22-diyl group, 12-docosene-1,22-diyl group, 13-docosene-1,22-diyl group, 14-docosene-1,22-diyl group, 15-docosene-1,22-diyl group, 16-docosene-1,22-diyl group, 17-docosene-1,22-diyl group, 18-docosene-1,22-diyl group, 19-docosene-1,22-diyl group, 20-docosene-1,22-diyl group;
2-tricosene-1,23-diyl group, 3-tricosene-1,23-diyl group, 4-tricosene-1,23-diyl group, 5-tricosene-1,23-diyl group, 6-tricosene-1,23-diyl group, 7-tricosene-1,23-diyl group, 8-tricosene-1,23-diyl group, 9-tricosene-1,23-diyl group, 10-tricosene-1,23-diyl group, 11-tricosene-1,23-diyl group, 12-tricosene-1,23-diyl group, 13-tricosene-1,23-diyl group, 14-tricosene-1,23-diyl group, 15-tricosene-1,23-diyl group, 16-tricosene-1,23-diyl group, 17-tricosene-1,23-diyl group, 18-tricosene-1,23-diyl group, 19-tricosene-1,23-diyl group, 20-tricosene-1,23-diyl group, 21-tricosene-1,23-diyl group;
2-tetracosene-1,24-diyl group, 3-tetracosene-1,24-diyl group, 4-tetracosene-1,24-diyl group, 5-tetracosene-1,24-diyl group, 6-tetracosene-1,24-diyl group, 7-tetracosene-1,24-diyl group, 8-tetracosene-1,24-diyl group, 9-tetracosene-1,24-diyl group, 10-tetracosene-1,24-diyl group, 11-tetracosene-1,24-diyl group, 12-tetracosene-1,24-diyl group, 13-tetracosene-1,24-diyl group, 14-tetracosene-1,24-diyl group, 15-tetracosene-1,24-diyl group, 16-tetracosene-1,24-diyl group, 17-tetracosene-1,24-diyl group, 18-tetracosene-1,24-diyl group, 19-tetracosene-1,24-diyl group, 20-tetracosene-1,24-diyl group, 21-tetracosene-1,24-diyl group, 22-tetracosene-1,24-diyl group;

2-pentacosene-1,25-diyl group, 3-pentacosene-1,25-diyl group, 4-pentacosene-1,25-diyl group, 5-pentacosene-1,25-diyl group, 6-pentacosene-1,25-diyl group, 7-pentacosene-1,25-diyl group, 8-pentacosene-1,25-diyl group, 8-pentacosene-1,25-diyl group, 9-pentacosene-1,25-diyl group, 10-pentacosene-1,25-diyl group, 11-pentacosene-1,25-diyl group, 12-pentacosene-1,25-diyl group, 13-pentacosene-1,25-diyl group, 14-pentacosene-1,25-diyl group, 15-pentacosene-1,25-diyl group, 16-pentacosene-1,25-diyl group, 17-pentacosene-1,25-diyl group, 18-pentacosene-1,25-diyl group, 19-pentacosene-1,25-diyl group, 20-pentacosene-1,25-diyl group, 21-pentacosene-1,25-diyl group, 22-pentacosene-1,25-diyl group, 23-pentacosene-1,25-diyl group;

2-hexacosene-1,26-diyl group, 3-hexacosene-1,26-diyl group, 4-hexacosene-1,26-diyl group, 5-hexacosene-1,26-diyl group, 6-hexacosene-1,26-diyl group, 7-hexacosene-1,26-diyl group, 8-hexacosene-1,26-diyl group, 9-hexacosene-1,26-diyl group,

10-hexacosene-1,26-diyl group, 11-hexacosene-1,26-diyl group, 12-hexacosene-1,26-diyl group, 13-hexacosene-1,26-diyl group, 14-hexacosene-1,26-diyl group, 15-hexacosene-1,26-diyl group, 16-hexacosene-1,26-diyl group, 17-hexacosene-1,26-diyl group, 18-hexacosene-1,26-diyl group, 19-hexacosene-1,26-diyl group, 20-hexacosene-1,26-diyl group, 21-hexacosene-1,26-diyl group, 22-hexacosene-1,26-diyl group, 23-hexacosene-1,26-diyl group, 24-hexacosene-1,26-diyl group;

2-heptacosene-1,27-diyl group, 3-heptacosene-1,27-diyl group, 4-heptacosene-1,27-diyl group, 5-heptacosene-1,27-diyl group, 6-heptacosene-1,27-diyl group, 7-heptacosene-1,27-diyl group, 8-heptacosene-1,27-diyl group, 9-heptacosene-1,27-diyl group, 10-heptacosene-1,27-diyl group, 11-heptacosene-1,27-diyl group, 12-heptacosene-1,27-diyl group, 13-heptacosene-1,27-diyl group, 14-heptacosene-1,27-diyl group, 15-heptacosene-1,27-diyl group, 16-heptacosene-1,27-diyl group, 17-heptacosene-1,27-diyl group, 18-heptacosene-1,27-diyl group, 19-heptacosene-1,27-diyl group, 20-heptacosene-1,27-diyl group, 21-heptacosene-1,27-diyl group, 22-heptacosene-1,27-diyl group, 23-heptacosene-1,27-diyl group, 24-heptacosene-1,27-diyl group, 25-heptacosene-1,27-diyl group;

2-octacosene-1,28-diyl group, 3-octacosene-1,28-diyl group, 4-octacosene-1,28-diyl group, 5-octacosene-1,28-diyl group, 6-octacosene-1,28-diyl group, 7-octacosene-1,28-diyl group, 8-octacosene-1,28-diyl group, 9-octacosene-1,28-diyl group, 10-octacosene-1,28-diyl group, 11-octacosene-1,28-diyl group, 12-octacosene-1,28-diyl group, 13-octacosene-1,28-diyl group, 14-octacosene-1,28-diyl group, 15-octacosene-1,28-diyl group, 16-octacosene-1,28-diyl group, 17-octacosene-1,28-diyl group, 18-octacosene-1,28-diyl group, 19-octacosene-1,28-diyl group, 20-octacosene-1,28-diyl group, 21-octacosene-1,28-diyl group, 22-octacosene-1,28-diyl group, 23-octacosene-1,28-diyl group, 24-octacosene-1,28-diyl group, 25-octacosene-1,28-diyl group, 26-octacosene-1,28-diyl group;

2-nonacosene-1,29-diyl group, 3-nonacosene-1,29-diyl group, 4-nonacosene-1,29-diyl group, 5-nonacosene-1,29-diyl group, 6-nonacosene-1,29-diyl group, 7-nonacosene-1,29-diyl group, 8-nonacosene-1,29-diyl group, 9-nonacosene-1,29-diyl group, 10-nonacosene-1,29-diyl group, 11-nonacosene-1,29-diyl group, 12-nonacosene-1,29-diyl group, 13-nonacosene-1,29-diyl group, 14-nonacosene-1,29-diyl group, 15-nonacosene-1,29-diyl group, 16-nonacosene-1,29-diyl group, 17-nonacosene-1,29-diyl group, 18-nonacosene-1,29-diyl group, 19-nonacosene-1,29-diyl group, 20-nonacosene-1,29-diyl group, 21-nonacosene-1,29-diyl group, 22-nonacosene-1,29-diyl group, 23-nonacosene-1,29-diyl group, 24-nonacosene-1,29-diyl group, 25-nonacosene-1,29-diyl group, 26-nonacosene-1,29-diyl group, 27-nonacosene-1,29-diyl group;

2-triacontene-1,30-diyl group, 3-triacontene-1,30-diyl group, 4-triacontene-1,30-diyl group, 5-triacontene-1,30-diyl group, 6-triacontene-1,30-diyl group, 7-triacontene-1,30-diyl group, 8-triacontene-1,30-diyl group, 9-triacontene-1,30-diyl group, 10-triacontene-1,30-diyl group, 11-triacontene-1,30-diyl group, 12-triacontene-1,30-diyl group, 13-triacontene-1,30-diyl group, 14-triacontene-1,30-diyl group, 15-triacontene-1,30-diyl group, 16-triacontene-1,30-diyl group, 17-triacontene-1,30-diyl group, 18-triacontene-1,30-diyl group, 19-triacontene-1,30-diyl group, 20-triacontene-1,30-diyl group, 21-triacontene-1,30-diyl group, 22-triacontene-1,30-diyl group, 23-triacontene-1,30-diyl group, 24-triacontene-1,30-diyl group, 25-triacontene-1,30-diyl group, 26-triacontene-1,30-diyl group, 27-triacontene-1,30-diyl group, and 28-triacontene-1,30-diyl group;

as well as branched alkenylene groups such as 1-methlethylene-1,2-diyl group, 2-methyl-1-propene-1,3-diyl group, 2-methyl-2-propene-1,3-diyl group, 2-methyl-1-butene-1,4-diyl group, 3-methyl-2-butene-1,4-diyl group, 2-methyl-3-butene-1,4-diyl group, 2,3-dimethyl-1,3-butadiene-1,4-diyl group, 3-ethyl-2-propene-1,5-diyl group, 4-methyl-3-propene-1,5-diyl group, 3-methyl-2,4-propadiene-1,5-diyl group, 3,4-diethyl-2-hexene-1,6-diyl group, 4-methyl-3-hexene-1,6-diyl group, 2-methyl-4-hexene-1,6-diyl group, 3,5-dimethyl-2,4-hexadiene-1,6-diyl group, 5-ethyl-3-methyl-2-heptene-1,7-diyl group, 5-methyl-3-heptene-1,7-diyl group, 4-n-propyl-4-heptene-1,7-diyl group, 3,6-dimethyl-5-heptene-1,7-diyl group, 5-ethyl-2,4-heptadiene-1,7-diyl group, 2,6-dimethyl-2,5-heptadiene-1,7-diyl group, 4-ethyl-3,5-heptadiene-1,7-diyl group, 4-ethyl-6,6-dimethyl-2-octene-1,8-diyl group, 5-n-propyl-3-octene-1,8-diyl group, 3-ethyl-4-octene-1,8-diyl group, 4-ethyl-2-methyl-6-i-propyl-5-octene-1,8-diyl group, 3,4,5-trimethyl-6-octene-1,8-diyl group, 5-ethyl-7-methyl-2,4-octadiene-1,8-diyl group, 3-methyl-2,5-octadiene-1,8-diyl group, 5-n-propyl-2,6-octadiene-1,8-diyl group, 4-methyl-2,4,6-octatriene-1,8-diyl group, 5-ethyl-2-nonene-1,9-diyl group, 3,5,6-trimethyl-3-nonene-1,9-diyl group, 2,4,5,7-tetramethyl-4-nonene-1,9-diyl group, 3,4-diethyl-5-nonene-1,9-diyl group, 4-i-propyl-6-nonene-1,9-diyl group, 3-ethyl-7-nonene-1,9-diyl group, 5-n-butyl-2-decene-1,10-diyl group, 6-i-propyl-3-decene-1,10-diyl group, 5-ethyl-4-decene-1,10-diyl group, 6,7-dimethyl-5-decene-1,10-diyl group, 4-ethyl-6-decene-1,10-diyl group, 5-methyl-7-decene-1,10-diyl group, 6-ethyl-4-methyl-8-decene-1,10-diyl group:
6-methyl-2-undecene-1,11-diyl group, 4-ethyl-3-undecene-1,11-diyl group, 5-methyl-4-undecene-1,11-diyl group, 7-ethyl-5-undecene-1,11-diyl group, 5-methyl-6-undecene-1,11-diyl group, 9-ethyl-7-undecene-1,11-diyl group, 3-methyl-8-undecene-1,11-diyl group, 4-ethyl-9-undecene-1,11-diyl group; 4-ethyl-2-dodecene-1,12-diyl group, 5-methyl-3-dodecene-1,12-diyl group, 6-ethyl-4-dodecene-1,12-diyl group, 7-methyl-5-dodecene-1,12-diyl group, 8-ethyl-6-dodecene-1,12-diyl group, 9-methyl-7-dodecene-1,12-diyl group, 10-ethyl-8-dodecene-1,12-diyl group, 2-methyl-9-dodecene-1,12-diyl group, 5-ethyl-10-dodecene-1,12-diyl group; 4,7,9-trimethyl-2-tridecene-1,13-diyl group, 10-methyl-3-tridecene-1,13-diyl group, 8-ethyl-4-tridecene-1,13-diyl group, 4-methyl-5-tridecene-1,13-diyl group, 5-ethyl-6-tridecene-1,13-diyl group, 3,6-diethyl-7-tridecene-1,13-diyl group, 5-methyl-8-tridecene-1,13-diyl group, 7-ethyl-9-tridecene-1,13-diyl group, 4-methyl-10-tridecene-1,13-diyl group, 6-ethyl-11-tridecene-1,13-diyl group;
7-methyl-2-tetradecene-1,14-diyl group, 8-ethyl-3-tetradecene-1,14-diyl group, 6-n-propyl-4-tetradecene-1,14-diyl group, 8-methyl-5-tetradecene-1,14-diyl group, 3-ethyl-6-tetradecene-1,14-diyl group, 10-methyl-7-tetradecene-1,14-diyl group, 6-i-propyl-8-tetradecene-1,14-diyl group, 5,7,11-trimethyl-9-tetradecene-1,14-diyl group, 5-ethyl-10-tetradecene-1,14-diyl group, 6-methyl-11-tetradecene-1,14-diyl group, 4-n-butyl-12-tetradecene-1,14-diyl group;
4-methyl-2-pentadecene-1,15-diyl group, 6-ethyl-3-pentadecene-1,15-diyl group, 8-methyl-4-pentadecene-1,15-diyl group, 10-ethyl-5-pentadecene-1,15-diyl group, 4,9-dimethyl-6-pentadecene-1,15-diyl group, 10-ethyl-7-pentadecene-1,15-diyl group, 6-methyl-8-pentadecene-1,15-diyl group, 8-n-propyl-9-pentadecene-1,15-diyl group, 5-methyl-10-pentadecene-1,15-diyl group, 4,7-diethyl-11-pentadecene-1,15-diyl group, 5-methyl-12-pentadecene-1,15-diyl group, 8-ethyl-13-pentadecene-1,15-diyl group;
8-i-propyl-2-hexadecene-1,16-diyl group, 6-methyl-3-hexadecene-1,16-diyl group, 8-ethyl-4-hexadecene-1,16-diyl group, 9-methyl-5-hexadecene-1,16-diyl group, 10-ethyl-6-hexadecene-1,16-diyl group, 5-methyl-7-hexadecene-1,16-diyl group, 5,10-dimethyl-8-hexadecene-1,16-diyl group, 5-ethyl-9-hexadecene-1,16-diyl group, 7,12-diethyl-10-hexadecene-1,16-diyl group, 5-ethyl-7-methyl-11-hexadecene-1,16-diyl group, 5-methyl-12-hexadecene-1,16-diyl group, 8-s-butyl-13-hexadecene-1,16-diyl group, 5-ethyl-14-hexadecene-1,16-diyl group;
11-methyl-2-heptadecene-1,17-diyl group, 9-ethyl-3-heptadecene-1,17-diyl group, 6-i-propyl-4-heptadecene-1,17-diyl group, 8-methyl-5-heptadecene-1,17-diyl group, 4-ethyl-6-heptadecene-1,17-diyl group, 10-methyl-7-heptadecene-1,17-diyl group, 5,11-dimethyl-8-heptadecene-1,17-diyl group, 5-ethyl-9-heptadecene-1,17-diyl group, 8-ethyl-10-heptadecene-1,17-diyl group, 7-methyl-11-heptadecene-1,17-diyl group, 5-i-propyl-12-heptadecene-1,17-diyl group, 9-ethyl-13-heptadecene-1,17-diyl group, 8-methyl-14-heptadecene-1,17-diyl group, 7-s-butyl-15-heptadecene-1,17-diyl group;
10,15-dimethyl-2-octadecene-1,18-diyl group, 6-ethyl-3-octadecene-1,18-diyl group, 10-methyl-4-octadecene-1,18-diyl group, 11-methyl-5-octadecene-1,18-diyl group, 12-ethyl-6-octadecene-1,18-diyl group, 10-methyl-7-octadecene-1,18-diyl group, 5-methyl-8-octadecene-1,18-diyl group, 8-ethyl-9-octadecene-1,18-diyl group, 7-methyl-10-octadecene-1,18-diyl group, 9-n-butyl-11-octadecene-1,18-diyl group, 7-methyl-12-octadecene-1,18-diyl group, 9-ethyl-13-octadecene-1,18-diyl group, 10-i-propyl-14-octadecene-1,18-diyl group, 7-methyl-15-octadecene-1,18-diyl group, 10-ethyl-16-octadecene-1,18-diyl group;
10-methyl-2-nonadecene-1,19-diyl group, 10,12-diethyl-3-nonadecene-1,19-diyl group, 6-methyl-4-nonadecene-1,19-diyl group, 7-ethyl-5-nonadecene-1,19-diyl group, 9-n-propyl-6-nonadecene-1,19-diyl group, 10-methyl-7-nonadecene-1,19-diyl group, 12-i-propyl-8-nonadecene-1,19-diyl group, 5,15-dimethyl-9-nonadecene-1,19-diyl group, 7-ethyl-13-methyl-10-nonadecene-1,19-diyl group, 6-methyl-11-nonadecene-1,19-diyl group, 6-ethyl-12-nonadecene-1,19-diyl group, 7,15-diethyl-13-nonadecene-1,19-diyl group, 9-s-butyl-14-nonadecene-1,19-diyl group, 8-methyl-15-nonadecene-1,19-diyl group, 10-ethyl-16-nonadecene-1,19-diyl group, 10-i-propyl-17-nonadecene-1,19-diyl group;
8-methyl-2-icosene-1,20-diyl group, 6-ethyl-3-icosene-1,20-diyl group, 10-i-propyl-4-icosene-1,20-diyl group, 11-n-propyl-5-icosene-1,20-diyl group, 12-methyl-6-icosene-1,20-diyl group, 11-ethyl-7-icosene-1,20-diyl group, 13-n-propyl-8-icosene-1,20-diyl group, 8-i-propyl-9-icosene-1,20-diyl group, 8-n-propyl-10-icosene-1,20-diyl group, 7-methyl-ll-icosene-1,20-diyl group, 8-ethyl-12-icosene-1,20-diyl group, 10-n-propyl-13-icosene-1,20-diyl group, 9-i-propyl-14-icosene-1,20-diyl group, 10-n-butyl-15-icosene-1,20-diyl group, 8-s-butyl-16-icosene-1,20-diyl group, 7-i-butyl-17-icosene-1,20-diyl group, 9-methyl-18-icosene-1,20-diyl group;
11-methyl-2-henicosene-1,21-diyl group, 12-n-butyl-3-henicosene-1,21-diyl group, 10-n-pentyl-4-henicosene-1,21-diyl group, 8-ethyl-5-henicosene-1,21-diyl group, 10-i-propyl-6-henicosene-1,21-diyl group, 5-n-propyl-7-henicosene-1,21-diyl group, 13-n-butyl-8-henicosene-1,21-diyl group, 15-s-butyl-9-henicosene-1,21-diyl group, 5-methyl-10-henicosene-1,21-diyl group, 15-ethyl-6-methyl-11-henicosene-1,21-diyl group, 8-ethyl-12-henicosene-1,21-diyl group,

7-methyl-13-henicosene-1,21-diyl group, 11-ethyl-14-henicosene-1,21-diyl group, 6-ethyl-15-henicosene-1,21-diyl group, 9-methyl-16-henicosene-1,21-diyl group, 5-ethyl-9-methyl-17-henicosene-1,21-diyl group, 10,10-dimethyl-18-henicosene-1,21-diyl group, 9-ethyl-19-henicosene-1,21-diyl group;

11-methyl-2-docosene-1,22-diyl group, 12-ethyl-3-docosene-1,22-diyl group, 13-i-propyl-4-docosene-1,22-diyl group, 10-n-propyl-5-docosene-1,22-diyl group, 10-n-butyl-6-docosene-1,22-diyl group, 15-s-butyl-7-docosene-1,22-diyl group, 11-i-butyl-8-docosene-1,22-diyl group, 5,15-dimethyl-9-docosene-1,22-diyl group, 8,14-diethyl-10-docosene-1,22-diyl group, 5-methyl-11-docosene-1,22-diyl group, 7-ethyl-12-docosene-1,22-diyl group, 10-methyl-13-docosene-1,22-diyl group, 10-ethyl-14-docosene-1,22-diyl group, 9-ethyl-15-docosene-1,22-diyl group, 8-methyl-16-docosene-1,22-diyl group, 7-i-propyl-17-docosene-1,22-diyl group, 10-i-butyl-18-docosene-1,22-diyl group, 9,10-dimethyl-19-docosene-1,22-diyl group, 13-ethyl-20-docosene-1,22-diyl group;

19-methyl-2-tricosene-1,23-diyl group, 10,15-dimethyl-3-tricosene-1,23-diyl group, 3,11,16-trimethyl-4-tricosene-1,23-diyl group, 12-ethyl-5-tricosene-1,23-diyl group,

6,13-diethyl-6-tricosene-1,23-diyl group, 4,12,18-triethyl-7-tricosene-1,23-diyl group, 18-i-propyl-8-tricosene-1,23-diyl group, 14-n-propyl-9-tricosene-1,23-diyl group, 8-n-butyl-10-tricosene-1,23-diyl group, 15-s-butyl-11-tricosene-1,23-diyl group, 5-i-butyl-12-tricosene-1,23-diyl group, 7-ethyl-9-methyl-13-tricosene-1,23-diyl group, 9-methyl-14-tricosene-1,23-diyl group, 4,18-dimethyl-15-tricosene-1,23-diyl group, 3,4,11-trimethyl-16-tricosene-1,23-diyl group, 9-ethyl-17-tricosene-1,23-diyl group, 10,13-diethyl-18-tricosene-1,23-diyl group, 5,8,21-triethyl-19-tricosene-1,23-diyl group, 15-i-propyl-20-tricosene-1,23-diyl group, 17-n-propyl-21-tricosene-1,23-diyl group;

16-n-butyl-2-tetracosene-1,24-diyl group, 11-s-butyl-3-tetracosene-1,24-diyl group, 8-i-butyl-4-tetracosene-1,24-diyl group, 18-ethyl-9-methyl-5-tetracosene-1,24-diyl group, 13-methyl-6-tetracosene-1,24-diyl group, 4,19-dimethyl-7-tetracosene-1,24-diyl group, 5,10,17-triethyl-8-tetracosene-1,24-diyl group, 6-ethyl-9-tetracosene-1,24-diyl group, 7,16-diethyl-10-tetracosene-1,24-diyl group, 5,9,18-triethyl-11-tetracosene-1,24-diyl group, 10-n-propyl-12-tetracosene-1,24-diyl group, 20-i-propyl-13-tetracosene-1,24-diyl group, 9-n-butyl-14-tetracosene-1,24-diyl group, 11-s-butyl-15-tetracosene-1,24-diyl group, 13-i-butyl-16-tetracosene-1,24-diyl group, 10-ethyl-13-methyl-17-tetracosene-1,24-diyl group, 6-methyl-18-tetracosene-1,24-diyl group, 5,7-dimethyl-19-tetracosene-1,24-diyl group, 4,8,13-trimethyl-20-tetracosene-1,24-diyl group, 18-ethyl-21-tetracosene-1,24-diyl group, 6,10-diethyl-22-tetracosene-1,24-diyl group;

9,13,16-trimethyl-2-pentacosene-1,25-diyl group, 12-n-propyl-3-pentacosene-1,25-diyl group, 11-i-propyl-4-pentacosene-1,25-diyl group, 20-n-butyl-5-pentacosene-1,25-diyl group, 17-i-butyl-6-pentacosene-1,25-diyl group, 15-s-butyl-7-pentacosene-1,25-diyl group, 15-ethyl-23-methyl-8-pentacosene-1,25-diyl group, 11-methyl-8-pentacosene-1,25-diyl group, 13,17-dimethyl-9-pentacosene-1,25-diyl group, 5,8,21-trimethyl-10-pentacosene-1,25-diyl group, 17-ethyl-11-pentacosene-1,25-diyl group, 8,18-dimethyl-12-pentacosene-1,25-diyl group, 10,15,18-trimethyl-13-pentacosene-1,25-diyl group, 4-n-propyl-14-pentacosene-1,25-diyl group, 20-i-propyl-15-pentacosene-1,25-diyl group, 8-n-butyl-16-pentacosene-1,25-diyl group, 11-s-butyl-17-pentacosene-1,25-diyl group, 5,22-dimethyl-18-pentacosene-1,25-diyl group, 5-i-butyl-19-pentacosene-1,25-diyl group, 9-methyl-13-ethyl-20-pentacosene-1,25-diyl group, 15-methyl-21-pentacosene-1,25-diyl group, 6,13-dimethyl-22-pentacosene-1,25-diyl group, 4,8,12-trimethyl-23-pentacosene-1,25-diyl group;

13-ethyl-2-hexacosene-1,26-diyl group, 5,16-diethyl-3-hexacosene-1,26-diyl group, 7,11,16-trimethyl-4-hexacosene-1,26-diyl group, 12-n-propyl-5-hexacosene-1,26-diyl group,

21-i-propyl-6-hexacosene-1,26-diyl group, 6-n-butyl-7-hexacosene-1,26-diyl group, 13-s-butyl-8-hexacosene-1,26-diyl group, 19-i-butyl-9-hexacosene-1,26-diyl group,. 13-ethyl-18-methyl-10-hexacosene-1,26-diyl group, 10-methyl-11-hexacosene-1,26-diyl group, 10,20-dimethyl-12-hexacosene-1,26-diyl group, 7,9,17-trimethyl-13-hexacosene-1,26-diyl group, 8-ethyl-14-hexacosene-1,26-diyl group,

5,22-diethyl-15-hexacosene-1,26-diyl group, 7,10,21-trimethyl-16-hexacosene-1,26-diyl group, 15-n-propyl-17-hexacosene-1,26-diyl group, 13-i-propyl-18-hexacosene-1,26-diyl group, 8-n-butyl-19-hexacosene-1,26-diyl group, 11-s-butyl-20-hexacosene-1,26-diyl group, 14-i-butyl-21-hexacosene-1,26-diyl group, 5-ethyl-21-methyl-22-hexacosene-1,26-diyl group, 7-methyl-23-hexacosene-1,26-diyl group, 8,14-dimethyl-24-hexacosene-1,26-diyl group; 7,16,24-trimethyl-2-heptacosene-1,27-diyl group, 9-ethyl-3-heptacosene-1,27-diyl group, 7,16-dimethyl-4-heptacosene-1,27-diyl group, 9,13,21-trimethyl-5-heptacosene-1,27-diyl group, 13-n-propyl-6-heptacosene-1,27-diyl group, 10-i-propyl-7-heptacosene-1,27-diyl group, 16-n-propyl-8-heptacosene-1,27-diyl group, 18-methyl-9-heptacosene-1,27-diyl group, 9-i-propyl-10-heptacosene-1,27-diyl group, 15-ethyl-7-methyl-11-heptacosene-1,27-diyl group, 25-methyl-12-heptacosene-1,27-diyl group, 8,21-dimethyl-13-heptacosene-1,27-diyl group, 5,11,23-trimethyl-14-heptacosene-1,27-diyl group, 9-ethyl-15-heptacosene-1,27-diyl group, 8,20-dimethyl-16-heptacosene-1,27-diyl group, 4,8,19-trimethyl-17-heptacosene-1,27-diyl group, 7-n-propyl-18-heptacosene-1,27-diyl group, 21-i-propyl-19-heptacosene-1,27-diyl group, 14-n-propyl-20-heptacosene-1,27-diyl group, 8-ethyl-21-heptacosene-1,27-diyl group, 11-i-propyl-22-heptacosene-1,27-diyl group, 5-ethyl-13-methyl-23-heptacosene-1,27-diyl group, 16-methyl-24-heptacosene-1,27-diyl group, 7-ethyl-25-heptacosene-1,27-diyl group;

14-ethyl-2-octacosene-1,28-diyl group, 20-methyl-3-octacosene-1,28-diyl group, 7,22-dimethyl-4-octacosene-1,28-diyl group, 19-ethyl-5-octacosene-1,28-diyl group, 11-methyl-6-octacosene-1,28-diyl group, 13,16-dimethyl-7-octacosene-1,28-diyl group, 13-ethyl-8-octacosene-1,28-diyl group, 6-methyl-9-octacosene-1,28-diyl group, 9,16-dimethyl-10-octacosene-1,28-diyl group, 7-ethyl-11-octacosene-1,28-diyl group, 16-methyl-12-octacosene-1,28-diyl group, 6,15-dimethyl-13-octacosene-1,28-diyl group, 22-ethyl-14-octacosene-1,28-diyl group, 6-methyl-15-octacosene-1,28-diyl group, 8,11-dimethyl-16-octacosene-1,28-diyl group, 23-ethyl-17-octacosene-1,28-diyl group, 4-methyl-18-octacosene-1,28-diyl group, 7,14-dimethyl-19-octacosene-1,28-diyl group, 13-ethyl-20-octacosene-1,28-diyl group, 8-methyl-21-octacosene-1,28-diyl group, 11,17-dimethyl-22-octacosene-1,28-diyl group, 10-ethyl-23-octacosene-1,28-diyl group, 9-methyl-24-octacosene-1,28-diyl group, 7,19-dimethyl-25-octacosene-1,28-diyl group, 12-ethyl-26-octacosene-1,28-diyl group;

15-methyl-2-nonacosene-1,29-diyl group, 14-methyl-3-nonacosene-1,29-diyl group, 12-methyl-4-nonacosene-1,29-diyl group, 13-methyl-5-nonacosene-1,29-diyl group, 11-methyl-6-nonacosene-1,29-diyl group, 10-methyl-7-nonacosene-1,29-diyl group, 25-methyl-8-nonacosene-1,29-diyl group, 24-methyl-9-nonacosene-1,29-diyl group, 23-methyl-10-nonacosene-1,29-diyl group, 22-methyl-11-nonacosene-1,29-diyl group, 21-methyl-12-nonacosene-1,29-diyl group, 20-methyl-13-nonacosene-1,29-diyl group, 19-methyl-14-nonacosene-1,29-diyl group, 18-methyl-15-nonacosene-1,29-diyl group, 27-methyl-16-nonacosene-1,29-diyl group, 26-methyl-17-nonacosene-1,29-diyl group, 25-methyl-18-nonacosene-1,29-diyl group, 24-methyl-19-nonacosene-1,29-diyl group, 23-methyl-20-nonacosene-1,29-diyl group, 20-methyl-21-nonacosene-1,29-diyl group, 19-methyl-22-nonacosene-1,29-diyl group, 18-methyl-23-nonacosene-1,29-diyl group, 17-methyl-24-nonacosene-1,29-diyl group, 16-methyl-25-nonacosene-1,29-diyl group, 6-methyl-26-nonacosene-1,29-diyl group, and 5-methyl-27-nonacosene-1,29-diyl group.

If G represents an optionally substituted straight-chained or branched alkynylene group having 2 - 30 carbon atoms, exemplary straight-chained or branched alkynylene groups having 2 - 30 carbon atoms include straight-chained alkynylene groups such as acetylene-1,2-diyl group, 1-propyne-1,3-diyl group, 2-propyne-1,3-diyl group, 1-butyne-1,4-diyl group, 2-butyne-1,4-diyl group, 3-butyne-1,4-diyl group, 1,3-butadiyne-1,4-diyl group, 2-pentyne-1,5-diyl group, 3-pentyne-1,5-diyl group, 2,4-pentadiyne-1,5-diyl group, 2-hexyne-1,6-diyl group, 3-hexyne-1,6-diyl group, 4-hexyne-1,6-diyl group, 2,4-hexadiyne-1,6-diyl group, 2-heptyne-1,7-diyl group, 3-heptyne-1,7-diyl group, 4-heptyne-1,7-diyl group, 5-heptyne-1,7-diyl group, 2,4-heptadiyne-1,7-diyl group, 2,5-heptadiyne-1,7-diyl group, 3,5-heptadiyne-1,7-diyl group, 2-octyne-1,8-diyl group, 3-octyne-1,8-diyl group, 4-octyne-1,8-diyl group, 5-octyne-1,8-diyl group, 6-octyne-1,8-diyl group, 2,4-octadiyne-1,8-diyl group, 2,5-octadiyne-1,8-diyl group, 2,6-octadiyne-1,8-diyl group, 2,4,6-octatriyne-1,8-diyl group, 2-nonyne-1,9-diyl group, 3-nonyne-1,9-diyl group, 4-nonyne-1,9-diyl group, 5-nonyne-1,9-diyl group, 6-nonyne-1,9-diyl group, 7-nonyne-1,9-diyl group, 2-decyne-1,10-diyl group, 3-decyne-1,10-diyl group, 4-decyne-1,10-diyl group, 5-decyne-1,10-diyl group, 6-decyne-1,10-diyl group, 7-decyne-1,10-diyl group, 8-decyne-1,10-diyl group;

2-undecyne-1,11-diyl group, 3-undecyne-1,11-diyl group, 4-undecyne-1,11-diyl group, 5-undecyne-1,11-diyl group, 6-undecyne-1,11-diyl group, 7-undecyne-1,11-diyl group, 8-undecyne-1,11-diyl group, 9-undecyne-1,11-diyl group;

2-dodecyne-1,12-diyl group, 3-dodecyne-1,12-diyl group, 4-dodecyne-1,12-diyl group, 5-dodecyne-1,12-diyl group, 6-dodecyne-1,12-diyl group, 7-dodecyne-1,12-diyl group, 8-dodecyne-1,12-diyl group, 9-dodecyne-1,12-diyl group, 10-dodecyne-1,12-diyl group;

2-tridecyne-1,13-diyl group, 3-tridecyne-1,13-diyl group, 4-tridecyne-1,13-diyl group, 5-tridecyne-1,13-diyl group, 6-tridecyne-1,13-diyl group, 7-tridecyne-1,13-diyl group, 8-tridecyne-1,13-diyl group, 9-tridecyne-1,13-diyl group, 10-tridecyne-1,13-diyl group, 11-tridecyne-1,13-diyl group;

2-tetradecyne-1,14-diyl group, 3-tetradecyne-1,14-diyl group, 4-tetradecyne-1,14-diyl group, 5-tetradecyne-1,14-diyl group, 6-tetradecyne-1,14-diyl group, 7-tetradecyne-1,14-diyl group, 8-tetradecyne-1,14-diyl group, 9-tetradecyne-1,14-diyl group, 10-tetradecyne-1,14-diyl group, 11-tetradecyne-1,14-diyl group, 12-tetradecyne-1,14-diyl group;

2-pentadecyne-1,15-diyl group, 3-pentadecyne-1,15-diyl group, 4-pentadecyne-1,15-diyl group, 5-pentadecyne-1,15-diyl group, 6-pentadecyne-1,15-diyl group, 7-pentadecyne-1,15-diyl group, 8-pentadecyne-1,15-diyl group, 9-pentadecyne-1,15-diyl group, 10-pentadecyne-1,15-diyl group, 11-pentadecyne-1,15-diyl group, 12-pentadecyne-1,15-diyl group, 13-pentadecyne-1,15-diyl group;

2-hexadecyne-1,16-diyl group, 3-hexadecyne-1,16-diyl group, 4-hexadecyne-1,16-diyl group, 5-hexadecyne-1,16-diyl group, 6-hexadecyne-1,16-diyl group, 7-hexadecyne-1,16-diyl group, 8-hexadecyne-1,16-diyl group, 9-hexadecyne-1,16-diyl group, 10-hexadecyne-1,16-diyl group, 11-hexadecyne-1,16-diyl group, 12-hexadecyne-1,16-diyl group, 13-hexadecyne-1,16-diyl group, 14-hexadecyne-1,16-diyl group;

2-heptadecyne-1,17-diyl group, 3-heptadecyne-1,17-diyl group, 4-heptadecyne-1,17-diyl group, 5-heptadecyne-1,17-diyl group, 6-heptadecyne-1,17-diyl group, 7-heptadecyne-1,17-diyl group, 8-heptadecyne-1,17-diyl group, 9-heptadecyne-1,17-diyl group, 10-heptadecyne-1,17-diyl group, 11-heptadecyne-1,17-diyl group, 12-heptadecyne-1,17-diyl group, 13-heptadecyne-1,17-diyl group, 14-heptadecyne-1,17-diyl group, 15-heptadecyne-1,17-diyl group;

2-octadecyne-1,18-diyl group, 3-octadecyne-1,18-diyl group, 4-octadecyne-1,18-diyl group, 5-octadecyne-1,18-diyl group, 6-octadecyne-1,18-diyl group, 7-octadecyne-1,18-diyl group, 8-octadecyne-1,18-diyl group, 9-octadecyne-1,18-diyl group, 10-octadecyne-1,18-diyl group, 11-octadecyne-1,18-diyl group, 12-octadecyne-1,18-diyl group, 13-octadecyne-1,18-diyl group, 14-octadecyne-1,18-diyl group, 15-octadecyne-1,18-diyl group, 16-octadecyne-1,18-diyl group;

2-nonadecyne-1,19-diyl group, 3-nonadecyne-1,19-diyl group, 4-nonadecyne-1,19-diyl group, 5-nonadecyne-1,19-diyl group, 6-nonadecyne-1,19-diyl group, 7-nonadecyne-1,19-diyl group, 8-nonadecyne-1,19-diyl group, 9-nonadecyne-1,19-diyl group, 10-nonadecyne-1,19-diyl group, 11-nonadecyne-1,19-diyl group, 12-nonadecyne-1,19-diyl group, 13-nonadecyne-1,19-diyl group, 14-nonadecyne-1,19-diyl group, 15-nonadecyne-1,19-diyl group, 16-nonadecyne-1,19-diyl group, 17-nonadecyne-1,19-diyl group;

2-icosyne-1,20-diyl group, 3-icosyne-1,20-diyl group, 4-icosyne-1,20-diyl group, 5-icosyne-1,20-diyl group, 6-icosyne-1,20-diyl group, 7-icosyne-1,20-diyl group, 8-icosyne-1,20-diyl group, 9-icosyne-1,20-diyl group, 10-icosyne-1,20-diyl group, 11-icosyne-1,20-diyl group, 12-icosyne-1,20-diyl group, 13-icosyne-1,20-diyl group, 14-icosyne-1,20-diyl group, 15-icosyne-1,20-diyl group, 16-icosyne-1,20-diyl group, 17-icosyne-1,20-diyl group, 18-icosyne-1,20-diyl group;

2-henicosyne-1,21-diyl group, 3-henicosyne-1,21-diyl group, 4-henicosyne-1,21-diyl group, 5-henicosyne-1,21-diyl group, 6-henicosyne-1,21-diyl group, 7-henicosyne-1,21-diyl group, 8-henicosyne-1,21-diyl group, 9-henicosyne-1,21-diyl group, 10-henicosyne-1,21-diyl group, 11-henicosyne-1,21-diyl group, 12-henicosyne-1,21-diyl group, 13-henicosyne-1,21-diyl group, 14-henicosyne-1,21-diyl group, 15-henicosyne-1,21-diyl group, 16-henicosyne-1,21-diyl group, 17-henicosyne-1,21-diyl group, 18-henicosyne-1,21-diyl group, 19-henicosyne-1,21-diyl group;

2-docosyne-1,22-diyl group, 3-docosyne-1,22-diyl group, 4-docosyne-1,22-diyl group, 5-docosyne-1,22-diyl group, 6-docosyne-1,22-diyl group, 7-docosyne-1,22-diyl group, 8-docosyne-1,22-diyl group, 9-docosyne-1,22-diyl group, 10-docosyne-1,22-diyl group, 11-docosyne-1,22-diyl group, 12-docosyne-1,22-diyl group, 13-docosyne-1,22-diyl group, 14-docosyne-1,22-diyl group, 15-docosyne-1,22-diyl group, 16-docosyne-1,22-diyl group, 17-docosyne-1,22-diyl group, 18-docosyne-1,22-diyl group, 19-docosyne-1,22-diyl group, 20-docosyne-1,22-diyl group;

2-tricosyne-1,23-diyl group, 3-tricosyne-1,23-diyl group, 4-tricosyne-1,23-diyl group, 5-tricosyne-1,23-diyl group, 6-tricosyne-1,23-diyl group, 7-tricosyne-1,23-diyl group, 8-tricosyne-1,23-diyl group, 9-tricosyne-1,23-diyl group, 10-tricosyne-1,23-diyl group, 11-tricosyne-1,23-diyl group, 12-tricosyne-1,23-diyl group, 13-tricosyne-1,23-diyl group, 14-tricosyne-1,23-diyl group, 15-tricosyne-1,23-diyl group, 16-tricosyne-1,23-diyl group, 17-tricosyne-1,23-diyl group, 18-tricosyne-1,23-diyl group, 19-tricosyne-1,23-diyl group, 20-tricosyne-1,23-diyl group, 21-tricosyne-1,23-diyl group;

2-tetracosyne-1,24-diyl group, 3-tetracosyne-1,24-diyl group, 4-tetracosyne-1,24-diyl group, 5-tetracosyne-1,24-diyl group, 6-tetracosyne-1,24-diyl group, 7-tetracosyne-1,24-diyl group, 8-tetracosyne-1,24-diyl group, 9-tetracosyne-1,24-diyl group, 10-tetracosyne-1,24-diyl group, 11-tetracosyne-1,24-diyl group, 12-tetracosyne-1,24-diyl group, 13-tetracosyne-1,24-diyl group, 14-tetracosyne-1,24-diyl group, 15-tetracosyne-1,24-diyl group, 16-tetracosyne-1,24-diyl group, 17-tetracosyne-1,24-diyl group, 18-tetracosyne-1,24-diyl group, 19-tetracosyne-1,24-diyl group, 20-tetracosyne-1,24-diyl group, 21-tetracosyne-1,24-diyl group, 22-tetracosyne-1,24-diyl group;

2-pentacosyne-1,25-diyl group, 3-pentacosyne-1,25-diyl group, 4-pentacosyne-1,25-diyl group, 5-pentacosyne-1,25-diyl group, 6-pentacosyne-1,25-diyl group, 7-pentacosyne-1,25-diyl group, 8-pentacosyne-1,25-diyl group, 8-pentacosyne-1,25-diyl group, 9-pentacosyne-1,25-diyl group, 10-pentacosyne-1,25-diyl group, 11-pentacosyne-1,25-diyl group, 12-pentacosyne-1,25-diyl group, 13-pentacosyne-1,25-diyl group, 14-pentacosyne-1,25-diyl group, 15-pentacosyne-1,25-diyl group, 16-pentacosyne-1,25-diyl group, 17-pentacosyne-1,25-diyl group, 18-pentacosyne-1,25-diyl group, 19-pentacosyne-1,25-diyl group, 20-pentacosyne-1,25-diyl group, 21-pentacosyne-1,25-diyl group, 22-pentacosyne-1,25-diyl group, 23-pentacosyne-1,25-diyl group;

2-hexacosyne-1,26-diyl group, 3-hexacosyne-1,26-diyl group, 4-hexacosyne-1,26-diyl group, 5-hexacosyne-1,26-diyl group, 6-hexacosyne-1,26-diyl group, 7-hexacosyne-1,26-diyl group,

8-hexacosyne-1,26-diyl group, 9-hexacosyne-1,26-diyl group, 10-hexacosyne-1,26-diyl group, 11-hexacosyne-1,26-diyl group, 12-hexacosyne-1,26-diyl group, 13-hexacosyne-1,26-diyl group, 14-hexacosyne-1,26-diyl group, 15-hexacosyne-1,26-diyl group, 16-hexacosyne-1,26-diyl group, 17-hexacosyne-1,26-diyl group, 18-hexacosyne-1,26-diyl group, 19-hexacosyne-1,26-diyl group, 20-hexacosyne-1,26-diyl group, 21-hexacosyne-1,26-diyl group, 22-hexacosyne-1,26-diyl group, 23-hexacosyne-1,26-diyl group, 24-hexacosyne-1,26-diyl group;

2-heptacosyne-1,27-diyl group, 3-heptacosyne-1,27-diyl group, 4-heptacosyne-1,27-diyl group, 5-heptacosyne-1,27-diyl group, 6-heptacosyne-1,27-diyl group, 7-heptacosyne-1,27-diyl group, 8-heptacosyne-1,27-diyl group, 9-heptacosyne-1,27-diyl group, 10-heptacosyne-1,27-diyl group, 11-heptacosyne-1,27-diyl group, 12-heptacosyne-1,27-diyl group, 13-heptacosyne-1,27-diyl group, 14-heptacosyne-1,27-diyl group, 15-heptacosyne-1,27-diyl group, 16-heptacosyne-1,27-diyl group, 17-heptacosyne-1,27-diyl group, 18-heptacosyne-1,27-diyl group, 19-heptacosyne-1,27-diyl group, 20-heptacosyne-1,27-diyl group, 21-heptacosyne-1,27-diyl group, 22-heptacosyne-1,27-diyl group, 2,3-heptacosyne-1,27-diyl group, 24-heptacosyne-1,27-diyl group, 25-heptacosyne-1,27-diyl group;

2-octacosyne-1,28-diyl group, 3-octacosyne-1,28-diyl group, 4-octacosyne-1,28-diyl group, 5-octacosyne-1,28-diyl group, 6-octacosyne-1,28-diyl group, 7-octacosyne-1,28-diyl group, 8-octacosyne-1,28-diyl group, 9-octacosyne-1,28-diyl group,

10-octacosyne-1,28-diyl group, 11-octacosyne-1,28-diyl group, 12-octacosyne-1,28-diyl group, 13-octacosyne-1,28-diyl group, 14-octacosyne-1,28-diyl group, 15-octacosyne-1,28-diyl group, 16-octacosyne-1,28-diyl group, 17-octacosyne-1,28-diyl group, 18-octacosyne-1,28-diyl group, 19-octacosyne-1,28-diyl group, 20-octacosyne-1,28-diyl group, 21-octacosyne-1,28-diyl group, 22-octacosyne-1,28-diyl group, 23-octacosyne-1,28-diyl group, 24-octacosyne-1,28-diyl group, 25-octacosyne-1,28-diyl group, 26-octacosyne-1,28-diyl group;

2-nonacosyne-1,29-diyl group, 3-nonacosyne-1,29-diyl group, 4-nonacosyne-1,29-diyl group, 5-nonacosyne-1,29-diyl group, 6-nonacosyne-1,29-diyl group, 7-nonacosyne-1,29-diyl group, 8-nonacosyne-1,29-diyl group, 9-nonacosyne-1,29-diyl group, 10-nonacosyne-1,29-diyl group, 11-nonacosyne-1,29-diyl group, 12-nonacosyne-1,29-diyl group, 13-nonacosyne-1,29-diyl group, 14-nonacosyne-1,29-diyl group, 15-nonacosyne-1,29-diyl group, 16-nonacosyne-1,29-diyl group, 17-nonacosyne-1,29-diyl group, 18-nonacosyne-1,29-diyl group, 19-nonacosyne-1,29-diyl group, 20-nonacosyne-1,29-diyl group, 21-nonacosyne-1,29-diyl group, 22-nonacosyne-1,29-diyl group, 23-nonacosyne-1,29-diyl group, 24-nonacosyne-1,29-diyl group, 25-nonacosyne-1,29-diyl group, 26-nonacosyne-1,29-diyl group, 27-nonacosyne-1,29-diyl group;

2-triacontyne-1,30-diyl group, 3-triacontyne-1,30-diyl group, 4-triacontyne-1,30-diyl group, 5-triacontyne-1,30-diyl group, 6-triacontyne-1,30-diyl group, 7-triacontyne-1,30-diyl group, 8-triacontyne-1,30-diyl group, 9-triacontyne-1,30-diyl group, 10-triacontyne-1,30-diyl group, 11-triacontyne-1,30-diyl group, 12-triacontyne-1,30-diyl group, 13-triacontyne-1,30-diyl group, 14-triacontyne-1,30-diyl group, 15-triacontyne-1,30-diyl group, 16-triacontyne-1,30-diyl group, 17-triacontyne-1,30-diyl group, 18-triacontyne-1,30-diyl group, 19-triacontyne-1,30-diyl group, 20-triacontyne-1,30-diyl group, 21-triacontyne-1,30-diyl group, 22-triacontyne-1,30-diyl group, 23-triacontyne-1,30-diyl group, 24-triacontyne-1,30-diyl group, 25-triacontyne-1,30-diyl group, 26-triacontyne-1,30-diyl group, 27-triacontyne-1,30-diyl group, and 28-triacontyne-1,30-diyl group;

as well as branched alkynylene groups such as 3-methyl-1-butyne-1,4-diyl group, 2-methyl-3-butyne-1,4-diyl group, 4-methyl-2-pentyne-1,5-diyl group, 2-methyl-3-pentyne-1,5-diyl group, 4-ethyl-2-hexyne-1,6-diyl group, 5-methyl-3-hexyne-1,6-diyl group, 2-methyl-4-hexyne-1,6-diyl group, 5-ethyl-6-methyl-2-heptyne-1,7-diyl group, 5-methyl-3-heptyne-1,7-diyl group, 3-n-propyl-4-heptyne-1,7-diyl group, 4,4-dimethyl-5-heptyne-1,7-diyl group, 6-methyl-2,4-heptadiyne-1,7-diyl group, 4-methyl-2,5-heptadiyne-1,7-diyl group, 2-methyl-3,5-heptadiyne-1,7-diyl group, 4-ethyl-6,6-dimethyl-2-octyne-1,8-diyl group, 5-n-propyl-3-octyne-1,8-diyl group, 3-ethyl-4-octyne-1,8-diyl group, 4-ethyl-2-methyl-5-octyne-1,8-diyl group, 3,4,5-trimethyl-6-octyne-1,8-diyl group, 7-methyl-2,4-octadiyne-1,8-diyl group, 4-methyl-2,5-octadiyne-1,8-diyl group, 5-n-propyl-2,6-octadiyne-1,8-diyl group, 5-ethyl-2-nonyne-1,9-diyl group, 5,6,7-trimethyl-3-nonyne-1,9-diyl group, 2,3,6,7-tetramethyl-4-nonyne-1,9-diyl group, 3,4-diethyl-5-nonyne-1,9-diyl group, 4-i-propyl-6-nonyne-1,9-diyl group, 3-ethyl-7-nonyne-1,9-diyl group, 5-n-butyl-2-decyne-1,10-diyl group, 6-i-propyl-3-decyne-1,10-diyl group, 7-ethyl-4-decyne-1,10-diyl group, 3,7-dimethyl-5-decyne-1,10-diyl group, 4-ethyl-6-decyne-1,10-diyl group, 5-methyl-7-decyne-1,10-diyl group, 6-ethyl-4-methyl-8-decyne-1,10-diyl group;

6-methyl-2-undecyne-1,11-diyl group, 6-ethyl-3-undecyne-1,11-diyl group, 7-methyl-4-undecyne-1,11-diyl group, 7-ethyl-5-undecyne-1,11-diyl group, 5-methyl-6-undecyne-1,11-diyl group, 9-ethyl-7-undecyne-1,11-diyl group, 3-methyl-8-undecyne-1,11-diyl group, 4-ethyl-9-undecyne-1,11-diyl group;

5-ethyl-2-dodecyne-1,12-diyl group, 6-methyl-3-dodecyne-1,12-diyl group, 8-ethyl-4-dodecyne-1,12-diyl group, 8-methyl-5-dodecyne-1,12-diyl group, 9-ethyl-6-dodecyne-1,12-diyl group, 6-methyl-7-dodecyne-1,12-diyl group, 10-ethyl-8-dodecyne-1,12-diyl group, 2-methyl-9-dodecyne-1,12-diyl group, 5-ethyl-10-dodecyne-1,12-diyl group, 4,7,9-trimethyl-2-tridecyne-1,13-diyl group, 10-methyl-3-tridecyne-1,13-diyl group, 8-ethyl-4-tridecyne-1,13-diyl group, 4-methyl-5-tridecyne-1,13-diyl group, 5-ethyl-6-tridecyne-1,13-diyl group, 3,6-diethyl-7-tridecyne-1,13-diyl group, 5-methyl-8-tridecyne-1,13-diyl group, 7-ethyl-9-tridecyne-1,13-diyl group, 4-methyl-10-tridecyne-1,13-diyl group, 6-ethyl-11-tridecyne-1,13-diyl group;

7-methyl-2-tetradecyne-1,14-diyl group, 8-ethyl-3-tetradecyne-1,14-diyl group, 6-n-propyl-4-tetradecyne-1,14-diyl group, 8-methyl-5-tetradecyne-1,14-diyl group, 3-ethyl-6-tetradecyne-1,14-diyl group, 10-methyl-7-tetradecyne-1,14-diyl group, 6-i-propyl-8-tetradecyne-1,14-diyl group, 5,7,11-trimethyl-9-tetradecyne-1,14-diyl group, 5-ethyl-10-tetradecyne-1,14-diyl group, 6-methyl-11-tetradecyne-1,14-diyl group, 4-n-butyl-12-tetradecyne-1,14-diyl group;

4-methyl-2-pentadecyne-1,15-diyl group, 6-ethyl-3-pentadecyne-1, 15-diyl group, 8-methyl-4-pentadecyne-1,15-diyl group, 10-ethyl-5-pentadecyne-1,15-diyl group, 4,9-dimethyl-6-pentadecyne-1,15-diyl group, 10-ethyl-7-pentadecyne-1,15-diyl group, 6-methyl-8-pentadecyne-1,15-diyl group, 8-n-propyl-9-pentadecyne-1,15-diyl group, 5-methyl-10-pentadecyne-1,15-diyl group, 4,7-diethyl-11-pentadecyne-1,15-diyl group, 5-methyl-12-pentadecyne-1,15-diyl group, 8-ethyl-13-pentadecyne-1,15-diyl group;

8-i-propyl-2-hexadecyne-1,16-diyl group, 6-methyl-3-hexadecyne-1,16-diyl group, 8-ethyl-4-hexadecyne-1,16-diyl group, 9-methyl-5-hexadecyne-1,16-diyl group, 10-ethyl-6-hexadecyne-1,16-diyl group, 5-methyl-7-hexadecyne-1,16-diyl group, 5,11-dimethyl-8-hexadecyne-1,16-diyl group, 5-ethyl-9-hexadecyne-1,16-diyl group, 7,13-diethyl-10-hexadecyne-1,16-diyl group, 5-ethyl-7-methyl-11-hexadecyne-1,16-diyl group, 5-methyl-12-hexadecyne-1,16-diyl group, 8-s-butyl-13-hexadecyne-1,16-diyl group, 5-ethyl-14-hexadecyne-1,16-diyl group;

11-methyl-2-heptadecyne-1,17-diyl group, 9-ethyl-3-heptadecyne-1,17-diyl group, 7-i-propyl-4-heptadecyne-1,17-diyl group, 8-methyl-5-heptadecyne-1,17-diyl group, 4-ethyl-6-heptadecyne-1,17-diyl group, 10-methyl-7-heptadecyne-1,17-diyl group, 5,11-dimethyl-8-heptadecyne-1,17-diyl group, 5-ethyl-9-heptadecyne-1,17-diyl group, 8-ethyl-10-heptadecyne-1,17-diyl group, 7-methyl-11-heptadecyne-1,17-diyl group, 5-i-propyl-12-heptadecyne-1,17-diyl group, 9-ethyl-13-heptadecyne-1,17-diyl group, 8-methyl-14-heptadecyne-1,17-diyl group, 7-s-butyl-15-heptadecyne-1,17-diyl group;

10,15-dimethyl-2-octadecyne-1,18-diyl group, 6-ethyl-3-octadecyne-1,18-diyl group, 10-methyl-4-octadecyne-1,18-diyl group, 11-methyl-5-octadecyne-1,18-diyl group, 12-ethyl-6-octadecyne-1,18-diyl group, 10-methyl-7-octadecyne-1,18-diyl group, 5-methyl-8-octadecyne-1,18-diyl group, 7-ethyl-9-octadecyne-1,18-diyl group, 7-methyl-10-octadecyne-1,18-diyl group, 8-n-butyl-11-octadecyne-1,18-diyl group, 7-methyl-12-octadecyne-1,18-diyl group, 9-ethyl-13-octadecyne-1,18-diyl group, 10-i-propyl-14-octadecyne-1,18-diyl group, 7-methyl-15-octadecyne-1,18-diyl group, 10-ethyl-16-octadecyne-1,18-diyl group;

10-methyl-2-nonadecyne-1,19-diyl group, 10,12-diethyl-3-nonadecyne-1,19-diyl group, 7-methyl-4-nonadecyne-1,19-diyl group, 9-ethyl-5-nonadecyne-1,19-diyl group, 9-n-propyl-6-nonadecyne-1,19-diyl group, 10-methyl-7-nonadecyne-1,19-diyl group, 12-i-propyl-8-nonadecyne-1,19-diyl group, 5,15-dimethyl-9-nonadecyne-1,19-diyl group, 7-ethyl-13-methyl-10-nonadecyne-1,19-diyl group, 6-methyl-11-nonadecyne-1,19-diyl group, 6-ethyl-12-nonadecyne-1,19-diyl group, 7,16-diethyl-13-nonadecyne-1,19-diyl group, 9-s-butyl-14-nonadecyne-1,19-diyl group, 8-methyl-15-nonadecyne-1,19-diyl group, 10-ethyl-16-nonadecyne-1,19-diyl group, 10-i-propyl-17-nonadecyne-1,19-diyl group;

8-methyl-2-icosyne-1,20-diyl group, 6-ethyl-3-icosyne-1,20-diyl group, 10-i-propyl-4-icosyne-1,20-diyl group, 11-n-propyl-5-icosyne-1,20-diyl group, 12-methyl-6-icosyne-1,20-diyl group, 11-ethyl-7-icosyne-1,20-diyl group, 13-n-propyl-8-icosyne-1,20-diyl group, 6-i-propyl-9-icosyne-1,20-diyl group, 5-n-propyl-10-icosyne-1,20-diyl group, 7-methyl-11-icosyne-1,20-diyl group, 8-ethyl-12-icosyne-1,20-diyl group, 10-n-propyl-13-icosyne-1,20-diyl group, 9-i-propyl-14-icosyne-1,20-diyl group, 10-n-butyl-15-icosyne-1,20-diyl group, 8-s-butyl-16-icosyne-1,20-diyl group, 7-i-butyl-17-icosyne-1,20-diyl group, 9-methyl-18-icosyne-1,20-diyl group;

11-methyl-2-henicosyne-1,21-diyl group, 12-n-butyl-3-henicosyne-1,21-diyl group, 10-n-pentyl-4-henicosyne-1,21-diyl group, 8-ethyl-5-henicosyne-1,21-diyl group, 10-i-propyl-6-henicosyne-1,21-diyl group, 5-n-propyl-7-henicosyne-1,21-diyl group, 13-n-butyl-8-henicosyne-1,21-diyl group, 15-s-butyl-9-henicosyne-1,21-diyl group, 5-methyl-10-henicosyne-1,21-diyl group, 15-ethyl-6-methyl-11-henicosyne-1,21-diyl group, 8-ethyl-12-henicosyne-1,21-diyl group, 7-methyl-13-henicosyne-1,21-diyl group, 11-ethyl-14-henicosyne-1,21-diyl group, 6-ethyl-15-henicosyne-1,21-diyl group, 9-methyl-16-henicosyne-1,21-diyl group, 5-ethyl-9-methyl-17-henicosyne-1,21-diyl group, 10,10-dimethyl-18-henicosyne-1,21-diyl group, 9-ethyl-19-henicosyne-1,21-diyl group;

11-methyl-2-docosyne-1,22-diyl group, 12-ethyl-3-docosyne-1,22-diyl group, 13-i-propyl-4-docosyne-1,22-diyl group, 10-n-propyl-5-docosyne-1,22-diyl group, 10-n-butyl-6-docosyne-1,22-diyl group, 15-s-butyl-7-docosyne-1,22-diyl group, 11-i-butyl-8-docosyne-1,22-diyl group, 5,15-dimethyl-9-docosyne-1,22-diyl group, 8,14-diethyl-10-docosyne-1,22-diyl group, 5-methyl-11-docosyne-1,22-diyl group, 7-ethyl-12-docosyne-1,22-diyl group, 10-methyl-13-docosyne-1,22-diyl group, 10-ethyl-14-docosyne-1,22-diyl group, 9-ethyl-15-docosyne-1,22-diyl group, 8-methyl-16-docosyne-1,22-diyl group, 7-i-propyl-17-docosyne-1,22-diyl group, 10-i-butyl-18-docosyne-1,22-diyl group, 9,10-dimethyl-19-docosyne-1,22-diyl group, 13-ethyl-20-docosyne-1,22-diyl group;

19-methyl-2-tricosyne-1,23-diyl group, 10,15-dimethyl-3-tricosyne-1,23-diyl group, 3,11,16-trimethyl-4-tricosyne-1,23-diyl group, 12-ethyl-5-tricosyne-1,23-diyl group, 6,13-diethyl-6-tricosyne-1,23-diyl group, 4,12,18-triethyl-7-tricosyne-1,23-diyl group, 18-i-propyl-8-tricosyne-1,23-diyl group, 14-n-propyl-9-tricosyne-1,23-diyl group, 8-n-butyl-10-tricosyne-1,23-diyl group, 15-s-butyl-11-tricosyne-1,23-diyl group, 5-i-butyl-12-tricosyne-1,23-diyl group, 7-ethyl-9-methyl-13-tricosyne-1,23-diyl group, 9-methyl-14-tricosyne-1,23-diyl group, 4,18-dimethyl-15-tricosyne-1,23-diyl group, 3,4,11-trimethyl-16-tricosyne-1,23-diyl group, 9-ethyl-17-tricosyne-1,23-diyl group, 10,13-diethyl-18-tricosyne-1,23-diyl group, 5,8,15-triethyl-19-tricosyne-1,23-diyl group, 15-i-propyl-20-tricosyne-1,23-diyl group, 17-n-propyl-21-tricosyne-1,23-diyl group;

16-n-butyl-2-tetracosyne-1,24-diyl group, 11-s-butyl-3-tetracosyne-1,24-diyl group, 8-i-butyl-4-tetracosyne-1,24-diyl group, 18-ethyl-9-methyl-5-tetracosyne-1,24-diyl group, 13-methyl-6-tetracosyne-1,24-diyl group, 4,19-dimethyl-7-tetracosyne-1,24-diyl group, 5,11,17-triethyl-8-tetracosyne-1,24-diyl group, 6-ethyl-9-tetracosyne-1,24-diyl group, 7,16-diethyl-10-tetracosyne-1,24-diyl group, 5,9,18-triethyl-11-tetracosyne-1,24-diyl group, 10-n-propyl-12-tetracosyne-1,24-diyl group, 20-i-propyl-13-tetracosyne-1,24-diyl group, 9-n-butyl-14-tetracosyne-1,24-diyl group, 11-s-butyl-15-tetracosyne-1,24-diyl group, 13-i-butyl-16-tetracosyne-1,24-diyl group, 10-ethyl-13-methyl-17-tetracosyne-1,24-diyl group, 6-methyl-18-tetracosyne-1,24-diyl group, 5,7-dimethyl-19-tetracosyne-1,24-diyl group, 4,8,13-trimethyl-20-tetracosyne-1,24-diyl group, 18-ethyl-21-tetracosyne-1,24-diyl group, 6,10-diethyl-22-tetracosyne-1,24-diyl group;

9,13,16-trimethyl-2-pentacosyne-1,25-diyl group, 12-n-propyl-3-pentacosyne-1,25-diyl group, 11-i-propyl-4-pentacosyne-1,25-diyl group, 20-n-butyl-5-pentacosyne-1,25-diyl group, 17-i-butyl-6-pentacosyne-1,25-diyl group, 15-s-butyl-7-pentacosyne-1,25-diyl group, 15-ethyl-23-methyl-8-pentacosyne-1,25-diyl group, 11-methyl-8-pentacosyne-1,25-diyl group, 13,17-dimethyl-9-pentacosyne-1,25-diyl group, 5,8,21-trimethyl-10-pentacosyne-1,25-diyl group, 17-ethyl-11-pentacosyne-1,25-diyl group, 8,18-diethyl-12-pentacosyne-1,25-diyl group, 10,15,18-trimethyl-13-pentacosyne-1,25-diyl group, 4-n-propyl-14-pentacosyne-1,25-diyl group, 20-i-propyl-15-pentacosyne-1,25-diyl group, 8-n-butyl-16-pentacosyne-1,25-diyl group, 11-s-butyl-17-pentacosyne-1,25-diyl group, 5,22-dimethyl-18-pentacosyne-1,25-diyl

group, 5-i-butyl-19-pentacosyne-1,25-diyl group, 9-methyl-13-ethyl-20-pentacosyne-1,25-diyl group, 15-methyl-21-pentacosyne-1,25-diyl group, 6,13-dimethyl-22-pentacosyne-1,25-diyl group, 4,8,12-trimethyl-23-pentacosyne-1,25-diyl group;

13-ethyl-2-hexacosyne-1,26-diyl group, 5,16-diethyl-3-hexacosyne-1,26-diyl group, 7,11,16-trimethyl-4-hexacosyne-1,26-diyl group, 12-n-propyl-5-hexacosyne-1,26-diyl group, 21-i-propyl-6-hexacosyne-1,26-diyl group, 6-n-butyl-7-hexacosyne-1,26-diyl group, 13-s-butyl-8-hexacosyne-1,26-diyl group, 19-i-butyl-9-hexacosyne-1,26-diyl group, 13-ethyl-18-methyl-10-hexacosyne-1,26-diyl group, 10-methyl-11-hexacosyne-1,26-diyl group, 10,20-dimethyl-12-hexacosyne-1,26-diyl group, 7,9,17-trimethyl-13-hexacosyne-1,26-diyl group, 8-ethyl-14-hexacosyne-1,26-diyl group,

5,22-diethyl-15-hexacosyne-1,26-diyl group, 7,10,21-trimethyl-16-hexacosyne-1,26-diyl group, 15-n-propyl-17-hexacosyne-1,26-diyl group, 13-i-propyl-18-hexacosyne-1,26-diyl group, 8-n-butyl-19-hexacosyne-1,26-diyl group, 11-s-butyl-20-hexacosyne-1,26-diyl group, 14-i-butyl-21-hexacosyne-1,26-diyl group, 5-ethyl-21-methyl-22-hexacosyne-1,26-diyl group, 7-methyl-23-hexacosyne-1,26-diyl group, 8,14-dimethyl-24-hexacosyne-1,26-diyl group; 7,16,24-trimethyl-2-heptacosyne-1,27-diyl group, 9-ethyl-3-heptacosyne-1,27-diyl group, 7,16-dimethyl-4-heptacosyne-1,27-diyl group, 9,13,21-trimethyl-5-heptacosyne-1,27-diyl group, 13-n-propyl-6-heptacosyne-1,27-diyl group, 10-i-propyl-7-heptacosyne-1,27-diyl group, 16-n-propyl-8-heptacosyne-1,27-diyl group, 18-methyl-9-heptacosyne-1,27-diyl group, 9-i-propyl-10-heptacosyne-1,27-diyl group, 15-ethyl-7-methyl-11-heptacosyne-1,27-diyl group, 25-methyl-12-heptacosyne-1,27-heptacosyne-1,27-diyl group, 8,21-dimethyl-13-heptacosyne-1,27-diyl group, 5,11,23-trimethyl-14-heptacosyne-1,27-diyl group, 9-ethyl-15-heptacosyne-1,27-diyl group, 8,20-dimethyl-16-heptacosyne-1,27-diyl group, 4,8,19-trimethyl-17-heptacosyne-1,27-diyl group, 7-n-propyl-18-heptacosyne-1,27-diyl group, 21-i-propyl-19-heptacosyne-1,27-diyl group, 14-n-propyl-20-heptacosyne-1,27-diyl group, 8-ethyl-21-heptacosyne-1,27-diyl group, 11-i-propyl-22-heptacosyne-1,27-diyl group, 5-ethyl-13-methyl-23-heptacosyne-1,27-diyl group, 16-methyl-24-heptacosyne-1,27-diyl group, 7-ethyl-25-heptacosyne-1,27-diyl group;

14-ethyl-2-octacosyne-1,28-diyl group, 20-methyl-3-octacosyne-1,28-diyl group, 7,22-dimethyl-4-octacosyne-1,28-diyl group, 19-ethyl-5-octacosyne-1,28-diyl group, 11-methyl-6-octacosyne-1,28-diyl group, 13,16-dimethyl-7-octacosyne-1,28-diyl group, 13-ethyl-8-octacosyne-1,28-diyl group, 6-methyl-9-octacosyne-1,28-diyl group, 9,16-dimethyl-10-octacosyne-1,28-diyl group, 7-ethyl-11-octacosyne-1,28-diyl group, 16-methyl-12-octacosyne-1,28-diyl group, 6,15-dimethyl-13-octacosyne-1,28-diyl group,

22-ethyl-14-octacosyne-1,28-diyl group, 6-methyl-15-octacosyne-1,28-diyl group, 8,11-dimethyl-16-octacosyne-1,28-diyl group, 23-ethyl-17-octacosyne-1,28-diyl group, 4-methyl-18-octacosyne-1,28-diyl group, 7,14-dimethyl-19-octacosyne-1,28-diyl group, 13-ethyl-20-octacosyne-1,28-diyl group, 8-methyl-21-octacosyne-1,28-diyl group, 11,17-dimethyl-22-octacosyne-1,28-diyl group, 10-ethyl-23-octacosyne-1,28-diyl group, 9-methyl-24-octacosyne-1,28-diyl group, 7,19-dimethyl-25-octacosyne-1,28-diyl group, 12-ethyl-26-octacosyne-1,28-diyl group;

15-methyl-2-nonacosyne-1,29-diyl group, 14-methyl-3-nonacosyne-1,29-diyl group, 12-methyl-4-nonacosyne-1,29-diyl group, 13-methyl-5-nonacosyne-1,29-diyl group, 11-methyl-6-nonacosyne-1,29-diyl group, 10-methyl-7-nonacosyne-1,29-diyl group, 25-methyl-8-nonacosyne-1,29-diyl group, 24-methyl-9-nonacosyne-1,29-diyl group, 23-methyl-10-nonacosyne-1,29-diyl group, 22-methyl-11-nonacosyne-1,29-diyl group, 21-methyl-12-nonacosyne-1,29-diyl group, 20-methyl-13-nonacosyne-1,29-diyl group, 19-methyl-14-nonacosyne-1,29-diyl group, 18-methyl-15-nonacosyne-1,29-diyl group, 27-methyl-16-nonacosyne-1,29-diyl group, 26-methyl-17-nonacosyne-1,29-diyl group, 25-methyl-18-nonacosyne-1,29-diyl group, 24-methyl-19-nonacosyne-1,29-diyl group, 23-methyl-20-nonacosyne-1,29-diyl group, 20-methyl-21-nonacosyne-1,29-diyl group, 19-methyl-22-nonacosyne-1,29-diyl group, 18-methyl-23-nonacosyne-1,29-diyl group, 17-methyl-24-nonacosyne-1,29-diyl group, 16-methyl-25-nonacosyne-1,29-diyl group, 6-methyl-26-nonacosyne-1,29-diyl group, and 5-methyl-27-nonacosyne-1,29-diyl group.

Typically, optionally substituted straight-chained alkylene groups having 1 - 30 carbon atoms are preferred as G; optionally substituted straight-chained groups having 2 - 15 carbon atoms are more preferred and straight-chained alkylene groups having 2 - 13 carbon atoms that may optionally be substituted by a hydroxyl group are further preferred; among these, ethane-1,2-diyl group, propane-1,3-diyl group, butane-1,4-diyl group, pentane-1,5-diyl group, hexane-1,6-diyl group, heptane-1,7-diyl group, octane-1,8-diyl group, nonane-1,9-diyl group, decane-1,10-diyl group, undecane-1,11-diyl group, dodecane-1,12-diyl group, tridecane-1,13-diyl group, 2-hydroxypropane-1,3-diyl group, 3-hydroxy-octane-1,8-diyl group, 3-hydroxynonane-1,9-diyl group, 3-hydroxydecane-1,10-diyl group and the like are particularly preferred.

The optionally substituted straight-chained or branched alkylene group having 1 - 30 carbon atoms, the optionally substituted straight-chained or branched alkenylene group having 2 - 30 carbon atoms and the optionally substituted straight-chained or branched alkynylene group having 2 - 30 carbon atoms, all being listed as candidates for G, are such that they bind to A in their 1-position and bind to E in their ω position or bind to E in their 1-position and bind to A in their ω position; preferably, they bind to A in their 1-position and bind to E in their ω position.

E represents a single bond or -O- and preferably represents a single bond.

J represents a single bond, an optionally substituted aromatic hydrocarbon group or an optionally substituted

heterocyclic group, with a single bond and an aromatic hydrocarbon group being preferred, and a single bond being more preferred.

If J is an optionally substituted aromatic hydrocarbon group or an optionally substituted heterocyclic group, exemplary substituents include $-(CH_2)_k-COOR^{7b}$, $-(CH_2)_1-$, $CONR^{8c}R^{9c}$, $-NR^{8d}R^{9d}$, hydroxyl group, etc. Here, k and l represent independently 0 or 1; $R^{7b}$ represents a hydrogen atom or a straight-chained or branched alkyl group having 1 - 6 carbon atoms; $R^{8c}$, $R^{9c}$, $R^{8c}$ and $R^{9d}$ represent each independently a hydrogen atom or a straight-chained or branched alkyl group having 1 - 3 carbon atoms. Except in the case where Q is a single bond, these substituents are preferably absent and in the case where Q is a single bond, a preferred substituent is $-(CH_2)_k-COOR^{7b}$ (where k and $R^{7b}$ have the same meanings as defined above). In the case where J is substituted, the number of substituents is from one to four, preferably one.

If J is an optionally substituted aromatic hydrocarbon group, the definition of the aromatic hydrocarbon group is the same as given for the aromatic hydrocarbon group in the case where it is used as Ar; preferred examples include p-phenylene group and m-phenylene group.

If J is an optionally substituted aromatic hydrocarbon group, preferred examples are unsubstituted p-phenylene group, unsubstituted m-phenylene group and -COOH substituted phenylene group.

If J is an optionally substituted heterocyclic group, the heterocyclic group means a 4-membered to 10-membered monocyclic or fused aliphatic or aromatic ring containing 1 - 4 hetero atoms which may be the same or different and are exemplified by an oxygen atom, a nitrogen atom and a sulfur atom; specific examples include oxetane, furan, dihydrofuran, tetrahydrofuran, pyran, dihydropyran, tetrahydropyran, dioxole, thiophene, dihydrothiophene, tetrahydrothiophene, thiopyran, dihydrothiopyran, tetrahydrothiopyran, pyrrole, dihydropyrrole, pyrrolidine, pyridine, dihydropyridine, tetrahydropyridine, piperidine, pyrazole, 2-pyrazoline, pyrazolidine, imidazole, imidazolidine, pyrimidine, pyrazine, pyridazine, oxazoline, piperazine, 1,2,3-triazole, 1,2,4-triazole, tetrazole, isoxazole, 1,3-oxazole, 1,2,3-oxadiazole, 1,2,4-oxadiazole, 1,2,5-oxadiazole, 1,3,4-oxadiazole, 1,2-thiazole, 1,3-thiazole, 1,2,3-thiadiazole, 1,2,4-thiadiazole, 1,2,5-thiadiazole, 1,3,4-thiadiazole, 1,3-dioxolan, 1,4-dioxane, oxazolidine, morpholine, indole, quinoline, isoquinoline, benzopyran, benzofuran, benzothiphene, benzodiazole, benzoxazole and benzothiazole. Preferred examples include furan and oxazole. The heterocyclic group as J means a group having one bond each in two different positions in these hetero rings other than the positions having substituents; preferred examples include furan-2,5-diyl group, 1,3-oxazole-2,4-diyl group and 1,3-oxazole-2,5-diyl group.

Preferred examples of the optionally substituted heterocyclic group as J include unsubstituted furan-2,5-diyl group, unsubstituted 1,3-oxazole-2,4-diyl group and unsubstituted 1,3-oxazole-2,5-diyl group.

If J is an optionally substituted aromatic hydrocarbon group or an optionally substituted heterocyclic group, it may be bound to E via any of the two bonds as long as it is bound to E via one bond and bound to Y via the other; preferably, J is bound to E in 4-position if it is 1,3-oxazole-2,4-diyl group and bound to E in 5-position if it is 1,3-oxazole-2,5-diyl group.

Y represents a single bond or -O- and preferably represents a single bond.

L represents a single bond, a straight-chained or branched alkylene group having 1 - 10 carbon atoms, a straight-chained or branched alkenylene group having 2 - 10 carbon atoms or a straight-chained or branched alkynylene group having 2 - 10 carbon atoms and a single bond is preferred; if J is an optionally substituted aromatic hydrocarbon group and Y is a single bond, L is preferably a single bond or a straight-chained alkylene group having 1 - 5 carbon atoms, among which a single bond and a straight-chained alkylene group having 1 - 3 carbon atoms are preferred, with a single bond and propane-1,3-dily group being particularly preferred; if J is an optionally substituted aromatic hydrocarbon group and Y is -O-, L is preferably one of straight-chained alkylene groups having 1 - 5 carbon atoms, among which straight-chained alkylene groups having 2 - 4 carbon atoms are preferred.

L represents a straight-chained or branched alkylene group having 1 - 10 carbon atoms, a straight-chained or branched alkenylene group having 2 - 10 carbon atoms, a straight-chained or branched alkynylene group having 2 - 10 carbon atoms, a straight-chained alkylene group having 1 - 5 carbon atoms, a straight-chained alkylene group having 1 - 3 carbon atoms or a straight-chained alkylene group having 2 - 4 carbon atoms; specific examples of these groups can appropriately be selected from the list of specific examples of G which represents an optionally substituted straight-chained or branched alkylene group having 1 - 30 carbon atoms, an optionally substituted straight-chained or branched alkenylene group having 2 - 30 carbon atoms or an optionally substituted straight-chained or branched alkynylene group having 2 - 30 carbon atoms, except that methylene group is added to the list.

Any of the two bonds of L may be bound to Y as long as it satisfies the condition that it is bound to Y via one bond and bound to Q via the other.

Q represents a single bond or one group selected from among the following formulae:

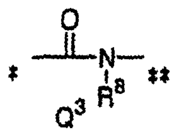
$Q^3$
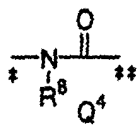
$Q^4$
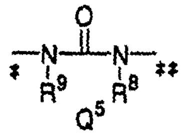
$Q^5$

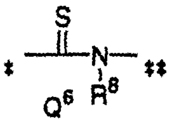
$Q^6$
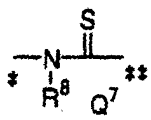
$Q^7$
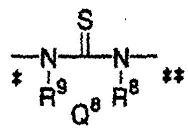
$Q^8$

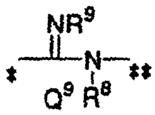
$Q^9$
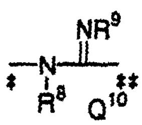
$Q^{10}$
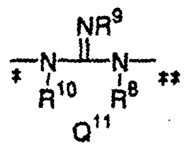
$Q^{11}$

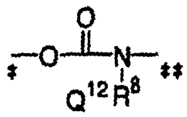
$Q^{12}$
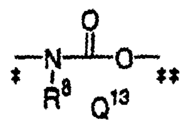
$Q^{13}$

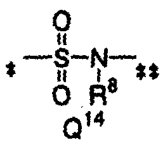
$Q^{14}$
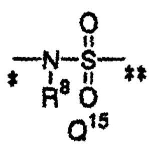
$Q^{15}$
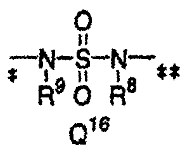
$Q^{16}$

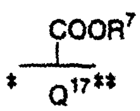
$Q^{17}$
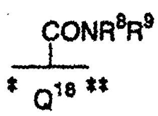
$Q^{18}$
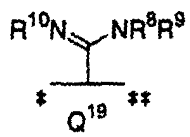
$Q^{19}$

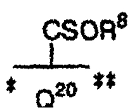
$Q^{20}$
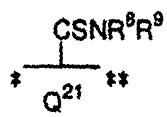
$Q^{21}$

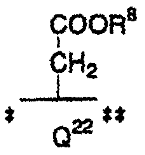
$Q^{22}$
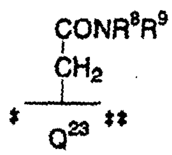
$Q^{23}$
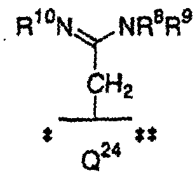
$Q^{24}$

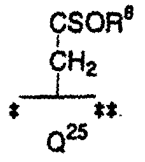
$Q^{25}$
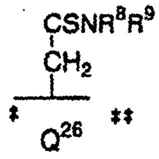
$Q^{26}$

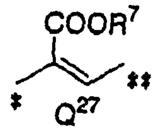
Q27

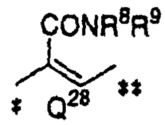
Q28

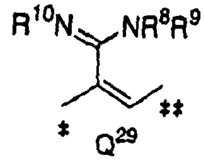
Q29

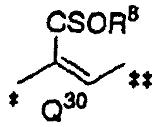
Q30

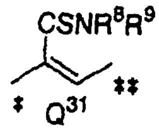
Q31

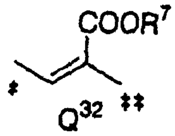
Q32

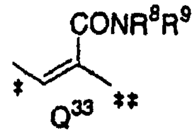
Q33

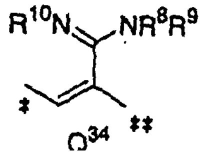
Q34

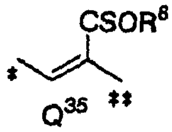
Q35

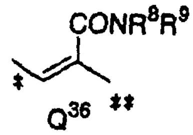
Q36

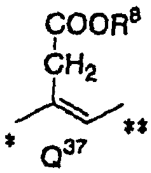
Q37

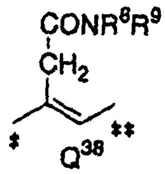
Q38

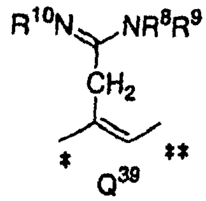
Q39

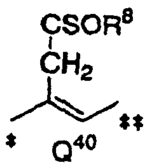
Q40

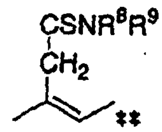
Q41

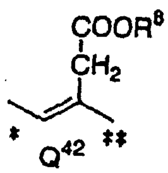
Q42

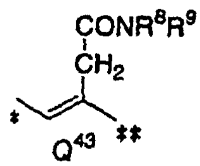
Q43

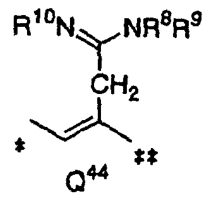
Q44

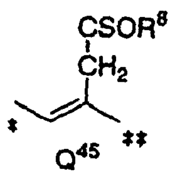
Q45

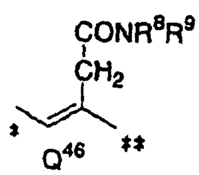
Q46

and

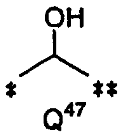
Q47
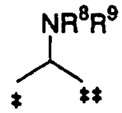
Q48
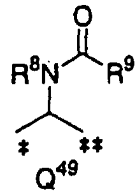
Q49
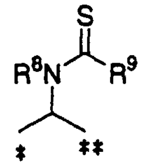
Q50
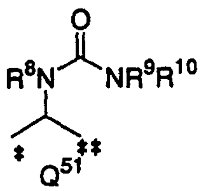
Q51
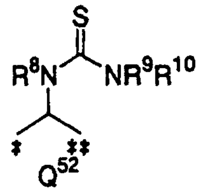
Q52
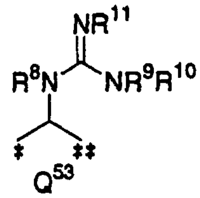
Q53
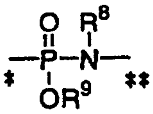
Q54
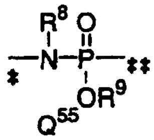
Q55
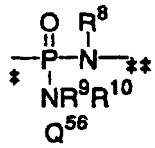
Q56
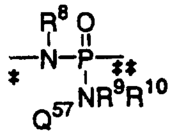
Q57
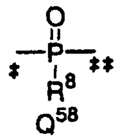
Q58
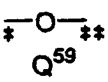
Q59
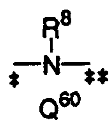
Q60
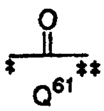
Q61
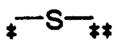
Q62
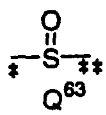
Q63
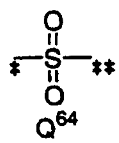
Q64
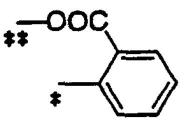
Q65
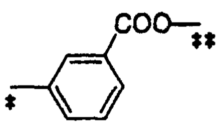
Q66
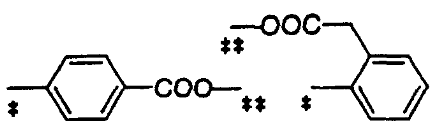
Q67 Q68
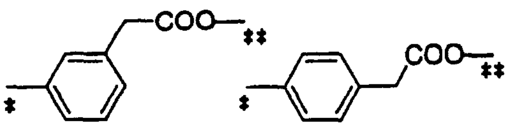
Q69 Q70
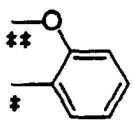
Q71
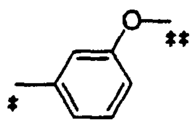
Q72
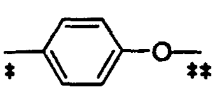
Q73
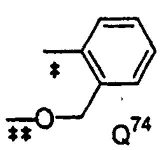
Q74
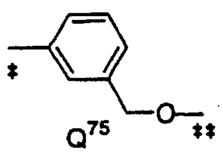
Q75
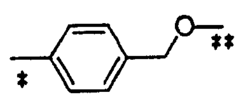
Q76
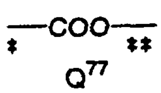
Q77
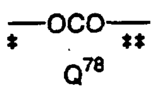
Q78

(where $R^7$ represents a hydrogen atom or a straight-chained or branched lower alkyl group having 1 - 6 carbon atoms,

and $R^8$ $R^9$, $R^{10}$ and $R^{11}$ represent each independently a hydrogen atom or a straight-chained or branched lower alkyl group having 1 - 3 carbon atoms); Q is preferably $Q^2$ (where examples of $Q^2$ include a single bond, $Q^{62}$, $Q^{63}$, $Q^{64}$, $Q^3$ (where $R^8$ has the same meaning as defined above), $Q^4$ (where $R^8$ has the same meaning as defined above), $Q^{17}$ (where $R^7$ has the same meaning as defined above), $Q^{32}$ (where $R^7$ has the same meaning as defined above) and $Q^{27}$ (where $R^7$ has the same meaning as defined above)}; considering the strength of antiandrogenic activity, cases where Q is $Q^{62}$, $Q^{63}$, $Q^{64}$ and $Q^3$, as well as $Q^4$ where $R^8$ is a hydrogen atom are more preferred, with $Q^{62}$, $Q^{63}$, $Q^{64}$ and $Q^3$ being particularly preferred. If Q is $Q^3$, the nitrogen atom and $R^8$
in $Q^3$ may preferably combine with Z to form a heterocyclic group. Considering peroral absorption, Q is more preferably $Q^{17}$ where $R^7$ is a hydrogen atom, or $Q^{32}$ where $R^7$ is a hydrogen atom, or $Q^{27}$ where $R^7$ is a hydrogen atom. If Z is -COOH, Q may preferably be a single bond considering peroral absorption.

Further referring to Q, it is bound to L in the position marked with * and bound to Z in the position marked with **.

Z represents a hydrogen atom, an optionally substituted
straight-chained or branched alkyl group having 1 - 10 carbon atoms, a straight-chained or branched alkenyl group having 2 - 10 carbon atoms that may optionally be substituted by a cycloalkyl group having 3 - 6 carbon atoms or a halogen atom, a straight-chained or branched alkynyl group having 2 - 10 carbon atoms that may optionally be substituted by a halogen atom, -O-$R^d$ (where $R^d$ represents a hydrogen atom or a protective group of a hydroxyl group), or -COOH.

If Z is an optionally substituted straight-chained or branched alkyl group having 1 - 10 carbon atoms, exemplary substituents include a halogen atom, a cycloalkyl group, a phenyl group optionally substituted by a straight-chained or branched alkyl group, a heterocyclic group, and a hydroxyl group. Said heterocyclic group may be exemplified by a furyl group. Examples of said halogen atom include a fluorine atom, a chlorine atom, a bromine atom and an iodine atom, with a fluorine atom being preferred. If said optionally substituted straight-chained or branched alkyl group having 1 - 10 carbon atoms is substituted by a halogen atom, the number of substituent halogen atoms ranges from one to ten, preferably from three to nine, and substitution by five halogen atoms is particularly preferred. In a preferred mode of substitution, all hydrogen atoms on a certain carbon atom are substituted by halogen atoms (as in the cases of trihalomethyl group, 1,1,3,3,3-pentahalopropyl group, etc.)

If Z is a straight-chained or branched alkenyl group having 2 - 10 carbon atoms that may optionally be substituted by a halogen atom or a straight-chained or branched alkynyl group having 2 - 10 carbon atoms that may optionally be substituted by a halogen atom, exemplary halogen atoms include a fluorine atom, a chlorine atom, a bromine atom and an iodine atom, with a fluorine atom being preferred. The number of substituent halogen atoms ranges from one to ten, preferably from three to nine, and substitution by five halogen atoms is particularly preferred. In a preferred mode of substitution, all hydrogen atoms on a certain carbon atom are substituted by halogen atoms.

If Z is an optionally substituted straight-chained or branched alkyl group having 1 - 10 carbon atoms, exemplary straight-chained or branched alkyl groups having 1 - 10 carbon atoms include straight-chained alkyl groups, i.e., methyl group, ethyl group, n-propyl group, n-butyl group, n-pentyl group, n-hexyl group, n-heptyl group, n-octyl group, n-nonyl group and n-decyl group, as well as branched alkyl groups such as 1-methylethyl group, 1-methylpropyl group, 2-methylpropyl group, 1-methylbutyl group, 2-methylbutyl group, 3-methylbutyl group, 1,1-dimethylpropyl group, 1,2-dimethylpropyl group, 2,2-dimethylpropyl group, 1-ethylpropyl group, 1-methylpentyl group, 2-methylpentyl group, 3-methylpentyl group, 4-methylpentyl group, 1,1-dimethylbutyl group, 1,2-dimethylbutyl group, 1,3-dimethylbutyl group, 2,2-dimethylbutyl group, 2,3-dimethylbutyl group, 3,3-dimethylbutyl group, 1-ethylbutyl group, 2-ethylbutyl group, 1-methylhexyl group, 2-methylhexyl group, 3-methylhexyl group, 4-methylhexyl group, 5-methylhexyl group, 1-ethylpentyl group, 2-ethylpentyl group, 3-ethylpentyl group, 1,1-dimethylpentyl group, 1,2-dimethylpentyl group, 1,3-dimethylpentyl group, 1,4-dimethylpentyl group, 2,2-dimethylpentyl group, 2,3-dimethylpentyl group, 2,4-dimethylpentyl group, 3,3-dimethylpentyl group, 3,4-dimethylpentyl group, 3,3-dimethylpentyl group, 3,4-dimethylpentyl group, 4,4-dimethylpentyl group, 1-propylbutyl group, 1-ethyl-1-methylbutyl group, 1-ethyl-2-methylbutyl group, 1-ethyl-3-methylbutyl group, 2-ethyl-1-methylbutyl group, 2-ethyl-2-methylbutyl group, 2-ethyl-3-methylbutyl group, 1,1,2-trimethylbutyl group, 1,1,3-trimethylbutyl group, 1,2,2-trimethylbutyl group, 1,2,3-trimethylbutyl group, 1,3,3-trimethylbutyl group, 2,2,3-trimethylbutyl group, 2,3,3-trimethylbutyl group, 1-methylheptyl group, 2-methylheptyl group, 3-methylheptyl group, 4-methylheptyl group, 5-methylheptyl group, 6-methylheptyl group, 1-ethylhexyl group, 2-ethylhexyl group, 3-ethylhexyl group, 4-ethylhexyl group, 1,1-dimethylhexyl group, 1,2-dimethylhexyl group, 1,3-dimethylhexyl group, 1,4-dimethylhexyl group, 1,5-dimethylhexyl group, 2,2-dimethylhexyl group, 2,3-dimethylhexyl group, 2,4-dimethylhexyl group, 2,5-dimethylhexyl group, 3,3-dimethylhexyl group, 3,4-dimethylhexyl group, 3,5-dimethylhexyl group, 4,4-dimethylhexyl group, 4,5-dimethylhexyl group, 5,5-dimethylhexyl group;
1-propylpentyl group, 2-propylpentyl group, 1-ethyl-1-methylpentyl group, 1-ethyl-2-methylpentyl group, 1-ethyl-3-methylpentyl group, 1-ethyl-4-methylpentyl group, 2-ethyl-1-methylpentyl group, 2-ethyl-2-methylpentyl group, 2-ethyl-3-methylpentyl group, 2-ethyl-4-methylpentyl group, 3-ethyl- 1-methylpentyl group, 3-ethyl-2-methylpentyl group, 3-ethyl-3-methylpentyl group, 3-ethyl-4-methylpentyl group, 1,1,2-trimethylpentyl group, 1,1,3-trimethylpentyl group, 1,1,4-trimethylpentyl group, 1,2,2-trimethylpentyl group, 1,2,3-trimethylpentyl group, 1,2,4-trimethylpentyl

group, 1,3,3-trimethylpentyl group, 1,3,4-trimethylpentyl group, 1,4,4-trimethylpentyl group, 2,2,3-trimethylpentyl group, 2,2,4-trimethylpentyl group, 2,3,3-trimethylpentyl group, 2,3,4-trimethylpentyl group, 2,4,4-trimethylpentyl group, 3,3,4-trimethylpentyl group, 3,4,4-trimethylpentyl group, 1-methyl-1-propylbutyl group, 2-methyl-1-propylbutyl group, 3-methyl-1-propylbutyl group, 1,1-diethylbutyl group, 1,2-diethylbutyl group, 2,2-diethylbutyl group, 1,2-dimethyl-1-ethylbutyl group, 1,3-dimethyl-1-ethylbutyl group, 2,2-dimethyl-1-ethylbutyl group, 2,3-dimethyl-1-ethylbutyl group, 3,3-dimethyl-1-ethylbutyl group, 1,1-dimethyl-2-ethylbutyl group, 1,2-dimethyl-2-ethylbutyl group, 1,3-dimethyl-2-ethylbutyl group, 2,3-dimethyl-2-ethylbutyl group, 3,3-dimethyl-2-ethylbutyl group, 1,1-diethyl-2-methylpropyl group, 1-methyloctyl group, 2-methyloctyl group, 3-methyloctyl group,
4-methyloctyl group, 5-methyloctyl group, 6-methyloctyl group, 7-methyloctyl group, 1-ethylheptyl group, 2-ethylheptyl group,
3-ethylheptyl group, 4-ethylheptyl group, 5-ethylheptyl group, 1,1-dimethylheptyl group, 1,2-dimethylheptyl group, 1,3-dimethylheptyl group, 1,4-dimethylheptyl group, 1,5-dimethylheptyl group, 1,6-dimethylheptyl group, 2,2-dimethylheptyl group, 2,3-dimethylheptyl group, 2,4-dimethylheptyl group, 2,5-dimethylheptyl group, 2,6-dimethylheptyl group, 3,3-dimethylheptyl group, 3,4-dimethylheptyl group, 3,5-dimethylheptyl group, 3,6-dimethylheptyl group, 4,4-dimethylheptyl group, 4,5-dimethylheptyl group, 4,6-dimethylheptyl group, 5,5-dimethylheptyl group, 5,6-dimethylheptyl group, 6,6-dimethylheptyl group;
1-propylhexyl group, 2-propylhexyl group, 3-propylhexyl group, 1-ethyl-1-methylhexyl group, 1-ethyl-2-methylhexyl group, 1-ethyl-3-methylhexyl group, 1-ethyl-4-methylhexyl group, 1-ethyl-5-methylhexyl group, 2-ethyl-1-methylhexyl group, 2-ethyl-2-methylhexyl group, 2-ethyl-3-methylhexyl group, 2-ethyl-4-methylhexyl group, 2-ethyl-5-methylhexyl group, 3-ethyl-1-methylhexyl group, 3-ethyl-2-methylhexyl group, 3-ethyl-3-methylhexyl group, 3-ethyl-4-methylhexyl group, 3-ethyl-5-methylhexyl group, 4-ethyl-1-methylhexyl group, 4-ethyl-2-methylhexyl group, 4-ethyl-3-methylhexyl group, 4-ethyl-4-methylhexyl group, 4-ethyl-5-methylhexyl group, 1,1,2-trimethylhexyl group, 1,1,3-trimethylhexyl group, 1,1,4-trimethylhexyl group, 1,1,5-trimethylhexyl group, 1,2,2-trimethylhexyl group, 1,2,3-trimethylhexyl group, 1,2,4-trimethylhexyl group, 1,2,5-trimethylhexyl group, 1,3,3-trimethylhexyl group, 1,3,4-trimethylhexyl group, 1,3,5-trimethylhexyl group, 1,4,4-trimethylhexyl group, 1,4,5-trimethylhexyl group, 1,5,5-trimethylhexyl group, 2,2,3-trimethylhexyl group, 2,2,4-trimethylhexyl group, 2,2,5-trimethylhexyl group, 2,3,3-trimethylhexyl group, 2,3,4-trimethylhexyl group, 2,3,5-trimethylhexyl group, 2,4,4-trimethylhexyl group, 2,4,5-trimethylhexyl group, 2,5,5-trimethylhexyl group, 3,3,4-trimethylhexyl group, 3,3,5-trimethylhexyl group, 3,4,4-trimethylhexyl group, 3,4,5-trimethylhexyl group, 3,5,5-trimethylhexyl group, 4,4,5-trimethylhexyl group, 4,5,5-trimethylhexyl group, 1-methyl-nonyl group, 2-methyl-nonyl group, 3-methyl-nonyl group, 4-methyl-nonyl group, 5-methyl-nonyl group, 6-methyl-nonyl group, 7-methyl-nonyl group, 8-methyl-nonyl group, and 9-methyl-nonyl group; straight-chained alkyl groups having 1 - 10 carbon atoms are preferred, among which methyl group, ethyl group, n-propyl group, i-propyl group, n-butyl group and n-pentyl group are particularly preferred.

If Z is a straight-chained or branched alkenyl group having 2 - 10 carbon atoms that may optionally be substituted by a halogen atom, exemplary straight-chained or branched alkenyl groups having 2 - 10 carbon atoms include straight-chained alkenyl groups such as vinyl group, 1-propenyl group, 2-propenyl group, 1-butenyl group, 2-butenyl group, 3-butenyl group, 1,3-butadienyl group, 2-pentenyl group, 3-pentenyl group, 2,4-pentadienyl group, 2-hexenyl group, 3-hexenyl group, 4-hexenyl group, 2,4-hexadienyl group, 2-heptenyl group, 3-heptenyl group, 4-heptenyl group, 5-heptenyl group, 2,4-heptadienyl group, 2,5-heptadienyl group, 3,5-heptadienyl group, 2-octenyl group, 3-octenyl group, 4-octenyl group, 5-octenyl group, 6-octenyl group, 2,4-octadienyl group, 2,5-octadienyl group, 2,6-octadienyl group, 2,4,6-octatrienyl group, 2-nonenyl group, 3-nonenyl group, 4-nonenyl group, 5-nonenyl group, 6-nonenyl group, 7-nonenyl group, 2-decenyl group, 3-decenyl group, 4-decenyl group, 5-decenyl group, 6-decenyl group, 7-decenyl group, 8-decenyl group;
as well as branched alkenyl groups such as 1-methylethenyl group, 2-methyl-1-propenyl group, 2-methyl-2-propenyl group, 2-methyl-1-butenyl group, 3-methyl-2-butenyl group, 2-methyl-3-butenyl group, 2,3-dimethyl-1,3-butadienyl group, 3-ethyl-2-propenyl group, 4-methyl-3-propenyl group, 3-methyl-2,4-propadienyl group, 3,4-diethyl-2-hexenyl group, 4-methyl-3-hexenyl group, 2-methyl-4-hexenyl group, 3,5-dimethyl-2,4-hexadienyl group, 5-ethyl-3-methyl-2-heptenyl group, 5-methyl-3-heptenyl group, 4-n-propyl-4-heptenyl group, 3,6-dimethyl-5-heptenyl group, 5-ethyl-2,4-heptadienyl group, 2,6-dimethyl-2,5-heptadienyl group, 4-ethyl-3,5-heptadienyl group, 4,6-dimethyl-2-octenyl group, 5-ethyl-3-octenyl group, 3-ethyl-4-octenyl group, 3-ethyl-5-octenyl group, 3,4-dimethyl-6-octenyl group, 5-ethyl-2,4-octadienyl group, 3-methyl-2,5-octadienyl group, 5-ethyl-2,6-octadienyl group, 4-methyl-2,4,6-octatrienyl group, 5-methyl-2-nonenyl group, 6-methyl-3-nonenyl group, 7-methyl-4-nonenyl group, 3-methyl-5-nonenyl group, 4-methyl-6-nonenyl group, 3-methyl-7-nonenyl group, etc.

If Z is a straight-chained or branched alkynyl group having 2 - 10 carbon atoms that may optionally be substituted by a halogen atom, exemplary straight-chained or branched alkynyl groups having 2 - 10 carbon atoms include straight-chained alkynyl groups such as ethynyl group, 1-propynyl group, 2-propynyl group, 1-butynyl group, 2-butynyl group, 3-butynyl group, 1,3-butadiynyl group, 2-pentynyl group, 3-pentynyl group, 2,4-pentadiynyl group, 2-hexynyl group, 3-hexynyl group, 4-hexynyl group, 2,4-hexadiynyl group, 2-heptynyl group, 3-heptynyl group, 4-heptynyl group,

5-heptynyl group, 2,4-heptadiynyl group, 2,5-heptadiynyl group, 3,5-heptadiynyl group, 2-octynyl group, 3-octynyl group, 4-octynyl group, 5-octynyl group, 6-octynyl group, 2,4-octadiynyl group, 2,5-octadiynyl group, 2,6-octadiynyl group, 2,4,6-octatriynyl group, 2-nonynyl group, 3-nonynyl group, 4-nonynyl group, 5-nonynyl group, 6-nonynyl group, 7-nonynyl group, 2-decynyl group, 3-decynyl group, 4-decynyl group, 5-decynyl group, 6-decynyl group, 7-decynyl group, 8-decynyl group;

as well as branched alkynyl groups such as 1-methyl-2-propynyl group, 3-methyl-1-butynyl group, 2-methyl-3-butynyl group, 4-methyl-2-pentynyl group, 2-methyl-3-pentynyl group, 4-ethyl-2-hexynyl group, 5-methyl-3-hexynyl group, 2-methyl-4-hexynyl group, 5-ethyl-6-methyl-2-heptynyl group, 5-methyl-3-heptynyl group, 3-n-propyl-4-heptynyl group, 4,4-dimethyl-5-heptynyl group, 6-methyl-2,4-heptadiynyl group, 4-methyl-2,5-heptadiynyl group, 2-methyl-3,5-heptadiynyl group, 6,6-dimethyl-2-octynyl group, 6-methyl-3-octynyl group, 3-ethyl-4-octynyl group, 4-methyl-5-octynyl group, 4,8-dimethyl-6-octynyl group, 7-methyl-2,4-octadiynyl group, 4-methyl-2,5-octadiynyl group, 5-ethyl-2,6-octadiynyl group, 5-methyl-2-nonynyl group, 6-methyl-3-nonynyl group, 7-methyl-4-nonynyl group, 8-methyl-5-nonynyl group, 4-methyl-6-nonynyl group, 3-methyl-7-nonynyl group, etc.

If Z is -O-$R^d$, $R^d$ is a hydrogen atom or a protective group for a hydroxyl group, and a hydrogen atom is preferred. The protective group for a hydroxyl group may be exemplified by the same protective groups for a hydroxyl group that were already mentioned for $R^a$, and preferred examples as well as particularly preferred examples are also the same as those mentioned for $R^a$.

Typically, preferred examples of Z are a straight-chained or branched alkyl group having 1 - 10 carbon atoms that may optionally be substituted by a halogen atom, a hydroxyl group, and a straight-chained or branched alkyl group having 1 - 10 carbon atoms that is substituted by any one group selected from the group consisting of a cycloalkyl group having 3 - 6 carbon atoms, a hydroxyl group, a heterocyclic group and an optionally substituted phenyl group; straight-chained or branched alkyl groups having 3 - 10 carbon atoms that are substituted by a halogen atom are preferred and among these, straight-chained or branched alkyl groups having 3 - 8 carbon atoms that are substituted by a fluorine atom are particularly preferred, with 4,4,5,5,5-pentafluoropentyl group being most preferred. Considering peroral absorption, Z is preferably -COOH. Further considering peroral absorption, Z may preferably be a hydrogen atom if Q is $Q^{17}$ where R7 is a hydrogen atom.

If Q is $Q^{65}$, $Q^{66}$, $Q^{67}$, $Q^{68}$, $Q^{69}$ or $Q^{70}$, Z is preferably a hydrogen atom or an unsubstituted straight-chained or branched alkyl group having 1 - 3 carbon atoms and, among these, a hydrogen atom is particularly preferred. If Q is $Q^{71}$, $Q^{72}$, $Q^{73}$, $Q^{74}$, $Q^{75}$ or $Q^{76}$, Z is preferably a hydrogen atom.

Compounds represented by the general formula (I) in which Z is -O-$R^d$ (where $R^d$ has the same meaning as defined above) or -COOH are also useful as intermediates for compounds represented by the general formula (I) in which Z is neither -O-$R^d$ (where $R^d$ has the same meaning as defined above) nor -COOH.

If Q is $Q^3$, the nitrogen atom and $R^8$ in $Q^3$ may combine with Z to form a heterocyclic group, as exemplified by morpholino group, pyrrolidinyl group, piperidino group, etc.

Referring further to the general formula (I), it is preferred that the dashed line in 4(5)-position signifies a single bond in combination with the solid line and $X^2$ signifies any one group selected from the group consisting of $-(CH_2)_pCO-NR^8Z^1$ (p represents an integer of at least 1, $R^8$ represents a straight-chained or branched lower alkyl group having 1 - 6 carbon atoms, and $Z^1$ represents a hydrogen atom or a straight-chained or branched alkyl group having 1 - 10 carbon atoms that may optionally be substituted by a halogen atom), $-(CH_2)_p-SO_2-Z^1$ (p and $Z^1$ have the same meanings as defined above), $-(CH_2)_p-SO-Z^1$ (p and $Z^1$ have the same meanings as defined above), $-Ph-O-(CH_2)_p-CO-NR^8Z^1$ (Ph represents a phenylene group and p, $R^8$ and $Z^1$ have the same meanings as defined above), and $-Ph-O-(CH_2)_p-H$ (p has the same meaning as defined above), with p being more referably an integer of 1 - 13;
speaking of the group represented by $-NR^8Z^1$ in $-(CH_2)_p-CO-NR^8Z^1$,
amino group and n-pentylamino group are preferred if p is 5;
if p is 6, dimethylamino group and diethylamino group are preferred;
if p is 7, dimethylamino group, ethylamino group, n-butylamino group, i-propylamino group, cyclohexylamino group, N-n-butyl-N-methylamino group, diethylamino group, methylamino group, N-ethyl-N-methylamino group, N-methyl-N-n-propylamino group, N-methyl-N-i-propylamino group, N-t-butyl-N-methylamino group, n-propylamino group, n-hexylamino group, i-pentylamino group, i-butylamino group, 2,2-dimethylpropylamino group, 1-ethylpropylamino group, di-n-hexylamino group, amino group and n-pentylamino group are preferred, with dimethylamino group, ethylamino group, i-propylamino group, N-n-butyl-N-methylamino group, diethylamino group, methylamino group, N-ethylN-methylamino group, N-methyl-N-n-propylamino group, N-methyl-N-i-propylamino group and n-propylamino group being more preferred, and with dimethylamino group, ethylamino group, N-n-butyl-N-methylamino group, diethylamino group, methylamino group, N-ethyl-N-methylamino group and N-methyl-N-i-propylamino group being particularly preferred;
if p is 8, dimethylamino group, diethylamino group and N-n-butyl-N-methylamino group are preferred;
if p is 9, amino group, n-pentylamino group, dimethylamino group, diethylamino group, N-ethyl-N-methylamino group, N-n-butyl-N-methylamino group and N-methyl-N-n-propylamino group are preferred, with dimethylamino group, diethylamino group, N-n-butyl-N-methylamino group and N-methyl-N-n-propylamino group being more preferred, and

dimethylamino group and N-methyl-N-n-propylamino being particularly preferred;
if p is 10, dimethylamino group, diethylamino group, N-ethyl-N-methylamino group, N-n-butyl-N-methylamino group and N-methyl-N-n-propylamino group are preferred, with dimethylamino group being more preferred;
if p is 11, dimethylamino group, diethylamino group, N-n-butyl-N-methylamino group, amino group and n-pentylamino group are preferred;
if p is 13, amino group and n-pentylamino group are preferred;
p in $-(CH_2)_p-SO_2-Z^1$ is preferably an integer of 5 - 13;
the group represented by $-Z^1$ in $-(CH_2)_p-SO_2-Z^1$ is preferably 4,4,5,5,5-pentafluoropentyl group;
p in $-(CH_2)_p-SO-Z^1$ is preferably an integer of 7 - 13;
the group represented by $-Z^1$ in $-(CH_2)_p-SO-Z^1$ is preferably 4,4,5,5,5-pentafluoropentyl group;
p in $-Ph-O-(CH_2)_p-CO-NR^8Z^1$ is preferably an integer of 1 - 7; the group represented by $-NR^8Z^1$ in $-Ph-O-(CH_2)_p-CO-NR^8Z^1$ is preferably amino group or n-pentylamino group;
speaking further of that group,
it is preferably amino group if p is 1;
if p is 3, amino group and n-pentylamino group are preferred;
if p is 7, amino group and n-pentylamino group are preferred;
p in $-Ph-O-(CH_2)_p-H$ is preferably 1.

Referring further to the general formula (I), it is preferred that the dashed line in 4(5) position signifies a single bond or a double bond in combination with the solid line and $X^2$ signifies any one group selected from the group consisting of $-(CH_2)_p-COOH$ (p is an integer of at least 1), $-(CH_2)_p-OH$ (p has the same meaning as defined above), $-Ph-O-(CH_2)_p-COOH$ (Ph represents a phenylene group and p has the same meaning as defined above), $-(CH_2)_p-CO-NR^8Z^2$ (p has the same meaning as defined above, $R^8$ represents a hydrogen atom or a straight-chained or branched lower alkyl group having 1 - 6 carbon atoms, $Z^2$ represents a straight-chained or branched alkyl group having 1 - 10 carbon atoms that is substituted by any one group selected from the group consisting of a cycloalkyl group, a hydroxyl group, a carboxyl group, a heterocyclic group and a phenyl group, or $-NR^8Z^2$ may be such that N, $R^8$ and $Z^2$ combine together to form a hetero ring), $-(CH_2)_p-Ph-O-(CH_2)_q-CO-NR^8Z^3$ (Ph, p and $R^8$ have the same meanings as defined above, q represents an integer of at least 1, and $Z^3$ represents a hydrogen atom or a straight-chained or branched alkyl group having 1 - 10 carbon atoms that may optionally be substituted by any one group selected from the group consisting of a cycloalkyl group, a hydroxyl group, a carboxyl group, a heterocyclic group and a phenyl group, or $-NR^8Z^3$ may be such that N, $R^8$ and $Z^3$ combine together to form a hetero ring) and $-(CH_2)_p-CH(COOH)-(CH_2)_3-CF_2-CF_3$ (p has the same meaning as defined above), with p being more preferably an integer of 1 - 13;
p in $-(CH_2)_p-COOH$ is preferably an integer of 5 - 13;
p in $-(CH_2)_p-OH$ is preferably an integer of 7 - 9;
p in $-Ph-O-(CH_2)_p-COOH$ is preferably an integer of 1 - 7;
p in $-(CH_2)_p-CO-NR^8Z^3$ is preferably an integer of 6 - 11; the group represented by $-NR^8Z^3$ in $-(CH_2)_p-CO-NR^8Z^3$ is preferably exemplified by, cyclohexylmethylamino group, cyclopropylmethylamino group, 3-hydroxypropylamino group, t-butylbenzylamino group, 2,2-diphenylethylamino group, N-methyl-N-benzylamino group, phenylamino group, benzylamino group, 2-phenylethylamino group, piperidino group, pyrrolidinyl group and morpholino group, with N-methyl-N-benzylamino group, benzylamino group, 2-phenylethylamino group, piperidino group, pyrrolidinyl group and morpholino group being more preferred, and piperidino group, pyrrolidinyl group and morpholino group being particularly preferred;
p in $-(CH_2)_p-Ph-O-(CH_2)_q-CO-NR^8Z^3$ is preferably 3;
q in $-(CH_2)_p-Ph-O-(CH_2)_q-CO-NR^8Z^3$ is preferably 3 or 4;
the group represented by $-NR^8Z^3$ in $-(CH_2)_p-Ph-O-(CH_2)_q-CO-NR^8Z^3$ is preferably exemplified by methylamino group, dimethylamino group and pyrrolidinyl group;
p in $-(CH_2)_p-CH(COOH)-(CH_2)_3-CF_2-CF_3$ is preferably 8;
p in $-(CH_2)_p-Ph-O-(CH_2)_q-COOH$ is preferably 3;
q in $-(CH_2)_p-Ph-O-(CH_2)_q-COOH$ is preferably 3 or 4.

Referring further to the general formula (I), it is preferred that the dashed line in 4(5) position signifies a single bond or a double bond in combination with the solid line and $X^1$ signifies any one group selected from the group consisting of $-(CH_2)_p-COOH$ (p is an integer of at least 1), $-(CH_2)_p-CH(COOH)-(CH_2)_3-CF_2-CF_3$ (p has the same meaning as defined above), $-(CH_2)_p-CH(COOMe)-(CH_2)_3-CF_2-CF_3$ (p has the same meaning as defined above), $-O-(CH_2)_p-COOH$ (p has the same meaning as defined above), $-O-(CH_2)_p-CH(COOH)-(CH_2)_3-CF_2-CF_3$ (p has the same meaning as defined above), $-(CH_2)_p-S-(CH_2)_3-CF_2-CF_3$ (p has the same meaning as defined above), $-(CH_2)_p-SO-(CH_2)_3-CF_2-CF_3$ (p has the same meaning as defined above), $-O-(CH_2)_p-SO-(CH_2)_3-CF_2-CF_3$ (p has the same meaning as defined above), $-O-(CH_2)_p-SO_2-(CH_2)_3-CF_2-CF_3$ (p has the same meaning as defined above), $-Ph-O-CH_3$ (Ph represents a phenylene group), $-Ph-O-(CH_2)_p-COOH$ (Ph and p have the same meanings as defined above), $-(CH_2)_p-CO-NR^8Z^3$ (p has the same meaning as defined above, $R^8$ represents a hydrogen atom or a straight-chained

or branched lower alkyl group having 1 - 6 carbon atoms, $Z^3$ represents a hydrogen atom or a straight-chained or branched alkyl group having 1 - 10 carbon atoms that may optionally be substituted by any one group selected from the group consisting of a cycloalkyl group, a hydroxyl group, a carboxyl group, a heterocyclic group and a phenyl group, or $-NR^8Z^3$ may be such that N, $R^8$ and $Z^3$ combine together to form a hetero ring), $-Ph-O-(CH_2)_p-CO-NR^8Z^3$ (Ph, p, $R^8$, $Z^3$ and $-NR^8Z^3$ have the same meanings as defined above) and $-O-(CH_2)_p-CO-NR^8Z^3$ (p, $R^8$, $Z^3$ and $-NR^8Z^3$ have the same meanings as defined above), with p being more preferably an integer of 3 - 13;
p in $-(CH_2)_p-COOH$ is preferably an integer of 7 - 11;
p in $-(CH_2)_p-CH(COOH)-(CH_2)_3-CF_2-CF_3$ is preferably 8;
p in $-(CH_2)_p-CH(COOMe)-(CH_2)_3-CF_2-CF_3$ is preferably 8;
p in $-O-(CH_2)_p-COOH$ is preferably an integer of 5 - 13;
p in $-O-(CH_2)_p-CH(COOH)-(CH_2)_3-CF_2-CF_3$ is preferably 8;
p in $-(CH_2)_p-S-(CH_2)_3-CF_2-CF_3$ is preferably 10;
p in $-(CH_2)_p-SO-(CH_2)_3-CF_2-CF_3$ is preferably 10;
p in $-O-(CH_2)_p-SO-(CH_2)_3-CF_2-CF_3$ is preferably an integer of 5 - 13;
p in $-O-(CH_2)_p-SO_2-(CH_2)_3-CF_2-CF_3$ is preferably an integer of 7 - 13;
p in $-Ph-O-(CH_2)_p-COOH$ is preferably an integer of 3 - 7;
p in $-(CH_2)_p-CO-NR^8Z^3$ is preferably an integer of 7 - 11;
the group represented by $-NR^8Z^3$ in $-(CH_2)_p-CO-NR^8Z^3$ is preferably exemplified by, amino group, n-pentylamino group, dimethylamino group, methylamino group, N-ethyl-N-methylamino group, N-methyl-N-n-propylamino group, diethylamino group, benzylamino group, N-n-butyl-N-methylamino group, 2-hydroxyethylamino group, morpholino group and piperidino group;
p in $-Ph-O-(CH_2)_p-CO-NR^8Z^3$ is preferably 7;
the group represented by $-NR^8Z^3$ in $-Ph-O-(CH_2)_p-CO-NR^8Z^3$ is preferably exemplified by amino group and n-pentylamino group;
p in $-O-(CH_2)_p-CO-NR^8Z^3$ is preferably an integer of 5 - 13; the group represented by $-NR^8Z^3$ in $-O-(CH_2)_p-CO-NR^8Z^3$ is preferably exemplified by amino group and n-pentylamino group.

Specifically, preferred examples of $X^1$ and $X^2$ are a hydrogen atom, 10-(4,4,5,5,5-pentafluoropentylsulfinyl) decyl group, 11-(4,4,5,5,5-pentafluoropentylsulfinyl)undecyl group, 12-(4,4,5,5,5-pentafluoropentylsulfinyl)dodecyl group, 10-(4,4,5,5,5-pentafluoropentylsulfonyl)decyl group, 11-(4,4,5,5,5-pentafluoropentylsulfonyl)undecyl group, 12-(4,4,5,5,5-pentafluoropentylsulfonyl)dodecyl group, 10-{N-(4,4,5,5,5-pentafluoropentyl)aminocarbonyl}decyl group, 11-{N-(4,4,5,5,5-pentafluoropentyl)aminocarbonyl}undecyl group, 9-{N-(5,5,6,6,6-pentafluorohexanoyl)amino) nonyl group, 10-{N-(5,5,6,6,6-pentafluorohexanoyl)amino}decyl group, 9-(4,4,5,5,5-pentafluoropentylsulfinyl)nonyloxy group, 10-(4,4,5,5,5-pentafluoropentylsulfinyl)decyloxy group, 11-(4,4,5,5,5-pentafluoropentylsulfinyl)undecyloxy group, 9-(4,4,5,5,5-pentafluoropentylsulfonyl)nonyloxy group, 10-(4,4,5,5,5-pentafluoropentylsulfonyl)decyloxy group, 11-(4,4,5,5,5-pentafluoropentylsulfonyl)undecyloxy group; 9-{N-(4,4,5,5,5-pentafluoropentyl)aminocarbonyl}nonyloxy group, 10-{N-(4,4,5,5,5-pentafluoropentyl)aminocarbonyl)decloxy group, 8-{N-(5,5,6,6,6-pentafluorohexanoyl)amino) octyloxy group, 9-{N-(5,5,6,6,6-pentafluorohexanoyl)amino)nonyloxy group, 4-{8-(4,4,5,5,5-pentafluoropentylsulfinyl) octyloxy)phenyl group, 4-{9-(4,4,5,5,5-pentafluoropentylsulfinyl)nonyloxy}phenyl group, 4-{8-(4,4,5,5,5-pentafluoropentylsulfonyl)octyloxy}phenyl group, 4-{9-(4,4,5,5,5-pentafluoropentylsulfonyl)nonyloxy)phenyl group, 4-[8-{N-(4,4,5,5,5-pentafluoropentyl)aminocarbonyl}octyloxy]phenyl group, 4-[9-{N-(4,4,5,5,5-pentafluoropentyl)aminocarbonyl}nonyloxy]phenyl group, 4-[7-{N-(5,5,6,6,6-pentafluorohexanoyl)amino}heptyloxy]phenyl group, 4-[8-{N-(5,5,6,6,6-pentafluorohexanoyl)amino}octyloxy]phenyl group, 6-[4-{N-(4,4,5,5,5-pentafluoropentyl)aminocarbonyl} phenyl]hexyl group, 5-[4-{N-(4,4,5,5,5-pentafluoropentyl)aminocarbonyl}phenyl]pentyloxy group, tridecyloxy group, 11-carboxy-15,15,16,16,16-pentafluorohexadecyl) group, 4-{{2-hydroxy-3-(4,4,5,5,5-pentafluoropentylsulfinylethyloxy)propyl}oxy)phenyl group, 4-hydroxy-9-(4,4,5,5,5-pentafluoropentylsulfinyl)nonyl group, 10-carboxy-14,14,15,15,15-pentafluoropentadecyloxy group, 9-carboxy-13,13,14,14,14-pentafluorotetradecyloxy group, 6-carboxy-10,10,11,11,11-pentafluoroundecyl group, 10-carboxy-14,14,15,15,15-pentafluoropentadecyl group, 14-carboxy-18,18,19,19,19-pentafluorononadecyl group, 9-carboxynonyloxy group, 6-carboxyhexyl group, 10-carboxydecyl group, 14-carboxytetradecyl group, 3-{4-(4-carboxybutyl)phenyl}propyl group, 3-{4-(4-carboxy-8,8,9,9,9-pentafluorononyl)phenyl}propyl group, 5-(4,4,5,5,5-pentafluoropentylsulfinyl)pentyl group, 9-(4,4,5,5,5-pentafluoropentylsulfinyl) nonyl group, 13-(4,4,5,5,5-pentafluoropentylsulfinyl)tridecyl group, 4-hydroxy-10-(4,4,5,5,5-pentafluoropentylsulfinyl) decyl group, 4-hydroxy-15,15,16,16,16-pentafluorohexadedecyl group, 9-{N-(4,4,5,5,5-pentafluoropentyl)aminocarbonyl}nonyl group, and 8-{N-(5,5,6,6,6-pentafluorohexanoyl)amino)octyl group;
5-carboxypentyl group, 7-carboxyheptyl group, 9-carboxynonyl group, 11-carboxyundecyl group, 13-carboxytridecyl group, 9-carboxy-13,13,14,14,14-pentafluorotetradecyl group, 9-methoxycarbonyl-13,13,14,14,14-pentafluorotetradecyl group, 5-carboxypentyloxy group, 7-carboxyheptyloxy group, 10-carboxydecyloxy group, 11-carboxyundecyloxy group, 13-carboxytridecyloxy group, 23-carboxytricosanyloxy group, 7-(N,N-dimethylaminocarbonyl)heptyl group, 7-(N-ethylaminocarbonyl)heptyl group, 7-{N-(cyclopropylmethyl)aminocarbonyl}heptyl group, 7-{N-(cyclohexylmethyl)aminocarbonyl)heptyl group, 7-(N-butylaminocarbonyl)heptyl group, 7-(N-isopropylaminocarbonyl)heptyl group, 7-(N-t-butylaminocarbonyl)heptyl group, 7-(N-cyclohexylaminocarbonyl)heptyl group, 7-{N-(3-hydroxypropyl)aminocarbonyl}heptyl group, 7-(N-methyl-N-butylaminocarbonyl)heptyl group, 7-(N,N-diethylaminocarbonyl)heptyl group, 7-(piperidinocarbonyl)heptyl group, 7-{N-(4-t-butylbenzyl)aminocarbonyl}heptyl group, 7-{N-(2,2-diphenylethyl)aminocarbonyl}heptyl group, 7-{N-(2-furylmethyl)aminocarbonyl}heptyl group, 7-(N-methylaminocarbonyl)heptyl group, 7-(N-methyl-N-ethylaminocarbonyl)heptyl group, 7-(N-methyl-N-propylaminocarbonyl)heptyl group, 7-(N-methyl-N-isopropylaminocarbonyl)heptyl group, 7-(N-methyl-N-benzylaminocarbonyl)heptyl group, 7-(1-pyrrolidinylcarbonyl)heptyl group, 7-(morpholinocarbonyl)heptyl group, 7-(N-methyl-N-t-butylaminocarbonyl)heptyl group, 7-(N-cyclopropylaminocarbonyl)heptyl group, 6-(N,N-dimethylaminocarbonyl)hexyl group, 6-(N,N-diethylaminocarbonyl)hexyl group, 6-(piperidinocarbonyl)hexyl group, 8-(N,N-dimethylaminocarbonyl)octyl group, 8-(N,N-diethylaminocarbonyl)octyl group, 8-(N-methyl-N-butylaminocarbonyl)octyl group, 8-(N-benzylaminocarbonyl)octyl group, 8-{N-(2-hydroxyethyl) aminocarbonyl}octyl group, 8-(piperidinocarbonyl)octyl group, 9-(N,N-dimethylaminocarbonyl)nonyl group, 9-(N,N-diethylaminocarbonyl)nonyl group, 9-(1-pyrrolidinylcarbonyl)nonyl group, 9-(N-methyl-N-ethylaminocarbonyl)nonyl group, 9-(N-methyl-N-butylaminocarbonyl)nonyl group, 9-(N-benzylaminocarbonyl)nonyl group, 9-(piperidinocarbonyl) nonyl group, 9-{N-(2-hydroxyethyl)aminocarbonyl}nonyl group, 9-(N-methyl-N-propylaminocarbonyl)nonyl group, 9-(morpholinocarbonyl)nonyl group, 10-(N,N-dimethylaminocarbonyl)decyl group, 10-(N,N-diethylaminocarbonyl)decyl group, 10-(N-methyl-N-ethylaminocarbonyl)decyl group, 10-(N-methyl-N-propylaminocarbonyl)decyl group, 10-(N-methyl-N-butylaminocarbonyl)decyl group, 10-(morpholinocarbonyl)decyl group, 11-(N,N-dimethylaminocarbonyl)undecyl group, 11-(N,N-diethylaminocarbonyl)undecyl group, 11-(piperidinocarbonyl)undecyl group, 11-(N-benzylaminocarbonyl)undecyl group, 11-(N-methyl-N-butylaminocarbonyl)undecyl group, 11-{N-(2-hydroxyethyl)aminocarbonyl) undecyl group, 7-{N-(2-hydroxyethyl)aminocarbonyl}heptyl group, 7-(N-propylaminocarbonyl)heptyl group, 7-(N-hexylaminocarbonyl)heptyl group, 7-(N-isopentylaminocarbonyl)heptyl group, 7-(N-isobutylaminocarbonyl)heptyl group, 7-(N-neopentylaminocarbonyl)heptyl group, 7-{N-(3-pentyl)aminocarbonyl}heptyl group, 7-(N,N-dihexylaminocarbonyl)heptyl group, 7-(N-phenylaminocarbonyl)heptyl group, 7-(N-benzylaminocarbonyl)heptyl group, 7-{N-(2-phenylethyl)aminocarbonyl}heptyl group, 5-(aminocarbonyl)pentyl group, 5-(N-pentylaminocarbonyl)pentyl group, 7-(aminocarbonyl)heptyl group, 7-(N-pentylaminocarbonyl)heptyl group, 9-(aminocarbonyl)nonyl group, 9-(N-pentylaminocarbonyl)nonyl group, 11-(aminocarbonyl)undecyl group, 11-(N-pentylaminocarbonyl)undecyl group, 13-(aminocarbonyl) tridecyl group, 13-(N-pentylaminocarbonyl)tridecyl group, 8-(N-methyl-N-ethylaminocarbonyl)octyl group, 8-(N-methyl-N-propylaminocarbonyl)octyl group, 8-(morpholinocarbonyl)octyl group, 8-(N-methylaminocarbonyl)octyl group, 10-(4,4,5,5,5-pentafluoropentylsulfanyl)decyl group, 7-hydroxyheptyl group, 8-hydroxyoctyl group, 9-hydroxynonyl group, 7-(4,4,5,5,5-pentafluoropentylsulfinyl)heptyl group, 7-(4,4,5,5,5-pentafluoropentylsulfonyl)heptyl group, 9-(4,4,5,5,5-pentafluoropentylsulfonyl)nonyl group, 13-(4,4,5,5,5-pentafluoropentylsulfonyl)tridecyl group;

4-(carboxymethoxy)phenyl group, 4-(3-carboxypropoxy)phenyl group, 4-(7-carboxyheptyloxy)phenyl group, 4-(carbamoylmethoxy)phenyl group, 4-(3-carbamoylpropoxy)phenyl group, 4-(7-carbamoylheptyloxy)phenyl group, 4-(3-N-pentylcarbamoylpropoxy)phenyl group, 4-(7-N-pentylcarbamoylheptyloxy)phenyl group, 4-methoxyphenyl group;

5-(4,4,5,5,5-pentafluoropentylsulfinyl)pentyloxy group, 7-(4,4,5,5,5-pentafluoropentylsulfinyl)heptyloxy group, 13-(4,4,5,5,5-pentafluoropentylsulfinyl)tridecyloxy group;

7-(4,4,5,5,5-pentafluoropentylsulfonyl)heptyloxy group, 13-(4,4,5,5,5-pentafluoropentylsulfonyl)tridecyloxy group;

4-{5-(4,4,5,5,5-pentafluoropentylsulfinyl)pentyloxy}phenyl group, 4-{7-(4,4,5,5,5-pentafluoropentylsulfinyl)heptyloxy} phenyl group, 4-{5-(4,4,5,5,5-pentafluoropentylsulfonyl)pentyloxy}phenyl group, 4-{7-(4,4,5,5,5-pentafluoropentylsulfonyl)heptyloxy}phenyl group;

3-{3-(3-carboxypropoxy)phenyl}propyl group, 3-{3-(4-carboxybutoxy)phenyl}propyl group, 3-[3-{3-N-methylaminocarbonyl)propoxy}phenyl]propyl group, 3-[3-{3-N,N-dimethylaminocarbonyl)propoxy}phenyl]propyl group, 3-[3-{3-(1-pyrrolidinylcarbonyl)propoxy}phenyl]propyl group, 3-[3-{4-N-methylaminocarbonyl)butoxy}phenyl]propyl group, 3-[3-{4-(N,N-dimethylaminocarbonyl)butoxy}phenyl]propyl group, 3-[3-{4-(1-pyrrolidinylcarbonyl)butoxy}phenyl]propyl group;

5-(aminocarbonyl)pentyloxy group, 5-(N-pentylaminocarbonyl)pentyloxy group, 7-(aminocarbonyl)heptyloxy group, 7-(N-pentylaminocarbonyl)heptyloxy group, 9-(aminocarbonyl)nonyloxy group, 9-(N-pentylaminocarbonyl)nonyloxy group, 11-(aminocarbonyl)undecyloxy group, 11-(N-pentylaminocarbonyl)undecyloxy group, 13-(aminocarbonyl)tridecyloxy group, and 13-(N-pentylaminocarbonyl)tridecyloxy group. More preferred are 10-(4,4,5,5,5-pentafluoropentylsulfinyl)decyl group, 11-(4,4,5,5,5-pentafluoropentylsulfinyl)undecyl group, 11-(4,4,5,5,5-pentafluoropentylsulfonyl)undecyl group, 9-(4,4,5,5,5-pentafluoropentylsulfinyl)nonyloxy group, 11-(4,4,5,5,5-pentafluoropentylsulfinyl)undecyloxy group, 9-(4,4,5,5,5-pentafluoropentylsulfonyl)nonyloxy group, 11-(4,4,5,5,5-pentafluoropentylsulfonyl)undecyloxy group, 9-carboxy-13,13,14,14,14-pentafluorotetradecyloxy group, 9-carboxynonyloxy group, 5-(4,4,5,5,5-pentafluoropentylsulfinyl)pentyl group, 9-(4,4,5,5,5-pentafluoropentylsulfinyl)nonyl group;

5-carboxypentyl group, 7-carboxyheptyl group, 9-carboxynonyl group, 11-carboxyundecyl group, 13-carboxytridecyl group, 9-carboxy-13,13,14,14,14-pentafluorotetradecyl group, 9-methoxycarbonyl-13,13,14,14,14-pentafluorotetradecyl group, 5-carboxypentyloxy group, 7-carboxyheptyloxy group, 10-carboxydecyloxy group, 11-carboxyundecyloxy

group, 13-carboxytridecyloxy group, 23-carboxytricosanyloxy group, 7-(N,N-dimethylaminocarbonyl)heptyl group, 7-(N-ethylaminocarbonyl)heptyl group, 7-{N-(cyclopropylmethyl)aminocarbonyl}heptyl group, 7-{N-(cyclohexylmethyl) aminocarbonyl}heptyl group, 7-(N-butylaminocarbonyl)heptyl group, 7-(N-isopropylaminocarbonyl)heptyl group, 7-(N-t-butylaminocarbonyl)heptyl group, 7-(N-cyclohexylaminocarbonyl)heptyl group, 7-{N-(3-hydroxypropyl)aminocarbonyl)heptyl group, 7-(N-methyl-N-butylaminocarbonyl)heptyl group, 7-(N,N-diethylaminocarbonyl)heptyl group, 7-(piperidinocarbonyl)heptyl group, 7-{N-(4-t-butylbenzyl)aminocarbonyl}heptyl group, 7-{N-(2,2-diphenylethyl)aminocarbonyl}heptyl group, 7-{N-(2-furylmethyl)aminocarbonyl}heptyl group, 7-(N-methylaminocarbonyl)heptyl group, 7-(N-methyl-N-ethylaminocarbonyl)heptyl group, 7-(N-methyl-N-propylaminocarbonyl)heptyl group, 7-(N-methyl-N-isopropylaminocarbonyl)heptyl group, 7-(N-methyl-N-benzylaminocarbonyl)heptyl group, 7-(1-pyrrolidinylcarbonyl)heptyl group, 7-(morpholinocarbonyl)heptyl group, 7-(N-methyl-N-t-butylaminocarbonyl)heptyl group, 7-(N-cyclopropylaminocarbonyl)heptyl group, 6-(N,N-dimethylaminocarbonyl)hexyl group, 6-(N,N-diethylaminocarbonyl)hexyl group, 6-(piperidinocarbonyl)hexyl group, 8-(N,N-dimethylaminocarbonyl)octyl group, 8-(N,N-diethylaminocarbonyl)octyl group, 8-(N-methyl-N-butylaminocarbonyl)octyl group, 8-(N-benzylaminocarbonyl)octyl group, 8-{N-(2-hydroxyethyl) aminocarbonyl}octyl group, 8-(piperidinocarbonyl)octyl group, 9-(N,N-dimethylaminocarbonyl)nonyl group, 9-(N,N-diethylaminocarbonyl)nonyl group, 9-(1-pyrrolidinylcarbonyl)nonyl group, 9-(N-methyl-N-ethylaminocarbonyl)nonyl group, 9-(N-methyl-N-butylaminocarbonyl)nonyl group, 9-(N-benzylaminocarbonyl)nonyl group, 9-(piperidinocarbonyl) nonyl group, 9-{N-(2-hydroxyethyl)aminocarbonyl}nonyl group, 9-(N-methyl-N-propylaminocarbonyl)nonyl group, 9-(morpholinocarbonyl)nonyl group, 10-(N,N-dimethylaminocarbonyl)decyl group, 10-(N,N-diethylaminocarbonyl)decyl group, 10-(N-methyl-N-ethylaminocarbonyl)decyl group, 10-(N-methyl-N-propylaminocarbonyl)decyl group, 10-(N-methyl-N-butylaminocarbonyl)decyl group, 10-(morpholinocarbonyl)decyl group, 11-(N,N-dimethylaminocarbonyl)undecyl group, 11-(N,N-diethylaminocarbonyl)undecyl group, 11-(piperidinocarbonyl)undecyl group, 11-(N-benzylaminocarbonyl)undecyl group, 11-(N-methyl-N-butylaminocarbonyl)undecyl group, 11-{N-(2-hydroxyethyl)aminocarbonyl) undecyl group, 7-{N-(2-hydroxyethyl)aminocarbonyl}heptyl group, 7-(N-propylaminocarbonyl)heptyl group, 7-(N-hexylaminocarbonyl)heptyl group, 7-(N-isopentylaminocarbonyl)heptyl group, 7-(N-isobutylaminocarbonyl)heptyl group, 7-(N-neopentylaminocarbonyl)heptyl group, 7-{N-(3-pentyl)aminocarbonyl}heptyl group, 7-(N,N-dihexylaminocarbonyl)heptyl group, 7-(N-phenylaminocarbonyl)heptyl group, 7-(N-benzylaminocarbonyl)heptyl group, 7-{N-(2-phenylethyl)aminocarbonyl}heptyl group, 5-(aminocarbonyl)pentyl group, 5-(N-pentylaminocarbonyl)pentyl group, 7-(aminocarbonyl)heptyl group, 7-(N-pentylaminocarbonyl)heptyl group, 9-(aminocarbonyl)nonyl group, 9-(N-pentylaminocarbonyl)nonyl group, 11-(aminocarbonyl)undecyl group, 11-(N-pentylaminocarbonyl)undecyl group, 13-(aminocarbonyl) tridecyl group, 13-(N-pentylaminocarbonyl)tridecyl group, 8-(N-methyl-N-ethylaminocarbonyl)octyl group, 8-(N-methyl-N-propylaminocarbonyl)octyl group, 8-(morpholinocarbonyl)octyl group, 8-(N-methylaminocarbonyl)octyl group, 10-(4,4,5,5,5-pentafluoropentylsulfanyl)decyl group, 7-hydroxyheptyl group, 8-hydroxyoctyl group, 9-hydroxynonyl group, 7-(4,4,5,5,5-pentafluoropentylsulfinyl)heptyl group, 7-(4,4,5,5,5-pentafluoropentylsulfonyl)heptyl group, 9-(4,4,5,5,5-pentafluoropentylsulfonyl)nonyl group, 13-(4,4,5,5,5-pentafluoropentylsulfonyl)tridecyl group; 4-(carboxymethoxy)phenyl group, 4-(3-carboxypropoxy)phenyl group, 4-(7-carboxyheptyloxy)phenyl group, 4-(carbamoylmethoxy)phenyl group, 4-(3-carbamoylpropoxy)phenyl group, 4-(7-carbamoylheptyloxy)phenyl group, 4-(3-N-pentylcarbamoylpropoxy)phenyl group, 4-(7-N-pentylcarbamoylheptyloxy)phenyl group, 4-methoxyphenyl group;
5-(4,4,5,5,5-pentafluoropentylsulfinyl)pentyloxy group, 7-(4,4,5,5,5-pentafluoropentylsulfinyl)heptyloxy group, 13-(4,4,5,5,5-pentafluoropentylsulfinyl)tridecyloxy group;
7-(4,4,5,5,5-pentafluoropentylsulfonyl)heptyloxy group, 13-(4,4,5,5,5-pentafluoropentylsulfonyl)tridecyloxy group;
4-{5-(4,4,5,5,5-pentafluoropentylsulfinyl)pentyloxy}phenyl group, 4-{7-(4,4,5,5,5-pentafluoropentylsulfinyl)heptyloxy} phenyl group, 4-{5-(4,4,5,5,5-pentafluoropentylsulfonyl)pentyloxy}phenyl group, 4-{7-(4,4,5,5,5-pentafluoropentylsulfonyl)heptyloxy group; 3-{3-(3-carboxypropoxy)phenyl)propyl group, 3-{3-(4-carboxybutoxy)phenyl)propyl group, 3-[3-{3-N-methylaminocarbonyl)propoxy)phenyl]propyl group, 3-[3-{3-N,N-dimethylaminocarbonyl)propoxy}phenyl]propyl group, 3-[3-{3-(1-pyrrolidinylcarbonyl)propoxy}phenyl]propyl group, 3-[3-{4-(N-methylaminocarbonyl)butoxy}phenyl] propyl group, 3-[3-(4-(N,N-dimethylaminocarbonyl)butoxy}phenyl}propyl group, and 3-[3-{4-(1-pyrrolidinylcarbonyl)butoxy}phenyl]propyl group;
as well as 5-(aminocarbonyl)pentyloxy group, 5-(N-pentylaminocarbonyl)pentyloxy group, 7-(aminocarbonyl)heptyloxy group, 7-(N-pentylaminocarbonyl)heptyloxy group, 9-(aminocarbonyl)nonyloxy group, 9-(N-pentylaminocarbonyl)nonyloxy group, 11-(aminocarbonyl)undecyloxy group, 11-(N-pentylaminocarbonyl)undecyloxy group, 13-(aminocarbonyl)tridecyloxy group, and 13-(N-pentylaminocarbonyl)tridecyloxy group.

Particularly preferred are 7-(N,N-dimethylaminocarbonyl)heptyl group, 7-(N-ethylaminocarbonyl)heptyl group, 7-(N-isopropylaminocarbonyl)heptyl group, 7-(N-methyl-N-butylaminocarbonyl)heptyl group, 7-(N,N-diethylaminocarbonyl)heptyl group, 7-(piperidinocarbonyl)heptyl group, 7-{N-(2-furylmethyl)aminocarbonyl}heptyl group, 7-(N-methylaminocarbonyl)heptyl group, 7-(N-methyl-N-ethylaminocarbonyl)heptyl group, 7-(N-methyl-N-propylaminocarbonyl)heptyl group, 7-(N-methyl-N-isopropylaminocarbonyl)heptyl group, 7-(N-methyl-N-benzylaminocarbonyl) heptyl group, 7-(1-pyrrolidinylcarbonyl)heptyl group, 7-(morpholinocarbonyl)heptyl group, 9-(N,N-dimethylaminocarbonyl)nonyl group, 9-(N,N-diethylaminocarbonyl)nonyl group, 9-(N-methyl-N-butylaminocarbonyl)nonyl group, 9-(N-methyl-N-propylaminocarbonyl)nonyl group, 9-(morpholinocarbonyl)nonyl group, 10-(N,N-dimethylaminocarbonyl)decyl group, 7-{N-(2-hydroxyethyl)aminocarbonyl}heptyl group, 7-(N-propylaminocarbonyl)heptyl group, 7-(N-benzylaminocarbonyl)heptyl group, 7-{N-(2-phenylethyl)aminocarbonyl}heptyl group, 3-[3-{3-N-methylaminocarbonyl)propoxy}phenyl]propyl group, 3-[3-{3-(N,N-dimethylaminocarbonyl)propoxy)phenyl]propyl group, and 3-[3-{4-(1-pyrrolidinylcarbonyl)butoxy}phenyl]propyl group. It should however be noted that $X^1$ and $X^2$ are not a hydrogen atom at the same time. Particularly preferred cases are such that $X^1$ is a hydrogen atom and $X^2$ is any one of the groups listed above except a hydrogen atom, as well as where $X^1$ is any one of the groups listed above except a hydrogen atom and $X^2$ is a hydrogen atom.

Preferred examples of the compound represented by the general formula (I) are listed below:

17β-hydroxy-11β-{10-(4,4,5,5,5-pentafluoropentylsulfinyl)decyl}-5α-androstan-3-one;
17β-hydroxy-11β-{9-(4,4,5,5,5-pentafluoropentylsulfinyl)nonyloxy}-5α-androstan-3-one;
17β-hydroxy-11β-{11-(4,4,5,5,5-pentafluoropentylsulfinyl)undecyloxy}-5α-androstan-3-one;
17β-hydroxy-11β-{9-(4,4,5,5,5-pentafluoropentylsulfonyl)nonyloxy}-5α-androstan-3-one;
17β-hydroxy-11β-{11-(4,4,5,5,5-pentafluoropentylsulfonyl)undecyloxy}-5α-androstan-3-one;
17β-hydroxy-11β-(9-carboxy-13,13,14,14,14-pentafluorotetradecyloxy)-5α-androstan-3-one;
17β-hydroxy-11β-(9-carboxynonyloxy)-5α-androstan-3-one;
17β-hydroxy-11β-{12-(4,4,5,5,5-pentafluoropentylsulfinyl)dodecyl}-5α-androstan-3-one;
17β-hydroxy-11β-{10-(4,4,5,5,5-pentafluoropentylsulfonyl)decyl)-5α-androstan-3-one;
17β-hydroxy-11β-{11-(4,4,5,5,5-pentafluoropentylsulfonyl)undecyl}-5α-androstan-3-one;
17β-hydroxy-11β-{12-(4,4,5,5,5-pentafluoropentylsulfonyl)dodecyl}-5α-androstan-3-one;
17β-hydroxy-11β-[10-{N-(4,4,5,5,5-pentafluoropentyl)aminocarbonyl}decyl]-5α-androstan-3-one;
17β-hydroxy-11β-[11-{N-(4,4,5,5,5-pentafluoropentyl)aminocarbonyl}undecyl]-5α-androstan-3-one;
17β-hydroxy-11β-[9-{N-(5,5,6,6,6-pentafluorohexanoyl)amino}nonyl]-5α-androstan-3-one ;
17β-hydroxy-11β-[10-{N-(5,5,6,6,6-pentafluorohexanoyl)amino)decyl]-5α-androstan-3-one;
17β-hydroxy-11β-{9-(4,4,5,5,5-pentafluoropentylsulfinyl)nonyloxy}-5α-androstan-3-one;
17β-hydroxy-11β-{10-(4,4,5,5,5-pentafluoropentylsulfinyl)decyloxy)-5α-androstan-3-one;
17β-hydroxy-11β-{11-(4,4,5,5,5-pentafluoropentylsulfinyl)undecyloxy}-5α-androstan-3-one;
17β-hydroxy-11β-{9-(4,4,5,5,5-pentafluoropentylsulfonyl)nonyloxy)-5α-androstan-3-one;
17β-hydroxy-11β-{10-(4,4,5,5,5-pentafluoropentylsulfonyl)decyloxy}-5α-androstan-3-one;
17β-hydroxy-11β-{11-(4,4,5,5,5-pentafluoropentylsulfonyl)undecyloxy}-5α-androstan-3-one;
17β-hydroxy-11β-[9-{N-(4,4,5,5,5-pentafluoropentyl)aminocarbonyl}nonyloxy]-5α-androstan-3-one;
17β-hydroxy-11β-[10-{N-(4,4,5,5,5-pentafluoropentyl)aminocarbonyl}decyloxy]-5α-androstan-3-one;
17β-hydroxy-11β-[8-{N-(5,5,6,6,6-pentafluorohexanoyl)amino}octyloxy]-5α-androstan-3-one;
17β-hydroxy-11β-[9-{N-(5,5,6,6,6-pentafluorohexanoyl)amino}nonyloxy]-5α-androstan-3-one;
17β-hydroxy-11β-[4-{8-(4,4,5,5,5-pentafluoropentylsulfinyl)octyloxy}phenyl]-5α-androstan-3-one;
17β-hydroxy-11β-[4-{9-(4,4,5,5,5-pentafluoropentylsulfinyl)nonyloxy}phenyl]-5α-androstan-3-one;
17β-hydroxy-11β-[4-{8-(4,4,5,5,5-pentafluoropentylsulfonyl)octyloxy)phenyl]-5α-androstan-3-one;
17β-hydroxy-11β-[4-{9-(4,4,5,5,5-pentafluoropentylsulfonyl)nonyloxy)phenyl]-5α-androstan-3-one;
17β-hydroxy-11β-(4-[8-{N-(4,4,5,5,5-pentafluoropenty)aminocarbonyl}octyloxy]phenyl)-5α-androstan-3-one;
17β-hydroxy-11β-(4-[9-{N-(4,4,5,5,5-pentafluoropenty)aminocarbonyl}nonyloxy]phenyl)-5α-androstan-3-one;
17β-hydroxy-11β-(4-[7-{N-(5,5,6,6,6-pentafluorohexanoyl)amino}heptyloxy]phenyl)-5α-androstan-3-one;
17β-hydroxy-11β-(4-[8-{N-(5,5,6,6,6-pentafluorohexanoyl)amino}octyloxy]phenyl)-5α-androstan-3-one;
17β-hydroxy-11β-(6-[4-{N-(4,4,5,5,5-pentafluoropentyl)aminocarbonyl}phenyl]hexyl)-5α-androstan-3-one;
17β-hydroxy-11β-(5-[4-{N-(4,4,5,5,5-pentafluoropentyl)aminocarbonyl}phenyl]pentyloxy)-5α-androstan-3-one;
17β-hydroxy-11β-tridecyloxy-5α-androstan-3-one;
17β-hydroxy-11β-(11-carboxy-15,15,16,16,16-pentafluorohexadecyl)-5α-androstan-3-one;
17β-hydroxy-11β-[4-{{2-hydroxy-3-(4,4,5,5,5-pentafluoropentylsulfinylethyloxy)propyl}oxy}phenyl]-5α-androstan-3-one;
17β-hydroxy-11β-{4-hydroxy-9-(4,4,5,5,5-pentafluoropentylsulfinyl)nonyl}-5α-androstan-3-one;
17β-hydroxy-11β-(10-carboxy-14,14,15,15,15-pentafluoropentadecyloxy)-5α-androstan-3-one;
17β-hydroxy-11β-(9-carboxy-13,13,14,14,14-pentafluorotetradecyloxy)-5α-androstan-3-one;
17β-hydroxy-11β-(6-carboxy-10,10,11,11,11-pentafluoroundecyl)-5α-androstan-3-one;
17β-hydroxy-11β-(10-carboxy-14,14,15,15,15-pentafluoropentadecyl)-5α-androstan-3-one;
17β-hydroxy-11β-(14-carboxy-18,18,19,19,19-pentafluorononadecyl)-5α-androstan-3-one;
17β-hydroxy-11β-(9-carboxynonyloxy)-5α-androstan-3-one;
17β-hydroxy-11β-(6-carboxyhexyl)-5α-androstan-3-one;
17β-hydroxy-11β-(10-carboxydecyl)-5α-androstan-3-one;

17β-hydroxy-11β-(14-carboxytetradecyl)-5α-androstan-3-one;
17β-hydroxy-11β-[3-{4-(4-carboxybutyl)phenyl}propyl]-5α-androstan-3-one;
17β-hydroxy-11β-[3-{4-(4-carboxy-8,8,9,9,9-pentafluorononyl)phenyl}propyl]-5α-androstan-3-one;
17β-hydroxy-11β-{5-(4,4,5,5,5-pentafluoropentylsulfinyl)pentyl}-5α-androstan-3-one;
17β-hydroxy-11β-{9-(4,4,5,5,5-pentafluoropentylsulfinyl)nonyl}-5α-androstan-3-one;
17β-hydroxy-11β-{13-(4,4,5,5,5-pentafluoropentylsulfinyl)tridecyl}-5α-androstan-3-one;
17β-hydroxy-11β-{4-hydroxy-10-(4,4,5,5,5-pentafluoropentylsulfinyl)decyl}-5α-androstan-3-one;
17β-hydroxy-11β-(4-hydroxy-15,15,16,16,16-pentafluorohexadecyl)-5α-androstan-3-one;
17β-hydroxy-11β-[9-{N-(4,4,5,5,5-pentafluoropentyl)aminocarbonyl}nonyl}-5α-androstan-3-one;
and
17β-hydroxy-11β-[8-{N-(5,5,6,6,6-pentafluorohexanoyl)amino}octyl]-5α-androstan-3-one;
17β-hydroxy-7β-{11-(4,4,5,5,5-pentafluoropentylsulfinyl)undecyl}-5α-androstan-3-one;
17β-hydroxy-7β-{11-(4,4,5,5,5-pentafluoropentylsulfonyl)undecyl}-5α-androstan-3-one;
17β-hydroxy-7β-{5-(4,4,5,5,5-pentafluoropentylsulfinyl)pentyl}-5α-androstan-3-one;
17β-hydroxy-7β-{9-(4,4,5,5,5-pentafluoropentylsulfinyl)nonyl}-5α-androstan-3-one;
17β-hydroxy-7β-(5-carboxypentyl)-5α-androstan-3-one;
17β-hydroxy-7β-(7-carboxyheptyl)-5α-androstan-3-one;
17β-hydroxy-7β-(9-carboxynonyl)-5α-androstan-3-one;
17β-hydroxy-7β-(11-carboxyundecyl)-5α-androstan-3-one;
17β-hydroxy-7β-(13-carboxytridecyl)-5α-androstan-3-one;
17β-hydroxy-7β-(9-carboxy-13,13,14,14,14-pentafluorotetradecyl)-5α-androstan-3-one;
17β-hydroxy-11β-(9-carboxy-13,13,14,14,14-pentafluorotetradecyl)-5α-androstan-3-one;
17β-hydroxy-11β-(9-methoxycarbonyl-13,13,14,14,14-pentafluorotetradecyl)-5α-androstan-3-one;
17β-hydroxy-11β-(5-carboxypentyloxy)-5α-androstan-3-one;
17β-hydroxy-11β-(7-carboxyheptyloxy)-5α-androstan-3-one;
17β-hydroxy-11β-(10-carboxydecyloxy)-5α-androstan-3-one;
17β-hydroxy-11β-(11-carboxyundecyloxy)-5α-androstan-3-one;
17β-hydroxy-11β-(13-carboxytridecyloxy)-5α-androstan-3-one;
17β-hydroxy-11β-(23-carboxytricosanyloxy)-5α-androstan-3-one;
17β-hydroxy-7α-{7-(N,N-dimethylaminocarbonyl)heptyl}-5α-androstan-3-one;
17β-hydroxy-7α-{7-(N-ethylaminocarbonyl)heptyl}-5α-androstan-3-one;
17β-hydroxy-7α-[7-{N-(cyclopropylmethyl)aminocarbonyl}heptyl]-5α-androstan-3-one;
17β-hydroxy-7α-[7-{N-(cyclohexylmethyl)aminocarbonyl}heptyl]-5α-androstan-3-one;
17β-hydroxy-7α-[7-(N-butylaminocarbonyl)heptyl]-5α-androstan-3-one;
17β-hydroxy-7α-[7-(N-(isopropylaminocarbonyl)heptyl]-5α-androstan-3-one;
17β-hydroxy-7α-[7-(N-t-butylaminocarbonyl)heptyl]-5α-androstan-3-one;
17β-hydroxy-7α-[7-(N-cyclohexylaminocarbonyl)heptyl]-5α-androstan-3-one;
17β-hydroxy-7α-[7-{N-(3-hydroxypropyl)aminocarbonyl}heptyl]-5α-androstan-3-one;
17β-hydroxy-7α-[7-(N-methyl-N-butylaminocarbonyl)heptyl]-5α-androstan-3-one;
17β-hydroxy-7α-[7-(N,N-diethylaminocarbonyl)heptyl]-5α-androstan-3-one;
17β-hydroxy-7α-[7-(piperidinocarbonyl)heptyl]-5α-androstan-3-one;
17β-hydroxy-7α-[7-{N-(4-t-butylbenzyl)aminocarbonyl}heptyl]-5α-androstan-3-one;
17β-hydroxy-7α-[7-{N-(2,2-diphenylethyl)aminocarbonyl}heptyl]-5α-androstan-3-one;
17β-hydroxy-7α-[7-{N-(2-furylmethyl)aminocarbonyl}heptyl]-5α-androstan-3-one;
17β-hydroxy-7α-[7-{7-(N-methylaminocarbonyl]heptyl]-5α-androstan-3-one;
17β-hydroxy-7α-[7-(N-methyl-N-ethylaminocarbonyl)heptyl]-5α-androstan-3-one;
17β-hydroxy-7α-[7-(N-methyl-N-propylaminocarbonyl)heptyl]-5α-androstan-3-one;
17β-hydroxy-7α-[7-(N-methyl-N-isopropylaminocarbonyl)heptyl]-5α-androstan-3-one; 17β-hydroxy-7α-[7-(N-methyl-N-benzylaminocarbonyl)heptyl]-5α-androstan-3-one;
17β-hydroxy-7α-[7-(1-pyrrolidinylcarbonyl)heptyl]-5α-androstan-3-one;
17β-hydroxy-7α-[7-(morpholinocarbonyl)heptyl]-5α-androstan-3-one;
17β-hydroxy-7α-[7-(N-methyl-N-t-butylaminocarbonyl)heptyl]-5α-androstan-3-one;
17β-hydroxy-7β-[7-(N-cyclopropylaminocarbonyl)heptyl]-5α-androstan-3-one;
17β-hydroxy-7α-[6-(N,N-dimethylaminocarbonyl)hexyl]-5α-androstan-3-one;
17β-hydroxy-7α-[6-(N,N-diethylaminocarbonyl)hexyl]-5α-androstan-3-one;
17β-hydroxy-7α-[6-(piperidinocarbonyl)hexyl]-5α-androstan-3-one;
17β-hydroxy-7α-[8-(N,N-dimethylaminocarbonyl)octyl]-5α-androstan-3-one;
17β-hydroxy-7α-[8-(N,N-diethylaminocarbonyl)octyl]-5α-androstan-3-one;

17β-hydroxy-7α-[8-(N-methyl-N-butylaminocarbonyl)octyl]-5α-androstan-3-one;
17β-hydroxy-7α-[8-(N-benzylaminocarbonyl)octyl]-5α-androstan-3-one;
17β-hydroxy-7α-[8-{N-(2-hydroxyethyl)aminocarbonyl}octyl]-5α-androstan-3-one;
17β-hydroxy-7α-[8-(piperidinocarbonyl)octyl]-5α-androstan-3-one;
17β-hydroxy-7α-[9-(N,N-dimethylaminocarbonyl)nonyl]-5α-androstan-3-one;
17β-hydroxy-7α-[9-(N,N-diethylaminocarbonyl)nonyl]-5α-androstan-3-one;
17β-hydroxy-7α-[9-(1-pyrrolidinylcarbonyl)nonyl]-5α-androstan-3-one;
17β-hydroxy-7α-[9-(N-methyl-N-ethylaminocarbonyl)nonyl]-5α-androstan-3-one;
17β-hydroxy-7α-[9-(N-methyl-N-butylaminocarbonyl)nonyl]-5α-androstan-3-one;
17β-hydroxy-7α-[9-(N-benzylaminocarbonyl)nonyl]-5α-androstan-3-one;
17β-hydroxy-7α-[9-(piperidinocarbonyl)nonyl]-5α-androstan-3-one:
17β-hydroxy-7α-[9-{N-(2-hydroxyethyl)aminocarbonyl)nonyl]-5α-androstan-3-one;
17β-hydroxy-7α-[9-(N-methyl-N-propylaminocarbonyl)nonyl]-5α-androstan-3-one;
17β-hydroxy-7α-[9-(morpholinocarbonyl)nonyl]-5α-androstan-3-one;
17β-hydroxy-7α-[10-(N,N-dimethylaminocarbonyl)decyl]-5α-androstan-3-one;
17β-hydroxy-7α-[10-(N,N-diethylaminocarbonyl)decyl]-5α-androstan-3-one;
17β-hydroxy-7α-[10-(N-methyl-N-ethylaminocarbonyl)decyl]-5α-androstan-3-one;
17β-hydroxy-7α-[10-N-methyl-N-propylaminocarbonyl)decyl]-5α-androstan-3-one;
17β-hydroxy-7α-[10-(N-methyl-N-butylaminocarbonyl)decyl]-5α-androstan-3-one;
17β-hydroxy-7α-[10-(morpholinocarbonyl)decyl]-5α-androstan-3-one;
17β-hydroxy-7α-[11-(N,N-dimethylaminocarbonyl)undecyl]-5α-androstan-3-one;
17β-hydroxy-7α-[11-(N,N-diethylaminocarbonyl)undecyl]-5α-androstan-3-one;
17β-hydroxy-7α-[11-(piperidinocarbonyl)undecyl]-5α-androstan-3-one;
17β-hydroxy-7α-[11-(N-benzylaminocarbonyl)undecyl]-5α-androstan-3-one;
17β-hydroxy-7α-[11-(N-methyl-N-butylaminocarbonyl)undecyl]-5α-androstan-3-one;
17β-hydroxy-7α-[11-{N-(2-hydroxyethyl)aminocarbonyl}undecyl]-5α-androstan-3-one;
17β-hydroxy-7α-[7-{N-(2-hydroxyethyl)aminocarbonyl}heptyl]-5α-androstan-3-one;
17β-hydroxy-7α-[7-(N-propylaminocarbonyl)heptyl]-5α-androstan-3-one;
17β-hydroxy-7α-[7-(N-hexylaminocarbonyl)heptyl]-5α-androstan-3-one;
17β-hydroxy-7α-[7-(N-isopentylaminocarbonyl)heptyl]-5α-androstan-3-one;
17β-hydroxy-7α-[7-(N-isobutylaminocarbonyl)heptyl]-5α-androstan-3-one;
17β-hydroxy-7α-[7-(N-neopentylaminocarbonyl)heptyl]-5α-androstan-3-one;
17β-hydroxy-7α-[7-{N-(3-pentyl)aminocarbonyl)heptyl]-5α-androstan-3-one;
17β-hydroxy-7α-[7-(N,N-dihexylaminocarbonyl)heptyl]-5α-androstan-3-one;
17β-hydroxy-7α-[7-(N-phenylaminocarbonyl)heptyl]-5α-androstan-3-one;
17β-hydroxy-7α-[7-(N-benzylaminocarbonyl)heptyl]-5α-androstan-3-one;
17β-hydroxy-7α-[7-{N-(2-phenylethyl)aminocarbonyl}heptyl]-5α-androstan-3-one;
17β-hydroxy-11β-(7-carboxyheptyl)-5α-androstan-3-one;
17β-hydroxy-11β-(8-carboxyoctyl)-5α-androstan-3-one;
17β-hydroxy-11β-(9-carboxynonyl)-5α-androstan-3-one;
17β-hydroxy-11β-(11-carboxyundecyl)-5α-androstan-3-one;
17β-hydroxy-7α-[5-(aminocarbonyl)pentyl]-5α-androstan-3-one;
17β-hydroxy-7α-[5-(N-pentylaminocarbonyl)pentyl]-5α-androstan-3-one;
17β-hydroxy-7α-[7-(aminocarbonyl)heptyl]-5α-androstan-3-one;
17β-hydroxy-7α-[7-(N-pentylaminocarbonyl)heptyl]-5α-androstan-3-one;
17β-hydroxy-7α-[9-(aminocarbonyl)nonyl]-5α-androstan-3-one;
17β-hydroxy-11β-[9-(aminocarbonyl)nonyl]-5α-androstan-3-one;
17β-hydroxy-7α-[9-(N-pentylaminocarbonyl)nonyl]-5α-androstan-3-one;
17β-hydroxy-11β-[9-(N-pentylaminocarbonyl)nonyl]-5 androstan-3-one;
17β-hydroxy-7α-[11-(aminocarbonyl)undecyl]-5α-androstan-3-one;
17β-hydroxy-11β-[11-(aminocarbonyl)undecyl]-5α-androstan-3-one;
17β-hydroxy-7α-[11-(N-pentylaminocarbonyl)undecyl]-5α-androstan-3-one;
17β-hydroxy-11β-[11-(N-pentylaminocarbonyl)undecyl]-5α-androstan-3-one;
17β-hydroxy-7α-[13-(aminocarbonyl)tridecyl]-5α-androstan-3-one;
17β-hydroxy-7α-[13-(N-pentylaminocarbonyl)tridecyl]-5α-androstan-3-one:
17β-hydroxy-11β-{7-(N,N-dimethylaminocarbonyl)heptyl}-5α-androstan-3-one;
17β-hydroxy-11β-[7-{7-(N-methylaminocarbonyl)heptyl]-5α-androstan-3-one;
17β-hydroxy-11β-[7-(N-methyl-N-ethylaminocarbonyl)heptyl]-5α-androstan-3-one;

17β-hydroxy-11β-[7-(N-methyl-N-propylaminocarbonyl)heptyl]-5α-androstan-3-one;
17β-hydroxy-11β-[7-(morpholinocarbonyl)heptyl]-5α-androstan-3-one;
17β-hydroxy-11β-[8-(N,N-dimethylaminocarbonyl)octyl]-5α-androstan-3-one;
17β-hydroxy-11β-[8-(N-methylaminocarbonyl)octyl]-5α-androstan-3-one;
17β-hydroxy-11β-[8-(N-methyl-N-ethylaminocarbonyl)octyl]-5α-androstan-3-one;
17β-hydroxy-11β-[8-(N-methyl-N-propylaminocarbonyl)octyl]-5α-androstan-3-one;
17β-hydroxy-11β-[8-(morpholinocarbonyl)octyl]-5α-androstan-3-one;
17β-hydroxy-11β-[9-(N,N-dimethylaminocarbonyl)nonyl]-5α-androstan-3-one;
17β-hydroxy-11β-[9-(N,N-diethylaminocarbonyl)nonyl]-5α-androstan-3-one;
17β-hydroxy-11β-[9-(N-methyl-N-butylaminocarbonyl)nonyl]-5α-androstan-3-one;
17β-hydroxy-11β-[9-(N-benzylaminocarbonyl)nonyl]-5α-androstan-3-one;
17β-hydroxy-11β-[9-(piperidinocarbonyl)nonyl]-5α-androstan-3-one;
17β-hydroxy-11β-[9-{N-(2-hydroxyethyl)aminocarbonyl}nonyl]-5α-androstan-3-one;
17β-hydroxy-11β-[10-(4,4,5,5,5-pentafluoropentylsulfanyl)decyl]-5α-androstan-3-one;
17β-hydroxy-7α-(7-hydroxyheptyl)-5α-androstan-3-one;
17β-hydroxy-7α-(8-hydroxyoctyl)-5α-androstan-3-one;
17β-hydroxy-7α-(9-hydroxynonyl)-5α-androstan-3-one;
17β-hydroxy-7α-[7-(4,4,5,5,5-pentafluoropentylsulfinyl)heptyl]-5α-androstan-3-one;
17β-hydroxy-7α-[13-(4,4,5,5,5-pentafluoropentylsulfinyl)tridecyl]-5α-androstan-3-one;
17β-hydroxy-7α-[7-(4,4,5,5,5-pentafluoropentylsulfonyl)heptyl]-5α-androstan-3-one;
17β-hydroxy-7α-[9-(4,4,5,5,5-pentafluoropentylsulfonyl)nonyl]-5α-androstan-3-one;
17β-hydroxy-7α-[13-(4,4,5,5,5-pentafluoropentylsulfonyl)tridecyl]-5α-androstan-3-one;
17β-hydroxy-7α-[4-(carboxymethoxy)phenyl]-5α-androstan-3-one;
17β-hydroxy-7α-[4-(3-carboxypropoxy)phenyl]-5α-androstan-3-one;
17β-hydroxy-11β-[4-(3-carboxypropoxy)phenyl]-5α-androstan-3-one;
17β-hydroxy-7α-[4-(7-carboxyheptyloxy)phenyl]-5α-androstan-3-one;
17β-hydroxy-11β-[4-(7-carboxyheptyloxy)phenyl]-5α-androstan-3-one;
17β-hydroxy-7α-[4-(carbamoylmethoxy)phenyl]-5α-androstan-3-one;
17β-hydroxy-7α-[4-(carbamoylmethoxy)phenyl]-5α-androstan-3-one;
17β-hydroxy-7α-[4-(3-carbamoylpropoxy)phenyl]-5α-androstan-3-one;
17β-hydroxy-11β-[4-(3-carbamoylpropoxy)phenyl)-5α-androstan-3-one;
17β-hydroxy-7α-[4-(7-carbamoylheptyloxy)phenyl]-5α-androstane-3-one;
17β-hydroxy-11β-[4-(7-carbamoylheptyloxy)phenyl]-5α-androstan-3-one;
17β-hydroxy-7α-[4-(3-N-pentylcarbamoylpropoxy)phenyl]-5α-androstan-3-one;
17β-hydroxy-11β-[4-(3-N-pentylcarbamoylpropoxy)phenyl]-5α-androstan-3-one;
17β-hydroxy-7α-[4-(7-N-pentylcarbamoylheptyloxy)phenyl]-5α-androstan-3-one;
17β-hydroxy-11β-[4-(7-N-pentylcarbamoylheptyloxy)phenyl]-5α-androstan-3-one;
17β-hydroxy-7α-[4-methoxyphenyl]-5α-androstan-3-one;
17β-hydroxy-11β-[4-methoxyphenyl]-5α-androstan-3-one;
17β-hydroxy-11β-[5-(4,4,5,5,5-pentafluoropentylsulfinyl)pentyloxy]-5α-androstan-3-one;
17β-hydroxy-11β-[7-(4,4,5,5,5-pentafluoropentylsulfinyl)heptyloxy]-5α-androstan-3-one;
17β-hydroxy-11β-[13-(4,4,5,5,5-pentafluoropentylsulfinyl)tridecyloxy]-5α-androstan-3-one;
17β-hydroxy-11β-[7-(4,4,5,5,5-pentafluoropentylsulfonyl)heptyloxy]-5α-androstan-3-one;
17β-hydroxy-11β-[13-(4,4,5,5,5-pentafluoropentylsulfonyl)tridecyloxy]-5α-androstan-3-one;
17β-hydroxy-11β-[4-(5-(4,4,5,5,5-pentafluoropentylsulfinyl)pentyloxy}phenyl]-5α-androstan-3-one;
17β-hydroxy-11β-[4-{7-(4,4,5,5,5-pentafluoropentylsulfinyl)heptyloxy}phenyl}-5α-androstan-3-one;
17β-hydroxy-11β-[4-{5-(4,4,5,5,5-pentafluoropentylsulfonyl)pentyloxy}phenyl]-5α-androstan-3-one;
17β-hydroxy-11β-[4-{7-(4,4,5,5,5-pentafluoropentylsulfonyl)heptyloxy)phenyl]-5α-androstan-3-one;
17β-hydroxy-7α-[3-{3-(3-carboxypropoxy)phenyl)propyl]-5α-androstan-3-one;
17β-hydroxy-7α-[3-{3-(4-carboxybutoxy)phenyl}propyl]-5α-androstan-3-one;
17β-hydroxy-7α-[3-[3-{3-(N-methylaminocarbonyl)propoxy}phenyl]propyl]-5α-androstan-3-one;
17β-hydroxy-7α-[3-[3-{3-(N,N-dimethylaminocarbonyl)propoxy}phenyl]propyl]-5α-androstan-3-one;
17β-hydroxy-7α-[3-[3-{3-(1-pyrrolidinylcarbonyl)propoxy}phenyl]propyl]-5α-androstan-3-one;
17β-hydroxy-7α-[3-[3-{4-(N-methylaminocarbonyl)butoxy}phenyl]propyl]-5α-androstan-3-one;
17β-hydroxy-7α-[3-[3-{4-(N,N-dimethylaminocarbonyl)butoxy}pheny]propyl]-5α-androstan-3-one;
17β-hydroxy-7α-[3-[3-{4-(1-pyrrolidinylcarbonyl)butoxy}phenyl]propyl]-5α-androstan-3-one;
17β-hydroxy-11β-[5-(aminocarbonyl)pentyloxy]-5α-androstan-3-one;
17β-hydroxy-11β-[5-(N-pentylaminocarbonyl)pentyloxy]-5α-androstan-3-one;

17β-hydroxy-11β-[7-(aminocarbonyl)heptyloxy]-5α-androstane-one;
17β-hydroxy-11β-[7-(N-pentylaminocarbonyl)heptyloxy]-5α-androstan-3-one;
17β-hydroxy-11β-[9-(aminocarbonyl)nonyloxy]-5α-androstane-one;
17β-hydroxy-11β-[9-(N-pentylaminocarbonyl)nonyloxy]-5α-androstan-3-one;
17β-hydroxy-11β-[11-(aminocarbonyl)undecyloxy]-5α-androstan-one;
17β-hydroxy-11β-[11-(N-pentylaminocarbonyl)undecyloxy]-5α-androstan-3-one;
17β-hydroxy-11β-[13-(aminocarbonyl)tridecyloxy]-5α-androstan-3-one;
17β-hydroxy-11β-[13-(N-pentylaminocarbonyl)tridecyloxy]-5α-androstan-3-one;
more preferred are the following:
17β-hydroxy-11β-{10-(4,4,5,5,5-pentafluoropentylsulfinyl)decyl}-5α-androstan-3-one;
17β-hydroxy-11β-{9-(4,4,5,5,5-pentafluoropentylsulfinyl)nonyloxy}-5α-androstan-3-one;
17β-hydroxy-11β-{11-(4,4,5,5,5-pentafluoropentylsulfinyl)undecyloxy}-5α-androstan-3-one;
17β-hydroxy-11β-{9-(4,4,5,5,5-pentafluoropentylsulfonyl)nonyloxy}-5α-androstan-3-one;
17β-hydroxy-11β-{11-(4,4,5,5,5-pentafluoropentylsulfonyl)undecyloxy}-5α-androstan-3-one;
17β-hydroxy-11β-(9-carboxy-13,13,14,14,14-pentafluorotetradecyloxy)-5α-androstan-3-one;
17β-hydroxy-11β-(9-carboxynonyloxy)-5α-androstan-3-one;
17β-hydroxy-7α-{11-(4,4,5,5,5-pentafluoropentylsulfinyl)undecyl}-5α-androstan-3-one;
17β-hydroxy-7α-{11-(4,4,5,5,5-pentafluoropentylsulfonyl)undecyl}-5α-androstan-3-one;
17β-hydroxy-7α-{5-(4,4,5,5,5-pentafluoropentylsulfinyl)pentyl}-5α-androstan-3-one;
17β-hydroxy-7α-{9-(4,4,5,5,5-pentafluoropentylsulfinyl)nonyl)-5α-androstan-3-one;
17β-hydroxy-7α-(5-carboxypentyl)-5α-androstan-3-one;
17β-hydroxy-7α-(7-carboxyheptyl)-5α-androstan-3-one;
17β-hydroxy-7α-(9-carboxynonyl)-5α-androstan-3-one;
17β-hydroxy-7α-(11-carboxyundecyl)-5α-androstan-3-one;
17β-hydroxy-7α-(13-carboxytridecyl)-5α-androstan-3-one;
17β-hydroxy-7α-(9-carboxy-13,13,14,14,14-pentafluorotetradecyl)-5α-androstan-3-one;
17β-hydroxy-11β-(9-carboxy-13,13,14,14,14-pentafluorotetradecyl)-5α-androstan-3-one;
17β-hydroxy-11β-(9-methoxycarbonyl-13,13,14,14,14-pentafluorotetradecyl)-5α-androstan-3-one;
17β-hydroxy-11β-(5-carboxypentyloxy)-5α-androstan-3-one
17β-hydroxy-11β-(7-carboxyheptyloxy)-5α-androstan-3-one;
17β-hydroxy-11β-(10-carboxydecyloxy)-5α-androstan-3-one;
17β-hydroxy-11β-(11-carboxyundecyloxy)-5α-androstan-3-one;
17β-hydroxy-11β-(13-carboxytridecyloxy)-5α-androstan-3-one;
17β-hydroxy-11β-(23-carboxytricosanyloxy)-5α-androstan-3-one;
17β-hydroxy-7α-{7-(N,N-dimethylaminocarbonyl)heptyl}-5α-androstan-3-one;
17β-hydroxy-7α-{7-(N-ethylaminocarbonyl)heptyl}-5α-androstan-3-one;
17β-hydroxy-7α-[7-{N-(cyclopropylmethyl)aminocarbonyl}heptyl]-5α-androstan-3-one;
17β-hydroxy-7α-[7-{N-(cyclohexylmethyl)aminocarbonyl}heptyl]-5α-androstan-3-one;
17β-hydroxy-7α-[7-(N-butylaminocarbonyl)heptyl]-5α-androstan-3-one;
17β-hydroxy-7α-[7-(N-(isopropylaminocarbonyl)heptyl]-5α-androstan-3-one;
17β-hydroxy-7α-[7-(N-t-butylaminocarbonyl)heptyl]-5α-androstan-3-one;
17β-hydroxy-7α-[7-(N-cyclohexylaminocarbonyl)heptyl]-5α-androstan-3-one;
17β-hydroxy-7α-[7-{N-(3-hydroxypropyl)aminocarbonyl}heptyl]-5α-androstan-3-one;
17β-hydroxy-7α-[7-(N-methyl-N-butylaminocarbonyl)heptyl]-5α-androstan-3-one;
17β-hydroxy-7α-[7-(N,N-diethylaminocarbonyl)heptyl]-5α-androstan-3-one;
17β-hydroxy-7α-[7-(piperidinocarbonyl)heptyl]-5α-androstan-3-one;
17β-hydroxy-7α-[7-{N-(4-t-butylbenzyl)aminocarbonyl}heptyl]-5α-androstan-3-one;
17β-hydroxy-7α-[7-{N-(2,2-diphenylethyl)aminocarbonyl}heptyl]-5α-androstan-3-one;
17β-hydroxy-7α-[7-{N-(2-furylmethyl)aminocarbonyl}heptyl]-5α-androstan-3-one;
17β-hydroxy-7α-(7-{7-(N-methylaminocarbonyl)heptyl]-5α-androstan-3-one;
17β-hydroxy-7α-[7-(N-methyl-N-ethylaminocarbonyl)heptyl]-5α-androstan-3-one;
17β-hydroxy-7α-[7-(N-methyl-N-propylaminocarbonyl)heptyl]-5α-androstan-3-one;
17β-hydroxy-7α-[7-(N-methyl-N-isopropylaminocarbonyl)heptyl]-5α-androstan-3-one;
17β-hydroxy-7α-[7-(N-methyl-N-benzylaminocarbonyl)heptyl]-5α-androstan-3-one;
17β-hydroxy-7α-[7-(1-pyrrolidinylcarbonyl)heptyl]-5α-androstan-3-one;
17β-hydroxy-7α-[7-(morpholinocarbonyl)heptyl]-5α-androstan-3-one;
17β-hydroxy-7α-[7-(N-methyl-N-t-butylaminocarbonyl)heptyl]-5α-androstan-3-one;
17β-hydroxy-7α-[7-(N-cyclopropylaminocarbonyl)heptyl]-5α-androstan-3-one;

17β-hydroxy-7α-[6-(N,N-dimethylaminocarbonyl)hexyl]-5α-androstan-3-one;
17β-hydroxy-7α-[6-(N,N-diethylaminocarbonyl)hexyl]-5α-androstan-3-one;
17β-hydroxy-7α-[6-(piperidinocarbonyl)hexyl]-5α-androstan-3-one;
17β-hydroxy-7α-[8-(N,N-dimethylaminocarbonyl)octyl]-5α-androstan-3-one;
17β-hydroxy-7α-[8-(N,N-diethylaminocarbonyl)octyl]-5α-androstan-3-one;
17β-hydroxy-7α-[8-(N-methyl-N-butylaminocarbonyl)octyl]-5α-androstan-3-one;
17β-hydroxy-7α-[8-(N-benzylaminocarbonyl)octyl]-5α-androstan-3-one;
17β-hydroxy-7α-[8-{N-(2-hydroxyethyl)aminocarbonyl}octyl]-5α-androstan-3-one;
17β-hydroxy-7α-[8-(piperidinocarbonyl)octyl]-5α-androstan-3-one;
17β-hydroxy-7α-[9-(N,N-dimethylaminocarbonyl)nonyl]-5α-androstan-3-one;
17β-hydroxy-7α-[9-(N,N-diethylaminocarbonyl)nonyl]-5α-androstan-3-one;
17β-hydroxy-7α-[9-(1-pyrrolidinylcarbonyl)nonyl]-5α-androstan-3-one;
17β-hydroxy-7α-[9-(N-methyl-N-ethylaminocarbonyl)nonyl]-5α-androstan-3-one;
17β-hydroxy-7α-[9-(N-methyl-N-butylaminocarbonyl)nonyl]-5α-androstan-3-one;
17β-hydroxy-7α-[9-(N-benzylaminocarbonyl)nonyl]-5α-androstan-3-one;
17β-hydroxy-7α-[9-(piperidinocarbonyl)nonyl]-5α-androstan-3-one;
17β-hydroxy-7α-[9-{N-(2-hydroxyethyl)aminocarbonyl)nonyl]-5α-androstan-3-one;
17β-hydroxy-7α-[9-(N-methyl-N-propylaminocarbonyl)nonyl]-5α-androstan-3-one;
17β-hydroxy-7α-[9-(morpholinocarbonyl)nonyl]-5α-androstan-3-one;
17β-hydroxy-7α-[10-(N,N-dimethylaminocarbonyl)decyl]-5α-androstan-3-one;
17β-hydroxy-7α-[10-(N,N-diethylaminocarbonyl)decyl]-5α-androstan-3-one;
17β-hydroxy-7α-[10-(N-methyl-N-ethylaminocarbonyl)decyl]-5α-androstan-3-one;
17β-hydroxy-7α-[10-N-methyl-N-propylaminocarbonyl)decyl]-5α-androstan-3-one;
17β-hydroxy-7α-[10-(N-methyl-N-butylaminocarbonyl)decyl]-5α-androstan-3-one;
17β-hydroxy-7α-[10-(morpholinocarbonyl)decyl]-5α-androstan-3-one;
17β-hydroxy-7α-[11-(N,N-dimethylaminocarbonyl)undecyl]-5α-androstan-3-one;
17β-hydroxy-7α-[11-(N,N-diethylaminocarbonyl)undecyl]-5α-androstan-3-one;
17β-hydroxy-7α-[11-(piperidinocarbonyl)undecyl]-5α-androstan-3-one;
17β-hydroxy-7α-[11-(N-benzylaminocarbonyl)undecyl]-5α-androstan-3-one;
17β-hydroxy-7α-[11-(N-methyl-N-butylaminocarbonyl)undecyl]-5α-androstan-3-one;
17β-hydroxy-7α-[11-(N-(2-hydroxyethyl)aminocarbonyl}undecyl]-5α-androstan-3-one;
17β-hydroxy-7α-[7-{N-(2-hydroxyethyl)aminocarbonyl)heptyl]-5α-androstan-3-one;
17β-hydroxy-7α-[7-(N-propylaminocarbonyl)heptyl]-5α-androstan-3-one;
17β-hydroxy-7α-[7-(N-hexylaminocarbonyl)heptyl]-5α-androstan-3-one;
17β-hydroxy-7α-[7-(N-isopentylaminocarbonyl)heptyl]-5α-androstan-3-one;
17β-hydroxy-7α-[7-(N-isobutylaminocarbonyl)heptyl]-5α-androstan-3-one;
17β-hydroxy-7α-[7-(N-neopentylaminocarbonyl)heptyl]-5α-androstan-3-one;
17β-hydroxy-7α-[7-{N-(3-pentyl)aminocarbonyl}heptyl]-5α-androstan-3-one;
17β-hydroxy-7α-[7-(N,N-dihexylaminocarbonyl)heptyl]-5α-androstan-3-one;
17β-hydroxy-7α-[7-(N-phenylaminocarbonyl)heptyl]-5α-androstan-3-one;
17β-hydroxy-7α-[7-(N-benzylaminocarbonyl)heptyl]-5α-androstan-3-one;
17β-hydroxy-7α-[7-{N-(2-phenylethyl)aminocarbonyl}heptyl]-5α-androstan-3-one;
17β-hydroxy-11β-(7-carboxyheptyl)-5α-androstan-3-one;
17β-hydroxy-11β-(8-carboxyoctyl)-5α-androstan-3-one;
17β-hydroxy-11β-(9-carboxynonyl)-5α-androstan-3-one;
17β-hydroxy-11β-(11-carboxyundecyl)-5α-androstan-3-one;
17β-hydroxy-7α-[5-(aminocarbonyl)pentyl]-5α-androstan-3-one;
17β-hydroxy-7α-[5-(N-pentylaminocarbonyl)pentyl]-5α-androstan-3-one;
17β-hydroxy-7α-[7-(aminocarbonyl)heptyl]-5α-androstan-3-one;
17β-hydroxy-7α-[7-(N-pentylaminocarbonyl)heptyl]-5α-androstan-3-one;
17β-hydroxy-7α-[9-(aminocarbonyl)nonyl]-5α-androstan-3-one;
17β-hydroxy-11β-[9-(aminocarbonyl)nonyl]-5α-androstan-3-one;
17β-hydroxy-7α-[9-(N-pentylaminocarbonyl)nonyl]-5α-androstan-3-one;
17β-hydroxy-11β-[9-(N-pentylaminocarbonyl)nonyl]-5α-androstan-3-one;
17β-hydroxy-7α-[11-(aminocarbonyl)undecyl]-5α-androstan-3-one;
17β-hydroxy-11β-[11-(aminocarbonyl)undecyl]-5α-androstan-3-one;
17β-hydroxy-7α-[11-(N-pentylaminocarbonyl)undecyl]-5α-androstan-3-one;
17β-hydroxy-11β-[11-(N-pentylaminocarbonyl)undecyl]-5α-androstan-3-one;

17β-hydroxy-7α-[13-(aminocarbonyl)tridecyl]-5α-androstan-3-one;
17β-hydroxy-7α-[13-(N-pentylaminocarbonyl)tridecyl]-5α-androstan-3-one;
17β-hydroxy-11β-(7-(N,N-dimethylaminocarbonyl)heptyl}-5α-androstan-3-one;
17β-hydroxy-11β-[7-{7-(N-methylaminocarbonyl)heptyl]-5α-androstan-3-one;
17β-hydroxy-11β-[7-(N-methyl-N-ethylaminocarbonyl)heptyl]-5α-androstan-3-one;
17β-hydroxy-11β-[7-(N-methyl-N-propylaminocarbonyl)heptyl]-5α-androstan-3-one;
17β-hydroxy-11β-[7-(morpholinocarbonyl)heptyl]-5α-androstan-3-one;
17β-hydroxy-11β-[8-(N,N-dimethylaminocarbonyl)octyl]-5α-androstan-3-one;
17β-hydroxy-11β-[8-(N-methylaminocarbonyl)octyl]-5α-androstan-3-one;
17β-hydroxy-11β-[8-(N-methyl-N-ethylaminocarbonyl)octyl]-5α-androstan-3-one;
17β-hydroxy-11β-[8-(N-methyl-N-propylaminocarbonyl)octyl]-5α-androstan-3-one;
17β-hydroxy-11β-[8-(morpholinocarbonyl)octyl]-5α-androstan-3-one;
17β-hydroxy-11β-[9-(N,N-dimethylaminocarbonyl)nonyl]-5α-androstan-3-one;
17β-hydroxy-11β-[9-(N,N-diethylaminocarbonyl)nonyl]-5α-androstan-3-one;
17β-hydroxy-11β-[9-(N-methyl-N-butylaminocarbonyl)nonyl]-5α-androstan-3-one;
17β-hydroxy-11β-[9-(N-benzylaminocarbonyl)nonyl]-5α-androstan-3-one;
17β-hydroxy-11β-[9-(piperidinocarbonyl)nonyl]-5α-androstan-3-one;
17β-hydroxy-11β-[9-{N-(2-hydroxyethyl)aminocarbonyl}nonyl]-5α-androstan-3-one;
17β-hydroxy-11β-[10-(4,4,5,5,5-pentafluoropentylsulfanyl)decyl]-5α-androstan-3-one;
17β-hydroxy-7α-(7-hydroxyheptyl)-5α-androstan-3-one;
17β-hydroxy-7α-(8-hydroxyoctyl)-5α-androstan-3-one;
17β-hydroxy-7α-(9-hydroxynonyl)-5α-androstan-3-one;
17β-hydroxy-7α-[7-(4,4,5,5,5-pentafluoropentylsulfinyl)heptyl]-5α-androstan-3-one;
17β-hydroxy-7α-[13-(4,4,5,5,5-pentafluoropentylsulfinyl)tridecyl]-5α-androstan-3-one;
17β-hydroxy-7α-[7-(4,4,5,5,5-pentafluoropentylsulfonyl)heptyl]-5α-androstan-3-one;
17β-hydroxy-7α-[9-(4,4,5,5,5-pentafluoropentylsulfonyl)nonyl]-5α-androstan-3-one;
17β-hydroxy-7α-[13-(4,4,5,5,5-pentafluoropentylsulfonyl)tridecyl]-5α-androstan-3-one;
17β-hydroxy-7α-[4-(carboxymethoxy)phenyl]-5α-androstan-3-one;
17β-hydroxy-7α-[4-(3-carboxypropoxy)phenyl]-5α-androstan-3-one;
17β-hydroxy-11β-[4-(3-carboxypropoxy)phenyl]-5α-androstan-3-one;
17β-hydroxy-7α-[4-(7-carboxyheptyloxy)phenyl]-5α-androstan-3-one;
17β-hydroxy-11β-[4-(7-carboxyheptyloxy)phenyl]-5α-androstan-3-one;
17β-hydroxy-7α-[4-(carbamoylmethoxy)phenyl]-5α-androstan-3-one;
17β-hydroxy-7α-[4-(carbamoylmethoxy)phenyl]-5α-androstan-3-one;
17β-hydroxy-7α-[4-(3-carbamoylpropoxy)phenyl]-5α-androstan-3-one;
17β-hydroxy-11β-[4-(3-carbamoylpropoxy)phenyl]-5α-androstan-3-one;
17β-hydroxy-7α-[4-(7-carbamoylheptyloxy)phenyl]-5α-androstan-3-one;
17β-hydroxy-11β-[4-(7-carbamoylheptyloxy)phenyl]-5α-androstan-3-one;
17β-hydroxy-7α-[4-(3-N-pentylcarbamoylpropoxy)phenyl]-5α-androstan-3-one;
17β-hydroxy-11β-[4-(3-N-pentylcarbamoylpropoxy)phenyl]-5α-androstan-3-one;
17β-hydroxy-7α-[4-(7-N-pentylcarbamoylheptyloxy)phenyl]-5α-androstan-3-one;
17β-hydroxy-11β-[4-(7-N-pentylcarbamoylheptyloxy)phenyl]-5α-androstan-3-one;
17β-hydroxy-7α-[4-methoxyphenyl]-5α-androstan-3-one;
17β-hydroxy-11β-[4-methoxyphenyl]-5α-androstan-3-one;
17β-hydroxy-11β-[5-(4,4,5,5,5-pentafluoropentylsulfinyl)pentyloxy]-5α-androstan-3-one;
17β-hydroxy-11β-[7-(4,4,5,5,5-pentafluoropentylsulfinyl)heptyloxy]-5α-androstan-3-one;
17β-hydroxy-11β-[13-(4,4,5,5,5-pentafluoropentylsulfinyl)tridecyloxy]-5α-androstan-3-one;
17β-hydroxy-11β-[7-(4,4,5,5,5-pentafluoropentylsulfonyl)heptyloxy]-5α-androstan-3-one;
17β-hydroxy-11β-[13-(4,4,5,5,5-pentafluoropentylsulfonyl)tridecyloxy]-5α-androstan-3-one;
17β-hydroxy-11β-[4-{5-(4,4,5,5,5-pentafluoropentylsulfinyl)pentyloxy}phenyl]-5α-androstan-3-one;
17β-hydroxy-11β-[4-{7-(4,4,5,5,5-pentafluoropentylsulfinyl)heptyloxy}phenyl]-5α-androstan-3-one;
17β-hydroxy-11β-[4-{5-(4,4,5,5,5-pentafluoropentylsulfonyl)pentyloxy}phenyl]-5α-androstan-3-one;
17β-hydroxy-11β-[4-{7-(4,4,5,5,5-pentafluoropentylsulfonyl)heptyloxy}phenyl]-5α-androstan-3-one;
17β-hydroxy-7α-[3-{3-(3-carboxypropoxy)phenyl}propyl]-5α-androstan-3-one;
17β-hydroxy-7α-[3-{3-(4-carboxybutoxy)phenyl}propyl]-5α-androstan-3-one;
17β-hydroxy-7α-[3-[3-{3-(N-methylaminocarbonyl)propoxy}phenyl]propyl]-5α-androstan-3-one;
17β-hydroxy-7α-[3-[3-{3-(N,N-dimethylaminocarbonyl)propoxy}phenyl]propyl]-5α-androstan-3-one;
17β-hydroxy-7α-[3-[3-{3-(1-pyrrolidinylcarbonyl)propoxy}phenyl]propyl]-5α-androstan-3-one;

17β-hydroxy-7α-[3-[3-{4-(N-methylaminocarbonyl)butoxy}phenyl]propyl]-5α-androstan-3-one;
17β-hydroxy-7α-[3-[3-{4-(N,N-dimethylaminocarbonyl)butoxy}phenyl]propyl]-5α-androstan-3-one;
17β-hydroxy-7α-[3-[3-{4-(1-pyrrolidinylcarbonyl)butoxy}phenyl]propyl]-5α-androstan-3-one;
17β-hydroxy-11β-[5-(aminocarbonyl)pentyloxy]-5α-androstan-3-one;
17β-hydroxy-11β-[5-(N-pentylaminocarbonyl)pentyloxy]-5α-androstan-3-one;
17β-hydroxy-11β-[7-(aminocarbonyl)heptyloxy]-5α-androstan-3-one;
17β-hydroxy-11β-[7-(N-pentylaminocarbonyl)heptyloxy]-5α-androstan-3-one;
17β-hydroxy-11β-[9-(aminocarbonyl)nonyloxy]-5α-androstan-3-one;
17β-hydroxy-11β-[9-(N-pentylaminocarbonyl)nonyloxy]-5α-androstan-3-one;
17β-hydroxy-11β-[11-(aminocarbonyl)undecyloxy]-5α-androstan-3-one;
17β-hydroxy-11β-[11-(N-pentylaminocarbonyl)undecyloxy]-5α-androstan-3-one;
17β-hydroxy-11β-[13-(aminocarbonyl)tridecyloxy]-5α-androstan-3-one;
17β-hydroxy-11β-[13-(N-pentylaminocarbonyl)tridecyloxyl-5α-androstan-3-one;
particularly preferred are the following:
17β-hydroxy-7α-{7-(N,N-dimethylaminocarbonyl)heptyl}-5α-androstan-3-one;
17β-hydroxy-7α-{7-(N-ethylaminocarbonyl)heptyl}-5α-androstan-3-one;
17β-hydroxy-7α-[7-(N-(isopropylaminocarbonyl)heptyl]-5α-androstan-3-one;
17β-hydroxy-7α-[7-(N-methyl-N-butylaminocarbonyl)heptyl]-5α-androstan-3-one;
17β-hydroxy-7α-[7-(N,N-diethylaminocarbonyl)heptyl]-5α-androstan-3-one;
17β-hydroxy-7α-[7-(piperidinocarbonyl)heptyl]-5α-androstan-3-one;
17β-hydroxy-7α-[7-{N-(2-furylmethyl)aminocarbonyl}heptyl]-5α-androstan-3-one;
17β-hydroxy-7α-[7-{7-(N-methylaminocarbonyl)heptyl]-5α-androstan-3-one;
17β-hydroxy-7α-[7-(N-methyl-N-ethylaminocarbonyl)heptyl]-5α-androstan-3-one;
17β-hydroxy-7α-[7-(N-methyl-N-propylaminocarbonyl)heptyl]-5α-androstan-3-one;
17β-hydroxy-7α-[7-(N-methyl-N-isopropylaminocarbonyl)heptyl]-5α-androstan-3-one;
17β-hydroxy-7α-[7-(N-methyl-N-benzylaminocarbonyl)heptyl]-5α-androstan-3-one;
17β-hydroxy-7α-[7-(1-pyrrolidinylcarbonyl)heptyl]-5α-androstan-3-one;
17β-hydroxy-7α-[7-(morpholinocarbonyl)heptyl]-5α-androstan-3-one;
17β-hydroxy-7α-[9-(N,N-dimethylaminocarbonyl)nonyl]-5α-androstan-3-one;
17β-hydroxy-7α-[9-(N,N-diethylaminocarbonyl)nonyl]-5α-androstan-3-one;
17β-hydroxy-7α-[9-(N-methyl-N-butylaminocarbonyl)nonyl]-5α-androstan-3-one;
17β-hydroxy-7α-[9-(N-methyl-N-propylaminocarbonyl)nonyl]-5α-androstan-3-one;
17β-hydroxy-7α-[9-(morpholinocarbonyl)nonyl]-5α-androstan-3-one;
17β-hydroxy-7α-[10-(N,N-dimethylaminocarbonyl)decyl]-5α-androstan-3-one;
17β-hydroxy-7α-[7-{N-(2-hydroxyethyl)aminocarbonyl}heptyl]-5α-androstan-3-one;
17β-hydroxy-7α-[7-(N-propylaminocarbonyl)heptyl]-5α-androstan-3-one;
17β-hydroxy-7α-[7-(N-benzylaminocarbonyl)heptyl]-5α-androstan-3-one;
17β-hydroxy-7α-[7-{N-(2-phenylethyl)aminocarbonyl}heptyl]-5α-androstan-3-one;
17β-hydroxy-11β-[9-(N,N-diethylaminocarbonyl)nonyl]-5α-androstan-3-one;
17β-hydroxy-7α-[3-[3-{3-(N-methylaminocarbonyl)propoxy}phenyl]propyl]-5α-androstan-3-one;
17β-hydroxy-7α-[3-[3-{3-(N,N-dimethylaminocarbonyl)propoxy}phenyl]propyl]-5α-androstan-3-one;
17β-hydroxy-7α-[3-[3-{4-(1-pyrrolidinylcarbonyl)butoxy}phenyl]propyl]-5α-androstan-3-one.

The structures of these compounds are shown below:

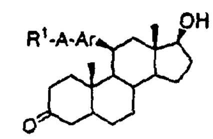

}-ArA-R[1];

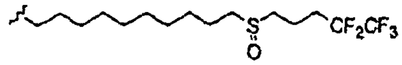
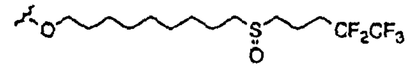
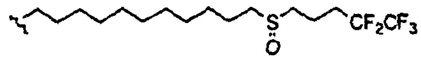
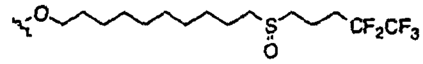
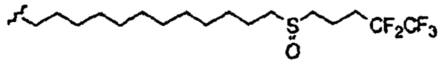
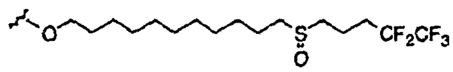
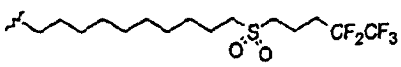
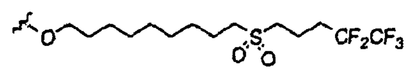
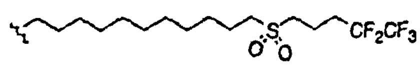
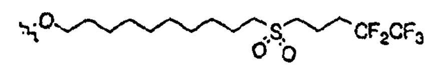
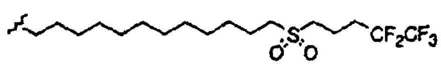
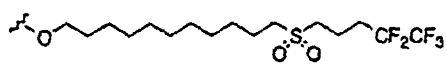
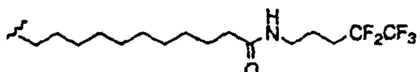
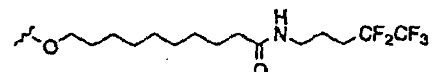
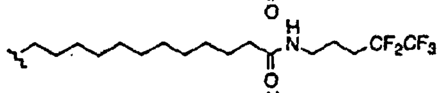
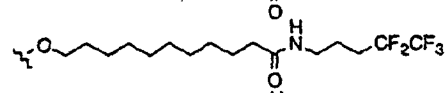
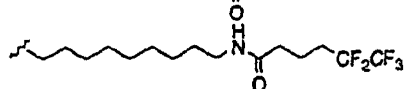
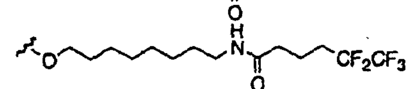
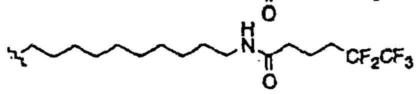
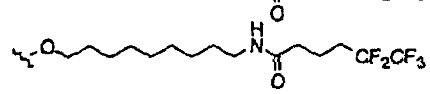
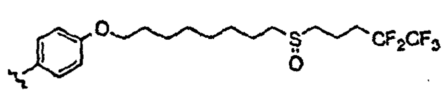
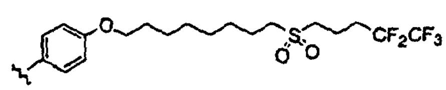
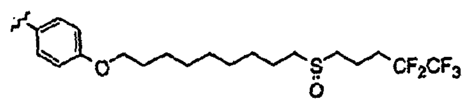
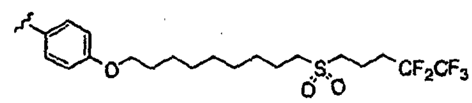
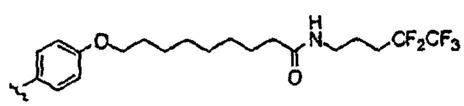
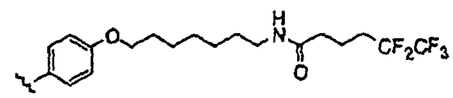
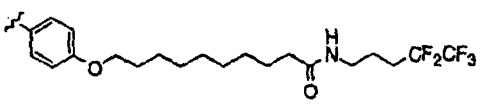
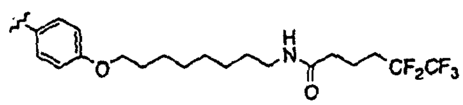
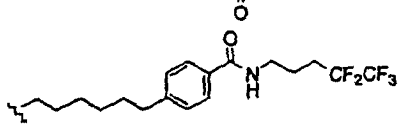
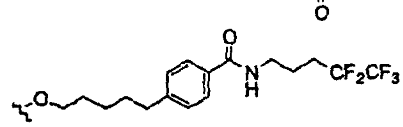
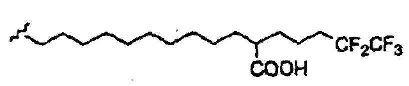
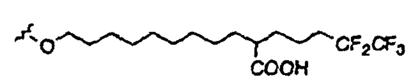
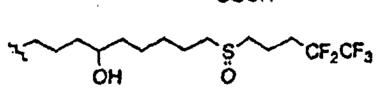
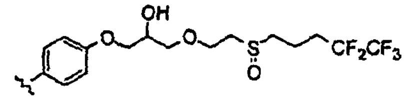
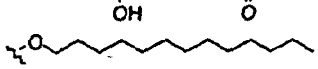

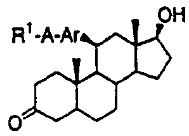

}-ArA-R$^1$;

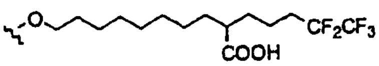

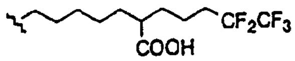

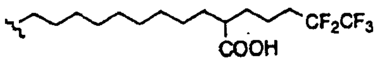

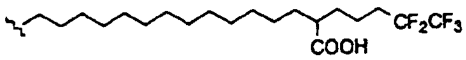

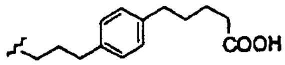

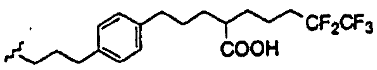

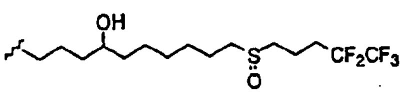

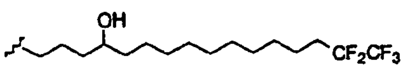

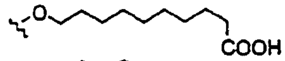

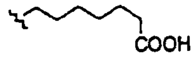

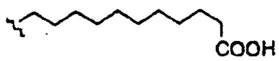

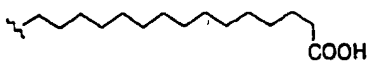

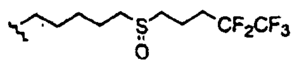

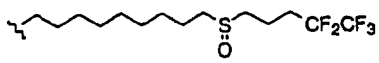

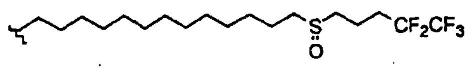

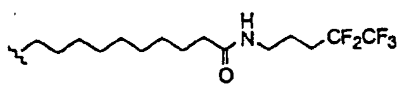

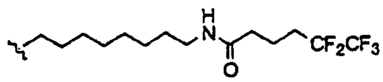

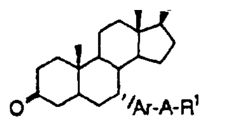

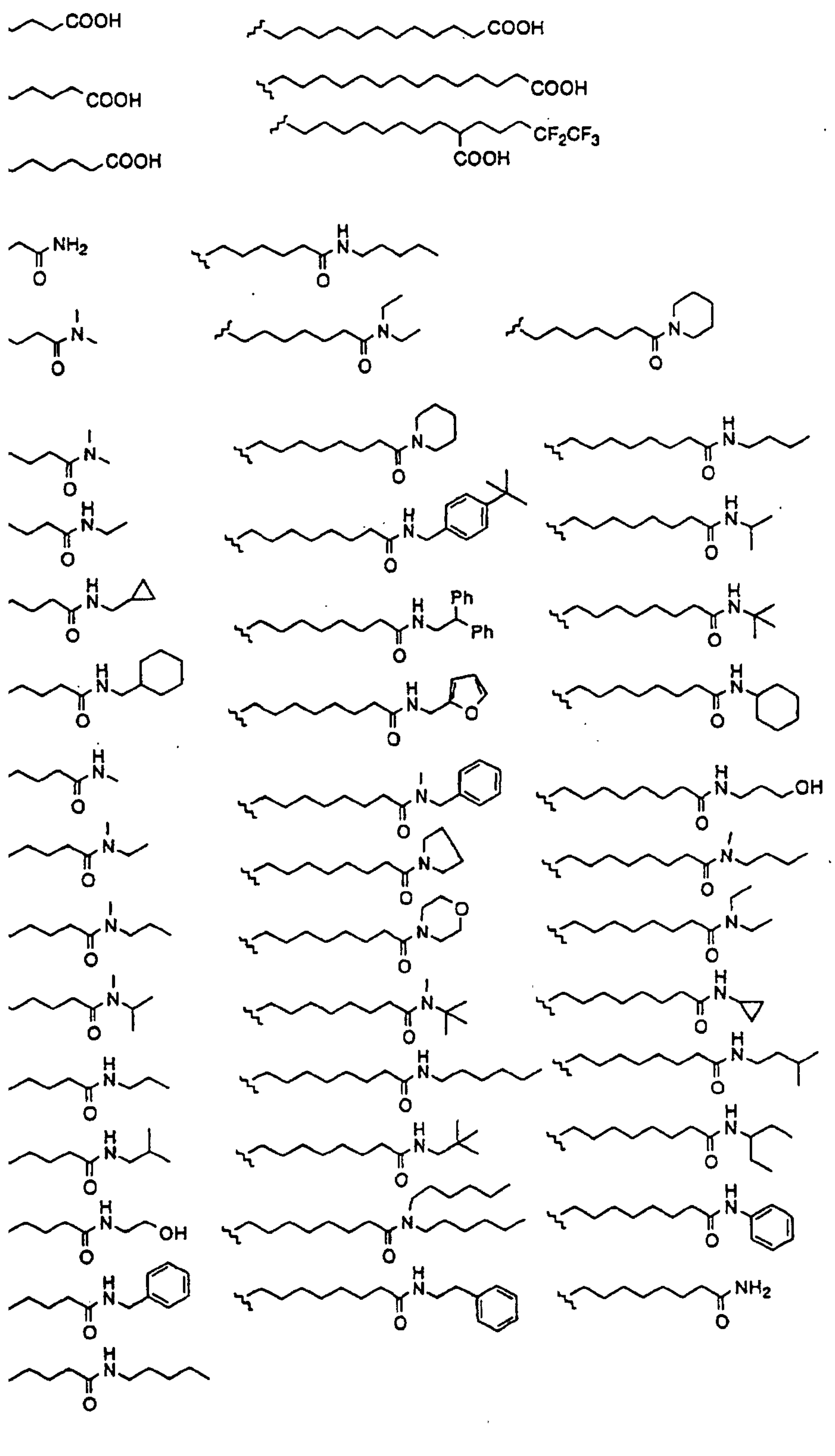

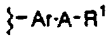 ; 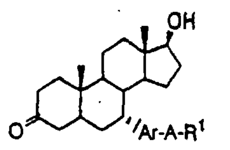

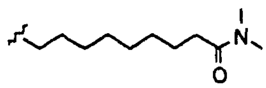 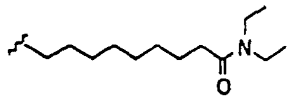 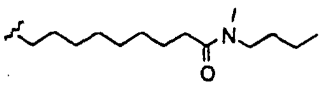

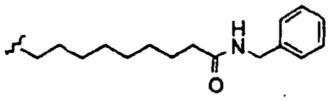 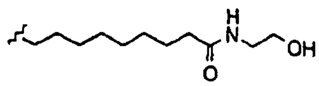 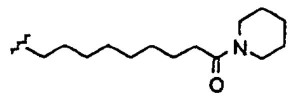

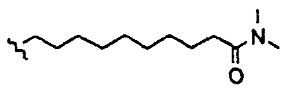 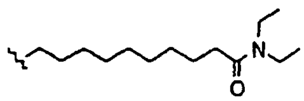 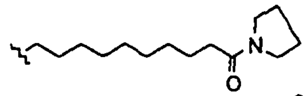

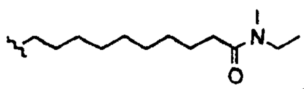 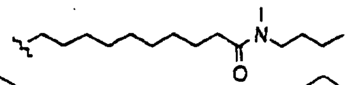 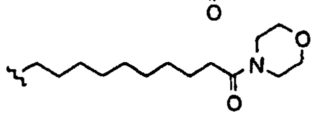

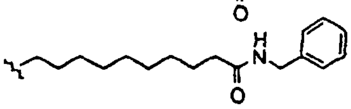 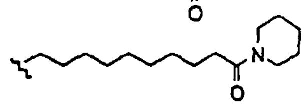 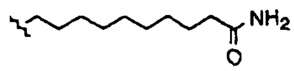

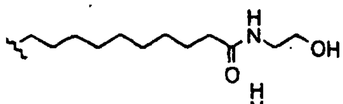 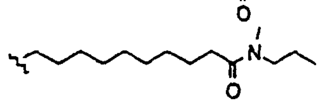

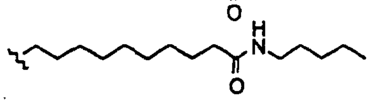

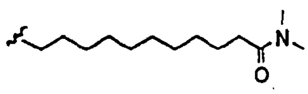 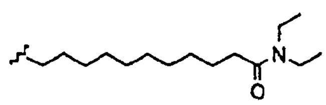

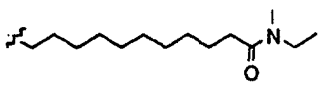 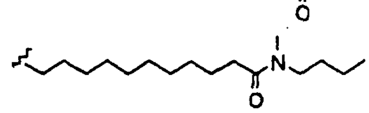

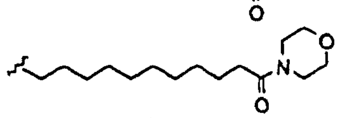 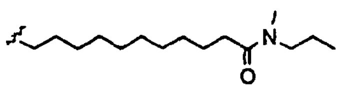

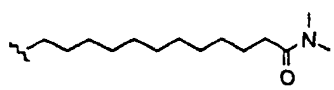 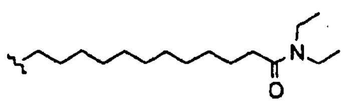

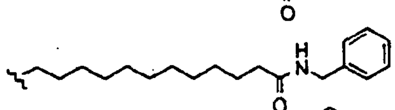 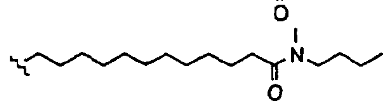

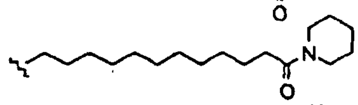 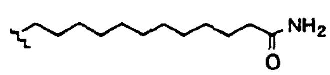

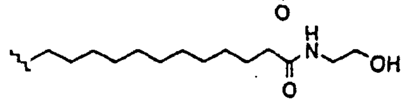 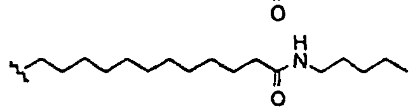

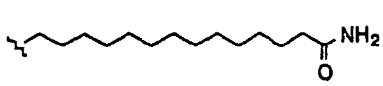 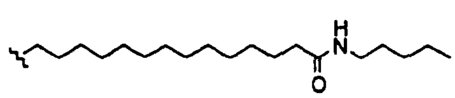

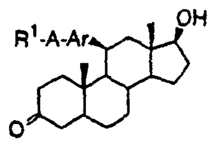

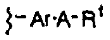 ;

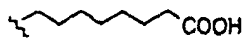

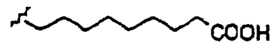

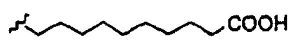

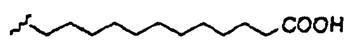

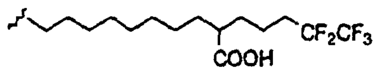

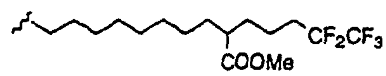

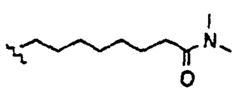

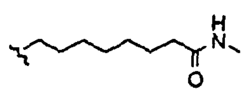

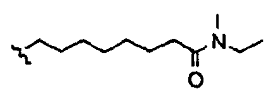

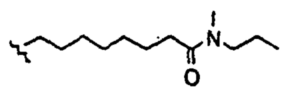

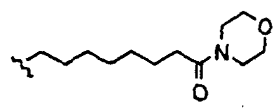

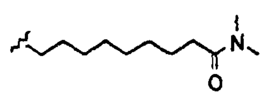

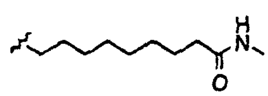

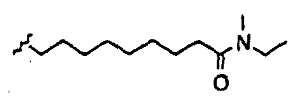

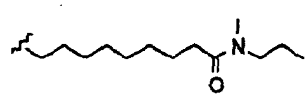

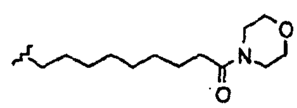

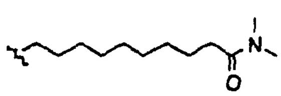

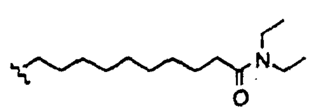

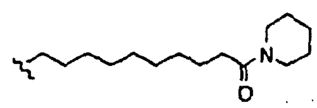

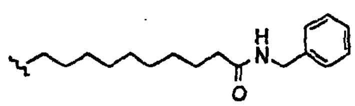

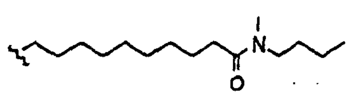

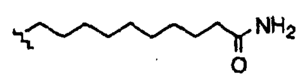

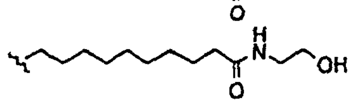

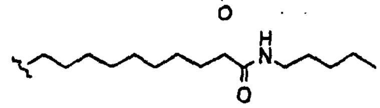

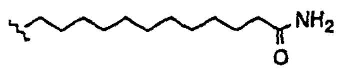

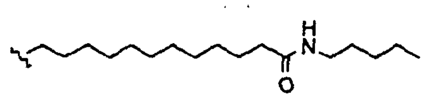

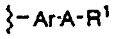 ;

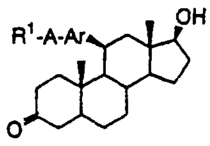

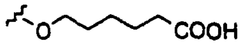

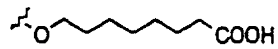

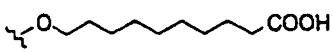

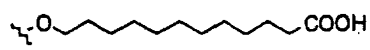

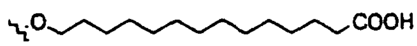

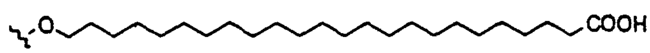

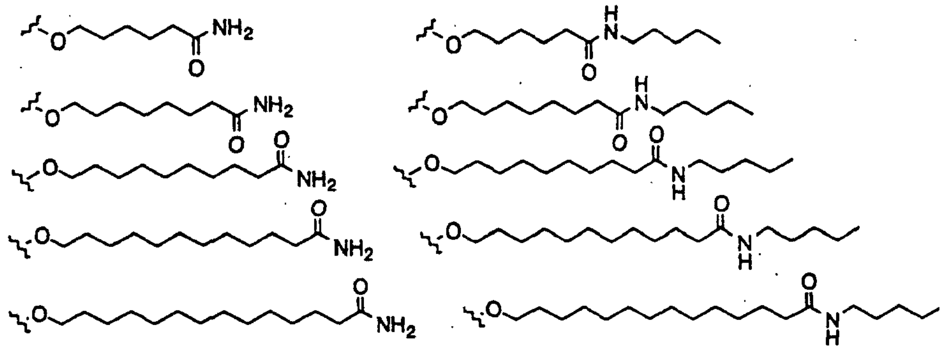

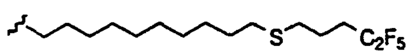

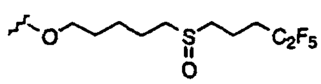

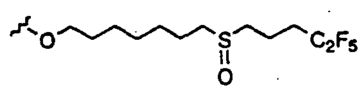

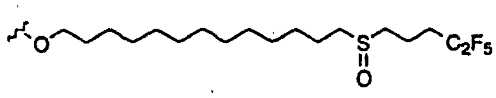

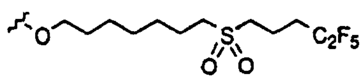

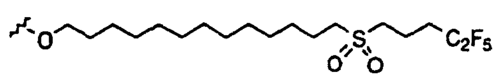

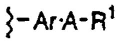

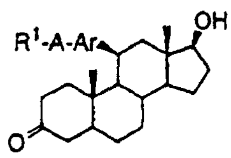

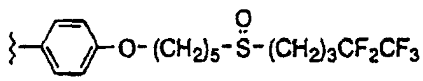

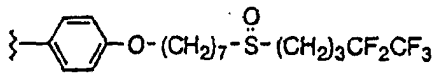

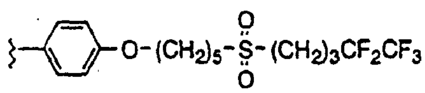

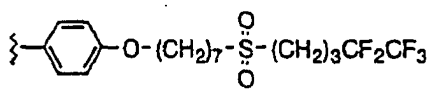,

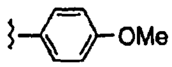

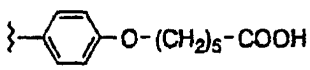

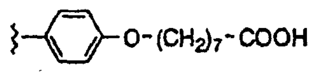

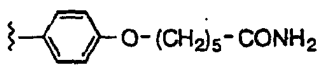

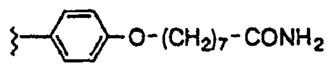

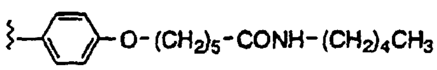

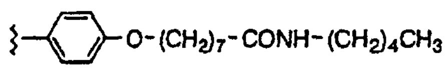

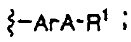 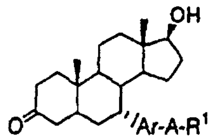

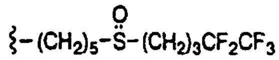 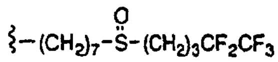 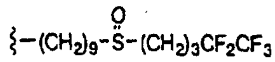

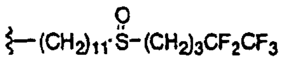 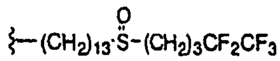

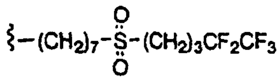 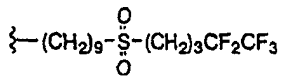 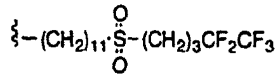

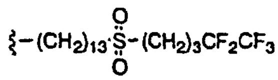

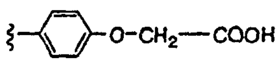 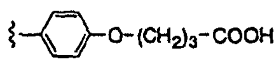 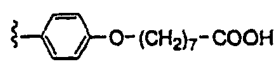

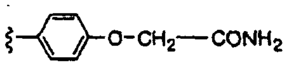 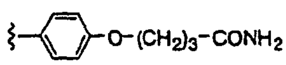 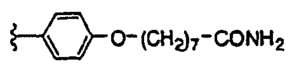

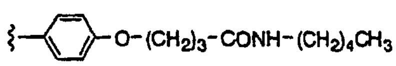 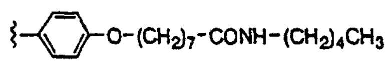

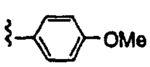 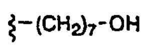 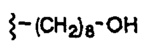 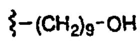

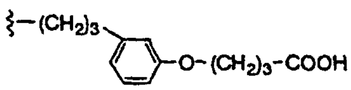 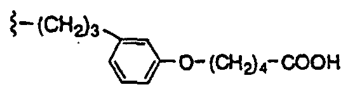

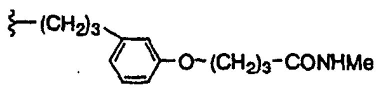 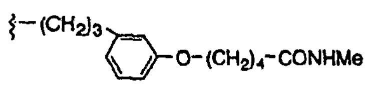

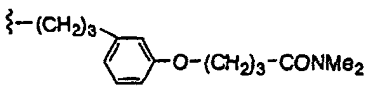 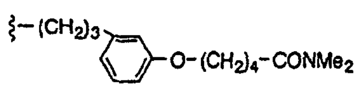

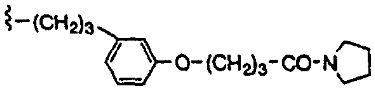 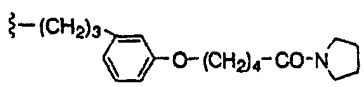

If the compounds represented by the general formula (I) contain one or more asymmetric carbon atom in their molecule, those compounds which have R and S configurations as absolute configuration for each of the contained asymmetric carbon atoms, as well as all mixtures of those compounds at any proportions are included within the scope of the invention.

Speaking of the substance of the invention which acts as antagonist against but not as agonist for the androgen receptor, the expression "not acting as agonist" means that in the following androgen receptor gene assay, the transcriptional activity value of the substance at any concentration of 0.1 nmol/L - 10 μmol/L is from one to five times the

transcriptional activity value for no addition of the substance which is taken as unity:

Twenty-four hours before transfection, 1.0 x $10^5$ HeLa cells (purchased from Dainippon Pharmaceutical Co., Ltd.) are cultured in phenol red free Dulbecco's modified Eagle medium (DMEM) containing 5% of charcoal-treated FBS (DCC-FBS) in 12-well microplates. Five hundred nanograms/well of MMTV-Luc vector (the reporter plasmid of luciferase having mouse tumor long terminal repeats containing the androgen response element: GM-CAT vector (A. T.C.C. No. 67282) purchased from A.T.C.C. provided that the chloramphenicol acetyl transferase gene was replaced by the firefly luciferase gene), 100 ng/well of pSG5-hAR (the expression vector of the human androgen receptor which harbors the androgen receptor gene under the control of SV40 promoter) and 5 ng/well of Renilla Luc vector (a vector for internal standard incorporating the sea pansy luciferase gene) are transfected into the HeLa cells. The transfection is performed in a liquid culture of the phenol red free DMEM using 3 mL/well of lipofectoamine (GibcoBRL). Nine hours after the transfection, the liquid culture is replaced by phenol red free DMEM/3% DCC-FBS containing 10 μmol/L of a compound of the invention which is represented by the general formula (I) or the substance of the invention which acts as antagonist against but not as agonist for the androgen receptor. The transcriptional activity value is measured 48 hours after the replacement of the liquid culture. Transcriptional activity is measured with a dual-luciferase reporter assay system (Promega). The transcriptional activity value is defined as the value for firefly luciferase divided by the value for sea pansy luciferase. To implement this assay, reference may be had to J. Biol. Chem., vol. 270, pp. 19998-20003, 1995.

WO97/49709 mentions hydroxyflutamide (the essence of the in vivo activity of flutamide) and bicaltamide as substances that act as antagonist against but not as agonist for the androgen receptor; however, according to the definition given in that publication, the expression "not acting as agonist" means that in an androgen reporter gene assay using CV-1 cells, the agonist efficiency value represented by the following formula is 0 - 20% at a concentration of 10 μmol/L or above and this definition is clearly and strictly distinguished from the definition of the expression "not acting as agonist" which is given in the present invention:

Agonist efficiency (%) = (transcriptional activity

value of screened non-steroid compound)/(maximum

transcriptional activity value by DHT) x 100

In the androgen receptor reporter gene assay used in defining the expression "not acting as agonist" in the invention, each of hydroxyflutamide and bicaltamide was found to act as agonist at a concentration of 10 μmol/L (see Example 1 in this specification).

The expression "acting as antagonist" means that in the following androgen receptor gene assay, the transcriptional activity value of 0.1 nmol/L of dihydrotestosterone (DHT) is inhibited to 0 - 50% at any concentration of 0.1 nmol/L - 10 μmol/L:

Twenty-four hours before transfection, 1.0 x $10^5$ HeLa cells are cultured in phenol red free DMEM/5% DCC-FBS on 12-well microplates. Five hundred nanograms/well of MMTV-Luc vector, 100 ng/well of pSG5/hAR and 5 ng/well of Renilla Luc vector are transfected into the HeLa cells. The transfection is performed in a liquid culture of the phenol red free DMEM using 3 mL/well of lipofectoamine. Nine hours after the transfection, the liquid culture is replaced by phenol red free DMEM/3% DCC-FBS containing 0.1 nmol/L of DHT and 1.0 mol/L of a compound of the invention which is represented by the general formula (I) or the substance of the invention which acts as antagonist against but not as agonist for the androgen receptor. The transcriptional activity value is measured 48 hours after the replacement of the liquid culture. Transcriptional activity is measured with a dual-luciferase reporter assay system. The transcriptional activity value is defined as the value for firefly luciferase divided by the value for sea pansy luciferase. To implement this assay, reference may be had to J. Biol. Chem., vol. 270, pp. 19998-20003, 1995.

Specific examples of the substance of the invention which acts as antagonist against but not as agonist for the androgen receptor may include compounds of the invention which are represented by the general formula (I).

The compounds of the invention which are represented by the general formula (I) and the substance of the invention which acts as antagonist against but not as agonist for the androgen receptor can also be obtained as their pharmaceutically acceptable salts. Pharmaceutically acceptable salts include inorganic acid salts such as hydrochlorides, hyrobromides, hydroiodides, sulfates and phosphates; organic acid salts such as formates, acetates, oxalates, maleates, fumarates, methanesulfonates, benzenesulfonates, p-toluenesulfonates, succinates, malonates, citrates, gluconates, mandelates, benzoates, salicylates, trifluoroacetates, tartrates, propionates and glutarates; inorganic base salts such as sodium salts, potassium salts, magnesium salts and zinc salts; and organic base salts such as ammonium salts.

The compounds of the invention which are represented by the general formula (I), their pharmaceutically

acceptable salts, as well as the substance of the invention which acts as antagonist against but not as agonist for the androgen receptor, and its pharmaceutically acceptable salts can also be obtained as their prodrugs. The prodrugs mean those compounds which undergo rapid transformation in living body to generate, typically by hydrolysis in the blood, the compounds of the invention which are represented by the general formula (I), their pharmaceutically acceptable salts, as well as the substance of the invention which acts as antagonist against but not as agonist for the androgen receptor, and its pharmaceutically acceptable salts. T. Higuchi and V. Stella give detailed accounts of the concept of prodrugs in "Prodrugs as Novel Delivery Systems", vol. 14 of the A.C.S. Symposium Series, American Chemical Society (1975). These prodrugs may or may not have activity on their own but they usually have little activity. Reference may also be had to D.E.V. Wilman, "Prodrugs in Cancer Chemotherapy" in Biochemical Society Transactions, vol. 14, pp. 375-382, the 615th Meeting, Belfast, 1986, and V.J. Stella et al., "Prodrugs: Chemical Methods for Targeted Drug Delivery" in Directed Drug Delivery, ed. by R. Borchardt et al., pp. 247-267, Humana Press, 1985. If compounds of the invention which are represented by the general formula (I) have the -COOH partial structure, specific examples of prodrugs include esters, carbonates, carbamates, etc. of such compounds.

The compounds of the invention which are represented by the general formula (I) can typically be produced by process A to process W, process B' to process L', process S' to process W', process U", process W" and process W' " that are set forth below, or depending on the end compound, partial modifications of process A to process W, process B' to process L', process S' to process W', process U", process W" and process W' " may be employed.

In the chemical formulae listed in process A to process W, process B' to process L', process S' to process W', process U", process W" and process W' ", $R^2$ represents the general formula (IV)

$$-G^2\text{-E-J-Y-L-}Q^2\text{-Z} \qquad \text{(IV)}$$

(wherein $G^2$ represents a single bond, a straight-chained or branched alkylene group having 1 - 26 carbon atoms, a straight-chained or branched alkenylene group having 2 - 26 carbon atoms or a straight-chained or branched alkynylene group having 2 - 26 carbon atoms; E, J, Y, L, $Q^2$ and Z have the same meanings as defined above, provided that $R^7$ and $R^8$ in $Q^2$ are preferably a hydrogen atom); $R^3$ represents a substituted silyl group, preferably t-butyldimethylsilyl group; $X^3$ represents a halogen atom or a substituted sulfonate group, preferably p-toluenesulfonate group or methanesulfonate group; $R^{12}$ represents a straight-chained or branched alkyl group having 1 - 6 carbon atoms, preferably methyl group and ethyl group; $R^e$ and $R^f$, when taken together with the carbon atoms in 3- and 17-positions to which they are bound, represent protected -(C=O)-, preferably 1,3-dioxane, 1,3-dioxolan, 1,3-dithian, etc., particularly preferably 1,3-dioxolan, etc.; $L^2$ represents a straight-chained or branched lower alkylene group having 1 - 10 carbon atoms, preferably ethane-1,2-diyl group, propane-1,3-diyl group and butane-1,4-diyl group.

$R^4$ represents the general formula (V)

$$-G^3\text{-E-J-Y-L-}Q^2\text{-Z} \qquad \text{(V)}$$

(wherein $G^3$ represents a straight-chained or branched alkylene group having 1 - 27 carbon atoms, a straight-chained or branched alkenylene group having 2 - 27 carbon atoms or a straight-chained or branched alkynylene group having 2 - 27 carbon atoms; E, J, Y, L, $Q^2$ and Z have the same meanings as defined above); $R^5$ represents a halogen atom, preferably a bromine atom or an iodine atom; $R^6$ represents a substituted silyl group, preferably trimethylsilyl group; $R^{13}$ represents a straight-chained or branched alkyl group having 1 - 6 carbon atoms that may optionally be substituted by a halogen atom, preferably trifluoromethyl group or 1,1,2,2,3,3,4,4,4-nonafluorobutyl group; $R^{14}$ represents a group represented by $-MgR^5$, $-ZnR^5$ or $-Sn(R^7)_3$, preferably a group represented by $-Sn(R^7)_3$; $G^4$ represents a straight-chained or branched alkylene group having 1 - 30 carbon atoms, a straight-chained or branched alkenylene group having 2 - 30 carbon atoms or a straight-chained or branched alkynylene group having 2 - 30 carbon atoms; the wavy line represents a single bond of trans configuration or cis configuration, preferably trans configuration, wih respect to the double bond.

Process A is for producing compound (6) represented by the general formula (I) in which $X^1$ is a group of β configuration that is represented by the general formula (II) in which Ar is a single bond, A is -O- and $R^1$ is $-CH_2\text{-}CH\text{=}CH\text{-}CH_2\text{-}R^2$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (7) represented by the general formula (I) in which $X^1$ is a group of β configuration that is represented by the general formula (II) in which Ar is a single bond, A is -O- and $R^1$ is $-(CH_2)_4\text{-}R^2$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound

(9) represented by the general formula (I) in which $X^1$ is a group of β configuration that is represented by the general formula (II) in which Ar is a single bond, A is -O- and $R^1$ is $-(CH_2)_4-G^2-S(O)-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (10) represented by the general formula (I) in which $X^1$ is a group of β configuration that is represented by the general formula (II) in which Ar is a single bond, A is -O- and $R^1$ is $-(CH_2)_4-G^2-S(O)_2-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; and compound (147) represented by the general formula (I) in which $X^1$ is a group of β configuration that is represented by the general formula (II) in which Ar is a single bond, A is -O- and $R^1$ is $-CH_2-CH=CH-CH_2-R^2$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a double bond.

## Process A

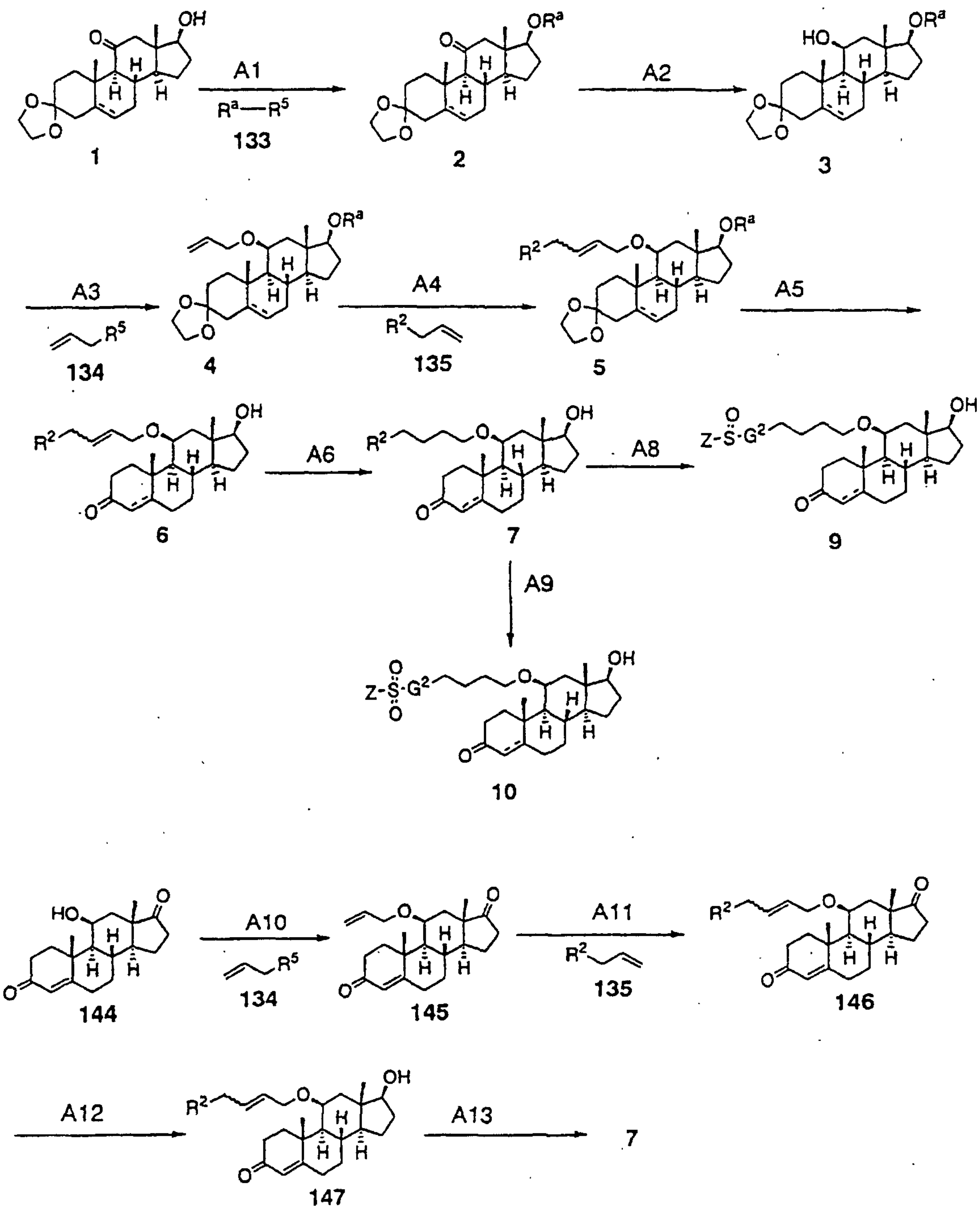


Step A1 is for producing compound (2) and implemented by reacting compound (1) with compound (133) in an inert solvent in the presence of a base.

The inert solvent to be used is not limited in any particular way as long as it does not participate in the reaction of interest; examples are halogen-containing solvents such as dichloromethane, chloroform and carbon tetrachloride, ether solvents such as ether, tetrahydrofuran, dioxane and dimethoxyethane, and aromatic solvents such as benzene, toluene, xylene, quinoline and chlorobenzene, preferably halogen-containing solvents such as dichloromethane, chloroform and carbon tetrachloride, with dichloromethane being more preferred. The base to be used may be exemplified by organic bases such as diisopropylethylamine, 4-dimethylaminopyridine, pyridine, triethylamine and N-methylmorpholine, preferably diisopropylethylamine. The reaction temperature which varies with the type of solvent and the like is typically in the range of 0°C - 50°C, preferably 10°C - 30°C. The reaction time which varies with the reaction temperature and the like is typically in the range of 10 minutes to 24 hours, preferably 30 minutes - 15 hours.

Step A2 is for synthesizing compound (3) and implemented by reacting compound (2) with a reducing agent in an inert solvent.

The inert solvent to be used is not limited in any particular way as long as it does not participate in the reaction; examples are ether solvents such as ether, tetrahydrofuran, dioxane and dimethoxyethane, and alcoholic solvents such as methanol and ethanol, preferably ether and tetrahydrofuran, with ether being more preferred. The reducing agent to be used may be exemplified by: metal hydrogen complex compounds such as aluminum lithium hydride, trimethoxyaluminum lithium hydride, tri-t-butoxyaluminum lithium hydride, aluminum lithium hydride-trichloroaluminum (alane), aluminum lithium hydride-boron trifluoride, aluminum hydride magnesium chloride, magnesium aluminum hydride, sodium aluminum hydride, sodium triethoxyaluminum hydride, sodium bis(methoxyethoxy)aluminum hydride, sodium boron hydride, sodium boron hydride-palladium/carbon, sodium boron hydrogensulfide, sodium boron hydrogencyanide, sodium trimethoxyboron hydride, lithium boron hydride, lithium boron hydrogencyanide, lithium triethylboron hydride, lithium tri-s-butylboron hydride, lithium tri-t-butylboron hydride, calcium boron hydride, potassium boron hydride, potassium triisopropoxyboron hydride, potassium tri-s-butylboron hydride, zinc boron hydride, tetramethylammonium boron hydride, and tetra-n-butylammonium cyanoboron hydride; metal hydrides such as diisobutylaluminum hydride, triphenyltin hydride, tri-n-butyltin hydride, diphenyltin hydride, di-n-butyltin hydride, triethyltin hydride, trimethyltin hydride, trichlorosilane/tri-n-butylamine, trichlorosilane/tri-n-propylamine, triethylsilane, trimethylsilane, diphenylsilane, phenylsilane, polymethylhydrosiloxane, dimethylphenylsilane, di-n-butylsilane, and methylphenylsilane; borane derivatives such as diborane, dimethylamine-borane, trimethylamine-borane, ethylenediamine-borane, pyridine-borane, dimethylsulfide-borane, 2,3-dimethyl-2-butylborane (thexylborane), bis-3-methyl-2-butylborane (disiamylborane), diisopinocanephenylborane, dicyclohexylborane, and 9-borabicyclo[3,3,1]nonane (9-BBN); preferred examples are metal hydrogen complex compounds such as aluminum lithium hydride, trimethoxyaluminum lithium hydride, tri-t-butoxyaluminum lithium hydride, aluminum lithium hydride-trichloroaluminum (alane), aluminum lithium hydride-boron trifluoride, aluminum hydride magnesium chloride, magnesium aluminum hydride, sodium aluminum hydride, sodium triethoxyaluminum hydride, sodium bis(methoxyethoxy)aluminum hydride, sodium boron hydride, sodium boron hydride-palladium/carbon, sodium boron hydrogensulfide, sodium boron hydrogencyanide, sodium trimethoxyboron hydride, lithium boron hydride, lithium boron hydrogencyanide, lithium triethylboron hydride, lithium tri-s-butylboron hydride, lithium tri-t-butylboron hydride, calcium boron hydride, potassium boron hydride, potassium triisopropoxyboron hydride, potassium tri-s-butylboron hydride, zinc boron hydride, tetramethylammonium boron hydride, and tetra-n-butylammonium cyanoboron hydride, with aluminum lithium hydride being more preferred. The reaction temperature which varies with the type of solvent and the like is typically in the range of -30°C - 100°C, preferably 0°C - 70°C. The reaction time which varies with the reaction temperature and the like is typically in the range of 10 minutes - 48 hours, preferably 30 minutes - 24 hours.

As a by-product of this step, there is formed a compound having the hydroxyl group in 11-position of compound (3) oriented in $\alpha$ configuration and this compound may be used to prepare compounds having $X^1$ in compound (6), compound (7), compound (9) and compound (10) oriented in $\alpha$ configuration.

Step A3 is for producing compound (4) and implemented by reacting compound (3) with a base in an inert solvent to make a salt of compound (3) and then reacting it with compound (134) in an inert solvent.

The inert solvent to be used is not limited in any particular way as long as it does not participate in the reaction; examples are halogen-containing solvents such as dichloromethane, chloroform and carbon tetrachloride, ether solvents such as ether, tetrahydrofuran, dioxane and dimethoxyethane, aromatic solvents such as benzene, toluene, xylene, quinoline and chlorobenzene, as well as cyclohexane, dimethyl sulfoxide, dimethylacetamide, dimethylimidazolidinone, dimethylformamide, and N-methylpyrrolidone; preferred examples are ether solvents such as ether, tetrahydrofuran, dioxane and dimethoxyethane, as well as dimethyl sulfoxide, dimethylacetamide, dimethylimidazolidinone, dimethylformamide, and N-methylpyrrolidone. The base to be used may be exemplified by metal hydrides such as sodium hydride, potassium hydride and calcium hydride, alkyllithium compounds such as methyllithium, ethyllithium, n-butyllithium and t-butyllithium, metal hydroxides such as lithium hydroxide, sodium hydroxide, potassium hydroxide, potassium hydroxide, calcium hydroxide, barium hydroxide and cesium hydroxide, metal amides such as sodium amide, potassium bistrimethylsilylamide, sodium bistrimethylsilylamide and lithium diisopropylamide, amines such as triethylamine, diisopropylethylamine, 1,8-diazabicyclo[5.4.0]-7-undecene, pyridine, dimethylamionopyridine and pyrazine, as well as sodium tetraborate, sodium iodide, lithium hexamethyldisilazane, sodium hexamethyldisilazane and potassium hexamethyldisilazane; preferred examples are metal hydrides such as sodium hydride, potassium hydride and calcium hydride, and alkyllithium compounds such as methyllithium, ethyllithium, n-butyllithium and t-butyllithium. The reaction temperature which varies with the type of solvent and the like is typically in the range of -30°C - 100°C, preferably 0°C - 70°C. The reaction temperature which varies with the reaction temperature and the like is typically in the range of 10 minutes - 48 hours, preferably 30 minutes - 24 hours.

Step A4 is for producing compound (5) and implemented by reacting compound (4) with compound (135) in an inert solvent in the presence of an organometallic catalyst.

The inert solvent to be used is not limited in any particular way as long as it does not participate in the reaction; preferred examples are halogen-containing solvents such as dichloromethane and chloroform, ether solvents such as ether, tetrahydrofuran, dioxane and dimethoxyethane, and aromatic solvents such as benzene, toluene, xylene, quinoline and chlorobenzene, with dichloromethane and dimethoxyethane being more preferred. The organometallic catalyst to be used is preferably benzylidene-bis(tricyclohexylphosphine)-dichlororuthenium. The reaction temperature which varies with the type of solvent and the like is typically in the range of -30°C ~ 100°C, preferably 0°C ~ 80°C. The reaction time which varies with the reaction temperature and the like is typically in the range of 10 minutes - 48 hours, preferably 30 minutes - 24 hours.

Step A5 is for producing compound (6) and implemented by reacting compound (5) with an acid in an aqueous solvent.

The inert solvent to be used is not limited in any particular way as long as it does not interfere with the reaction; examples are mixed solvents consisting of water and ether solvents such as ether, tetrahydrofuran and dioxane, alcoholic solvents such as methanol and ethanol, or ketonic solvents such as acetone, with hydrous acetone being preferred. The acid to be used may be exemplified by inorganic acids such as hydrochloric acid, hydrobromic acid, hydroiodic acid, sulfuric acid and phosphoric acid, and organic acids such as acetic acid, p-toluenesulfonic acid, pyridinium-p-toluenesulfonate, with hydrochloric acid being preferred. The reaction temperature which varies with the type of solvent and the like is typically in the range of 0°C - 100°C (preferably 30°C - 80°C). The reaction time which varies with the reaction temperature and the like is typically in the range of 15 minutes - 24 hours (preferably 30 minutes - 10 hours).

Step A6 is for producing compound (7) and implemented by performing catalytic reduction in an alcoholic solvent or an inert solvent.

The solvent to be used may be exemplified by alcoholic solvents such as methanol, ethanol, n-propanol, i-propanol, n-butanol, s-butanol, t-butanol, pentanol, hexanol, cyclopropanol, cyclobutanol, cyclopentanol, cyclohexanol, ethylene glycol, 1,3-propanediol, 1,4-butanediol and 1,5-pentanediol, ether solvents such as ether, tetrahydrofuran, dioxane and dimethoxyethane, aromatic solvents such as benzene, toluene, xylene, quinoline and chlorobenzene, halogen-containing solvents such as dichloromethane, chloroform and carbon tetrachloride, as well as cyclohexane, dimethyl sulfoxide, dimethylacetamide, dimethylimidazolidinone, dimethylformamide, N-methylpyrrolidone, ethyl acetate, acetonitrile and nitromethane; preferred examples are ethanol, dioxane, benzene and ethyl acetate.

The condition to be used in catalytic reduction is a homogeneous system such as hydrogen-chlorotris(triphenylphosphine)rhodium(I), hydrogen-chlorotris(triparatolylphosphine)rhodium(I), hydrogen-chlorotris(triparamethoxyphenylphosphine)rhodium(I), hydrogen-hydridecarbonyltris(triphenylphosphine)rhodium(I), hydrogen-rhodium(II) acetate, hydrogen-ruthenium(II) acetate, hydrogen-chlorohydridetris(triphenylphosphine) ruthenium(II), hydrogen-carboxylatohydridetris(triphenylphosphine)ruthenium(II), hydrogen-hydridecarbonyltris(triphenylphosphine)iridium(I), hydrogen-platinum(II)-tin chloride complex, hydrogen-pentacyanocobalt(II) complex, hydrogen-tricyanobipyridine cobalt(II) complex, hydrogen-bis(dimethylglyoximato)cobalt(II) complex, hydrogen-methyl benzoate-tricarbonylchromium complex, hydrogen-bis(tricarbonylcyclopentadienylchromium), hydrogen-pentacarbonyliron, hydrogen-bis(cyclopentadienyl)dicarbonyltitanium, hydrogen-hydridecarbonylcobalt complex, hydrogen-octacarbonyldicobalt, hydrogen-hydridecarbonylrhodium, hydrogen-chromium(III) acetylacetonato-triisobutylaluminum, hydrogen-cobalt(II) acetylacetonato-triisobutylaluminum, or hydrogen-nickel(II)-2-hexanoato-triethylaluminum, or an inhomogeneous system condition such as hydrogen-platinum dioxide, hydrogen-platinum/carbon, hydrogen-palladium/carbon, hydrogen-palladium/barium sulfate, hydrogen-palladium/calcium carbonate, hydrogen-Raney nickel, hydrogen-copper chromite, hydrogen-rhodium/carbon, hydrogen-rhodium/alumina, hydrogen-ruthenium dioxide, or hydrogen-ruthenium/carbon; preferred examples are hydrogen-chlorotris(triphenylphosphine)rhodium(I), hydrogen-palladium/carbon, hydrogen-palladium/calcium carbonate, etc.

The reaction temperature is typically in the range of 0°C - 100°C, preferably 0°C - 60°C. The reaction time which varies with the reaction temperature and the like is typically in the range of 10 minutes - 24 hours, preferably 10 minutes - 6 hours.

Step A8 is for producing compound (9) in the case where $Q^2$ in $R^2$ in compound (7) is -S- and implemented by reacting compound (7) with an oxidizing agent in an inert solvent.

The inert solvent to be used is not limited in any particular way as long as it does not participate in the reaction and examples include halogen-containing solvents such as dichloromethane, chloroform and carbon tetrachloride, aromatic solvents such as benzene, toluene, xylene, quinoline and chlorobenzene, alcoholic solvents such as methanol and ethanol, ether solvents such as tetrahydrofuran, as well as water, and mixtures thereof; preferred examples are dichloromethane, methanol and a mixture of tetrahydrofuran and water.

The oxidizing agent to be used may be exemplified by organic peroxides such as t-butyl perbenzoate, t-butyl peracetate, t-butyl hydroperoxide, t-amyl hydroperoxide, dibenzoyl peroxide, di-p-nitrobenzoyl peroxide and di-p-chlorobenzoyl peroxide, organic peracids such as perbenzoic acid, metachloroperbenzoic acid, p-nitroperbenzoic acid, monoperoxyphthalic acid, performic acid, peracetic acid, trifluoroperacetic acid and peroxylauric acid, halogens such

as hypochlorous acid, sodium hypochlorite, potassium hypobromite, potassium hypoiodite, sodium chlorate, potassium chlorate, sodium bromate, potassium bromate, sodium iodate, potassium iodate, perchloryl fluoride, orthoperiodic acid, sodium metaperiodate, potassium metaperiodate, N-bromoacetamide, N-bromosuccinimide, N-bromophthalimide, isocyanuric chloride, isocyanuric bromide, N-bromocaprolactam, 1-chlorobenzotriazole, 1,3-dibromo-5,5-dimethylhydantoin, sodium N-chloro-p-toluenesulfonamide (chloramine T), sodium N-chlorobenzenesulfonamide (chloramine B), t-butyl hypochlorite, t-butyl hypobromite, t-butyl hypoiodite, iodosylbenzene acetate and iodosylbenzene, as well as peroxomonosulfuric acid, OXONE (registered trademark) and hydrogen peroxide; preferred examples are sodium periodate and OXONE (registered trademark).

The reaction temperature which varies with the type of solvent and the like is typically in the range of -20°C ~ 30°C (preferably -10°C - 10°C). The reaction time which varies with the reaction temperature and the like is typically in the range of 15 minutes - 24 hours (preferably 30 minutes - 15 hours).

Step A9 is for producing compound (10) in the case where $Q^2$ in $R^2$ in compound (7) is -S- and implemented by reacting compound (7) with an oxidizing agent in an inert solvent.

The inert solvent to be used is not limited in any particular way as long as it does not participate in the reaction and examples include halogen-containing solvents such as dichloromethane, chloroform and carbon tetrachloride, aromatic solvents such as benzene, toluene, xylene, quinoline and chlorobenzene, alcoholic solvents such as methanol and ethanol, ether solvents such as tetrahydrofuran, as well as water, and mixtures thereof; preferred examples are dichloromethane, methanol and a mixture of tetrahydrofuran and water.

The oxidizing agent to be used may be exemplified by organic peroxides such as t-butyl perbenzoate, t-butyl peracetate, t-butyl hydroperoxide, t-amyl hydroperoxide, dibenzoyl peroxide, di-p-nitrobenzoyl peroxide and di-p-chlorobenzoyl peroxide, organic peracids such as perbenzoic acid, metachloroperbenzoic acid, p-nitroperbenzoic acid, monoperoxyphthalic acid, performic acid, peracetic acid, trifluoroperacetic acid and peroxylauric acid, halogens such as hypochlorous acid, sodium hypochlorite, potassium hypobromite, potassium hypoiodite, sodium chlorate, potassium chlorate, sodium bromate, potassium bromate, sodium iodate, potassium iodate, perchloryl fluoride, orthoperiodic acid, sodium metaperiodate, potassium metaperiodate, N-bromoacetamide, N-bromosuccinimide, N-bromophthalimide, isocyanuric chloride, isocyanuric bromide, N-bromocaprolactam, 1-chlorobenzotriazole, 1,3-dibromo-5,5-dimethylhydantoin, sodium N-chloro-p-toluenesulfonamide (chloramine T), sodium N-chlorobenzenesulfonamide (chloramine B), t-butyl hypochlorite, t-butyl hypobromite, t-butyl hypoiodite, iodosylbenzene acetate and iodosylbenzene, as well as peroxomonosulfuric acid, OXONE (registered trademark) and hydrogen peroxide; a preferred example is OXONE (registered trademark).

The reaction temperature which varies with the type of solvent and the like is typically in the range of 0 °C ~ 100 °C (preferably 10°C - 50°C). The reaction time which varies with the reaction temperature and the like is typically in the range of 15 minutes - 24 hours (preferably 30 minutes - 15 hours).

Step A10 is for producing compound (145) and implemented by reacting compound (144) with a base in an inert solvent to make a salt of compound (144) and then reacting it with compound (134) in an inert solvent. The reaction is performed as in the aforementioned step A3 in process A.

A compound having the hydroxyl group in 11-position of compound (144) oriented in β configuration is commercially available and using this compound in place of compound (144), one can obtain a compound having $X^1$ in compound (7) oriented in β configuration.

Step A11 is for producing compound (146) and implemented by reacting compound (145) with compound (135) in an inert solvent in the presence of an organometallic catalyst. The reaction is performed as in the aforementioned step A4 in process A.

Step A12 is for producing compound (147) and implemented by reacting compound (146) with a reducing agent in an optionally miscible inert solvent.

The inert solvent to be used is not limited in any particular way as long as it does not interfere with the reaction; examples are ether solvents such as ether, tetrahydrofuran, dioxane and dimethoxyethane, alcoholic solvents such as methanol and ethanol, aromatic solvents such as benzene, toluene, xylene, quinoline and chlorobenzene, and amines such as pyridine and triethylamine; preferred examples are alcoholic solvents such as methanol and ethanol, with methanol being more preferred. The reducing agent to be used may be exemplified by: metal hydrogen complex compounds such as aluminum lithium hydride, trimethoxyaluminum lithium hydride, tri-t-butoxyaluminum lithium hydride, aluminum lithium hydride-trichloroaluminum (alane), aluminum lithium hydride-boron trifluoride, aluminum hydride magnesium chloride, magnesium aluminum hydride, sodium aluminum hydride, sodium triethoxyaluminum hydride, sodium bis(methoxyethoxy)aluminum hydride, sodium boron hydride, sodium boron hydride-palladium/carbon, sodium boron hydrogensulfide, sodium boron hydrogencyanide, sodium trimethoxyboron hydride, lithium boron hydride, lithium boron hydrogencyanide, lithium triethylboron hydride, lithium tri-s-butylboron hydride, lithium tri-t-butylboron hydride, calcium boron hydride, potassium boron hydride, potassium triisopropoxyboron hydride, potassium tri-s-butylboron hydride, zinc boron hydride, tetramethylammonium boron hydride, and tetra-n-butylammonium cyanoboron hydride; metal hydrides such as diisobutylaluminum hydride, triphenyltin hydride, tri-n-butyltin hydride, diphenyltin

hydride, di-n-butyltin hydride, triethyltin hydride, trimethyltin hydride, trichlorosilane/tri-n-butylamine, trichlorosilane/tri-n-propylamine, triethylsilane, trimethylsilane, diphenylsilane, phenylsilane, polymethylhydrosiloxane, dimethylphenylsilane, di-n-butylsilane, and methylphenylsilane; borane derivatives such as diborane, dimethylamine-borane, trimethylamine-borane, ethylenediamine-borane, pyridine-borane, dimethylsulfide-borane, 2,3-dimethyl-2-butylborane (thexylborane), bis-3-methyl-2-butylborane (disiamylborane), diisopinocanephenylborane, dicyclohexylborane, and 9-borabicyclo[3,3,1]nonane (9-BBN); preferred examples are metal hydrogen complex compounds such as aluminum lithium hydride, trimethoxyaluminum lithium hydride, tri-t-butoxyaluminum lithium hydride, aluminum lithium hydride-trichloroaluminum (alane), aluminum lithium hydride-boron trifluoride, aluminum hydride magnesium chloride, magnesium aluminum hydride, sodium aluminum hydride, sodium triethoxyaluminum hydride, sodium bis(methoxyethoxy)aluminum hydride, sodium boron hydride, sodium boron hydride-palladium/carbon, sodium boron hydrogensulfide, sodium boron hydrogencyanide, sodium trimethoxyboron hydride, lithium boron hydride, lithium boron hydrogencyanide, lithium triethylboron hydride, lithium tri-s-butylboron hydride, lithium tri-t-butylboron hydride, calcium boron hydride, potassium boron hydride, potassium triisopropoxyboron hydride, potassium tri-s-butylboron hydride, zinc boron hydride, tetramethylammonium boron hydride, and tetra-n-butylammonium cyanoboron hydride, with sodium boron hydride being more preferred. The reaction temperature which varies with the type of solvent and the like is typically in the range of -30°C ~ 100°C, preferably 0°C ~ 70°C. The reaction time which varies with the reaction temperature and the like is typically in the range of 10 minutes - 48 hours, preferably 30 minutes - 24 hours.

Step A13 is for producing compound (7) and implemented by performing catalytic reduction in an alcoholic solvent or an inert solvent. The reaction is performed as in the aforementioned step A6 in process A.

Process B is for producing compound (17) represented by the general formula (I) in which $X^1$ is a group of β configuration that is represented by the general formula (II) in which Ar is a single bond, A is -O- and $R^1$ is -G-S-Z, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (18) represented by the general formula (I) in which $X^1$ is a group of β configuration that is represented by the general formula (II) in which Ar is a single bond, A is -O- and $R^1$ is -G-S(O)-Z-, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; and compound (19) represented by the general formula (I) in which $X^1$ is a group of β configuration that is represented by the general formula (II) in which Ar is a single bond, A is -O- and $R^1$ is -G-S(O)$_2$-Z, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

Process B

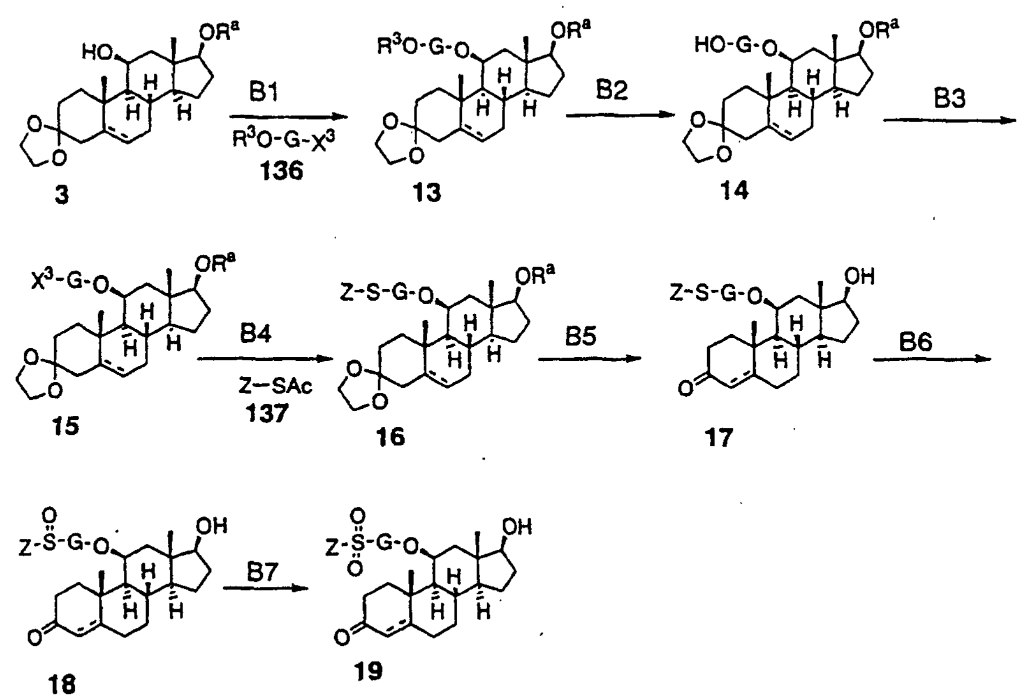


Step B1 is for producing compound (13) and implemented by reacting compound (3) with a base in an inert solvent to make a salt of compound (3) and then reacting it with compound (136) in an inert solvent. The reaction is performed as in the aforementioned step A3 in process A.

Step B2 is for producing compound (14) and implemented by reacting compound (13) with a deprotecting agent, namely by removing the substituted silyl group, in an inert solvent.

The inert solvent to be used is not limited in an particular way as long as it does not interfere with the reaction; examples include ether solvents such as ether, tetrahydrofuran, dioxane and dimethoxyethane, as well as dimethylformamide and water, with tetrahydrofuran being preferred. The deprotecting agent to be used is not limited in any particular way and may be exemplified by fluorides such as hydrogen fluoride, hydrogen fluoride-pyridine, sodium fluoride, potassium fluoride and tetra-n-butylammonium fluoride, and organic acids such as formic acid, acetic acid and p-toluenesulfonic acid, with tetra-n-butylammonium fluoride being preferred.

The reaction temperature which varies with the type of solvent and the like is typically in the range of 0°C - 80°C (preferably 0°C - 50°C). The reaction time which varies with the reaction temperature and the like is typically in the range of 15 minutes - 24 hours (preferably 30 minutes - 15 hours).

Step B3 is for producing compound (15) and implemented by reacting compound (14) with a sulfonyl chloride compound in an amine-containing solvent or reacting compound (14) with a halogenating agent in an inert solvent.

The amine-containing solvent to be used is not limited in any particularly way and may be exemplified by triethylamine, diisopropylethylamine, 1,8-diazabicyclo[5.4.0]-7-undecene and pyridine, with pyridine and triethylamine being preferred.

The sulfonyl chloride compound to be used is not limited in any particular way and may be exemplified by p-toluenesulfonyl chloride, benzenesulfonyl chloride, methanesulfonyl chloride and trifluoromethanesulfonyl chloride, with methanesulfonyl chloride and trifluoromethanesulfonyl chloride being preferred.

The inert solvent to be used is not limited in any particular way as long as it does not participate in the reaction of interest; examples include ether solvents such as ether, tetrahydrofuran, dioxane and dimethoxyethane, aromatic solvents such as benzene, toluene, xylene, quinoline and chlorobenzene, nitrile-containing solvents such as acetonitrile, halogen-containing solvents such as dichloromethane, chloroform and carbon tetrachloride, as well as cyclohexane, dimethyl sulfoxide, dimethylacetamide, dimethylimidazolidinone, dimethylformamide, N-methylpyrrolidone and ethyl acetate, with benzene and dichloromethane being preferred.

The halogenating agent to be used may be exemplified by chlorinating agents such as carbon tetrachloride-triphenylphosphine, thionyl chloride, sulfuryl chloride, N-chlorosuccinimide-triphenylphosphine, N-chlorosuccinimide-dimethyl sulfide, phosphorus trichloride and phosphorus pentachloride, and brominating agents such as carbon tetrabromide-triphenylphosphine, N-bromosuccinimide-triphenylphosphine, N-bromosuccinimide-dimethyl sulfide, phosphorus tribromide and phosphorus pentabromide, and preferred examples are carbon tetrabromide-triphenylphosphine and thionyl chloride. The reaction temperature is typically in the range of 0°C - 80°C, preferably 10°C - 40°C. The reaction time which varies with the reaction temperature is typically in the range of 10 minutes - 10 hours, preferably 30 minutes - 3 hours.

Step B4 is for producing compound (16) and implemented by reacting compound (137) with a metal alkoxide in an alcoholic solvent to make a reactive derivative of compound (137) and then reacting it with compound (15) in an alcoholic solvent.

The alcoholic solvent to be used is not limited in any particular way and may be exemplified by methanol, ethanol, n-propanol, i-propanol and mixed solvents containing them, and preferred examples are methanol and a methanol-tetrahydrofuran mixed solvent.

The metal alkoxide to be used is not limtied in any particular way and may be exemplified by sodium methoxide and sodium ethoxide, with sodium methoxide being preferred.

The reaction temperature which varies with the solvent and other conditions is typically in the range of 0°C - 80°C, preferably 10°C - 40°C. The reaction time which varies with the reaction temperature and the like is typically in the range of 10 minutes - 10 hours, preferably 30 minutes - 8 hours.

Step B5 is for producing compound (17) and implemented by reacting compound (16) with an acid in an aqueous solvent. The reaction is performed as in the aforementioned step A5 in process A.

Step B6 is for producing compound (18) and implemented by reacting compound (17) with an oxidizing agent in an inert solvent. The reaction is performed as in the aforementioned step A8 in process A.

Step B7 is for producing compound (19) and implemented by reacting compound (18) with an oxidizing agent in an inert solvent. The reaction is performed as in the aforementioned step A9 in process A.

Process B' is an alternative method for producing compound (243) having the dashed line in compound (17) forming a double bond together with the solid line and compound (244) having the dashed line in compound (17) forming a single bond together with the solid line.

Process B'

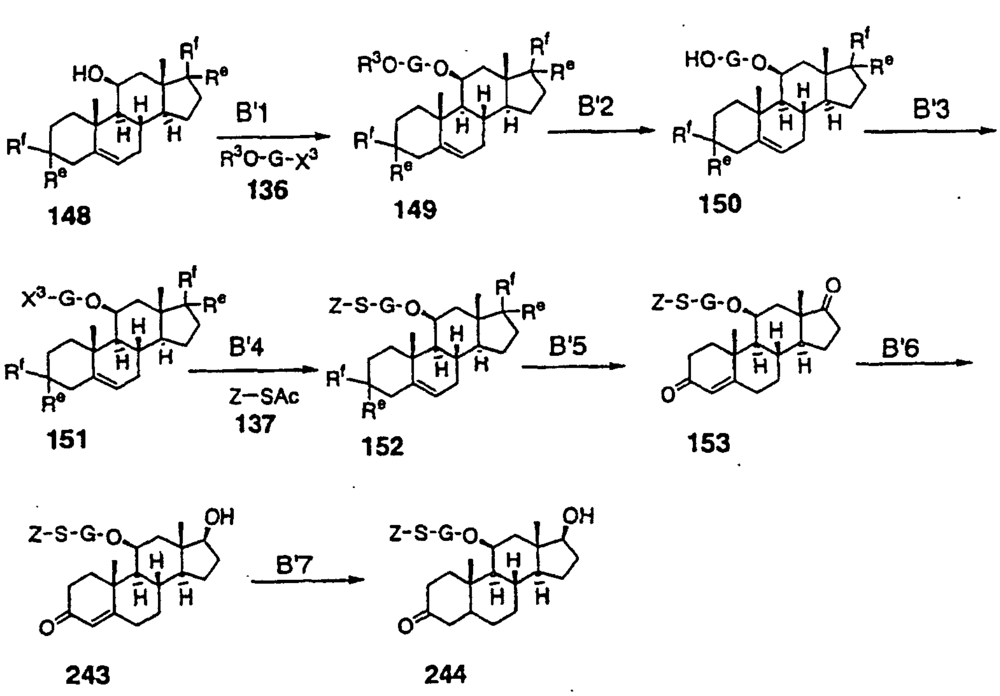


Step B'1 is for producing compound (149) and implemented by reacting compound (148) with a base in an inert solvent to make a salt of compound (148) and then reacting it with compound (136) in an inert solvent. The reaction is performed as in the aforementioned step A3 in process A.

Step B'2 is for producing compound (150) and implemented by reacting compound (149) with a deprotecting

agent, namely by removing the substituted silyl group, in an inert solvent. The reaction is performed as in the aforementioned step B2 in process B.

Step B'3 is for producing compound (151) and implemented by reacting compound (150) with a sulfonyl chloride compound in an amine-containing solvent or reacting compound (150) with a halogenating agent in an inert solvent. The reaction is performed as in the aforementioned step B3 in process B.

Step B'4 is for producing compound (152) and implemented by reacting compound (137) with a metal alkoxide in an alcoholic solvent to make a reactive derivative of compound (137) and then reacting it with compound (151) in an alcoholic solvent. The reaction is performed as in the aforementioned step B4 in process B.

Step B'5 is for producing compound (153) and implemented by reacting compound (152) with an acid in an aqueous solvent. The reaction is performed as in the aforementioned step A5 in process A.

Step B'6 is for producing compound (243) and implemented by reacting compound (153) with a reducing agent in an optionally miscible inert solvent. The reaction is performed as in the aforementioned step A12 in process A.

Step B'7 is for producing compound (244) and implemented by performing catalytic reduction of compound (243) in an alcoholic solvent or an inert solvent or reducing compound (243) with a reducing agent in an optionally miscible inert solvent.

The solvent to be used in performing catalytic reduction may be exemplified by alcoholic solvents such as methanol, ethanol, n-propanol, i-propanol, n-butanol, s-butanol, t-butanol, pentanol, hexanol, cyclopropanol, cyclobutanol, cyclopentanol, cyclohexanol, ethylene glycol, 1,3-propanediol, 1,4-butanediol and 1,5-pentanediol, ether solvents such as ether, tetrahydrofuran, dioxane and dimethoxyethane, aromatic solvents such as benzene, toluene, xylene, quinoline and chlorobenzene, halogen-containing solvents such as dichloromethane, chloroform and carbon tetrachloride, amine-containing solvents such as pyridine and triethylamine, as well as cyclohexane, dimethyl sulfoxide, dimethylacetamide, dimethylimidazolidinone, dimethylformamide, N-methylpyrrolidone, ethyl acetate, acetonitrile and nitromethane; preferred examples are ethanol, ether, dioxane, and pyridine.

The condition to be used in catalytic reduction is a homogeneous system such as hydrogen-chlorotris(triphenylphosphine)rhodium(I), hydrogen-chlorotris(triparatolylphosphine)rhodium(I), hydrogen-chlorotris(triparamethoxyphenylphosphine)rhodium(I), hydrogen-hydridecarbonyltris(triphenylphosphine)rhodium(I), hydrogen-rhodium(II) acetate, hydrogen-ruthenium(II) acetate, hydrogen-chlorohydridetris(triphenylphosphine)ruthenium(II), hydrogen-carboxylatohydridetris(triphenylphosphine)ruthenium(II), hydrogen-hydridecarbonyltris(triphenylphosphine)iridium(I), hydrogen-platinum(II)-tin chloride complex, hydrogen-pentacyanocobalt(II) complex, hydrogen-tricyanobipyridine cobalt(II) complex, hydrogen-bis(dimethylglyoximato)cobalt(II) complex, hydrogen-methyl benzoate-tricarbonylchromium complex, hydrogen-bis(tricarbonylcyclopentadienylchromium), hydrogen-pentacarbonyliron, hydrogen-bis(cyclopentadienyl)dicarbonyltitanium, hydrogen-hydridecarbonylcobalt complex, hydrogen-octacarbonyldicobalt, hydrogen-hydridecarbonylrhodium, hydrogen-chromium(III) acetylacetonato-triisobutylaluminum, hydrogen-cobalt(II) acetylacetonato-triisobutylaluminum, or hydrogen-nickel(II)-2-hexanoato-triethylaluminum, or an inhomogeneous system condition such as hydrogen-platinum dioxide, hydrogen-platinum/carbon, hydrogen-palladium/carbon, hydrogen-palladium/barium sulfate, hydrogen-palladium/calcium carbonate, hydrogen-Raney nickel, hydrogen-copper chromite, hydrogen-rhodium/carbon, hydrogen-rhodium/alumina, hydrogen-ruthenium dioxide, or hydrogen-ruthenium/carbon; preferred examples are hydrogen-chlorotris(triphenylphosphine)rhodium(I), hydrogen-palladium/carbon, hydrogen-palladium/calcium carbonate, etc.

The reaction temperature is typically in the range of 0°C - 100°C, preferably 0°C - 60°C. The reaction time which varies with the reaction temperature and the like is typically in the range of 10 minutes - 24 hours, preferably 10 minutes - 6 hours.

The inert solvent to be used in the reaction with the reducing agent is not limited in any particular way as long as it does not participate in the reaction; examples include ether solvents such as ether, tetrahydrofuran, dioxane and dimethoxyethane, aromatic solvents such as benzene, toluene, xylene, quinoline and chlorobenzene, halogen-containing solvents such as dichloromethane, chloroform and carbon tetrachloride, amine-containing solvents such as pyridine and triethylamine, as well as cyclohexane, dimethyl sulfoxide, dimethylacetamide, dimethylimidazolidinone, dimethylformamide, N-methylpyrrolidone, acetonitrile and nitromethane; preferred examples are tetrahydrofuran, benzene, toluene and pyridine.

The reducing agent to be used may be exemplified by metals such as sodium/liquid ammonia, lithium/liquid ammonia, lithium/methylamine, lithium/ethylamine, lithium/ethylenediamine, sodium/hexamethylphosphamide-t-butanol, sodium/ethanol, sodium/t-butanol-terrahydrofuran and sodium/toluene-t-amyl alcohol, metal hydrides such as triphenyltin hydride, tri-n-butyltin hydride, diphenyltin hydride, di-n-butyltin hydride, triethyltin hydride, trimethyltin hydride, trichlorosilane/tri-n-butylamine, trichlorosilane/tri-n-propylamine, triethylsilane, trimethylsilane, diphenylsilane, phenylsilane, polymethylhydrosiloxane, dimethylphenylsilane, di-n-butylsilane and methylphenylsilane, metal hydrogen complex compounds such as lithium aluminum hydride/copper(I) iodide, trimethoxyaluminum lithium hydride/copper(I) bromide, tri-t-butoxyaluminum lithium hydride/copper(I) bromide, sodium boron hydride, sodium boron hydride-palladium/carbon, sodium boron hydrogensulfide, sodium boron hydrogencyanide, sodium trimethoxyboron hydride,

lithium boron hydride, lithium boron hydrogencyanide, lithium triethylboron hydride, lithium tri-s-butylboron hydride, lithium tri-t-butylboron hydride, calcium boron hydride, potassium boron hydride, potassium triisopropoxyboron hydride, potassium tri-s-butylboron hydride, zinc boron hydride, tetramethylammonium boron hydride and tetra-n-butylammonium cyanoboron hydride; preferred examples are sodium/liquid ammonia, lithium/liquid ammonia, triphenyltin hydride, tri-n-butyltin hydride, lithium aluminum hydride/copper(I) iodide, trimethoxyaluminum lithium hydride/copper(I) bromide, sodium boron hydride and potassium tri-s-butylboron hydride.

The reaction temperature which varies with the type of reducing agent is typically in the range of -80°C - 100°C, preferably -78°C - 80°C. The reaction time which varies with the reaction temperature and the like is typically in the range of 10 minutes - 24 hours, preferably 10 minutes - 6 hours.

Process C is for producing compound (25) represented by the general formula (I) in which $X^1$ is a group of β configuration that is represented by the general formula (II) in which Ar is a single bond, A is -O- and $R^1$ is -G-CONH-Z, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

## Process C

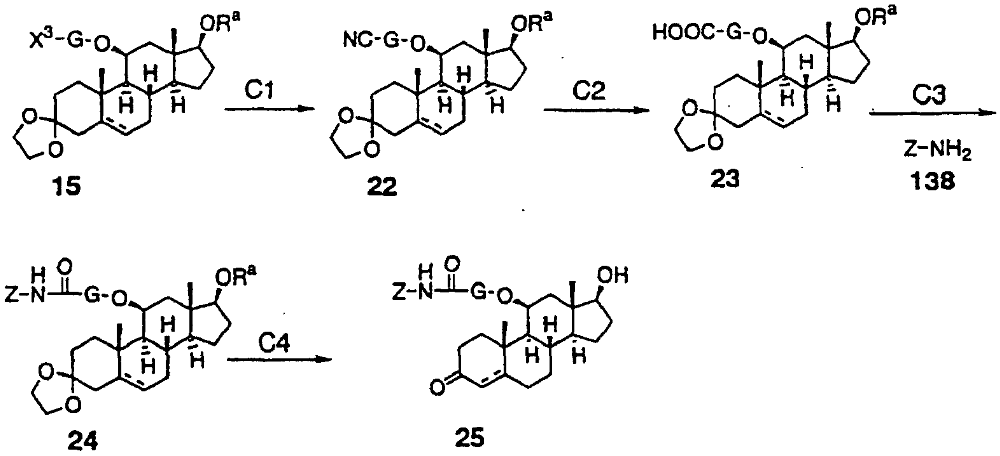


15 22 23 24 25

Step C1 is for producing compound (22) and implemented by reacting compound (15) with a cyanylating agent in an inert solvent.

The inert solvent to be used is not limited in any particular way as long as it does not participate in the reaction; examples include ether solvents such as ether, tetrahydrofuran, dioxane and dimethoxyethane, aromatic solvents such as benzene, toluene, xylene, quinoline and chlorobenzene, halogen-containing solvents such as dichloromethane, chloroform and carbon tetrachloride, as well as cyclohexane, dimethyl sulfoxide, dimethylacetamide, dimethylimidazolidinone, dimethylformamide, N-methylpyrrolidone, methyl acetate, acetonitrile and nitromethane; a preferred example is dimethyl sulfoxide.

The cyanlyating agent to be used may be exemplified by lithium cyanide, sodium cyanide, potassium cyanide, etc. and sodium cyanide is preferred. The reaction temperature is typically in the range of 0°C - 80°C, preferably 10°C - 40°C. The reaction time which varies with the reaction temperature and the like is typically in the range of 10 minutes - 24 hours, preferably 1 hour - 15 hours.

Step C2 is for producing compound (23) and implemented by hydrolyzing compound (22) in the presence of a base.

The solvent to be used is not limited in any particular way as long as it is used in ordinary hydrolytic reaction; examples can be alcoholic solvents such as methanol and ethanol, ether solvents such as tetrahydrofuran and dioxane, water, and mixtures thereof; preferred examples are water and hydrous alcoholic solvents such as water-ethanol.

The base to be used is not limited in any particular way as long as it does not affect other portions of the compound; preferred examples are metal hydroxides such as lithium hydroxide, sodium hydroxide, potassium hydroxide, calcium hydroxide, barium hydroxide and cesium hydroxide, with sodium hydroxide and potassium hydroxide being particularly preferred.

The reaction temperature is typically in the range of 0°C - 100°C, preferably 50°C - 100°C.

The reaction time which varies with the reaction temperature and the like is typically in the range of 10 minutes

- 48 hours, preferably 5 hours - 48 hours.

Step C3 is for producing compound (24) and implemented by reacting compound (23) or reactive derivatives thereof (acid halides, mixed acid anhydrides or active esters) with compound (138) or acid addition salts thereof in an inert solvent.

The reaction is performed by, for example, the acid halide method, the mixed acid anhydride method, the active ester method or the condensation method. The acid halide method is implemented by reacting compound (23) with a halogenating agent (e.g. thionyl chloride, chloride oxalate, phosphorus pentachloride, etc.) in an inert solvent to prepare an acid halide which is then reacted with compound (138) or an acid addition salt in an inert solvent in the presence or absence of a base (preferably in it's presence). The base to be used may be exemplified by organic amines such as triethylamine, N-methylmorpholine, pyridine and 4-dimethylaminopyridine, alkali metal bicarbonates such as sodium bicarbonate and potassium bicarbonate, and alkali metal carbonates such as sodium carbonate and potassium carbonate; organic amines are preferred (with triethylamine being particularly preferred).

The solvent to be used is not limited in any particular way as long as it does not participate in the reaction; examples include hydrocarbon solvents such as hexane, cyclohexane, benzene, toluene and xylene, halogen-containing solvents such as dichloromethane, 1,2-dichloroethane and carbon tetrachloride, ether solvents such as ether, tetrahydrofuran and dioxane, ketonic solvents such as acetone, amide-containing solvents such as N,N-dimethylacetamide, N,N-dimethylformamide and N-methyl-2-pyrrolidone, and sulfoxide-containing solvents such as dimethyl sulfoxide; preferred examples are hydrocarbon solvents, halogen-containing solvents and ether solvents, and more preferred examples are ether solvents (with tetrahydrofuran being particularly preferred). The reaction temperature varies with the type of solvent and the like; however, for both the reaction of a halogenating agent with compound (23) and the reaction of an acid halide with compound (138) or its acid addition salt, the range is typically between -20 °C and 150 °C; preferably, the temperature for the reaction between a halogenating agent and compound (23) is in the range of -10°C ~ 50°C, and the temperature for the reaction between an acid halide and compound (138) or its acid addition salt is in the range of 0°C - 100°C. The reaction time which varies with the reaction temperature and the like is typically in the range of 15 minutes - 24 hours (preferably, 30 minutes - 15 hours).

The mixed acid anhydride method is implemented by reacting a $C_1$-$C_6$ alkyl halogenocarbonate (where $C_1$-$C_6$ refers to a straight-chained or branched alkyl group having 1 - 6 carbon atoms), a di-$C_1$-$C_6$ alkylcyanophosphoric acid or a diarylphosphorylazide with compound (23) to prepare a mixed acid anhydride which is then reacted with compound (138) or an acid addition salt thereof. The reaction for preparing a mixed acid anhydride is performed by reacting a $C_1$-$C_6$ alkyl halogenocarbonate such as methyl chlorocarbonate, ethyl chlorocarbonate, isobutyl chlorocarbonate or hexyl chlorocarbonate (preferably ethyl chlorocarbonate or isobutyl chlorocarbonate), a di-$C_1$-$C_6$ alkylcyanophosphoric acid such as dimethylcyanophosphoric acid, diethylcyanophosphoric acid or dihexylcyanophosphoric acid or a diarylphosphoric acid azide such as diphenylphosphoric acid azide, di-(p-nitrophenyl)phosphoric acid azide or dinaphthylphosphoric acid azide (preferably diphenylphosphoric acid azide) with compound (23), preferably in an inert solvent in the presence of a base.

The base and the inert solvent to be used are the same as those used when the acid halide method is employed in the step under consideration. The reaction temperature which varies with the type of solvent and the like is typically in the range of -20°C - 50°C (preferably 0°C - 30°C). The reaction time which varies with the reaction temperature and the like is typically in the range of 15 minutes - 24 hours (preferably 30 minutes - 15 hours).

The reaction between a mixed acid anhydride and compound (138) or its acid addition salt is performed in an inert solvent in the presence or absence (preferably the presence) of a base, and the base and the inert solvent to be used are the same as those used in the above acid halide method. The reaction temperature which varies with the type of solvent and the like is typically in the range of - 20°C - 50°C (preferably 0°C - 30°C). The reaction time which varies with the reaction temperature and the like is typically in the range of 15 minutes - 24 hours (preferably 30 minutes - 15 hours). If a di-$C_1$-$C_6$ alkylcyanophosphoric acid or a diarylphosphoric acid azide is used in the process under consideration, compound (23) may be directly reacted with compound (138) or its acid addition salt in the presence of a base.

The active esterification method is implemented by reacting compound (23) with an active esterifying agent (e.g. N-hydroxy compounds such as N-hydroxysuccinimide and N-hydroxybenzotriazole) in the presence of a condensing agent (e.g. dicyclohexylcarbodiimide or carbonyldiimidazole) to prepare an active ester which is then reacted with compound (138) or an acid addition salt thereof. The reaction for preparing an active ester is preferably performed in an inert solvent and the inert solvent to be used may be exemplified by ether solvents such as ether, tetrahydrofuran, dioxane and dimethoxyethane, halogen-containing solvents such as dichloromethane, chloroform and carbon tetrachloride, as well as dimethylformamide, ethyl acetate, acetonitrile, etc.; preferred examples are dichloromethane, acetonitrile, ethyl acetate, etc. The reaction temperature varies with the type of solvent and the like; the temperature for the active esterification reaction is typically in the range of -20°C ~ 50°C (preferably -10°C ~ 30°C), and the temperature for the reaction between the active ester compound and compound (138) or its acid addition salt is typically in the range of -20°C - 50°C (preferably -10°C - 30°C). The reaction time varies with the reaction temperature and the like;

however, for both reactions, it is typically in the range of 15 minutes - 24 hours (preferably 30 minutes - 15 hours).

The condensation method is performed by directly reacting compound (23) with compound (138) or an acid addition salt thereof in the presence of a condensing agent [e.g. dicyclohexylcarbodiimide, carbonyldiimidazole, or 1-(N,N-dimethylaminopropyl)-3-ethylcarbodiimide hydrochloride]. This reaction is performed as in the aforementioned reaction for preparing the active ester.

Step C4 is for producing compound (25) and implemented by reacting compound (24) with an acid in an aqueous solvent and this reaction is performed as in the aforementioned step A5 in process A.

Process C' is a method of producing compound (245) having the dashed line in compound (25) forming a double bond together with the solid line, and compound (158) having the dashed line in compound (25) forming a single bond together with the solid line.

Process C'

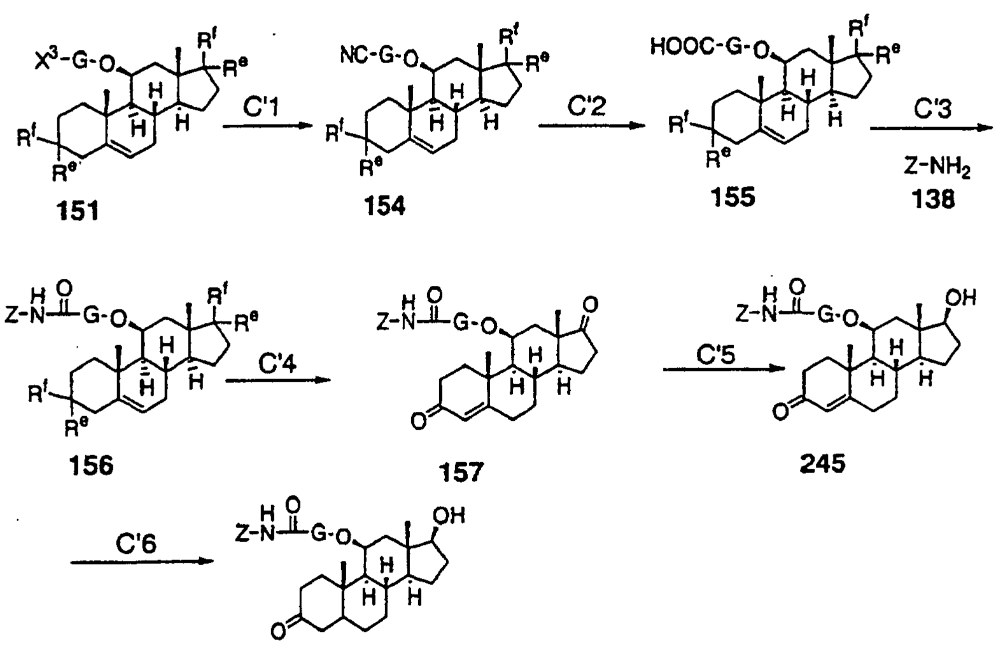

151
154
155
156
157
245
158

Step C'1 is for producing compound (154) and implemented by reacting compound (151) with a cyanylating agent in an inert solvent. The reaction is performed as in the aforementioned step C1 in process C.

Step C'2 is for producing compound (155) and implemented by hydrolyzing compound (154) in the presence of a base. The reaction is performed as in the aforementioned step C2 in process C.

Step c'3 is for producing compound (156) and implemented by reacting compound (155) or reactive derivatives thereof (acid halides, mixed acid anhydrides or active esters) with compound (138) or acid addition salts thereof in an inert solvent. The reaction is performed as in the aforementioned step C3 in process C.

Step C'4 is for producing compound (157) and implemented by reacting compound (156) with an acid in an aqueous solvent. The reaction is performed as in the aforementioned step A5 in process A.

Step C'5 is for producing compound (245) and implemented by reacting compound (157) with a reducing agent in an optionally miscible inert solvent. The reaction is performed as in the aforementioned step A12 in process A.

Step C'6 is for producing compound (158) and implemented by performing catalytic reduction of compound (245) in an alcoholic solvent or an inert solvent or reacting compound (245) with a reducing agent in an optionally miscible inert solvent.

The solvent to be used in performing catalytic reduction may be exemplified by alcoholic solvents such as methanol, ethanol, n-propanol, i-propanol, n-butanol, s-butanol, t-butanol, pentanol, hexanol, cyclopropanol, cyclobutanol, cyclopentanol, cyclohexanol, ethylene glycol, 1,3-propanediol, 1,4-butanediol and 1,5-pentanediol, ether solvents such as ether, tetrahydrofuran, dioxane and dimethoxyethane, aromatic solvents such as benzene, toluene, xylene, quinoline and chlorobenzene, halogen-containing solvents such as dichloromethane, chloroform and carbon tetrachloride, amine-containing solvents such as pyridine and triethylamine, as well as cyclohexane, dimethyl sulfoxide,

dimethylacetamide, dimethylimidazolidinone, dimethylformamide, N-methylpyrrolidone, ethyl acetate, acetonitrile and nitromethane; preferred examples are ethanol, ether, dioxane, and pyridine.

The condition to be used in catalytic reduction is a homogeneous system such as hydrogen- chlorotris(triphenylphosphine)rhodium(I), hydrogen-chlorotris(triparatolylphosphine)rhodium(I), hydrogen-chlorotris(triparamethoxyphenylphosphine)rhodium(I), hydrogen-hydridecarbonyltris(triphenylphosphine)rhodium(I), hydrogen-rhodium(II) acetate, hydrogen-ruthenium(II) acetate, hydrogen-chlorohydridetris(triphenylphosphine)ruthenium(II), hydrogen-carboxylatohydridetris(triphenylphosphine)ruthenium(II), hydrogen-hydridecarbonyltris(triphenylphosphine)iridium(I), hydrogen-platinum(II)-tin chloride complex, hydrogen-pentacyanocobalt(II) complex, hydrogen-tricyanobipyridine cobalt(II) complex, hydrogen-bis(dimethylglyoximato)cobalt (II) complex, hydrogen-methyl benzoate-tricarbonylchromium complex, hydrogen-bis(tricarbonylcyclopentadienylchromium), hydrogen-pentacarbonyliron, hydrogen-bis(cyclopentadienyl)dicarbonyltitanium, hydrogen-hydridecarbonylcobalt complex, hydrogen-octacarbonyldicobalt, hydrogen-hydridecarbonylrhodium, hydrogen-chromium(III) acetylacetonato-triisobutylaluminum, hydrogen-cobalt(II) acetylacetonato-triisobutylaluminum, or hydrogen-nickel(II)-2-hexanoato-triethylaluminum, or an inhomogeneous system condition such as hydrogen-platinum dioxide, hydrogen-platinum/carbon, hydrogen-palladium/carbon, hydrogen-palladium/barium sulfate, hydrogen-palladium/calcium carbonate, hydrogen-Raney nickel, hydrogen-copper chromite, hydrogen-rhodium/carbon, hydrogen-rhodium/alumina, hydrogen-ruthenium dioxide, or hydrogen-ruthenium/carbon; preferred examples are hydrogen-chlorotris(triphenylphosphine)rhodium(I), hydrogen-palladium/carbon, hydrogen-palladium/calcium carbonate, etc.

The reaction temperature is typically in the range of 0°C - 100°C, preferably 0°C - 60°C. The reaction time which varies with the reaction temperature and the like is typically in the range of 10 minutes - 24 hours, preferably 10 minutes - 6 hours.

The inert solvent to be used in the reaction with the reducing agent is not limited in any particular way as long as it does not participate in the reaction; examples include ether solvents such as ether, tetrahydrofuran, dioxane and dimethoxyethane, aromatic solvents such as benzene, toluene, xylene, quinoline and chlorobenzene, halogen-containing solvents such as dichloromethane, chloroform and carbon tetrachloride, amine-containing solvents such as pyridine and triethylamine, as well as cyclohexane, dimethyl sulfoxide, dimethylacetamide, dimethylimidazolidinone, dimethylformamide, N-methylpyrrolidone, acetonitrile and nitromethane; preferred examples are tetrahydrofuran, benzene, toluene and pyridine.

The reducing agent to be used may be exemplified by metals such as sodium/liquid ammonia, lithium/liquid ammonia, lithium/methylamine, lithium/ethylamine, lithium/ethylenediamine, sodium/hexamethylphosphamide-t-butanol, sodium/ethanol, sodium/t-butanol-terrahydrofuran, sodium/toluene-t-amyl alcohol, metal hydrides such as triphenyltin hydride, tri-n-butyltin hydride, diphenyltin hydride, di-n-butyltin hydride, triethyltin hydride, trimethyltin hydride, trichlorosilane/tri-n-butylamine, trichlorosilane/tri-n-propylamine, triethylsilane, trimethylsilane, diphenylsilane, phenylsilane, polymethylhydrosiloxane, dimethylphenylsilane, di-n-butylsilane and methylphenylsilane; metal hydrogen complex compounds such as lithium aluminum hydride/copper(I) iodide, trimethoxyaluminum lithium hydride/copper(I) bromide, tri-t-butoxyaluminum lithium hydride/copper(I) bromide, sodium boron hydride, sodium boron hydride-palladium/carbon, sodium boron hydrogensulfide, sodium boron hydrogencyanide, sodium trimethoxyboron hydride, lithium boron hydride, lithium boron hydrogencyanide, lithium triethylboron hydride, lithium tri-s-butylboron hydride, lithium tri-t-butylboron hydride, calcium boron hydride, potassium boron hydride, potassium triisopropoxyboron hydride, potassium tri-s-butylboron hydride, zinc boron hydride, tetramethylammonium boron hydride and tetra-n-butylammonium cyanoboron hydride; preferred examples are sodium/liquid ammonia, lithium/liquid ammonia, triphenyltin hydride, tri-n-butyltin hydride, lithium aluminum hydride/copper(I) iodide, trimethoxyaluminum lithium hydride/copper(I) bromide, sodium boron hydride and potassium tri-s-butylboron hydride.

The reaction temperature which varies with the type of reducing agent is typically in the range of -80°C ~ 100°C, preferably -78°C ~ 80°C. The reaction time which varies with the reaction temperature and the like is typically in the range of 10 minutes - 24 hours, preferably 10 minutes - 6 hours.

Process D is for producing compound (30) represented by the general formula (I) in which $X^1$ is a group of β configuration that is represented by the general formula (II) in which Ar is a single bond, A is -O- and $R^1$ is -G-NHCO-Z, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

## Process D

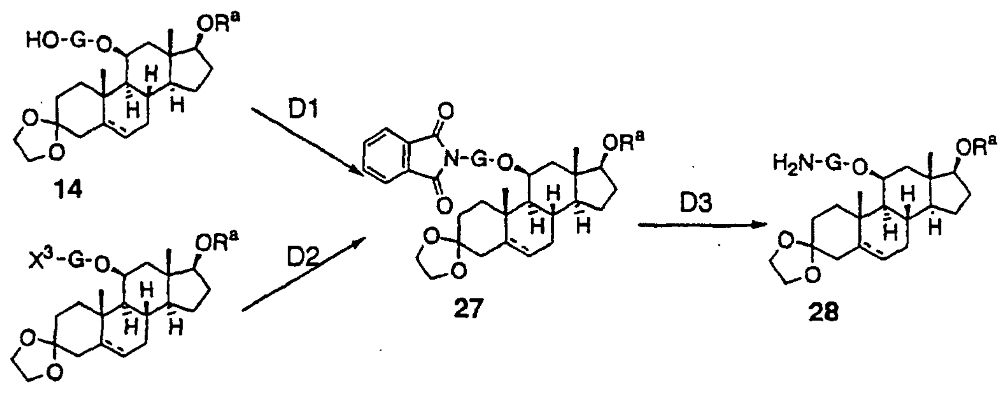


14

15

27

28

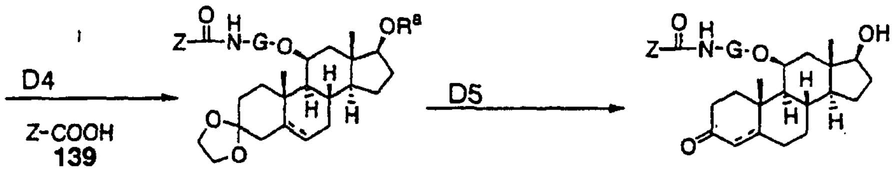


29

30

Step D1 is for producing compound (27) and implemented by reacting compound (14) with phthalimide in an inert solvent in the presence of an azodicarboxylic acid dialkyl ester (preferably diethyl azodicarboxylate) and a phosphine compound (preferably triphenylphosphine).

The inert solvent to be used is not limited in any particular way as long as it does not participate in the reaction; examples are ether solvents such as ether, tetrahydrofuran, dioxane and dimethoxyethane, and aromatic solvents such as benzene, toluene, xylene, quinoline and chlorobenzene, with tetrahydrofuran being preferred. The reaction temperature which varies with the type of solvent and the like is typically in the range of 0°C - 50°C (preferably 10°C - 30°C). The reaction time which varies with the reaction temperature and the like is typically in the range of 15 minutes - 48 hours (preferably 30 minutes - 24 hours).

Step D2 is an alternative step for producing compound (27) and implemented by reacting compound (15) with a metal salt of phthalimide (preferably phthalimide potassium) in an inert solvent.

The inert solvent to be used is not limited in any particular way as long as it does not participate in the reaction; examples include ether solvents such as ether, tetrahydrofuran, dioxane and dimethoxyethane, aromatic solvents such as benzene, toluene, xylene, quinoline and chlorobenzene, halogen-containing solvents such as dichloromethane, as well as dimethyl sulfoxide, dimethylacetamide, dimethylimidazolidinone, dimethylformamide, and N-methylpyrrolidone; a preferred example is tetrahydrofuran.

The reaction temperature which varies with the type of solvent and the like is typically in the range of 0°C - 50°C (preferably 10°C - 30°C). The reaction time which varies with the reaction temperature and the like is typically in the range of 15 minutes - 48 hours (preferably 30 minutes - 24 hours).

Step D3 is for producing compound (28) and implemented by reacing compound (27) with an amine-containing compound (preferably hydrazine) in an alcoholic solvent.

The alcoholic solvent to be used is not limited in any particular way as long as it does not interfere with the reaction and examples are methanol, ethanol, n-propyl alcohol and i-propyl alcohol, with ethanol being preferred.

The reaction temperature which varies with the type of solvent and the like is typically in the range of 0°C - 50°C (preferably 10°C - 30°C). The reaction time which varies with the reaction temperature and the like is typically in the range of 15 minutes - 48 hours (preferably 30 minutes - 24 hours).

Step D4 is for producing compound (29) and implemented by reacting compound (139) or reactive derivatives thereof (acid halides, mixed acid anhydrides or active esters) with compound (28) or acid addition salts thereof in an inert solvent. The reaction is performed as in the aforementioned step C3 in process C.

Step D5 is for producing compound (30) and implemented by reacting compound (29) with an acid in an aqueous solvent. The reaction is performed as in the aforementioned step A5 in process A.

Process D' is a method of producing compound (246) having the dashed line in compound (30) forming a

double bond together with the solid line, and compound (163) having the dashed line in compound (30) forming a single bond together with the solid line.

## Process D'

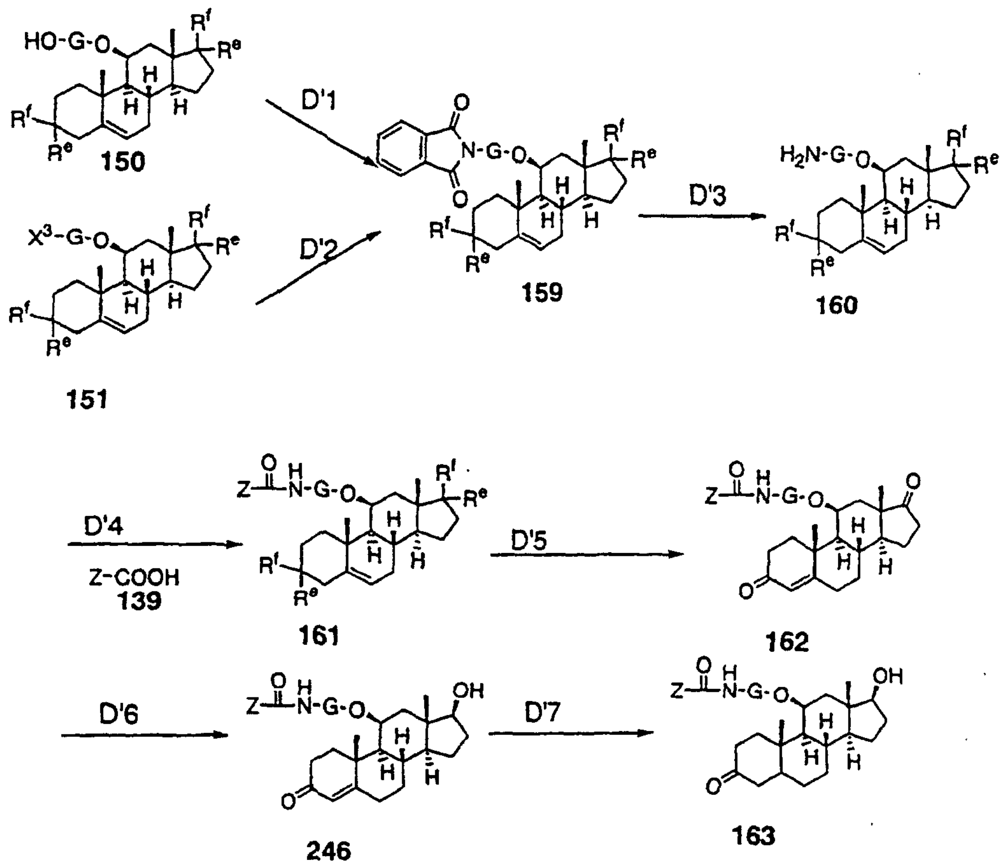


Step D'1 is for producing compound (159) and implemented by reacting compound (150) with phthalimide in an inert solvent in the presence of an azodicarboxylic acid dialkyl ester (preferably diethyl azodicarboxylate) and a phosphine compound (preferably triphenylphosphine). The reaction is performed as in the aforementioned step D1 in process D.

Step D'2 is an alternative step for producing compound (159) and implemented by reacting compound (151) with a metal salt of phthalimide (preferably phthalimide potassium) in an inert solvent. The reaction is performed as in the aforementioned step D2 in process D.

Step D'3 is for producing compound (160) and implemented by reacing compound (159) with an amine-containing compound (preferably hydrazine) in an alcoholic solvent. The reaction is performed as in the aforementioned step D3 in process D.

Step D'4 is for producing compound (161) and implemented by reacting compound (139) or reactive derivatives thereof (acid halides, mixed acid anhydrides or active esters) with compound (160) or acid addition salts thereof in an inert solvent. The reaction is performed as in the aforementioned step D4 in process D.

Step D'5 is for producing compound (162) and implemented by reacting compound (161) with an acid in an aqueous solvent. The reaction is performed as in the aforementioned step C4 in process C.

Step D'6 is for producing compound (246) and implemented by reacting compound (162) with a reducing agent in an optionally miscible inert solvent. The reaction is performed as in the aforementioned step A12 in process A.

Step D'7 is for producing compound (163) and implemented by performing catalytic reduction of compound (246) in an alcoholic solvent or an inert solvent or by reacting compound (246) with a reducing agent in an optionally miscible inert solvent. The reaction is performed as in the aforementioned step C'6 in process C'.

Process E is for producing compound (35) represented by the general formula (I) in which $X^1$ is a group of β

configuration that is represented by the general formula (II) in which Ar is a single bond, A is a methylene group and $R^1$ is $-CH=CH-CH_2-R^4$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (36) represented by the general formula (I) in which $X^1$ is a group of β configuration that is represented by the general formula (II) in which Ar is a single bond, A is a methylene group and $R^1$ is $-(CH_2)_3-R^4$, $X^2$ is a hydrogen atom, $R^a$ is a hydrogen atom, $R^b$ and $R^c$, when taken together with the carbon atom in 3-position to which they are bound, are -(C=O), and the dashed line together with the solid line is a single bond or a double bond; compound (38) represented by the general formula (I) in which $X^1$ is a group of β configuration that is represented by the general formula (II) in which Ar is a single bond, A is a methylene group and $R^1$ is $-(CH_2)_3-G^3-S(O)-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; and compound (39) represented by the general formula (I) in which $X^1$ is a group of β configuration that is represented by the general formula (II) in which Ar is a single bond, A is a methylene group and $R^1$ is $-(CH_2)_3-G^3-S(O)2-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

Process E

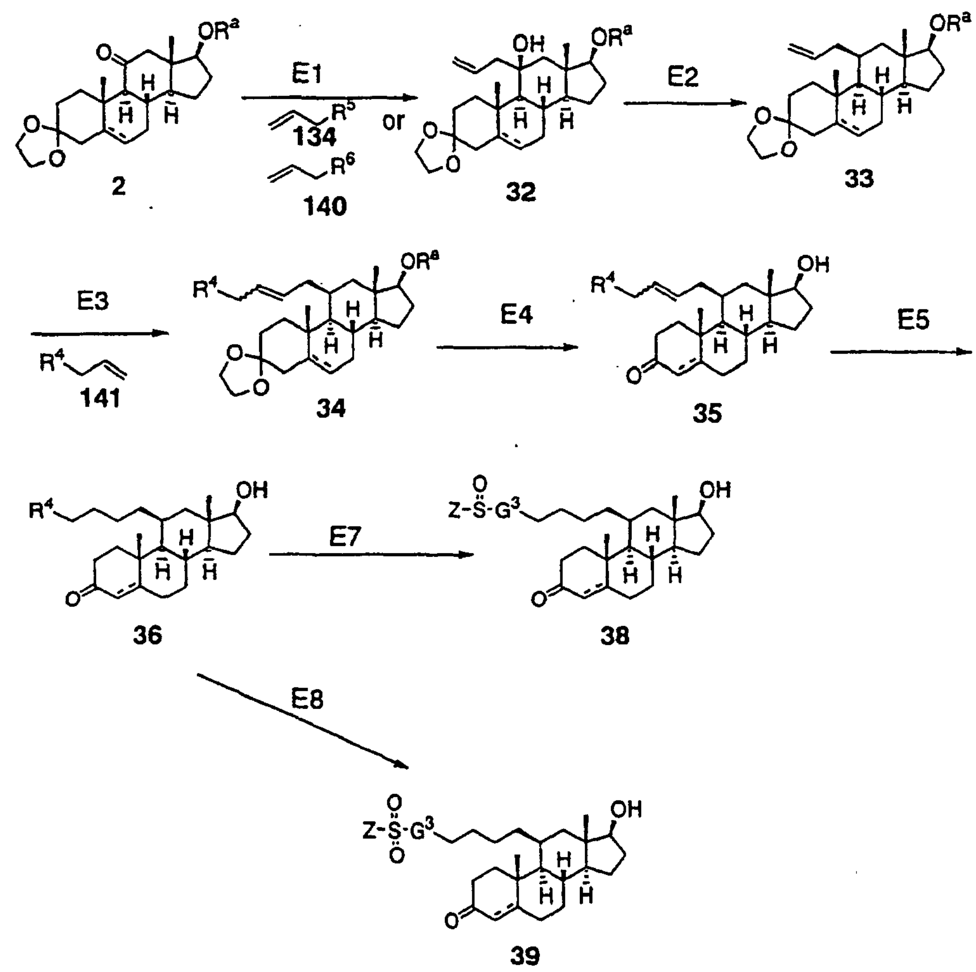

2
134
140
32
33
141
34
35
36
38
39

Step E1 is for producing compound (32) and implemented by reacting compound (134) with a metal (preferably magnesium) or an alkyllithium (preferably t-butyllithium) in an inert solvent to make a reactive derivative of compound (134) and reacting it with compound (2) in an inert solvent. The inert solvent to be used is not limited in any particular way as long as it does not participate in the reaction; preferred examples are ether solvents such as ether, tetrahydrofuran, dioxane and dimethoxyethane, and tetrahydrofuran is more preferred.

The reaction temperature which varies with the type of solvent and the like is typically in the range of 0°C -

80°C (preferably 10°C - 50°C). The reaction time which varies with the reaction temperature and the like is typically in the range of 15 minutes - 24 hours (preferably 30 minutes - 15 hours).

The reaction of interest can also be implemented by reacting compound (2) with compound (140) in an inert solvent in the presence of an activator.

The inert solvent to be used is not limited in any particular way as long as it does not participate in the reaction; preferred examples are ether solvents such as ether, tetrahydrofuran, dioxane and dimethoxyethane, and halogen-containing solvents such as dichloromethane; more preferred examples are tetrahydrofuran and dichloromethane.

The activator to be used is not limited in any particular way as long as it does not interfere with the reaction; examples are fluorides such as tetra-n-butylammonium fluoride, and Lewis acids such as aluminum trichloride, ethylaluminum dichloride, titanium tetrachloride, boron trifluoride, and trimethylsilyl trifluoromethanesulfonate; a preferred example is trimethylsilyl trifluoromethanesulfonate.

The reaction temperature which varies with the type of solvent and the like is typically in the range of -78°C ~ 50°C (preferably -60°C ~ 30°C). The reaction time which varies with the reaction temperature and the like is typically in the range of 15 minutes - 24 hours (preferably 30 minutes - 5 hours).

Step E2 is for producing compound (33) and implemented by reacting compound (32) with a reducing agent in an inert solvent in the presence of an additive.

The inert solvent to be used is not limited in any particular way as long as it does not participate in the reaction; examples include aromatic solvents such as benzene, toluene, xylene, quinoline and chlorobenzene, halogen-containing solvents such as dichloromethane, chloroform and carbon tetrachloride, and ether solvents such as ether, tetrahydrofuran, dioxane and dimethoxyethane; preferred examples are benzene, toluene and dichloromethane.

The additive to be used is not limited in any particular way as long as it does not interfer with the reaction and preferred examples are 1,1'-thiocarbonyl diimidazole, phosgene, benzoyl chloride, zinc iodide, boron trifluoride ($BF_3$), etc.

The reducing agent to be used may be exemplified by metal hydrides such as triphenyltin hydride, tri-n-butyltin hydride, diphenyltin hydride, di-n-butyltin hydride, triethyltin hydride, trimethyltin hydride, trichlorosilane/tri-n-butylamine, trichlorosilane/tri-n-propylamine, triethylsilane, trimethylsilane, diphenylsilane, phenylsilane, polymethylhydrosiloxane, dimethylphenylsilane, di-n-butylsilane and methylphenylsilane; and metal hydrogen complex compounds such as lithium aluminum hydride, trimethoxyaluminum lithium hydride, tri-t-butoxyaluminum lithium hydride, lithium aluminum hydride-trichloroaluminum (alane), lithium aluminum hydride-boron trifluoride, aluminum hydride magnesium chloride, magnesium aluminum hydride, sodium aluminum hydride, sodium triethoxyaluminum hydride, sodium bis(methoxyethoxy)aluminum hydride, sodium boron hydride, sodium boron hydride-palladium/carbon, sodium boron hydrogensulfide, sodium boron hydrogencyanide, sodium trimethoxyboron hydride, lithium boron hydride, lithium boron hydrogencyanide, lithium triethylboron hydride, lithium tri-s-butylboron hydride, lithium tri-t-butylboron hydride, calcium boron hydride, potassium boron hydride, potassium triisopropoxyboron hydride, potassium tri-s-butylboron hydride, zinc boron hydride, tetramethylammonium boron hydride, and tetra-n-butylammonium cyanoboron hydride; preferred examples are tri-n-butyltin hydride, triethylsilane and sodium boron hydrogencyanide.

The reaction temperature which varies with the type of solvent and the like is typically in the range of 0°C ~ 150°C (preferably 10°C ~ 100°C). The reaction time which varies with the reaction temperature and the like is typically in the range of 15 minutes - 24 hours (preferably 30 minutes - 15 hours).

In this step, a compound having the allyl group in the 11-position of compound (33) oriented in $\alpha$ configuration forms as a by-product and this may be used to give compounds having $X^1$ in compound (35), compound (36), compound (38) and compound (39) oriented in $\alpha$ configuration.

Step E3 is for producing compound (34) and implemented by reacting compound (33) with compound (141) in an inert solvent in the presence of an organometallic catalyst. The reaction is performed as in the aforementioned step A4 in process A.

Step E4 is for producing compound (35) and implemented by reacting compound (34) with an acid in an aqueous solvent. The reaction is performed as in the aforementioned step A5 in process A.

Step E5 is for producing compound (36) and implemented by performing catalytic reduction in an alcoholic solvent or an inert solvent. The reaction is performed as in the aforementioned step A6 in process A.

Step E7 is for producing compound (38) in the case where $Q^2$ in $R^4$ in compound (36) is -S- and implemented by reacting compound (36) with an oxidizing agent in an inert solvent. The reaction is performed as in the aforementioned step A8 in process A.

Step E8 is for producing compound (39) in the case where $Q^2$ in $R^4$ in compound (36) is -S- and implemented by reacting compound (36) with an oxidizing agent in an inert solvent. The reaction is performed as in the aforementioned step A9 in process A.

Process E' is an alternative method of producing compound (247) having the dashed line in compound (35) forming a double bond together with the solid line, and compound (248) having the dashed line in compound (36) forming a single bond together with the solid line.

Process E'

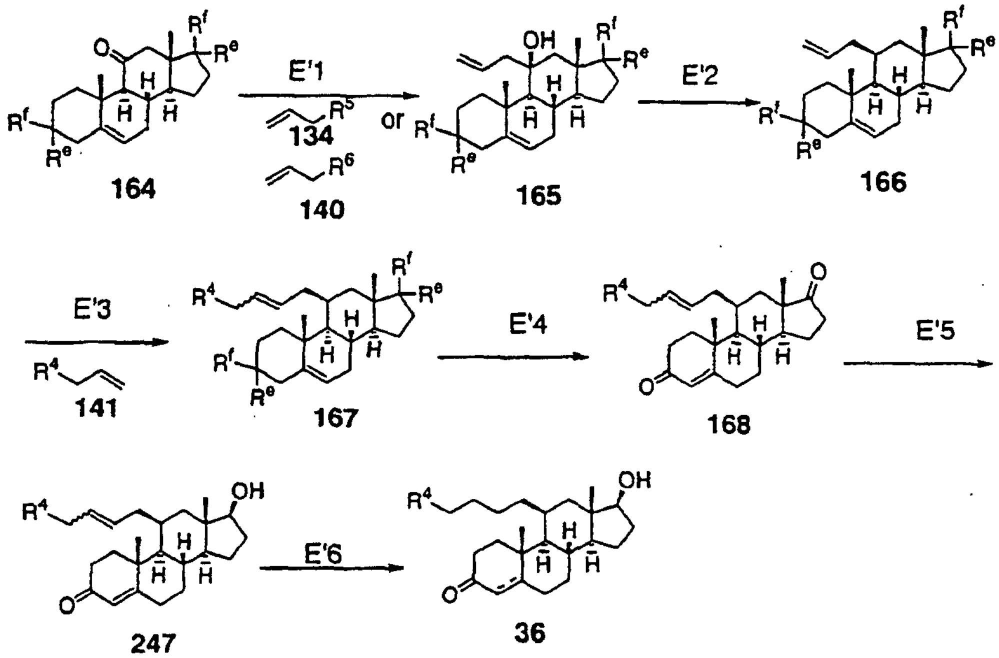


Step E'1 is for producing compound (165) and implemented by reacting compound (134) with a metal (preferably magnesium) or an alkyllithium (preferably t-butyllithium) in an inert solvent to make a reactive derivative of compound (134) and reacting it with compound (164) in an inert solvent. Alternatively, step E'1 may be implemented by reacting compound (164) with compound (140) in an inert solvent in the presence of an activator. The reaction is performed as in the aforementioned step E1 in process E.

Step E'2 is for producing compound (166) and implemented by reacting compound (166) with a reducing agent in an inert solvent in the presence of an additive. The reaction is performed as in the aforementioned step E2 in process E.

In this step, a compound having the allyl group in the 11-position of compound (166) oriented in $\alpha$ configuration forms as a by-product and this may be used to give compounds having $X^1$ in compound (35) and compound (36) oriented in $\alpha$ configuration.

Step E'3 is for producing compound (167) and implemented by reacting compound (166) with compound (141) in an inert solvent in the presence of an organometallic catalyst. The reaction is performed as in the aforementioned step E3 in process E.

Step E'4 is for producing compound (168) and implemented by reacting compound (167) with an acid in an aqueous solvent. The reaction is performed as in the aforementioned step D'5 in process D'.

Step E'5 is for producing compound (247) and implemented by reacting compound (168) with a reducing agent in an optionally miscible inert solvent. The reaction is performed as in the aforementioned step A12 in process A.

Step E'6 is for producing compound (36) and implemented by performing catalytic reduction in an alcoholic solvent or an inert solvent. The reaction is performed as in the aforementioned step A6 in process A.

Process F is for producing compound (49) represented by the general formula (I) in which $X^1$ is a group of $\beta$ configuration that is represented by the general formula (II) in which Ar is a single bond, A is a methylene group and $R^1$ is $-CH_2-G^4-S(O)-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond, and compound (52) represented by the general formula (I) in which $X^1$ is a group of $\beta$ configuration that is represented by the general formula (II) in which Ar is a single bond, A is a methylene group and $R^1$ is $-CH_2-G^4-S(O)_2-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

Process F

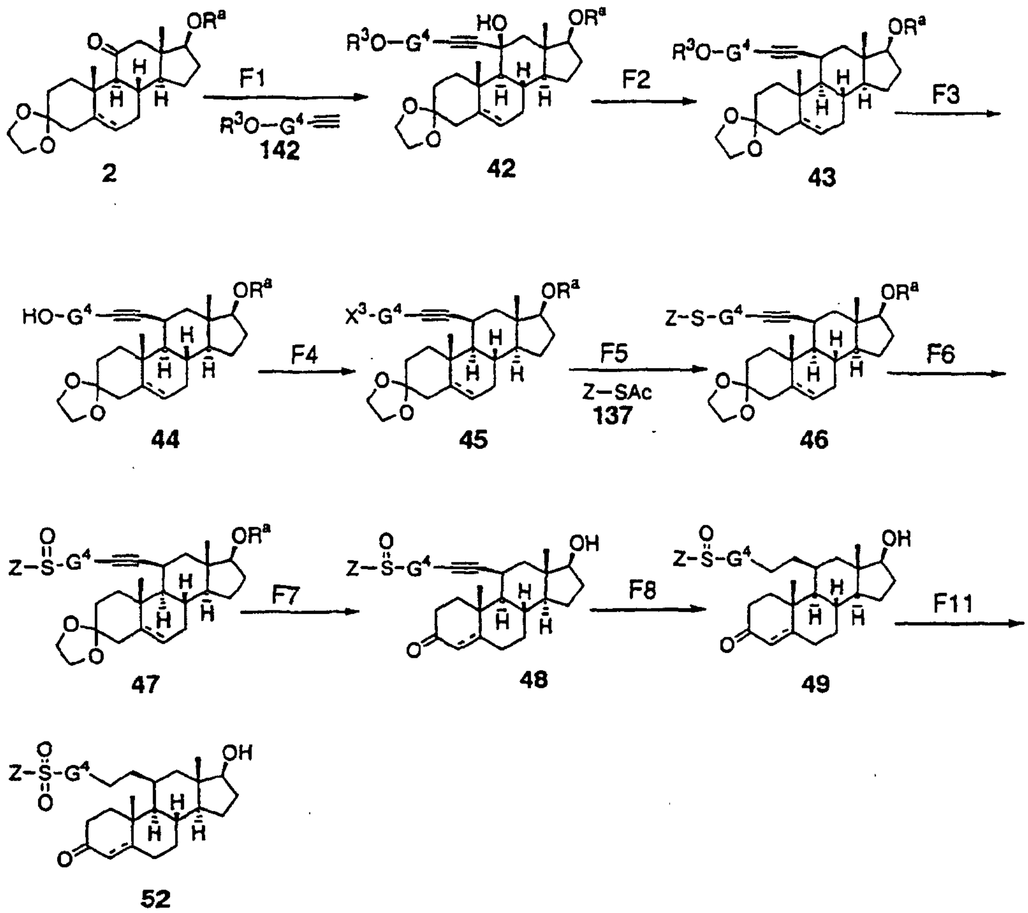


2 42 43 44 45 46 47 48 49 52

Step F1 is for producing compound (42) and implemented by reacting compound (142) with an alkyllithium (preferably n-butyllithium) in an inert solvent to make a reactive derivative of compound (142) and reacting it with compound (2) in an inert solvent.

The inert solvent to be used is not limited in any particular way as long as it does not participate in the reaction; preferred examples are ether solvents such as ether, tetrahydrofuran, dioxane and dimethoxyethane, and tetrahydrofuran is more preferred.

The reaction temperature which varies with the type of solvent and the like is typically in the range of 0 °C - 80 °C (preferably 10 °C - 50 °C). The reaction time which varies with the reaction temperature and the like is typically in the range of 15 minutes - 24 hours (preferably 30 minutes - 15 hours).

Step F2 is for producing compound (43) and implemented by reacting compound (42) with a reducing agent in an inert solvent in the presence of an additive. The reaction is performed as in the aforementioned step E2 in process E.

In this step, a compound having the substituent in the 11-position of compound (43) oriented in $\alpha$ configuration forms as a by-product and this may be used to give compounds having $X^1$ in compound (49) and compound (52) oriented in $\alpha$ configuration.

Step F3 is for producing compound (44) and implemented by reacting compound (43) with a deprotecting agent, namely, removing the substituted silyl group, in an inert solvent. The reaction is performed as in the aforementioned step B2 in process B.

Step F4 is for producing compound (45) and implemented by reacting compound (44) with a sulfonyl chloride compound in an amine-containing solvent or reacting compound (44) with a halogenating agent in an inert solvent. The reaction is performed as in the aforementioned stepp B3 in process B.

Step F5 is for producing compound (46) and implemented by reacting compound (137) with a metal alkoxide in an alcoholic solvent to make a reactive derivative of compound (137) and reacting it with compound (45) in an

alcoholic solvent. The reaction is performed as in the aforementioned step B4 in process B.

Step F6 is for producing compound (47) and implemented by reacting compound (46) with an oxidizing agent in an inert solvent. The reaction is performed as in the aforementioned step A8 in process A.

Step F7 is for producing compound (48) and implemented by reacting compound (47) with an acid in an aqueous solvent. The reaction is performed as in the aforementioned step A5 in process A.

Step F8 is for producing compound (49) and implemented by performing catalytic reduction of compound (48) in an alcoholic solvent or an inert solvent.

The solvent to be used may be exemplified by alcoholic solvents such as methanol, ethanol, n-propanol, i-propanol, n-butanol, s-butanol, t-butanol, pentanol, hexanol, cyclopropanol, cyclobutanol, cyclopentanol, cyclohexanol, ethylene glycol, 1,3-propanediol, 1,4-butanediol, and 1,5-pentanediol, ether solvents such as ether, tetrahydrofuran, dioxane and dimethoxyethane, aromatic solvents such as benzene, toluene, xylene, quinoline and chlorobenzene, halogen-containing solvents such as dichloromethane, chloroform and carbon tetrachloride, as well as cyclohexane, dimethyl sulfoxide, dimethylacetamide, dimethylimidazolidinone, dimethylformamide, N-methylpyrrolidone, ethyl acetate, acetonitrile and nitromethane; a preferred example is ethyl acetate.

The condition to be used in catalytic reduction is a homogeneous system such as hydrogen-chlorotris(triphenylphosphine)rhodium(I), hydrogen-chlorotris(triparatolylphosphine)rhodium(I), hydrogen-chlorotris(triparamethoxyphenylphosphine)rhodium(I), hydrogen-hydridecarbonyltris(triphenylphosphine)rhodium(I), hydrogen-rhodium(II) acetate, hydrogen-ruthenium(II) acetate, hydrogen-chlorohydridetris(triphenylphosphine)ruthenium(II), hydrogen-carboxylatohydridetris(triphenylphosphine)ruthenium(II), hydrogen-hydridecarbonyltris(triphenylphosphine)iridium(I), hydrogen-platinum(II)-tin chloride complex, hydrogen-pentacyanocobalt(II) complex, hydrogen-tricyanobipyridine cobalt(II) complex, hydrogen-bis(dimethylglyoximato)cobalt(II) complex, hydrogen-methyl benzoate-tricarbonylchromium complex, hydrogen-bis(tricarbonylcyclopentadienylchromium), hydrogen-pentacarbonyliron, hydrogen-bis(cyclopentadienyl)dicarbonyltitanium, hydrogen-hydridecarbonylcobalt complex, hydrogen-octacarbonyldicobalt, hydrogen-hydridecarbonylrhodium, hydrogen-chromium(III) acetylacetonato-triisobutylaluminum, hydrogen-cobalt(II) acetylacetonato-triisobutylaluminum, or hydrogen-nickel(II)-2-hexanoato-triethylaluminum, or an inhomogeneous system condition such as hydrogen-platinum dioxide, hydrogen-platinum/carbon, hydrogen-palladium/carbon, hydrogen-palladium/barium sulfate, hydrogen-palladium/calcium carbonate, hydrogen-Raney nickel, hydrogen-copper chromite, hydrogen-rhodium/carbon, hydrogen-rhodium/alumina, hydrogen-ruthenium dioxide, or hydrogen-ruthenium/carbon; a preferred example is hydrogen palladium/carbon.

The reaction temperature is typically in the range of 0°C - 100°C, preferably 0°C - 60°C. The reaction time which varies with the reaction temperature and the like is typically in the range of 10 minutes - 24 hours, preferably 10 minutes- 6 hours.

As an ancillary to this reaction, conversion to a single bond may occasionally be effected if the dashed line forms a double bond together with the solid line.

Step F11 is for producing compound (52) and implemented by reacting compound (49) with an oxidizing agent in an inert solvent. The reaction is performed as in the aforementioned step B7 in process B.

Process F' is an alternative method of producing compound (249) having the dashed line in compound (48) forming a double bond together with the solid line.

Process F'

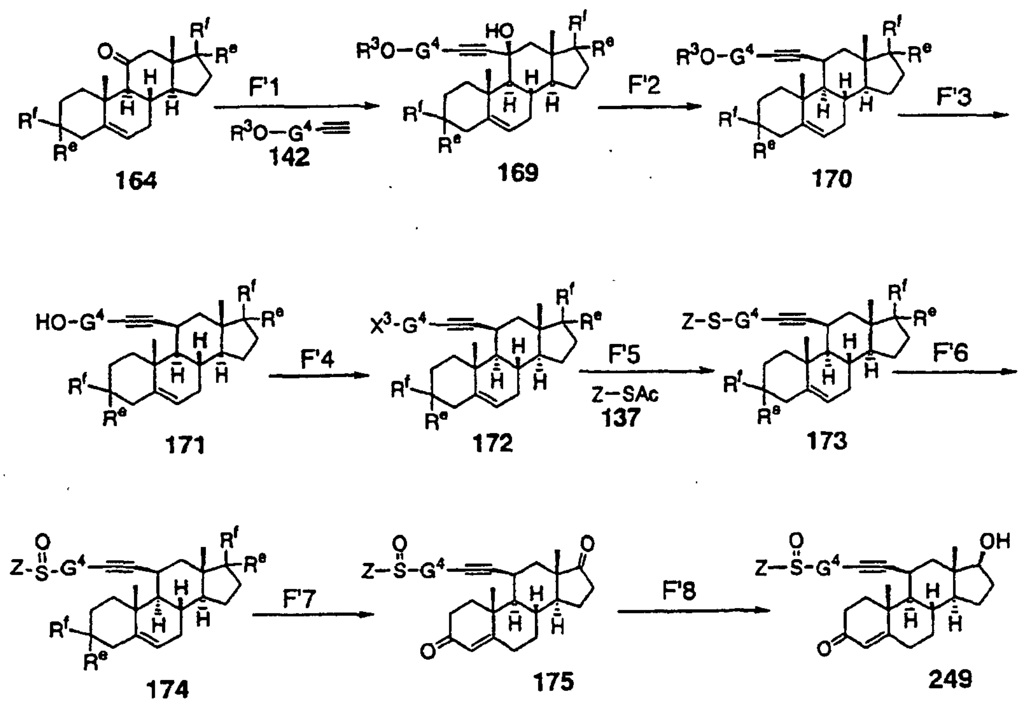


Step F'1 is for producing compound (169) and implemented by reacting compound (142) with an alkyllithium (preferably n-butyllithium) in an inert solvent to make a reactive derivative of compound (142) and reacting it with compound (164) in an inert solvent. The reaction is performed as in the aforementioned step F1 in process F.

Step F'2 is for producing compound (170) and implemented by reacting compound (169) with a reducing agent in an inert solvent in the presence of an additive. The reaction is performed as in the aforementioned step F2 in process F.

In this step, a compound having the substituent in the 11-position of compound (170) oriented in $\alpha$ configuration forms as a by-product and this may be used to give a compound having $X^1$ in compound (249) oriented in $\alpha$ configuration.

Step F'3 is for producing compound (171) and implemented by reacting compound (170) with a deprotecting agent, namely, removing the substituted silyl group, in an inert solvent. The reaction is performed as in the aforementioned step F3 in process F.

Step F'4 is for producing compound (172) and implemented by reacting compound (171) with a sulfonyl chloride compound in an amine-containing solvent or reacting compound (171) with a halogenating agent in an inert solvent. The reaction is performed as in the aforementioned stepp F4 in process F.

Step F'5 is for producing compound (173) and implemented by reacting compound (137) with a metal alkoxide in an alcoholic solvent to make a reactive derivative of compound (137) and reacting it with compound (172) in an alcoholic solvent. The reaction is performed as in the aforementioned step F5 in process F.

Step F'6 is for producing compound (174) and implemented by reacting compound (173) with an oxidizing agent in an inert solvent. The reaction is performed as in the aforementioned step F6 in process F.

Step F'7 is for producing compound (175) and implemented by reacting compound (174) with an acid in an aqueous solvent. The reaction is performed as in the aforementioned step E'4 in process E.

Step F'8 is for producing compound (249) and implemented by reacting compound (175) with a reducing agent in an optionally miscible inert solvent. The reaction is performed as in the aforementioned step E'5 in process E.

Process G is for producing compound (56) represented by the general formula (I) in which $X^1$ is a group of $\beta$ configuration that is represented by the general formula (II) in which Ar is a single bond, A is a methylene group and $R^1$ is $-CH_2-G^4-COOH$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond, and compound (57) represented by the general formula (I) in which $X^1$ is a group of $\beta$ configuration that is represented by the general formula (II) in which Ar is a single bond, A is a methylene group and $R^1$ is $-CH_2-G^4-CONH-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single

bond or a double bond.

## Process G

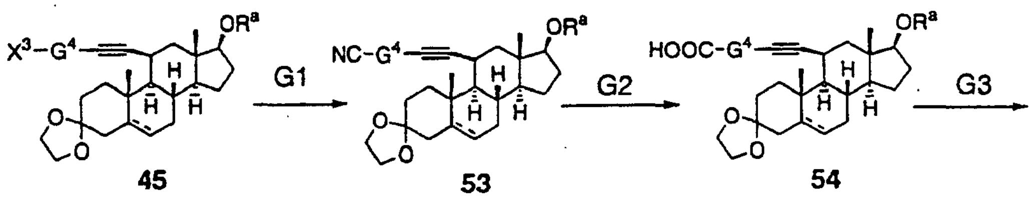


45 53 54

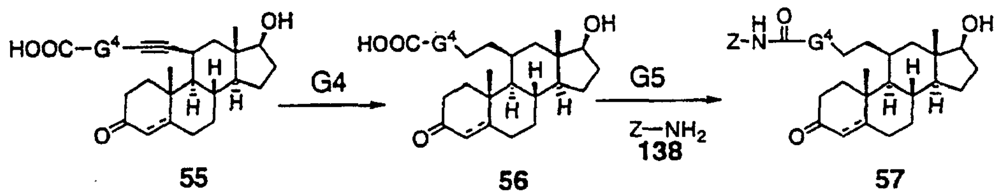


55 56 57

Step G1 is for producing compound (53) and implemented by reacting compound (45) with a cyanylating agent in an inert solvent. The reaction is performed as in the aforementioned step C1 in process C.

Step G2 is for producing compound (54) and implemented by hydrolyzing compound (53) in the presence of a base. The reaction is performed as in the aforementioned step C2 in process C.

Step G3 is for producing compound (55) and implemented by reacting compound (54) with an acid in an aqueous solvent. The reaction is performed as in the aforementioned step A5 in process A.

Step G4 is for producing compound (56) and implemented by performing catalytic reduction in an alcoholic solvent or an inert solvent. The reaction is performed as in the aforementioned step F8 in process F.

As an ancillary to this reaction, conversion to a single bond may occasionally be effected if the dashed line forms a double bond together with the solid line.

Step G5 is for producing compound (57) and implemented by reacting compound (56) or reactive derivatives thereof (acid halides, mixed acid anhydrides or active esters) with compound (138) or acid addition salts thereof in an inert solvent. The reaction is performed as in the aforementioned step C3 in process C.

Process G' is an alternative method of producing compound (250) having the dashed line in compound (55) forming a double bond together with the solid line.

## Process G'

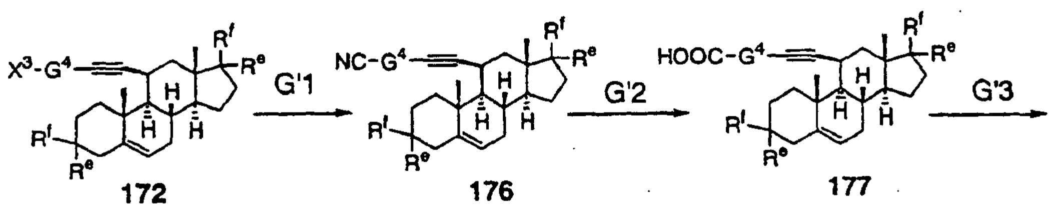


172 176 177

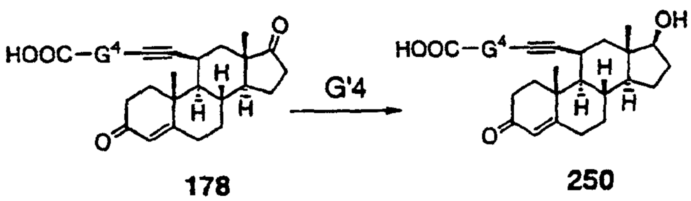


178 250

Step G'1 is for producing compound (176) and implemented by reacting compound (172) with a cyanylating agent in an inert solvent. The reaction is performed as in the aforementioned step G1 in process G.

Step G'2 is for producing compound (177) and implemented by hydrolyzing compound (176) in the presence of a base. The reaction is performed as in the aforementioned step G2 in process G.

Step G'3 is for producing compound (178) and implemented by reacting compound (177) with an acid in an aqueous solvent. The reaction is performed as in the aforementioned step F'7 in process F'.

Step G'4 is for producing compound (250) and implemented by reacing compound (178) with a reducing agent in an optionally miscible inert solvent. The reaction is performed as in the aforementioned step F'8 in process F'.

Process H is for producing compound (63) represented by the general formula (I) in which $X^1$ is a group of β configuration that is represented by the general formula (II) in which Ar is a single bond, A is a methylene group and $R^1$ is $-CH_2-G^4-NHCO-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

Process H

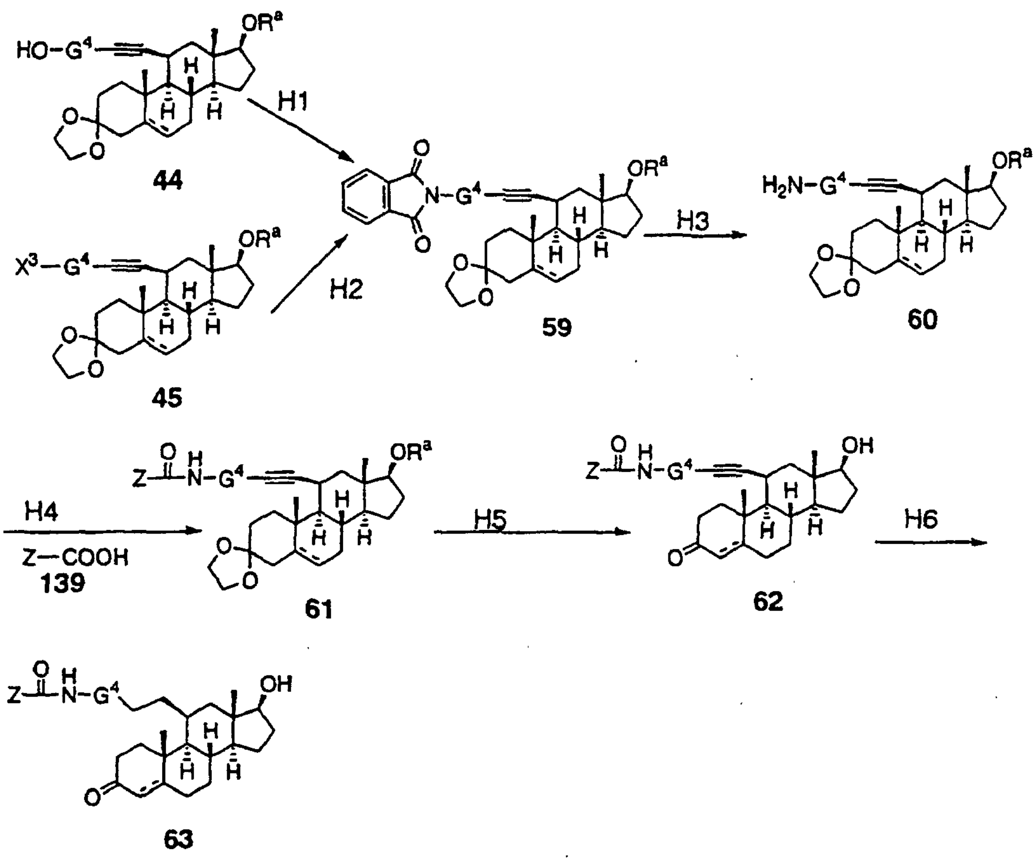


44, 45, 59, 60, 61, 62, 63

Step H1 is for producing compound (59) and implemented by reacting compound (44) with phthalimide in an inert solvent in the presence of an azodicarboxylic acid dialkyl ester (preferably diethyl azodicarboxylate) and a phosphine compound (preferably triphenylphosphine). The reaction is performed as in the aforementioned step D1 in process D.

Step H2 is an alternative step for producing compound (59) and implemented by reacting compound (45) with a metal salt of phthalimide (preferably phthalimide potassium) in an inert solvent. The reaction is performed as in the aforementioned step D2 in process D.

Step H3 is for producing compound (60) and implemented by reacting compound (59) with an amine-containing compound (preferably hydrazine) in an alcoholic solvent. The reaction is performed as in the aforementioned step D3 in process D.

Step H4 is for producing compound (61) and implemented by reacting compound (139) or reactive derivatives

thereof (acid halides, mixed acid anhydrides or active esters) with compound (60) or acid addition salts thereof in an inert solvent. The reaction is performed as in the aforementioned step C3 in process C.

Step H5 is for producing compound (62) and implemented by reacting compound (61) with an acid in an aqueous solvent. The reaction is performed as in the aforementioned step A5 in process A.

Step H6 is for producing compound (63) and implemented by performing catalytic reduction in an alcoholic solvent or an inert solvent. The reaction is performed as in the aforementioned step F8 in process F.

As an ancillary to this reaction, conversion to a single bond may occasionally be effected if the dashed line forms a double bond together with the solid line.

Process H' is an alternative method of producing compound (251) having the dashed line in compound (62) forming a double bond together with the solid line.

Process H'

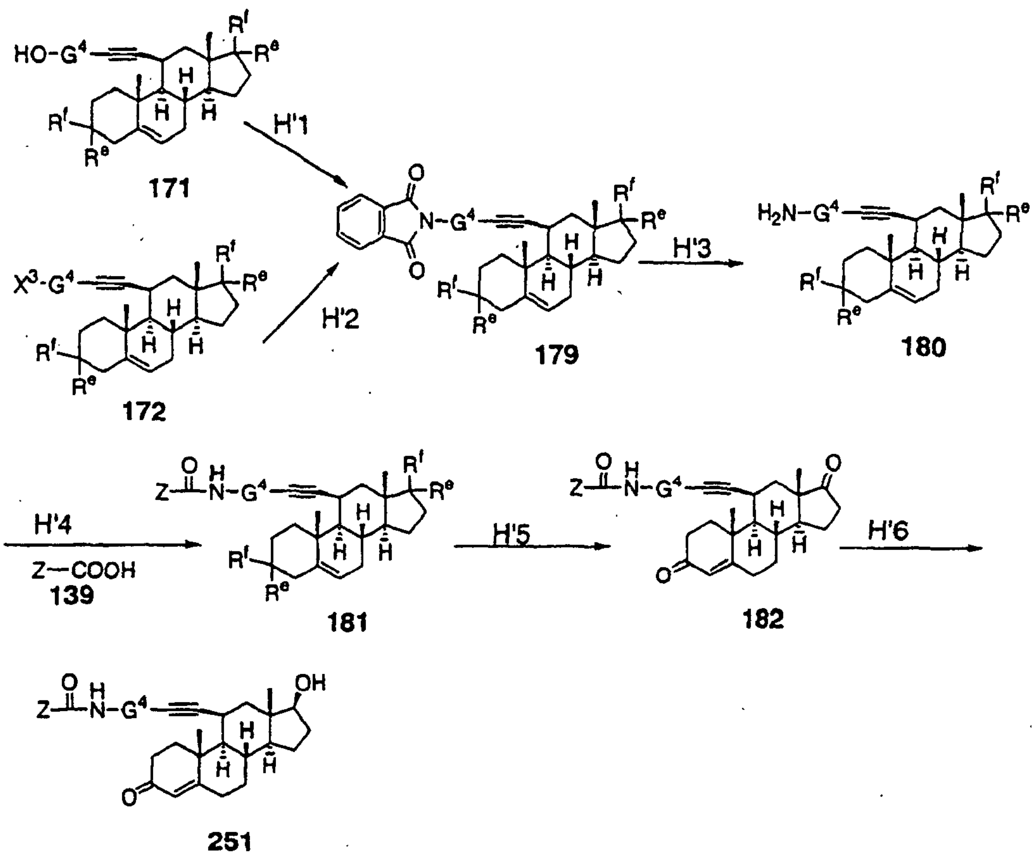


171, 172, 179, 180, 181, 182, 251

Step H'1 is for producing compound (179) and implemented by reacting compound (171) with phthalimide in an inert solvent in the presence of an azodicarboxylic acid dialkyl ester (preferably diethyl azodicarboxylate) and a phosphine compound (preferably triphenylphosphine). The reaction is performed as in the aforementioned step H1 in process H.

Step H'2 is an alternative step for producing compound (179) and implemented by reacting compound (172) with a metal salt of phthalimide (preferably phthalimide potassium) in an inert solvent. The reaction is performed as in the aforementioned step H2 in process H.

Step H'3 is for producing compound (180) and implemented by reacting compound (179) with an amine-containing compound (preferably hydrazine) in an alcoholic solvent. The reaction is performed as in the aforementioned step H3 in process H.

Step H'4 is for producing compound (181) and implemented by reacting compound (139) or reactive derivatives thereof (acid halides, mixed acid anhydrides or active esters) with compound (180) or acid addition salts thereof in an inert solvent. The reaction is performed as in the aforementioned step H4 in process H.

Step H'5 is for producing compound (182) and implemented by reacting compound (181) with an acid in an

aqueous solvent. The reaction is performed as in the aforementioned step G'3 in process G'.

Step H'6 is for producing compound (251) and implemented by reacting compound (182) with a reducing agent in an optionally miscible inert solvent. The reaction is performed as in the aforementioned step G'4 in process G'.

Process I is for producing compound (70) represented by the general formula (I) in which $X^1$ is a group of β configuration that is represented by the general formula (II) in which Ar is an aromatic hydrocarbon group (preferably a p-phenylene group), A is -O- and $R^1$ is $-CH_2-CH=CH-CH_2-R^2$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (71) represented by the general formula (I) in which $X^1$ is a group of β configuration that is represented by the general formula (II) in which Ar is an aromatic hydrocarbon group (preferably a p-phenylene group), A is -O- and $R^1$ is $-(CH_2)_4-R^2$, $X^2$ is a hydrogen atom, $R^a$ is a hydrogen atom, $R^b$ and $R^c$, when taken together with the carbon atom in 3-position to which they are bound, are -(C=O), and the dashed line together with the solid line is a single bond or a double bond; compound (73) represented by the general formula (I) in which $X^1$ is a group of β configuration that is represented by the general formula (II) in which Ar is an aromatic hydrocarbon group (preferably a p-phenylene group), A is -O- and $R^1$ is $-(CH_2)_4-G^2-S(O)-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; and compound (74) represented by the general formula (I) in which $X^1$ is a group of β configuration that is represented by the general formula (II) in which Ar is an aromatic hydrocarbon group (peferably a p-phenylene group), A is -O- and $R^1$ is $-(CH_2)_4-G^2-S(O)_2-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

Process I

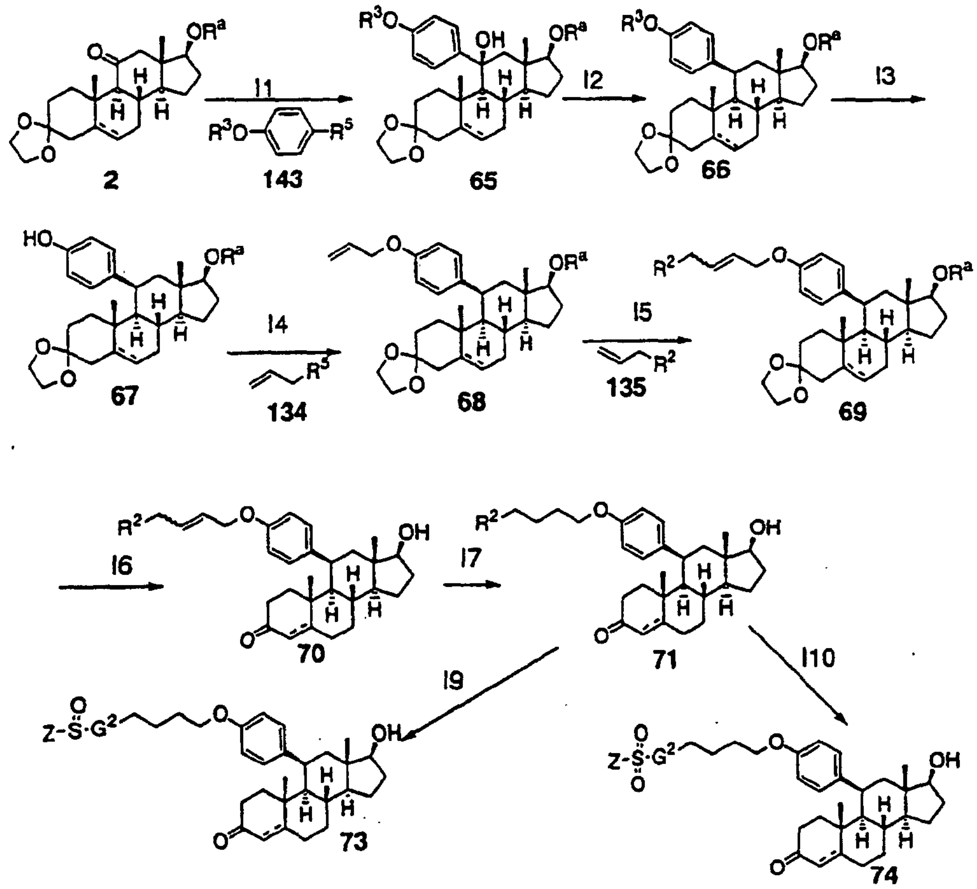


Step I1 is for producing compound (65) and implemented by reacting compound (143) with a metal (preferably magnesium) or an alkyllithium (preferably n-butyllithium) in an inert solvent to make a reactive derivative of compound

(143) and reacting it with compound (2) in an inert solvent. The inert solvent to be used is not limited in any particular way as long as it does not participate in the reaction; preferred examples are ether solvents such as ether, tetrahydrofuran, dioxane and dimethoxyethane, and tetrahydrofuran is more preferred. The reaction temperature which varies with the type of solvent and the like is typically in the range of 0°C - 80°C (preferably 10°C - 50°C). The reaction time which varies with the reaction temperature and the like is typically in the range of 15 minutes - 24 hours (preferably 30 minutes - 15 hours).

Step 12 is for producing compound (66) and implemented by reacting compound (65) with a reducing agent in an inert solvent in the presence of an additive. The reaction is performed as in the aforementioned step E2 in process E.

In this step, a compound having the $-C_6H_4-OR^3$ in the 11-position of compound (66) oriented in α configuration forms as a by-product and this may be used to give compounds having $X^1$ in compound (70), compound (71), compound (73) and compound (74) oriented in α configuration.

For the synthesis of compound (66) and a compound having the $-C_6H_4-OR^3$ in the 11-position of compound (66) oriented in α configuration, reference may be had to the methods of introducing a variety of aromatic hydrocaron groups as disclosed in Tetrahedron, vol. 52, 1529-1542, 1996.

Step I3 is for producing compound (67) and implemented by reacting compound (66) with a deprotecting agent, namely, removing the substituted silyl group, in an inert solvent.

The inert solvent to be used is not limited in an particular way as long as it does not interfere with the reaction; examples include ether solvents such as ether, tetrahydrofuran, dioxane and dimethoxyethane, as well as dimethylformamide and water, with tetrahydrofuran being preferred. The deprotecting agent to be used is not limited in any particular way and may be exemplified by fluorides such as hydrogen fluoride, hydrogen fluoride-pyridine, sodium fluoride, potassium fluoride and tetra-n-butylammonium fluoride, and organic acids such as formic acid, acetic acid and p-toluenesulfonic acid, with tetra-n-butylammonium fluoride being preferred.

The reaction temperature which varies with the type of solvent and the like is typically in the range of 0°C - 80°C (preferably 0°C - 50°C). The reaction time which varies with the reaction temperature and the like is typically in the range of 15 minutes - 24 hours (preferably 30 minutes - 15 hours).

Step 14 is for producing compound (68) and implemented by reacting compound (67) with a base in an inert solvent to make a salt of compound (67) and then reacting it with compound (134) in an inert solvent. The reaction is performed as in the aforementioned step A3 in process A.

Step I5 is for producing compound (69) and implemented by reacting compound (68) with compound (135) in an inert solvent in the presence of an organometallic catalyst. The reaction is performed as in the aforementioned step A4 in process A.

Step 16 is for producing compound (70) and implemented by reacting compound (69) with an acid in an aqueous solvent. The reaction is performed as in the aforementioned step A5 in process A.

Step 17 is for producing compound (71) and implemented by performing catalytic reduction in an alcoholic solvent or an inert solvent. The reaction is performed as in the aforementioned step A6 in process A.

Step I9 is for producing compound (73) in the case where $Q^2$ in $R^2$ in compound (71) is -S- and implemented by reacting compound (71) with an oxidizing agent in an inert solvent. The reaction is performed as in step A8 in process A.

Step I10 is for producing compound (74) in the case where $Q^2$ in $R^2$ in compound (74) is -S- and implemented by reacting compound (71) with an oxidizing agent in an inert solvent. The reaction is performed as in the aforementioned step A9 in process A.

Process I' is an alternative method of producing compound (252) having the dashed line in compound (70) forming a double bond together with the dashed line.

## Process I'

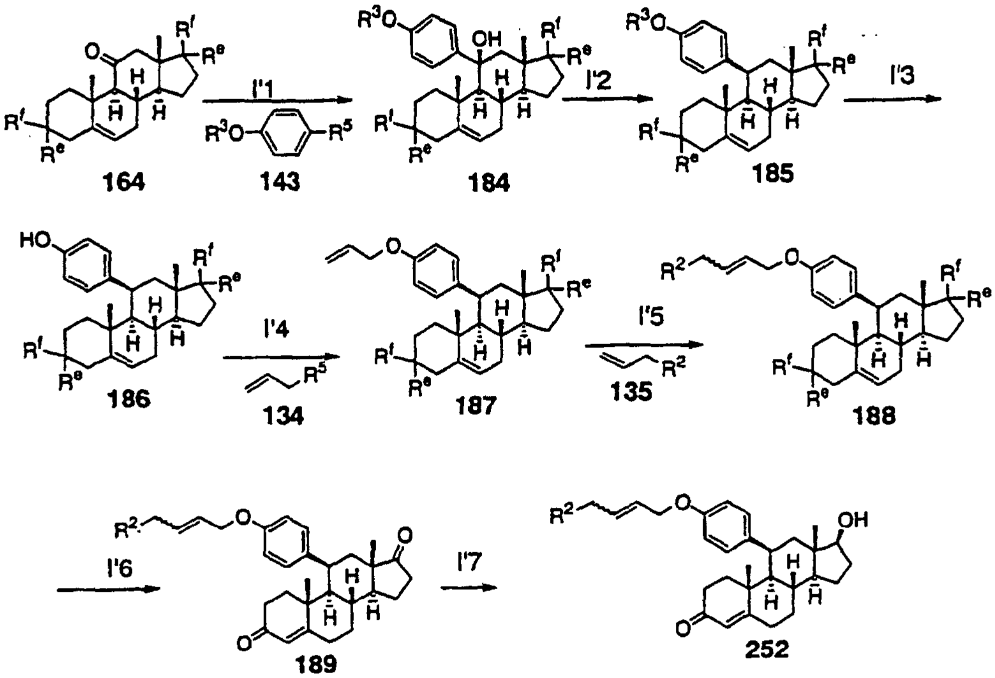


Step I'1 is for producing compound (184) and implemented by reacting compound (143) with a metal (preferably magnesium) or an alkyllithium (preferably n-butyllithium) in an inert solvent to make a reactive derivative of compound (143) and reacting it with compound (164) in an inert solvent. The reaction is performed as in the aforementioned step I1 in process I.

Step I'2 is for producing compound (185) and implemented by reacting compound (184) with a reducing agent in an inert solvent in the presence of an additive. The reaction is performed as in the aforementioned step I2 in process I.

In this step, a compound having the $-C_6H_4-OR^3$ in the 11-position of compound (185) oriented in $\alpha$ configuration forms as a by-product and this may be used to give a compound having $X^1$ in compound (252) oriented in $\alpha$ configuration.

For the synthesis of compound (185) and a compound having the $-C_6H_4-OR^3$ in the 11-position of compound (185) oriented in $\alpha$ configuration, reference may be had to the methods of introducing a variety of aromatic hydrocaron groups as disclosed in Tetrahedron, vol. 52, 1529-1542, 1996.

Step I'3 is for producing compound (186) and implemented by reacting compound (185) with a deprotecting agent, namely, removing the substituted silyl group, in an inert solvent. The reaction is performed as in the aforementioned step I3 in process I.

Step I'4 is for producing compound (187) and implemented by reacting compound (186) with a base in an inert solvent to make a salt of compound (186) and then reacting it with compound (134) in an inert solvent. The reaction is performed as in the aforementioned step I4 in process I.

Step I'5 is for producing compound (188) and implemented by reacting compound (187) with compound (135) in an inert solvent in the presence of an organometallic catalyst. The reaction is performed as in the aforementioned step I5 in process I.

Step I'6 is for producing compound (189) and implemented by reacting compound (188) with an acid in an aqueous solvent. The reaction is performed as in the aforementioned step H'5 in process H'.

Step I'7 is for producing compound (252) and implemented by reacting compound (189) with a reducing agent in an optionally miscible inert solvent. The reaction is performed as in the aforementioned step H'6 in process H'.

Process J is for producing compound (81) represented by the general formula (I) in which $X^1$ is a group of $\beta$ configuration that is represented by the general formula (II) in which Ar is an aromatic hydrocarbon group (preferably a p-phenylene group), A is -O- and $R^1$ is -G-S-Z, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (82) represented by the general formula (I) in which $X^1$ is a group of $\beta$ configuration that is represented by the general formula (II) in which Ar is an aromatic hydrocarbon

group (preferably a p-phenylene group), A is -O- and $R^1$ is -G-S(O)-Z, $X^2$ is a hydrogen atom, $R^a$ is a hydrogen atom, $R^b$ and $R^c$, when taken together with the carbon atom in 3-position to which they are bound, are -(C=O), and the dashed line together with the solid line is a single bond or a double bond; and compound (83) represented by the general formula (I) in which $X^1$ is a group of β configuration that is represented by the general formula (II) in which Ar is an aromatic hydrocarbon group (preferably a p-phenylene group), A is -O- and $R^1$ is $-G^2-S(O)_2-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

## Process J

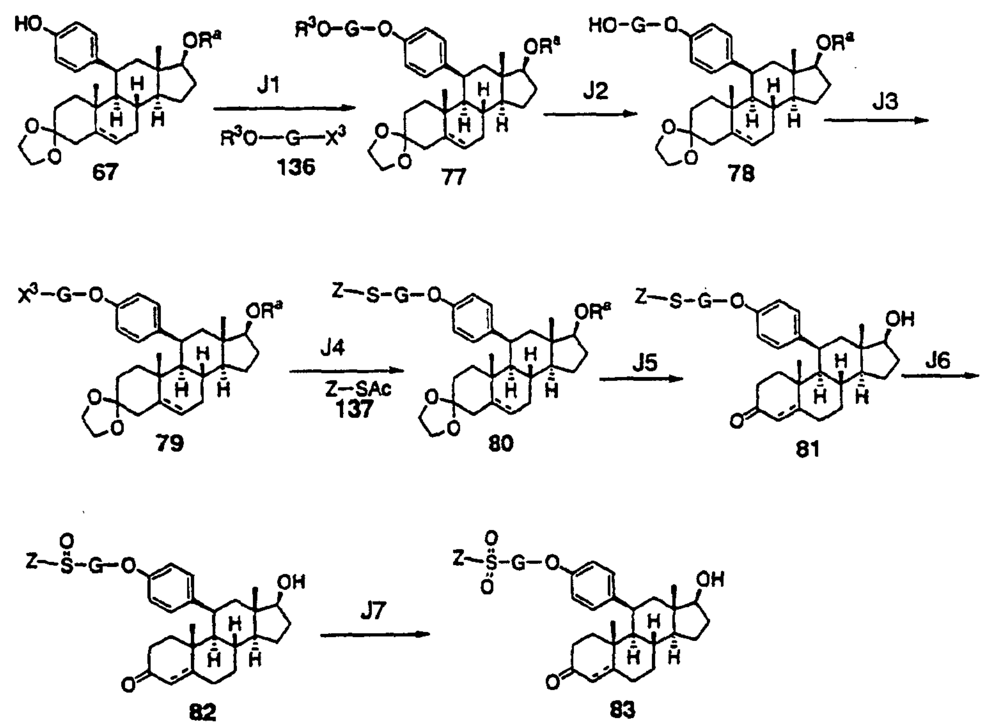

67 77 78 79 80 81 82 83

Step J1 is for producing compound (77) and implemented by reacting compound (67) with a base in an inert solvent to make a salt of compound (67) and reacting it with compound (136) in an inert solvent. The reaction is performed as in the aforementioned step A3 in process A.

Step J2 is for producing compound (78) and implemented by reacting compound (77) with a deprotecting agent, namely, removing the substituted silyl group, in an inert solvent. The reaction is performed as in the aforementioned step B2 in process B.

Step J3 is for producing compound (79) and implemented by reacting compound (78) with a sulfonyl chloride compound in an amine-containing solvent or reacting compound (78) with a halogenating agent in an inert solvent. The reaction is performed as in the aforementioned step B3 in process B.

Step J4 is for producing compound (80) and implemented by reacting compound (137) with a metal alkoxide in an alcoholic solvent to make a reactive derivative of compound (137) and reacting it with compound (79) in an alcoholic solvent. The reaction is performed as in the aforementioned step B4 in process B.

Step J5 is for producing compound (81) and implemented by reacting compound (80) with an acid in an aqueous solvent. The reaction is performed as in the aforementioned step A5 in process A.

Step J6 is for producing compound (82) and implemented by reacting compound (81) with an oxidizing agent in an inert solvent. The reaction is performed as in the aforementioned step A8 in process A.

Step J7 is for producing compound (83) and implemented by reacting compound (82) with an oxidizing agent in an inert solvent. The reaction is performed as in the aforementioned step B7 in process B.

Process J' is an alternative method for producing compound (253) having the dashed line in compound (81) forming a double bond together with the solid line, and compound (254) having the dashed line in compound (81) forming a single bond together with the solid line.

## Process J'

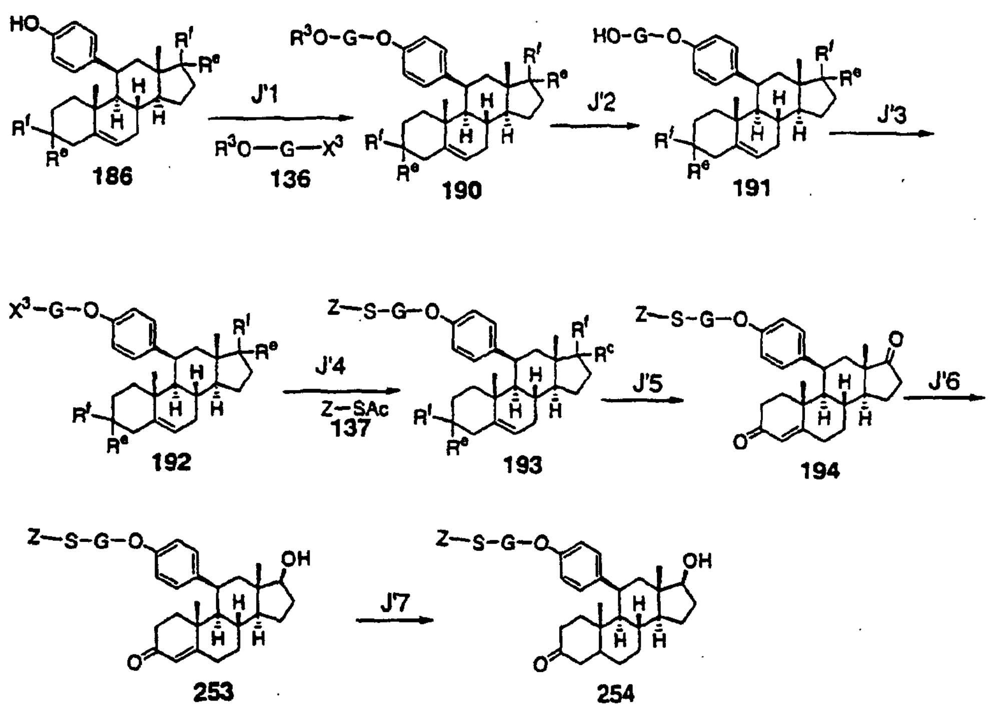


Step J'1 is for producing compound (190) and implemented by reacting compound (186) with a base in an inert solvent to make a salt of compound (186) and reacting it with compound (136) in an inert solvent. The reaction is performed as in the aforementioned step J1 in process J.

Step J'2 is for producing compound (191) and implemented by reacting compound (190) with a deprotecting agent, namely, removing the substituted silyl group, in an inert solvent. The reaction is performed as in the aforementioned step J2 in process J.

Step J'3 is for producing compound (192) and implemented by reacting compound (191) with a sulfonyl chloride compound in an amine-containing solvent or reacting compound (191) with a halogenating agent in an inert solvent. The reaction is performed as in the aforementioned step J3 in process J.

Step J'4 is for producing compound (193) and implemented by reacting compound (137) with a metal alkoxide in an alcoholic solvent to make a reactive derivative of compound (137) and reacting it with compound (192) in an alcoholic solvent. The reaction is performed as in the aforementioned step J4 in process J.

Step J'5 is for producing compound (194) and implemented by reacting compound (193) with an acid in an aqueous solvent. The reaction is performed as in the aforementioned step I'6 in process I'.

Step J'6 is for producing compound (253) and implemented by reacting compound (194) with a reducing agent in an optionally miscible inert solvent. The reaction is performed as in the aforementioned step I'7 in process I'.

Step J'7 is for producing compound (254) and implemented by performing catalytic reduction of compound (253) in an alcobolic solvent or an inert solvent or reacting compound (253) with a reducing agent in an optionally miscible inert solvent. The reaction is performed as in the aforementioned step C'6 in process C'.

Process K is for producing compound (89) represented by the general formula (I) in which $X^1$ is a group of β configuration that is represented by the general formula (II) in which Ar is an aromatic hydrocarbon group (preferably a p-phenylene group), A is -O- and $R^1$ is -G-CONH-Z, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

Process K

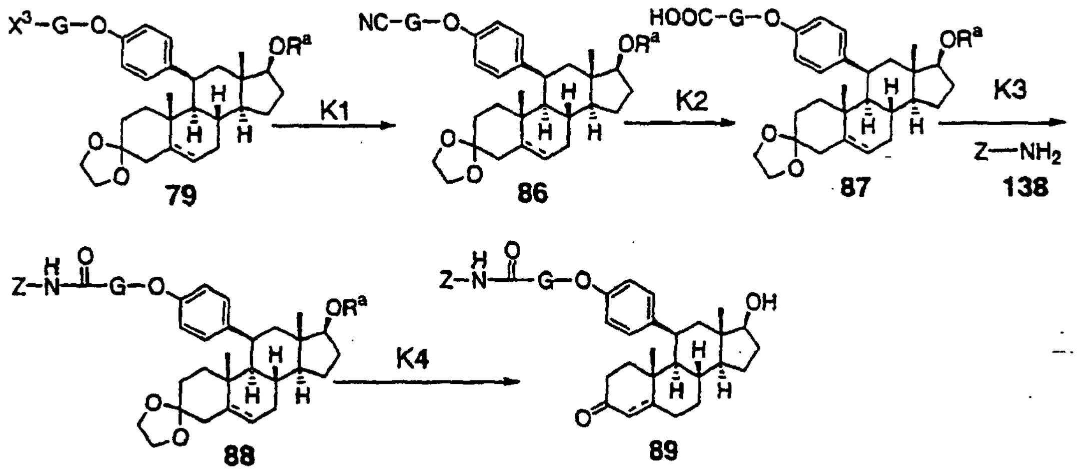


79 86 87 138 88 89

Step K1 is for producing compound (86) and implemented by reacting compound (79) with a cyanylating agent in an inert solvent. The reaction is performed as in the aforementioned step C1 in process C.

Step K2 is for producing compound (87) and implemented by hydrolyzing compound (86) in the presence of a base. The reaction is performed as in the aforementioned step C2 in process C.

Step K3 is for producing compound (88) and implemented by reacting compound (87) or reactive derivatives thereof (acid halides, mixed acid anhydrides or active esters) with compound (138) or acid addition salts thereof in an inert solvent. The reaction is performed as in the aforementioned step C3 in process C.

Step K4 is for producing compound (89) and implemented by reacting compound (88) with an acid in an aqueous solvent. The reaction is performed as in the aforementioned step A5 in process A.

Process K' is an alternative method for producing compound (255) having the dashed line in compound (89) forming a double bond together with the solid line, and compound (199) having the dashed line in compound (89) forming a single bond together with the solid line.

## Process K'

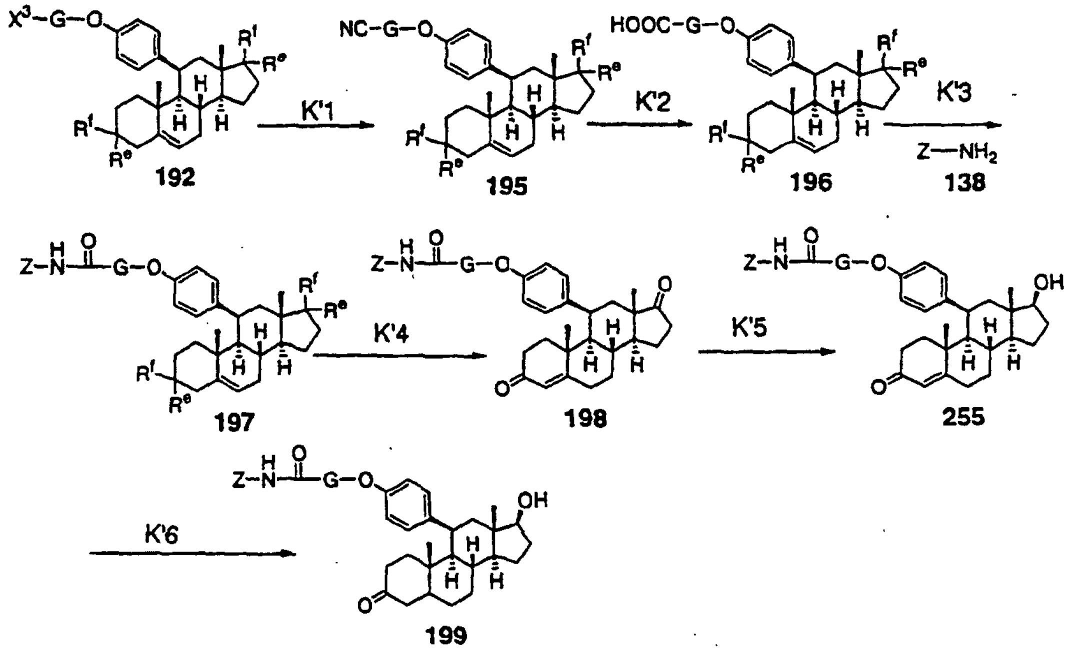


Step K'1 is for producing compound (195) and implemented by reacting compound (192) with a cyanylating agent in an inert solvent. The reaction is performed as in the aforementioned step K1 in process K.

Step K'2 is for producing compound (196) and implemented by hydrolyzing compound (195) in the presence of a base. The reaction is performed as in the aforementioned step K2 in process K.

Step K'3 is for producing compound (197) and implemented by reacting compound (196) or reactive derivatives thereof (acid halides, mixed acid anhydrides or active esters) with compound (138) or acid addition salts thereof in an inert solvent. The reaction is performed as in the aforementioned step K3 in process K.

Step K'4 is for producing compound (198) and implemented by reacting compound (197) with an acid in an aqueous solvent. The reaction is performed as in the aforementioned step J'5 in process J'.

Step K'5 is for producing compound (255) and implemented by reacting compound (198) with a reducing agent in an optionally miscible inert solvent. The reaction is performed as in the aforementioned step J'6 in process J'.

Step K'6 is for producing compound (199) and implemented by performing catalytic reduction of compound (255) in an alcobolic solvent or an inert solvent or reacting compound (255) with a reducing agent in an optionally miscible inert solvent. The reaction is performed as in the aforementioned step C'6 in process C'.

Process L is for producing compound (94) represented by the general formula (I) in which $X^1$ is a group of β configuration that is represented by the general formula (II) in which Ar is an aromatic hydrocarbon group (preferably a p-phenylene group), A is -O- and $R^1$ is -G-NHCO-Z, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

## Process L

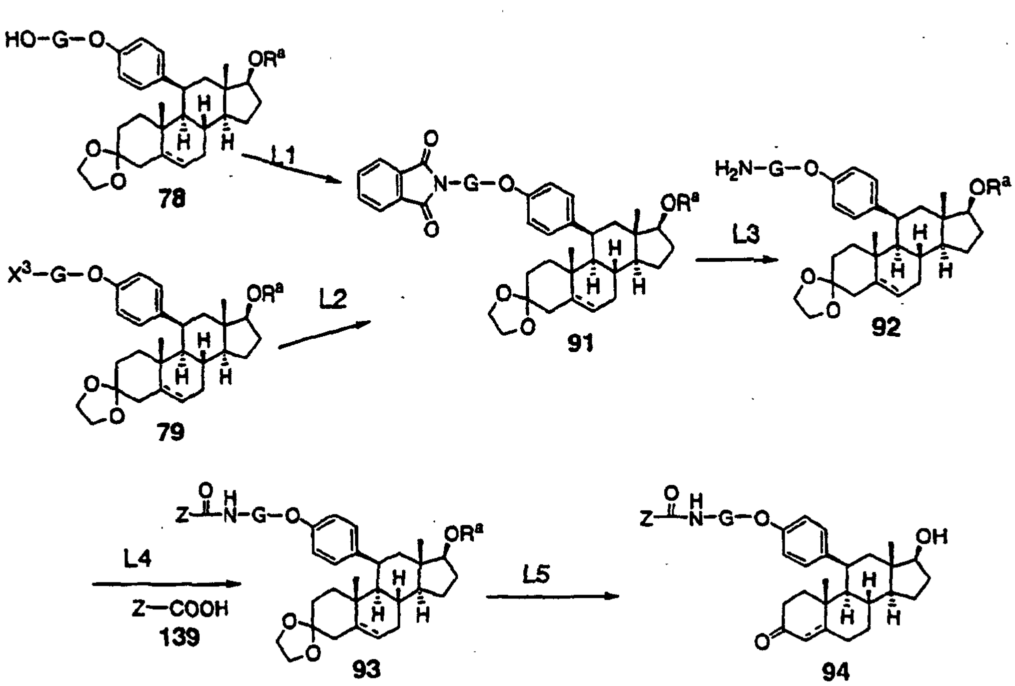


78 79 91 92 93 94

Step L1 is for producing compound (91) and implemented by reacting compound (78) with phthalimide in an inert solvent in the presence of an azodicarboxylic acid dialkyl ester (preferably diethyl azodicarboxylate) and a phosphine compound (preferably triphenylphosphine). The reaction is performed as in the aforementioned step D1 in process D.

Step L2 is an alternative step for producing compound (91) and implemented by reacting compound (79) with a metal salt of phthalimide (preferably phthalimide potassium) in an inert solvent. The reaction is performed as in the aforementioned step D2 in process D.

Step L3 is for producing compound (92) and implemented by reacing compound (91) with an amine-containing compound (preferably hydrazine) in an alcoholic solvent. The reaction is performed as in the aforementioned step D3 in process D.

Step L4 is for producing compound (93) and implemented by reacting compound (139) or reactive derivatives thereof (acid halides, mixed acid anhydrides or active esters) with compound (92) or acid addition salts thereof in an inert solvent. The reaction is performed as in the aforementioned step C3 in process C.

Step L5 is for producing compound (94) and implemented by reacting compound (93) with an acid in an aqueous solvent. The reaction is performed as in the aforementioned step A5 in process A.

Process L' is a method of producing compound (256) having the dashed line in compound (94) forming a double bond together with the solid line, and compound (199) having the dashed line in compound (94) forming a single bond together with the solid line.

## Process L'

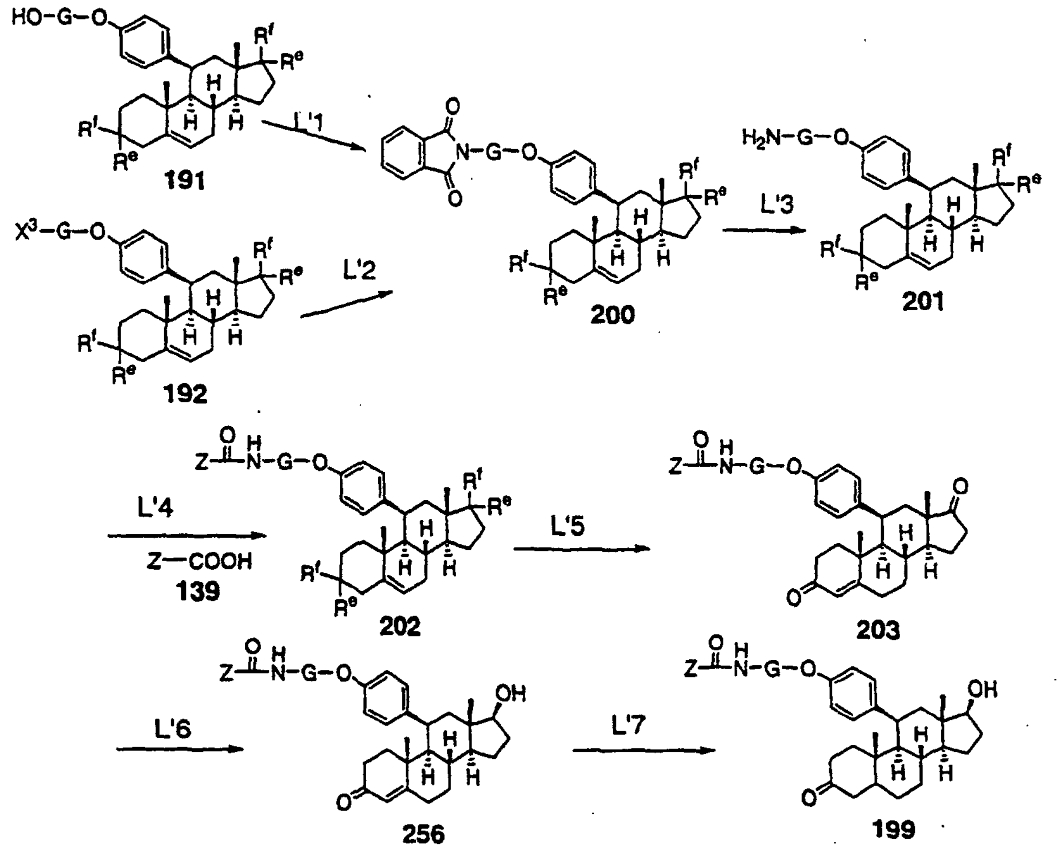


191 192 200 201 202 203 256 199

Step L'1 is for producing compound (200) and implemented by reacting compound (191) with phthalimide in an inert solvent in the presence of an azodicarboxylic acid dialkyl ester (preferably diethyl azodicarboxylate) and a phosphine compound (preferably triphenylphosphine). The reaction is performed as in the aforementioned step L1 in process L.

Step L'2 is an alternative step for producing compound (200) and implemented by reacting compound (192) with a metal salt of phthalimide (preferably phthalimide potassium) in an inert solvent. The reaction is performed as in the aforementioned step L2 in process L.

Step L'3 is for producing compound (201) and implemented by reacting compound (200) with an amine-containing compound (preferably hydrazine) in an alcoholic solvent. The reaction is performed as in the aforementioned step L3 in process L.

Step L'4 is for producing compound (202) and implemented by reacting compound (139) or reactive derivatives thereof (acid halides, mixed acid anhydrides or active esters) with compound (201) or acid addition salts thereof in an inert solvent. The reaction is performed as in the aforementioned step L4 in process L.

Step L'5 is for producing compound (203) and implemented by reacting compound (202) with an acid in an aqueous solvent. The reaction is performed as in the aforementioned step K'4 in process K'.

Step L'6 is for producing compound (256) and implemented by reacting compound (203) with a reducing agent in an optionally miscible inert solvent. The reaction is performed as in the aforementioned step K'5 in process K.

Step L'7 is for producing compound (199) and implemented by performing catalytic reduction of compound (256) in an alcoholic solvent or an inert solvent or by reacting compound (256) with a reducing agent in an optionally miscible inert solvent. The reaction is performed as in the aforementioned step C'6 in process C'.

Process M is for producing compound (102) represented by the general formula (I) in which $X^1$ is a hydrogen atom, $X^2$ is a group of $\alpha$ configuration that is represented by the general formula (II) in which Ar is a single bond, A is -O-and $R^1$ is $-(CH_2)_4$- $R^2$, $R^a$ is a hydrogen atom, $R^b$ and $R^c$, when taken together with the carbon atom in 3-position to which they are bound, are -(C=O)-, and the dashed line together with the solid line is a single bond or a double bond; compound (104) represented by the general formula (I) in which $X^1$ is a hydrogen atom, $X^2$ is a group of $\alpha$ configuration that is represented by the general formula (II) in which Ar is a single bond, A is -O- and $R^1$ is $-(CH_2)_4$-$G^2$-S(O)-Z, $R^a$

is a hydrogen atom, $R^b$ and $R^c$, when taken together with the carbon atom in 3-position to which they are bound, are -(C=O)-, and the dashed line together with the solid line is a single bond or a double bond; and compound (105) represented by the general formula (I) in which $X^1$ is a hydrogen atom, $X^2$ is a group of α configuration that is represented by the general formula (II) in which Ar is a single bond, A is -O- and $R^1$ is $-(CH_2)_4-G^2-S(O)_2-Z$, $R^a$ is a hydrogen atom, $R^b$ and $R^c$, when taken together with the carbon atom in 3-position to which they are bound, are -(C=O)-, and the dashed line together with the solid line is a single bond or a double bond.

Process M

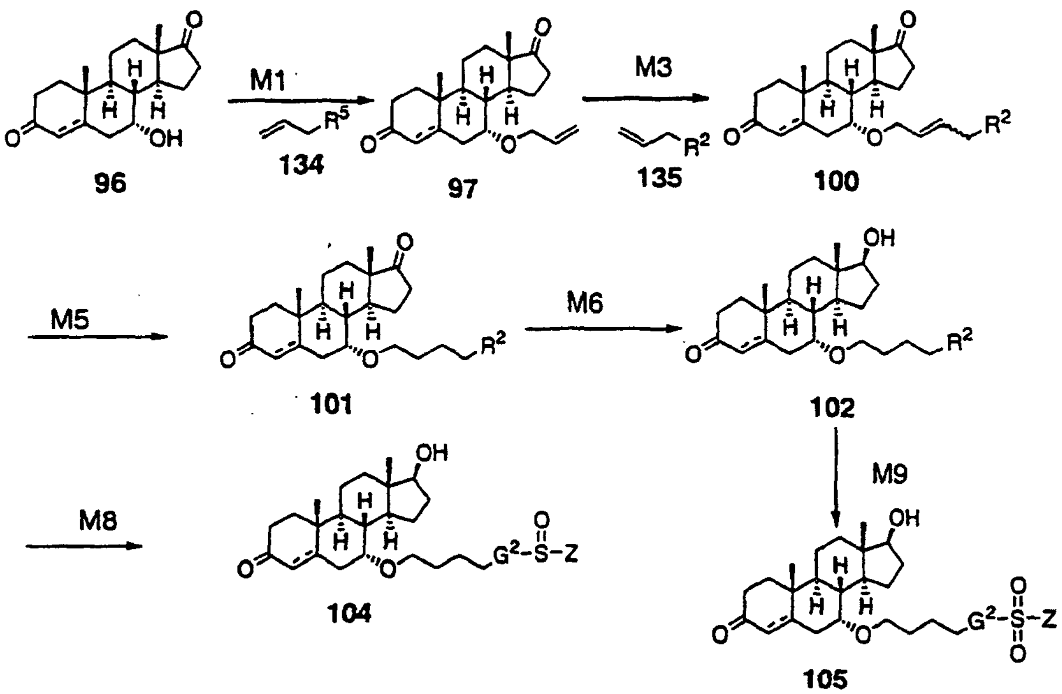


Step M1 is for producing compound (97) and implemented by reacting compound (96) with a base in an inert solvent to make a salt of compound (96) and then reacting it with compound (134) in an inert solvent. The reaction is performed as in the aforementioned step A3 in process A.

A compound having the hydroxyl group in 7-position of compound (96) oriented in β configuration is also known by being disclosed in, for example, J. Org. Che., 26, 2856-2859 (1961) and by using this compound in place of compound (96), one can obtain compounds having $X^2$ in compound (102), compound (104) and compound (105) oriented in β configuration.

Step M3 is for producing compound (100) and implemented by reacting compound (97) with compound (135) in an inert solvent in the presence of an organometallic catalyst. The reaction is performed as in the aforementioned step A4 in process A.

Step M5 is for producing compound (101) and implemented by performing catalytic reduction in an alcoholic solvent or an inert solvent. The reaction is performed as in the aforementioned step A6 in process A.

Step M6 is for producing compound (102) and implemented by reacting compound (101) with a reducing agent in an optionally miscible inert solvent.

The inert solvent to be used is not limited in any particular way as long as it does not interfere with the reaction; examples are ether solvents such as ether, tetrahydrofuran, dioxane and dimethoxyethane, alcoholic solvents such as methanol and ethanol, aromatic solvents such as benzene, toluene, xylene, quinoline and chlorobenzene, and amines such as pyridine and triethylamine, and preferred examples are alcoholic solvents such as methanol and ethanol, with metanol being more preferred. The reducing agent to be used may be exemplified by: metal hydrogen complex compounds such as aluminum lithium hydride, trimethoxyaluminum lithium hydride, tri-t-butoxyaluminum lithium hydride, aluminum lithium hydride-trichloroaluminum (alane), aluminum lithium hydride-boron trifluoride, aluminum hydride magnesium chloride, magnesium aluminum hydride, sodium aluminum hydride, sodium triethoxyaluminum hydride, sodium bis(methoxyethoxy)aluminum hydride, sodium boron hydride, sodium boron hydride-palladium/carbon, sodium boron hydrogensulfide, sodium boron hydrogencyanide, sodium trimethoxyboron hydride, lithium boron hydride, lithium boron hydrogencyanide, lithium triethylboron hydride, lithium tri-s-butylboron hydride, lithium tri-t-butylboron hydride, calcium boron hydride, potassium boron hydride, potassium triisopropoxyboron hydride, potassium tri-s-butylboron hydride, zinc boron hydride, tetramethylammonium boron hydride, and tetra-n-butylammonium cyanoboron hydride; metal hydrides such as diisobutylaluminum hydride, triphenyltin hydride, tri-n-butyltin hydride, diphenyltin hydride, di-n-butyltin hydride, triethyltin hydride, trimethyltin hydride, trichlorosilane/tri-n-butylamine, trichlordsilane/tri-n-propylamine, triethylsilane, trimethylsilane, diphenylsilane, phenylsilane, polymethylhydrosiloxane, dimethylphenylsilane, di-n-butylsilane, and methylphenylsilane; borane derivatives such as diborane, dimethylamine-borane, trimethylamine-borane, ethylenediamine-borane, pyridine-borane, dimethylsulfide-borane, 2,3-dimethyl-2-butylborane (thexylborane), bis-3-methyl-2-butylborane (disiamylborane), diisopinocanephenylborane, dicyclohexylborane, and 9-borabicyclo[3,3,1]nonane (9-BBN); preferred examples are metal hydrogen complex compounds such as aluminum lithium hydride, trimethoxyaluminum lithium hydride, tri-t-butoxyaluminum lithium hydride, aluminum lithium hydride-trichloroaluminum (alane), aluminum lithium hydride-boron trifluoride, aluminum hydride magnesium chloride, magnesium aluminum hydride, sodium aluminum hydride, sodium triethoxyaluminum hydride, sodium bis(methoxyethoxy)aluminum hydride, sodium boron hydride, sodium boron hydride-palladium/carbon, sodium boron hydrogensulfide, sodium boron hydrogencyanide, sodium trimethoxyboron hydride, lithium boron hydride, lithium boron hydrogencyanide, lithium triethylboron hydride, lithium tri-s-butylboron hydride, lithium tri-t-butylboron hydride, calcium boron hydride, potassium boron hydride, potassium triisopropoxyboron hydride, potassium tri-s-butylboron hydride, zinc boron hydride, tetramethylammonium boron hydride, and tetra-n-butylammonium cyanoboron hydride, with sodium boron hydride being more preferred. The reaction temperature which varies with the type of solvent and the like is typically in the range of -30°C - 100°C, preferably 0°C - 70°C. The reaction time which varies with the reaction temperature and the like is typically in the range of 10 minutes - 48 hours, preferably 30 minutes - 24 hours.

Step M8 is for producing compound (104) in the case where $Q^2$ in $R^2$ in compound (102) is -S- and implemented by reacting compound (102) with an oxidizing agent in an inert solvent. The reaction is performed as in the aforementioned step A8 in process A.

Step M9 is for producing compound (105) in the case where $Q^2$ in $R^2$ in compound (102) is -S- and implemented by reacting compound (102) with an oxidizing agent in an inert solvent. The reaction is perfdormed as in the aforementioned step A9 in process A.

Process N is for producing compound (112) represented by the general formula (I) in which $X^1$ is a hydrogen atom, $X^2$ is a group of α configuration that is represented by the general formula (II) in which Ar is a single bond, A is a methylene group and $R^1$ is $-CH_2-CH=CH-CH_2-R^4$, $R^a$ is a hydrogen atom, $R^b$ and $R^c$, when taken together with the carbon atom in 3-position to which they are bound, are -(C=O)-, and the dashed line together with the solid line is a double bond; compound (113) represented by the general formula (I) in which $X^1$ is a hydrogen atom, $X^2$ is a group of α configuration that is represented by the general formula (II) in which Ar is a single bond, A is a methylene group and $R^1$ is $-(CH_2)_3-R^4$, $R^a$ is a hydrogen atom, $R^b$ and $R^c$, when taken together with the carbon atom in 3-position to which they are bound, are -(C=O)-, and the dashed line together with the solid line is a double bond; compound (114) represented by the general formula (I) in which $X^1$ is a hydrogen atom, $X^2$ is a group of α configuration that is represented by the general formula (II) in which Ar is a single bond, A is a methylene group and $R^1$ is $-(CH_2)_3-R^4$, $R^a$ is a hydrogen atom, $R^b$ and $R^c$, when taken together with the carbon atom in 3-position to which they are bound, are -(C=O), and the dashed line together with the solid line is a single bond; compound (115) represented by the general formula (I) in which $X^1$ is a hydrogen atom, $X^2$ is a group of α configuration represented by the general formula (II) in which Ar is a single bond, A is a methylene group and $R^1$ is $-(CH_2)_3-G^3-S(O)-Z$, $R^a$ is a hydrogen atom, $R^b$ and $R^c$, when taken together with the carbon atom in 3-position to which they are bound, are -(C=O), and the dashed line together with the solid line is a double bond; compound (116) represented by the general formula (I) in which $X^1$ is a hydrogen atom and $X^2$ is a group of α configuration that is represented by the general formula (II) in which Ar is a single bond, A is a methylene group and $R^1$ is $-(CH_2)_3-G^3-S(O)_2-Z$, $R^a$ is a hydrogen atom, $R^b$ and $R^c$, when taken together with the carbon atom in 3-position to which they are bound, are -(C=O)-, and the dashed line together with the solid line is a double bond; compound (117) represented by the general formula (I) in which $X^1$ is a hydrogen atom, $X^2$ is a group of α configuration represented by the general formula (II) in which Ar is a single bond, A is a methylene group and $R^1$ is $-(CH_2)_3-G^3-S(O)-Z$, $R^a$ is a hydrogen atom, $R^b$ and $R^c$, when taken together with the carbon atom in 3-position to which they are bound, are -(C=O), and the dashed line together with the solid line is a single bond; and compound (118) represented by the general formula (I) in which $X^1$ is a hydrogen atom and $X^2$ is a group of α configuration that is represented by the general formula (II) in which Ar is a single bond, A is a methylene group and $R^1$ is $-(CH_2)_3-G^3-S(O)_2-Z$, $R^a$ is a hydrogen atom, $R^b$ and $R^c$, when taken together with the carbon atom in 3-position to which they are bound, are -(C=O)-, and the dashed line together with the solid line is a single bond.

## Process N

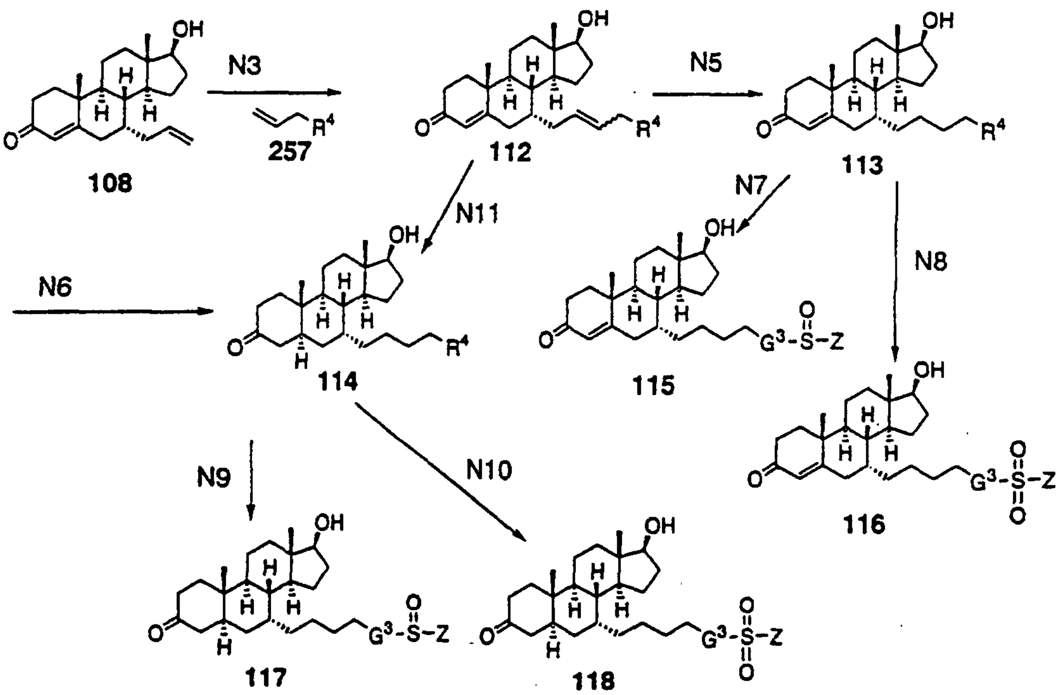

Step N3 is for producing compound (112) and implemented by reacting compound (108) with compound (257) in an inert solvent in the presence of an organometallic catalyst. The reaction is performed as in the aforementioned step A4 in process A.

Step N5 is for producing compound (113) and implemented by performing catalytic reduction in an alcoholic solvent or an inert solvent. The reaction is performed as in the aforementioned step A6 in process A.

Step N6 is for producing compound (114) and implemented by performing catalytic reduction in an alcoholic solvent or an inert solvent.

The solvent to be used may be exemplified by alcoholic solvents such as methanol, ethanol, n-propanol, i-propanol, n-butanol, s-butanol, t-butanol, pentanol, hexanol, cyclopropanol, cyclobutanol, cyclopentanol, cyclohexanol, ethylene glycol, 1,3-propanediol, 1,4-butanediol, and 1,5-pentanediol, ether solvents such as ether, tetrahydrofuran, dioxane and dimethoxyethane, aromatic solvents such as benzene, toluene, xylene, quinoline and chlorobenzene, halogen-containing solvents such as dichloromethane, chloroform and carbon tetrachloride, as well as cyclohexane, dimethyl sulfoxide, dimethylacetamide, dimethylimidazolidinone, dimethylformamide, N-methylpyrrolidone, ethyl acetate, acetonitrile and nitromethane; preferred examples are methanol and ethanol.

The condition to be used in catalytic reduction is a homogeneous system such as hydrogen-chlorotris(triphenylphosphine)rhodium(I), hydrogen-chlorotris(triparatolylphosphine)rhodium(I), hydrogen-chlorotris(triparamethoxyphenylphosphine)rhodium(I), hydrogen-hydridecarbonyltris(triphenylphosphine)rhodium(I), hydrogen-rhodium(II) acetate, hydrogen-ruthenium(II) acetate, hydrogen-chlorohydridetris(triphenylphosphine)ruthenium(II), hydrogen-carboxylatohydridetris(triphenylphosphine)ruthenium(II), hydrogen-hydridecarbonyltris(triphenylphosphine)iridium(I), hydrogen-platinum(II)-tin chloride complex, hydrogen-pentacyanocobalt(II) complex, hydrogen-tricyanobipyridine cobalt(II) complex, hydrogen-bis(dimethylglyoximato)cobalt(II) complex, hydrogen-methyl benzoate-tricarbonylchromium complex, hydrogen-bis(tricarbonylcyclopentadienylchromium), hydrogen-pentacarbonyliron, hydrogen-bis(cyclopentadienyl)dicarbonyltitanium, hydrogen-hydridecarbonylcobalt complex, hydrogen-octacarbonyldicobalt, hydrogen-hydridecarbonylrhodium, hydrogen-chromium(III) acetylacetonato-triisobutylaluminum, hydrogen-cobalt(II) acetylacetonato-triisobutylaluminum, or hydrogen-nickel(II)-2-hexanoato-triethylaluminum, or an inhomogeneous system condition such as hydrogen-platinum dioxide, hydrogen-platinum/carbon, hydrogen-palladium/carbon, hydrogen-palladium/barium sulfate, hydrogen-palladium/calcium carbonate, hydrogen-Raney nickel, hydrogen-copper chromite, hydrogen-rhodium/carbon, hydrogen-rhodium/alumina, hydrogen-ruthenium dioxide, or hydrogen-ruthenium/carbon; a preferred example is hydrogen-palladium/carbon.

The reaction temperature is typically in the range of 0°C - 100°C, preferably 0°C - 60°C. The reaction time which varies with the reaction temperature and the like is typically in the range of 10 minutes - 24 hours, preferably 10

minutes - 6 hours.

Step N7 is for producing compound (115) in the case where $Q^4$ in $R^2$ in compound (113) is -S- and implemented by reacting compound (113) with an oxidizing agent in an inert solvent. The reaction is performed as in the aforementioned step A8 in process A.

Step N8 is for producing compound (116) in the case where $Q^4$ in $R^2$ in compound (113) is -S- and implemented by reacting compound (113) with an oxidizing agent in an inert solvent. The reaction is performed as in the aforementioned step A9 in process A.

Step N9 is for producing compound (117) in the case where $Q^4$ in $R^2$ in compound (114) is -S- and implemented by reacting compound (114) with an oxidizing agent in an inert solvent. The reaction is performed as in the aforementioned step A8 in process A.

Step N10 is for producing compound (118) in the case where $Q^4$ in $R^2$ in compound (114) is -S- and implemented by reacting compound (114) with an oxidizing agent in an inert solvent. The reaction is performed as in the aforementioned step A9 in process A.

Step N11 is an alternative method of producing compound (114) and implemented by performing catalytic reduction in an alcoholic solvent or an inert solvent. The reaction is performed as in the aforementioned step N6 in process N.

Process O is for producing compound (126) represented by the general formula (I) in which $X^1$ is a hydrogen atom, $X^2$ is a group of $\alpha$ configuration that is represented by the general formula (II) in which Ar is an aromatic hydrocarbon group (preferably a p-phenylene group), A is -O- and $R^1$ is $-CH_2-CH=CH-CH_2-R^2$, $R^a$ is a hydrogen atom, $R^b$ and $R^c$, when taken together with the carbon atom in 3-position to which they are bound, are -(C=O)-, and the dashed line together with the solid line is a double bond; compound (127) represented by the general formula (I) in which $X^1$ is a hydrogen atom, $X^2$ is a group of $\alpha$ configuration that is represented by the general formula (II) in which Ar is an aromatic hydrocarbon group (preferably a p-phenylene group), A is -O- and $R^1$ is $-(CH_2)_4-R^2$, $R^a$ is a hydrogen atom, $R^b$ and $R^c$, when taken together with the carbon atom in 3-position to which they are bound, are -(C=O), and the dashed line together with the solid line is a double bond; compound (128) represented by the general formula (I) in which $X^1$ is a hydrogen atom, $X^2$ is a group of $\alpha$ configuration that is represented by the general formula (II) in which Ar is an aromatic hydrocarbon grouop (preferably a p-phenylene group), A is -O- and $R^1$ is $-(CH_2)_4-R^2$, $R^a$ is a hydrogen atom, $R^b$ and $R^c$, when taken together with the carbon atom in 3-position to which they are bound, are -(C=O), and the dashed line together with the solid line is a single bond; compound (129) represented by the general formula (I) in which $X^1$ is a hydrogen atom, $X^2$ is a group of $\alpha$ configuration represented by the general formula (II) in which Ar is an aromatic hydrocarbon group (preferably a p-phenylene group), A is -O-and $R^1$ is $-(CH_2)_4-G^2-S(O)-Z$, $R^a$ is a hydrogen atom, $R^b$ and $R^c$, when taken together with the carbon atom in 3-position to which they are bound, are -(C=O), and the dashed line together with the solid line is a double bond; compound (130) represented by the general formula (I) in which $X^1$ is a hydrogen atom and $X^2$ is a group of $\alpha$ configuration that is represented by the general formula (II) in which Ar is an aromatic hydrocarbon group, A is -O- and $R^1$ is $-(CH_2)_4-G^2-S(O)_2-Z$, $R^a$ is a hydrogen atom, $R^b$ and $R^c$, when taken together with the carbon atom in 3-position to which they are bound, are -(C=O)-, and the dashed line together with the solid line is a double bond; compound (131) represented by the general formula (I) in which $X^1$ is a hydrogen atom, $X^2$ is a group of $\alpha$ configuration represented by the general formula (II) in which Ar is an aromatic hydrocarbon group (preferably a p-phenylene group), A is -O- and $R^1$ is $-(CH_2)_4-G^2-S(O)-Z$, $R^a$ is a hydrogen atom, $R^b$ and $R^c$, when taken together with the carbon atom in 3-position to which they are bound, are -(C=O), and the dashed line together with the solid line is a single bond; and compound (132) represented by the general formula (I) in which $X^1$ is a hydrogen atom and $X^2$ is a group of $\alpha$ configuration that is represented by the general formula (II) in which Ar is an aromatic hydrocarbon group (preferably a p-phenylene group), A is -O- and $R^1$ is $-(CH_2)_4-G^2-S(O)_2-Z$, $R^a$ is a hydrogen atom, $R^b$ and $R^c$, when taken together with the carbon atom in 3-position to which they are bound, are -(C=O)-, and the dashed line together with the solid line is a single bond.

Process O

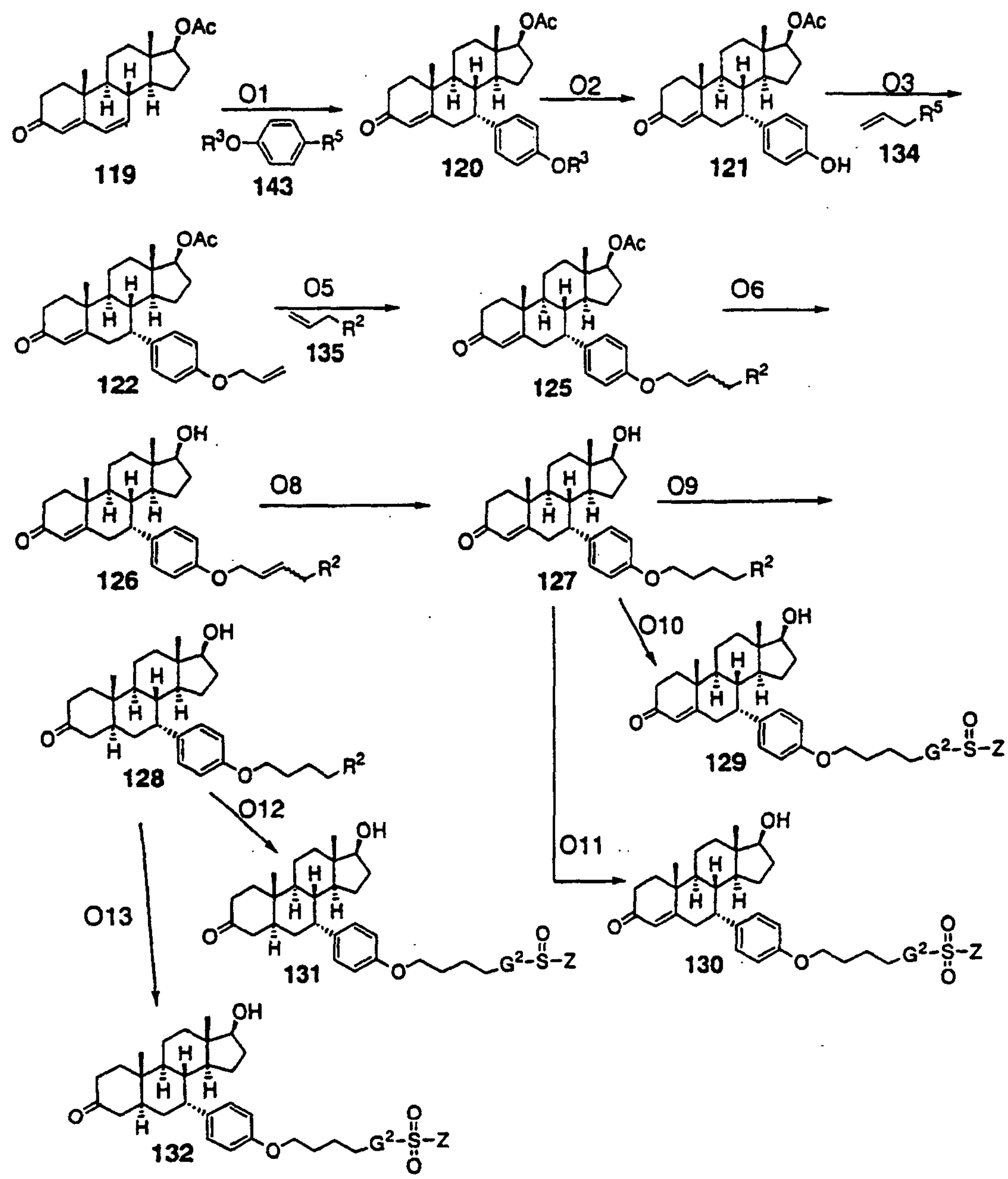


Step O1 is for producing compound (120) and implemented by reacting compound (143) with a metal (referably magnesium) or an alkyllithium (preferably t-butyllithium) in an inert solvent to make a reactive derivative of compound (143) and reacting it with compound (119) in an inert solvent in the presence of an additive (preferably tetrakis[(tri-n-butylphosphine)copper(I) iodide]).

The inert solvent to be used is not limited in any particular way as long as it does not participate in the reaction; preferred examples are ethers such as ether, tetrahydrofuran, dioxane and dimethoxyethane, with ether being further preferred. The reaction temperature which varies with the type of solvent and the like is typically in the range of -78°C ~ 80°C (preferably -78°C ~ 50°C). The reaction time which varies with the reaction temperature and the like is typically in the range of 15 minutes - 24 hours (preferably 30 minutes - 15 hours).

As a by-product of the production of compound (12), there is formed a compound having the $-C_6H_4-OR^3$ in 7-position of compound (12) and by using this compound in place of compound (120), one can obtain compounds having $X^2$ in compound (126), compound (127), compound (128), compound (129), compound (130), compound (131) and compound (132) oriented in β configuration.

Step O2 is for producing compound (121) and implemented by reacting compound (120) with a deprotecting agent, namely by removing the substituted silyl group, in an inert solvent.

The inert solvent to be used is not limited in an particular way as long as it does not interfere with the reaction; examples include ether solvents such as ether, tetrahydrofuran, dioxane and dimethoxyethane, as well as dimethylformamide and water, with tetrahydrofuran being preferred. The deprotecting agent to be used is not limited in any particular way and may be exemplified by fluorides such as hydrogen fluoride, hydrogen fluoride-pyridine, sodium fluoride, potassium fluoride and tetra-n-butylammonium fluoride, inorganic acids such as hydrochloric acid, hydrobromic acid, hydroiodic acid, sulfuric acid and phosphoric acid, and organic acids such as formic acid, acetic acid and p-toluenesulfonic acid, with tetra-n-butylammonium fluoride being preferred.

The reaction temperature which varies with the type of solvent and the like is typically in the range of 0°C - 80°C (preferably 0°C - 50°C). The reaction time which varies with the reaction temperature and the like is typically in the range of 15 minutes - 24 hours (preferably 30 minutes - 15 hours).

Step O3 is for producing compound (122) and implemented by reacting compound (121) with a base in an inert solvent to make a salt of compound (121) and then reacting it with compound (134) in an inert solvent. The reaction is performed as in-the aforementioned step A3 in process A.

Step O5 is for producing compound (125) and implemented by reacting compound (122) with compound (135) in an inert solvent in the presence of an organometallic catalyst. The rection is performed as in the aforementioned step A4 in process A.

Step O6 is for producing compound (126) and implemented by hydrolyzing compound (125) in water or a water-soluble solvent in the presence of a base or an acid (preferably a base).

The water-soluble solvent to be used is not limited in any particular way and may be exemplified by alcoholic solvents such as methanol, ethanol, n-propanol and i-propanol, ether solvents such as tetrahydrofuran and dioxane, as well as dimethylformamide, with methanol being preferred.

The base to be used is not limited in any particular way and may be exemplified by metal hydroxides such as lithium hydroxide, sodium hydroxide, potassium hydroxide, potassium hydroxide, calcium hydroxide, barium hydroxide and cesium hydroxide, and carbonates such as potassium carbonate and sodium carbonate, with sodium hydroxide being preferred.

The acid to be used is not limited in any particular way and may be exemplified by inorganic acids such as hydrochloric acid, hydrobromic acid, hydroiodic acid, sulfuric acid and phosphoric acid, with hydrochloric acid being preferred.

The reaction temperature which varies with the type of solvent and the like is typically in the range of 0°C - 100°C (preferably 0°C - 80°C). The reaction time which varies with the reaction temperature and the like is typically in the range of 15 minutes - 24 hours (preferably 30 minutes - 15 hours).

Step O8 is for producing compound (127) and implemented by performing catalytic reduction in an alcoholic solvent or an inert solvent. The reaction is performed as in the aforementioned step A6 in process A.

Step O9 is for producing compound (128) and implemented by performing catalytic reduction in an alcoholic solvent or an inert solvent. The reaction is performed as in the aforementioned step N6 in process N.

Step O10 is for producing compound (129) in the case where $Q^2$ in $R^2$ in compound (127) is -S- and implemented by reacting compound (127) with an oxidizing agent in an inert solvent. The reaction is performed as in the aforementioned step A8 in process A.

Step O11 is for producing compound (130) in the case where $Q^2$ in $R^2$ in compound (127) is -S- and implemented by reacting compound (127) with an oxidizing agent in an inert solvent. The reaction is performed as in the aforementioned step A9 in process A.

Step O12 is for producing compound (131) in the case where $Q^2$ in $R^2$ in compound (128) is -S- and implemented by reacting compound (128) with an oxidizing agent in an inert solvent. The reaction is performed as in the aforementioned step A8 in process A.

Step O13 is for producing compound (132) in the case where $Q^2$ in $R^2$ in compound (128) is -S- and implemented by reacting compound (128) with an oxidizing agent in an inert solvent. The reaction is performed as in the aforementioned step A9 in process A.

Process P is for producing compound (217) represented by the general formula (I) in which $X^2$ is a hydrogen atom, $X^1$ is a group of β configuration that is represented by the general formula (II) in which Ar is single bond, A is a methylene group and $R^1$ is a group represented by the general formula (III) in which G is $-G^4-CH_2-$, E is a single bond, J is an optionally substituted aromatic hydrocarbon group (preferably a p-phenylene group), Y is a single bond, L is $L^2$, Q is $Q^{17}$, with $R^7$ in $Q^{17}$ being a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

Process P

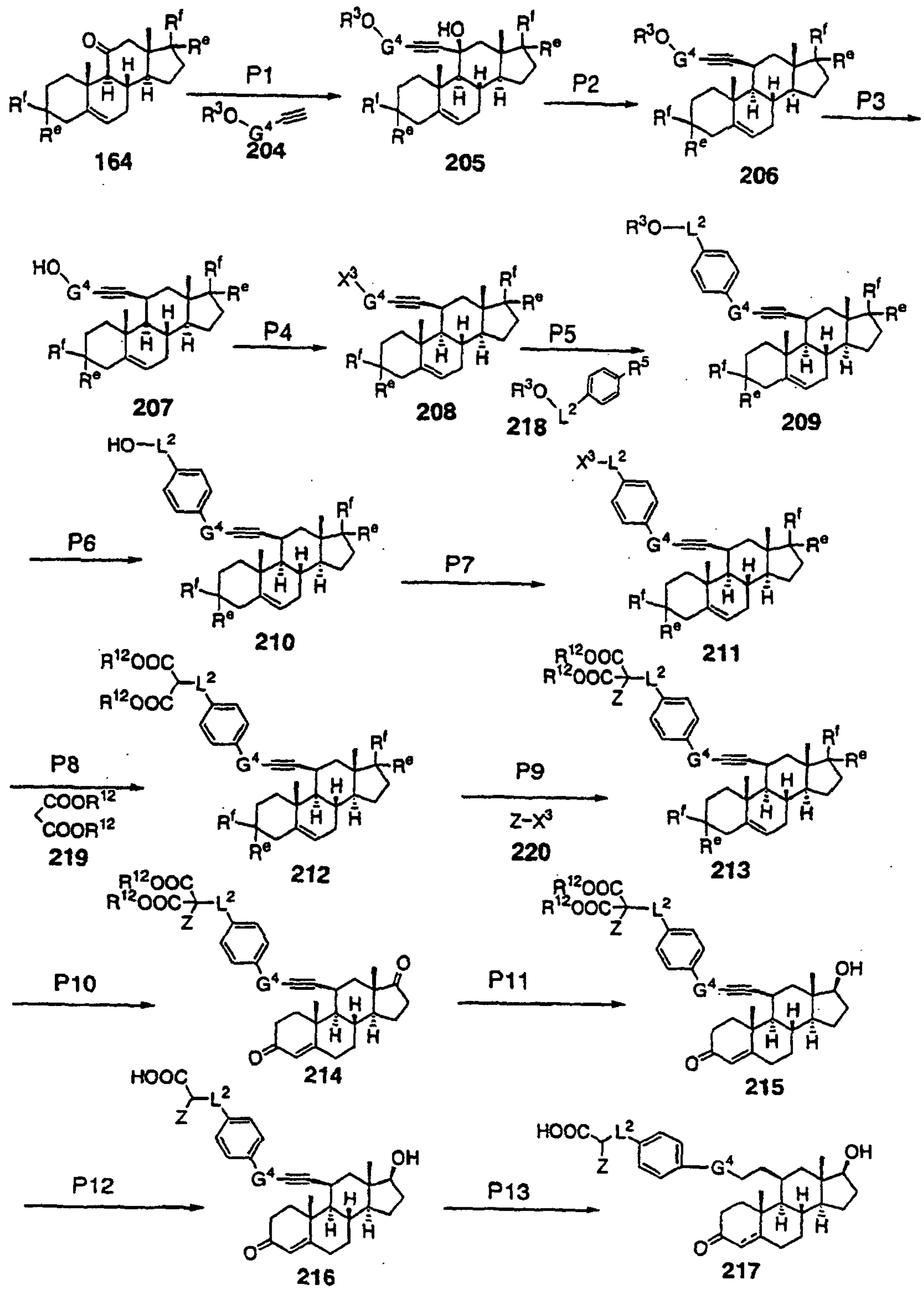
164
P1
204
205
P2
206
P3
207
P4
208
P5
218
209
P6
210
P7
211
P8
219
212
P9
Z-X3
220
213
P10
214
P11
215
P12
216
P13
217

## Process P (continued)

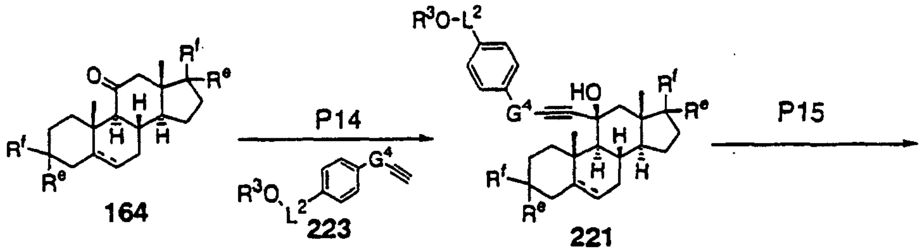

164 223 221

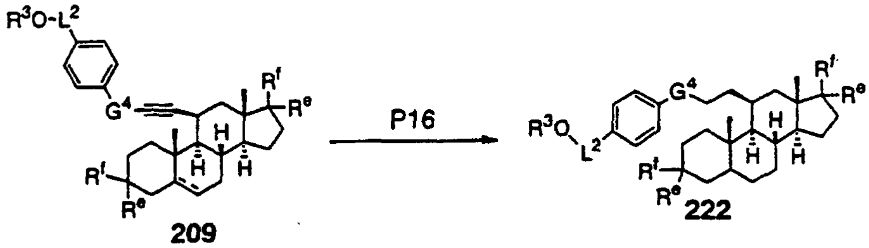

209 222

Step P1 is for producing compound (205) and implemented by reacting compound (204) with an alkyllithium (preferably n-butyllithium) in an inert solvent to make a reactive derivative of compound (204) and reacting it with compound (164) in an inert solvent. The reaction is performed as in the aforementioned step F1 in process F.

Step P2 is for producing compound (206) and implemented by reacting compound (205) with a reducing agent in an inert solvent in the presence of an additive. The reaction is performed as in the aforementioned step F2 in process F.

In this step, a compound having the substituent in the 11-position of compound (206) oriented in α configuration forms as a by-product and this may be used to give a compound having $X^1$ in compound (217) oriented in α configuration.

Step P3 is for producing compound (207) and implemented by reacting compound (206) with a deprotecting agent, namely, removing the substituted silyl group, in an inert solvent. The reaction is performed as in the aforementioned step F3 in process F.

Step P4 is for producing compound (208) and implemented by reacting compound (207) with a sulfonyl chloride compound in an amine-containing solvent or reacting compound (207) with a halogenating agent in an inert solvent. The reaction is performed as in the aforementioned stepp F4 in process F.

Step P5 is for producing compound (209) and implemented by reacting compound (218) with a metal (preferably magnesium) or an alkyllithium (preferably n-butyllithium) in an inert solvent to make a reactive derivative of compound (218) and reacting it with compound (208) in an inert solvent. The reaction is performed as in the aforementioned step I1 in process I.

Step P6 is for producing compound (210) and implemented by reacting compound (209) with a deprotecting agent, namely, removing the substituted silyl group, in an inert solvent. The reaction is performed as in the aforementioned step P3 in process P.

Step P7 is for producing compound (211) and implemented by reacting compound (210) with a sulfonyl chloride compound in an amine-containing solvent or reacting compound (210) with a halogenating agent in an inert solvent. The reaction is performed as in the aforementioned stepp P4 in process P.

Step P8 is for producing compound (212) and implemented by reacting compound (219) with a base in an inert solvent to make a reactive derivative of compound (219) and then reacting it with compound (211) in an inert solvent.

The inert solvent to be used is not limited in any particular way as long as it does not participate in the reaction; examples are ether solvents such as ether, tetrahydrofuran, dioxane and dimethoxyethane, aromatic solvents such as benzene, toluene, xylene, quinoline and chlorobenzene, as well as dimethyl sulfoxide, dimethylacetamide, dimethylimidazolidinone, dimethylformamide, and N-methylpyrrolidone; preferred examples are ether solvents such as tetrahydrofuran, as well as dimethylformamide. The base to be used may be exemplified by metal alkoxides such as sodium

alkoxide and potassium t-butoxide, metal hydrides such as sodium hydride, potassium hydride and calcium hydride, alkyllithium compounds such as methyllithium, ethyllithium, n-butyllithium and t-butyllithium, metal amides such as sodium amide, potassium bistrimethylsilylamide, sodium bistrimethylsilylamide and lithium diisopropylamide, as well as carbnates such as cesium carbonate, potassium carbonate and sodium carbonate; preferred examples are metal hydrides such as sodium hydride, metal amides such as lithium diisopropylamide, and carbonates such as cesium carbonate. The reaction temperature which varies with the type of solvent and the like is typically in the range of -78°C ~ 80°C, preferably 0°C ~ 30°C. The reaction time which varies with the reaction temperature and the like is typically in the range of 10 minutes - 24 hours, preferably 30 minutes - 15 hours.

Step P9 is for producing compound (213) and implemented by reacting compound (212) with a base in an inert solvent to make a reactive derivative of compound (212) and then reacting it with compound (220) in an inert solvent. The reaction is performed as in the aforementioned step P8 in process P.

If Z is a hydrogen atom in process P, step P9 may be omitted.

Step P10 is for producing compound (214) and implemented by reacting compound (213) with an acid in an aqueous solvent. The reaction is performed as in the aforementioned step L'5 in process L'.

Step P11 is for producing compound (215) and implemented by reacting compound (214) with a reducing agent in an optionally miscible inert solvent. The reaction is performed as in the aforementioned step L'6 in process L'.

Step P12 is for producing compound (216) and implemented by reacting compound (215) with an acid, a base or a metal salt in an hydrous alcohol or an inert solvent.

The solvents to be used are not limited in any particular way as long as they do not interfere with the reaction; examples are mixtures of water and alcoholic solvents such as methanol, ethanol, n-propanol, i-propanol, n-butanol, s-butanol and t-butanol, amine-containing solvents such as pyridine, as well as dimethyl sulfoxide, dimethylacetamide, dimethylimidazolidinone and dimethylformamide; preferred examples are a mixture of water and an alcoholic solvent such as methanol or ethanol, and dimethyl sulfoxide.

The acid to be used may be exemplified by inorganic acids such as hydrochloric acid, hydrobromic acid, hydroiodic acid, sulfuric acid and phosphoric acid, with hydrochloric acid and hydrobromic acid being preferred.

The base to be used may be exemplified by metal hydroxides such as lithium hydroxide, sodium hydroxide, potassium hydroxide, potassium hydroxide, calcium hydroxide, barium hydroxide and cesium hydroxide, with sodium hydroxide and potassium hydroxide being preferred.

The metal salt to be used can be lithium chloride, sodium cyanide, etc., with lithium chloride being preferred.

The reaction temperature which varies with the type of solvent and the like is typically in the range of 25°C - 180°C, preferably 40°C - 150°C. The reaction time which varies with the reaction temperature and the like is typically in the range of 10 minutes - 24 hours, preferably 30 minutes - 15 hours.

Step P13 is for producing compound (217) and implemented by performing catalytic reduction of compound (216) in an alcoholic solvent or an inert solvent. The reaction is performed as in the aforementioned step F8 in process F.

As an ancillary to this reaction, conversion to a single bond may occasionally be effected if the dashed line forms a double bond together with the solid line.

Steps P14 and P15 provide an alternative method of producing compound (209).

Step P14 is for producing compound (221) and implemented by reacting compound (223) with an alkyllithium (preferably n-butyllithium) in an inert solvent to make a reactive derivative of compound (223) and reacting it with compound (164) in an inert solvent. The reaction is performed as in the aforementioned step P1 in process P.

Step P15 is for producing compound (209) and implemented by reacting compound (209) with a reducing agent in an inert solvent in the presence of an additive. The reaction is performed as in the aforementioned step P2 in process P.

In this step, a compound having the substituent in the 11-position of compound (209) oriented in $\alpha$ configuration forms as a by-product and this may be used to give a compound having $X^1$ in compound (217) oriented in $\alpha$ configuration.

Step P16 is for producing compound (222) and implemented by performing catalytic reduction of compound (209) in an alcoholic solvent or an inert solvent. The reaction is performed as in the aforementioned step P13 in process P.

By subjecting compound (222) to step P6 as in the case of compound (209), one can produce a compound having the dashed line in compound (217) forming a single bond together with the solid line.

Process Q is for producing compound (234) represented by the general formula (I) in which $X^2$ is a hydrogen atom, $X^1$ is a group of $\beta$ configuration that is represented by the general formula (II) in which Ar is single bond, A is a methylene group and $R^1$ is a group represented by the general formula (III) in which G is $-(CH_2)_2-CH(OH)-G^3-$, E, J, Y and L are single bonds, and Q is $Q^{63}$, $R^a$ is a hydrogen atom, $R^b$ and $R^c$, when taken together with the carbon atom in 3-position to which they are bound, are -(C=O)-, and the dashed line together with the solid line is a single bond or a double bond; and compound (235) represented by the general formula (I) in which $X^2$ is a hydrogen atom, $X^1$ is a group of $\beta$ configuration that is represented by the general formula (II) in which Ar is single bond, A is a methylene

group and $R^1$ is a group represented by the general formula (III) in which G is $-(CH_2)_2-CH(OH)-G^3-$, E, J, Y and L are single bonds, and Q is $Q^{64}$, $R^a$ is a hydrogen atom, $R^b$ and $R^c$, when taken together with the carbon atom in 3-position to which they are bound, are -(C=O)-, and the dashed line together with the solid line is a single bond or a double bond.

Process Q

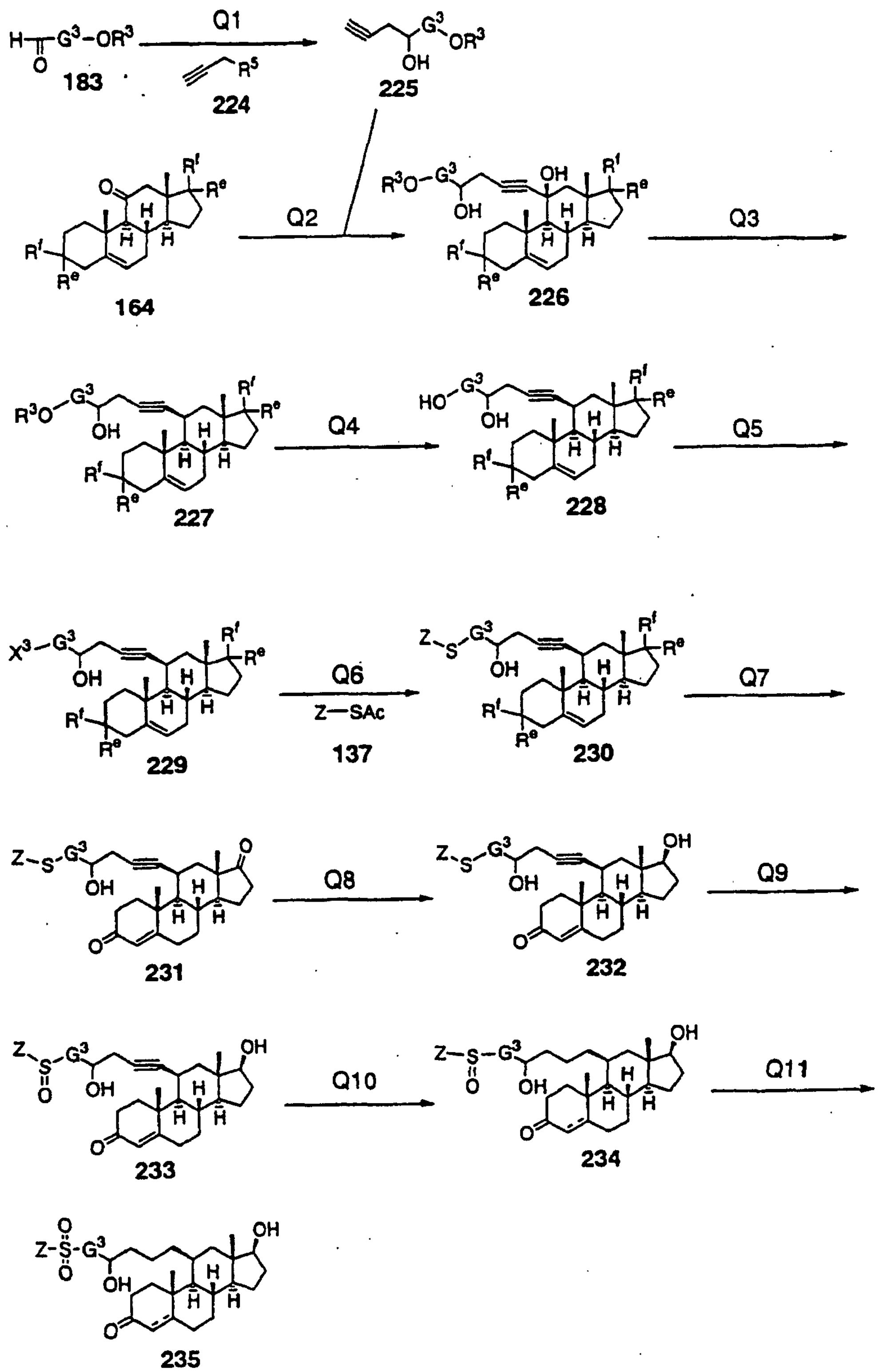


183 224 225
164 226
227 228
229 137 230
231 232
233 234
235

Step Q1 is for producing compound (225) and implemented by reacting compound (224) with a metal (preferably magnesium) or an alkyllithium (preferably t-butyllithium) in an inert solvent in the presence or absence (preferably the presence) of an additive {preferably mercury (II) chloride} to make a reactive derivative of compound (224) and reacting it with compound (183) in an inert solvent.

The inert solvent to be used is not limited in any particular way as long as it does not participate in the reaction; preferred examples are ether solvents such as ether, tetrahydrofuran, dioxane and dimethoxyethane, with ether and tetrahydrofuran being more preferred.

The reaction temperature which varies with the type of solvent and the like is typically in the range of 0°C - 80°C (preferably 10°C - 50°C). The reaction time which varies with the reaction temperature and the like is typically in the range of 15 minutes - 24 hours (preferably 30 minutes - 15 hours).

Step Q2 is for producing compound (226) and implemented by reacting compound (225) with an alkyllithium (preferably n-butyllithium) in an inert solvent to make a reactive derivative of compound (225) and reacting it with compound (164) in an inert solvent. The reaction is performed as in the aforementioned step F1 in process F.

Step Q3 is for producing compound (227) and implemented by reacting compound (226) with a reducing agent in an inert solvent in the presence of an additive. The reaction is performed as in the aforementioned step F2 in process F.

As a by-product of this step, there is formed a compound having the substituent in 11-position of compound (227) oriented in $\alpha$ configuration; by using this compound, one can obtain compounds having $X^1$ in compound (234) and compound (235) oriented in $\alpha$ configuration.

Step Q4 is for producing compound (228) and implemented by reacting compound (227) with a deprotecting agent, namely, removing the substituted silyl group, in an inert solvent. The reaction is performed as in the aforementioned step F3 in process F.

Step Q5 is for producing compound (229) and implemented by reacting compound (228) with a sulfonyl chloride compound in an amine-containing solvent or reacting compound (228) with a halogenating agent in an inert solvent. The reaction is performed as in the aforementioned step F4 in process F.

Step Q6 is for producing compound (230) and implemented by reacting compound (137) with a metal alkoxide in an alcoholic solvent to make a reactive derivative of compound (137) and reacting it with compound (229) in an alcoholic solvent. The reaction is performed as in the aforementioned step F5 in process F.

Step Q7 is for producing compound (231) and implemented by reacting compound (230) with an acid in an aqueous solvent. The reaction is performed as in the aforementioned step P10 in process P.

Step Q8 is for producing compound (232) and implemented by reacting compound (231) with a reducing agent in an optionally miscible inert solvent. The reaction is performed as in the aforementioned step P11 in process P.

Step Q9 is for producing compound (233) and implemented by reacting compound (232) with an oxidizing agent in an inert solvent. The reaction is performed as in the aforementioned step F6 in process F.

Step Q10 is for producing compound (234) and implemented by performing catalytic reduction of compound (233) in an alcoholic solvent or an inert solvent. The reaction is performed as in the aforementioned step F8 in process F.

As an ancillary to this reaction, conversion to a single bond may occasionally be effected if the dashed line forms a double bond together with the solid line.

Step Q11 is for producing compound (235) and implemented by reacting compound (234) with an oxidizing agent in an inert solvent. The reaction is performec as in the aforementioned step B7 in process B.

In process Q, step Q5, step Q6, step Q9 and step Q11 may be omitted and by so doing, one can produce a compound represented by the general formula (I) in which $X^2$ is a hydrogen atom, $X^1$ is a group of $\beta$ configuration that is represented by the general formula (II) in which Ar is single bond, A is a methylene group and $R^1$ is a group represented by the general formula (III) in which G is $-(CH_2)_2-CH(OH)-G^3-$, E, J, Y, L and Q are single bonds, and Z is $-O-R^d$, $R^a$ is a hydrogen atom, $R^b$ and $R^c$, when taken together with the carbon atom in 3-position to which they are bound, are -(C=O)-, and the dashed line together with the solid line is a single bond or a double bond.

In process Q, the hydroxyl group on G may optionally be subjected to a protecting reaction and a deprotecting reaction in any desired steps.

Process R is a method for producing compound (242) represented by the general formula (I) in which $X^2$ is a hydrogen atom, $X^1$ is a group of $\beta$ configuration that is represented by the general formula (II) in which Ar is single bond, A is a methylene group and $R^1$ is a group represented by the general formula (III) in which G is $-(CH_2)_2-CH(OH)$ $-G^3-$, E, J, Y, L and Q are single bonds, and Z is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

## Process R

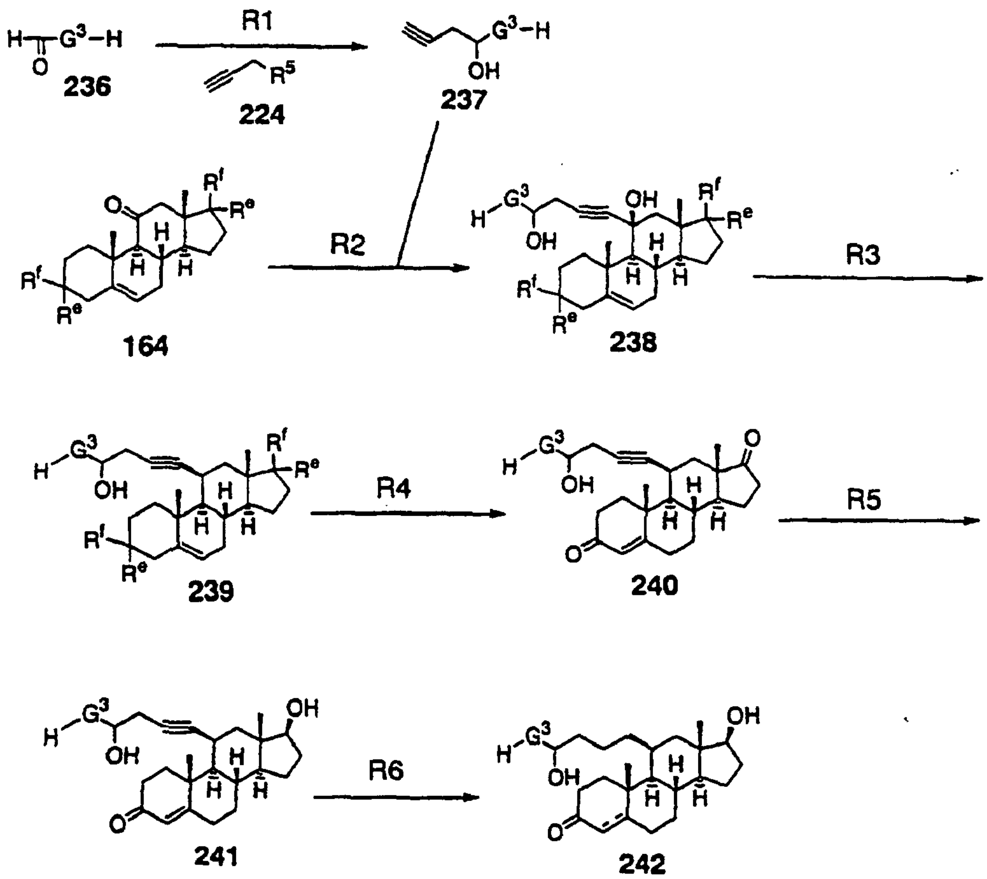


236 224 237 164 238 239 240 241 242

Step R1 is for producing compound (237) and implemented by reacting compound (224) with a metal (preferably magnesium) or an alkyllithium (preferably t-butyllithium) in an inert solvent in the presence or absence (preferably the presence) of an additive {preferably mercury(II) chloride} to make a reactive derivative of compound (224) and reacting it with compound (236) in an inert solvent. The reaction is performed as in the aforementioned step Q1 in process Q.

Step R2 is for producing compound (238) and implemented by reacting compound (237) with an alkyllithium (preferably n-butyllithium) in an inert solvent to make a reactive derivative of compound (237) and reacting it with compound (164) in an inert solvent. The reaction is performed as in the aforementioned step Q2 in process Q.

Step R3 is for producing compound (239) and implemented by reacting compound (238) with a reducing agent in an inert solvent in the presence of an additive. The reaction is performed as in the aforementioned step Q3 in process Q.

As a by-product of this step, there is formed a compound having the substituent in 11-position of compound (239) oriented in $\alpha$ configuration; by using this compound, one can obtain a compound having $X^1$ in compound (242) oriented in $\alpha$ configuration.

Step R4 is for producing compound (240) and implemented by reacting compound (239) with an acid in an aqueous solvent. The reaction is performed as in the aforementioned step Q7 in process Q.

Step R5 is for producing compound (241) and implemented by reacting compound (240) with a reducing agent in an optionally miscible inert solvent. The reaction is performed as in the aforementioned step Q8 in process Q.

Step R6 is for producing compound (242) and implemented by performing catalytic reduction of compound (241) in an alcoholic solvent or an inert solvent. The reaction is performed as in the aforementioned step Q10 in process Q.

As an ancillary to this reaction, conversion to a single bond may occasionally be effected if the dashed line forms a double bond together with the solid line.

In process R, the hydroxyl group on G may optionally be subjected to a protecting reaction and a deprotecting reaction in any desired steps.

Process S is for producing compound (244) represented by the general formula (I) in which $X^1$ is a group of β configuration that is represented by the general formula (II) in which Ar is a single bond, A is -O- and $R^1$ is $-CH_2-CH_2-CH_2-CH_2-G^2-COOH$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; and compound (245) represented by the general formula (I) in which $X^1$ is a group of β configuration that is represented by the general formula (II) in which Ar is a single bond, A is -O- and $R^1$ is $-CH_2-CH_2-CH_2-CH_2-G^2-CON(R^7)Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

Process S

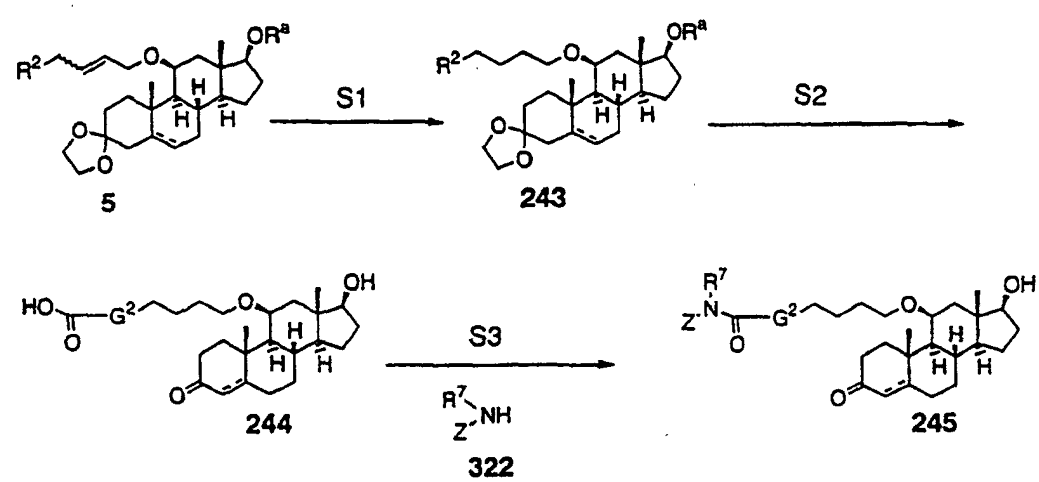

5
243
244
322
245

Step S1 is for producing compound (243) and implemented by performing catalytic reduction in an alcoholic solvent or an inert solvent. The reaction is performed as in the aforementioned step A6 in process A.

Step S2 is for producing compound (244) in the case where $Q^2$ in $R^2$ in compound (243) is $Q^{17}$ and Z is a hydrogen atom and this is implemented by reacting compound (243) with an acid in an aqueous solvent. The reaction is performed as in the aforementioned step AS in process A.

Step S3 is for producing compound (245) and implemented by reacting compound (244) or reactive derivatives thereof (acid halides, mixed acid anhydrides or active esters) with compound (322) or acid addition salts thereof in an inert solvent. The reaction is performed as in the aforementioned step C3 in process C.

In process S, compound (245) can be produced whether the sequence of reaction steps is S2 → S3 → S1 or S2 → S1 → S2.

Process S' is for producing compound (251) represented by the general formula (I) in which $X^1$ is a group of β configuration that is represented by the general formula (II) in which Ar is a single bond, A is -O- and $R^1$ is $-CH_2-CH_2-CH_2-G^3-SO-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; and compound (254) represented by the general formula (I) in which $X^1$ is a group of β configuration that is represented by the general formula (II) in which Ar is a single bond, A is -O- and $R^1$ is $-CH_2-CH_2-CH_2-G^3-SO_2-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

Process S'

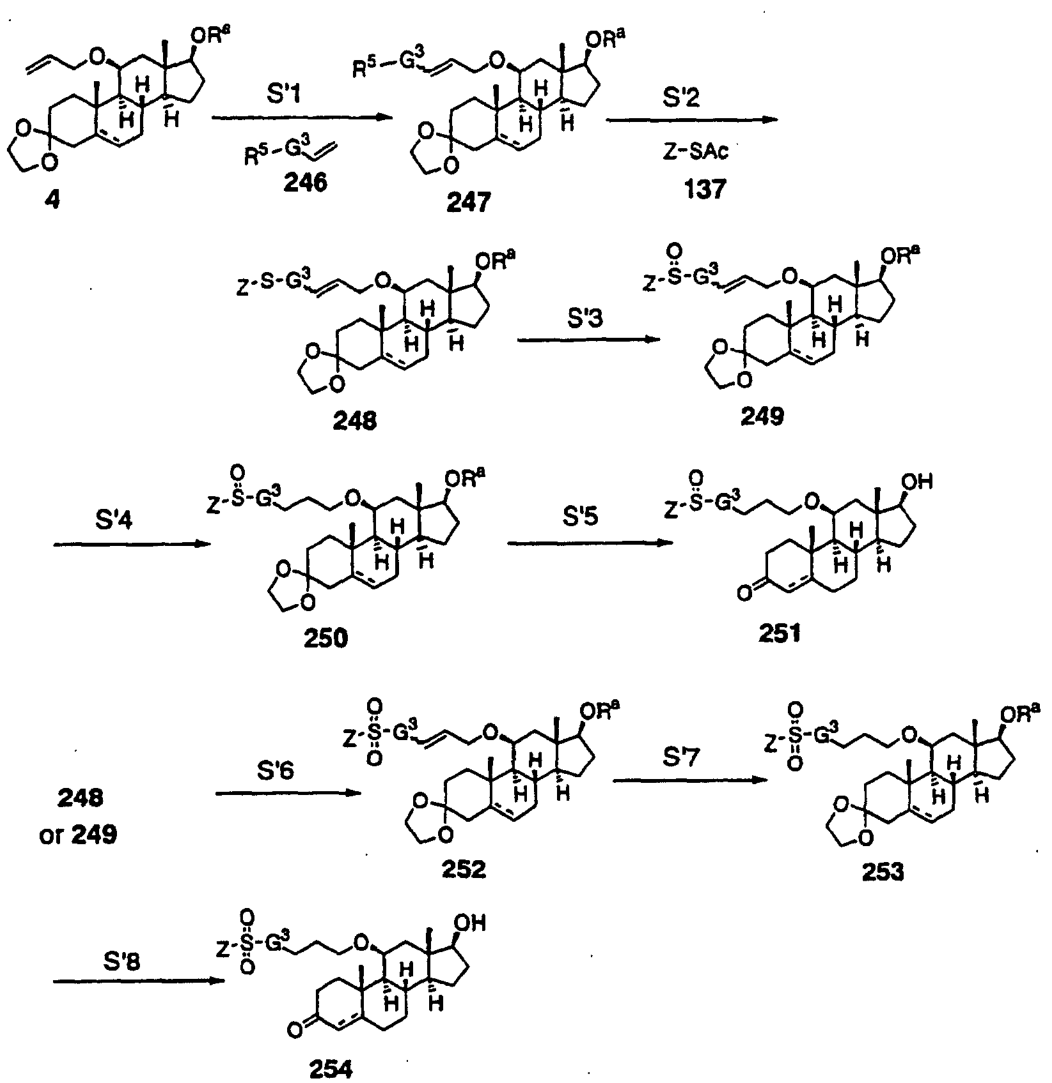


4, 246, 247, 137, 248, 249, 250, 251, 248 or 249, 252, 253, 254

Step S'1 is for producing compound (247) and implemented by reacting compound (4) with compound (246) in an inert solvent in the presence of an organometallic catalyst. The reaction is performed as in the aforementioned step A4 in process A.

Step S'2 is for producing compound (248) and implemented by reacting compound (137) with a metal alkoxide in an alcoholic solvent to make a reactive derivative of compound (137) and reacting it with compound (247) in an alcoholic solvent. The reaction is performed as in the aforementioned step B4 in process B.

Step S'3 is for producing compound (249) and implemented by reacting compound (248) with an oxidizing agent in an inert solvent. The reaction is performed as in the aforementioned step A8 in process A.

Step S'4 is for producing compound (250) and implemented by performing catalytic reduction in an alcoholic solvent or an inert solvent. The reaction is performed as in the aforementioned step A6 in process A.

Step S'5 is for producing compound (251) and implemented by reacting compound (250) with an acid in an aqueous solvent. The reaction is performed as in the aforementioned step A5 in process A.

Step S'6 is for producing compound (252) and implemented by reacting compound (248) or compound (249) with an oxidizing agent in an inert solvent. The reaction is performed as in the aforementioned step A9 in process A.

Step S'7 is for producing compound (253) and implemented by performing catalytic reduction in an alcoholic solvent or an inert solvent. The reaction is performed as in the aforementioned step A6 in process A.

This step can also be implemented by using compound (250) as the starting material.

Step S'8 is for producing compound (254) and implemented by reacting compound (253) with an acid in an aqueous solvent. The reaction is performed as in the aforementioned step A5 in process A.

This step can also be implemented by using compound (251) as the starting material.

In process S', compound (251) can be produced from compound (247) whether the sequence of reaction steps is S'4 → S'2 → S'5 → S'3, or S'5 → S'4 → S'2 → S'3, or S'4 → S'5 → S'2 → S'3, or S'5 → S'2 → S'3 → S'4, and compound (254) can be produced from compound (247) whether the sequence of reaction steps is S'4 → S'2 → S'5 → S'6, or S'5 → S'4 → S'2 → S'6, or S'4 → S'5 → S'2 → S'6, or S'5 → S'2 → S'6 → S'4.

Process T is for producing compound (261) represented by the general formula (I) in which $X^1$ is a group of β configuration that is represented by the general formula (II) in which Ar is a single bond, A is a methylene group and $R^1$ is $-CH_2-CH_2-CH_2-G^2-COOR^7$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (262) represented by the general formula (I) in which $X^1$ is a group of β configuration that is represented by the general formula (II) in which Ar is a single bond, A is a methylene group and $R^1$ is $-CH_2-CH_2-CH_2-G^2-COOH$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; and compound (263) represented by the general formula (I) in which $X^1$ is a group of β configuration that is represented by the general formula (II) in which Ar is a single bond, A is a methylene group and $R^1$ is $-CH_2-CH_2-CH_2-G^2-CON(R^7)-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond.

## Process T

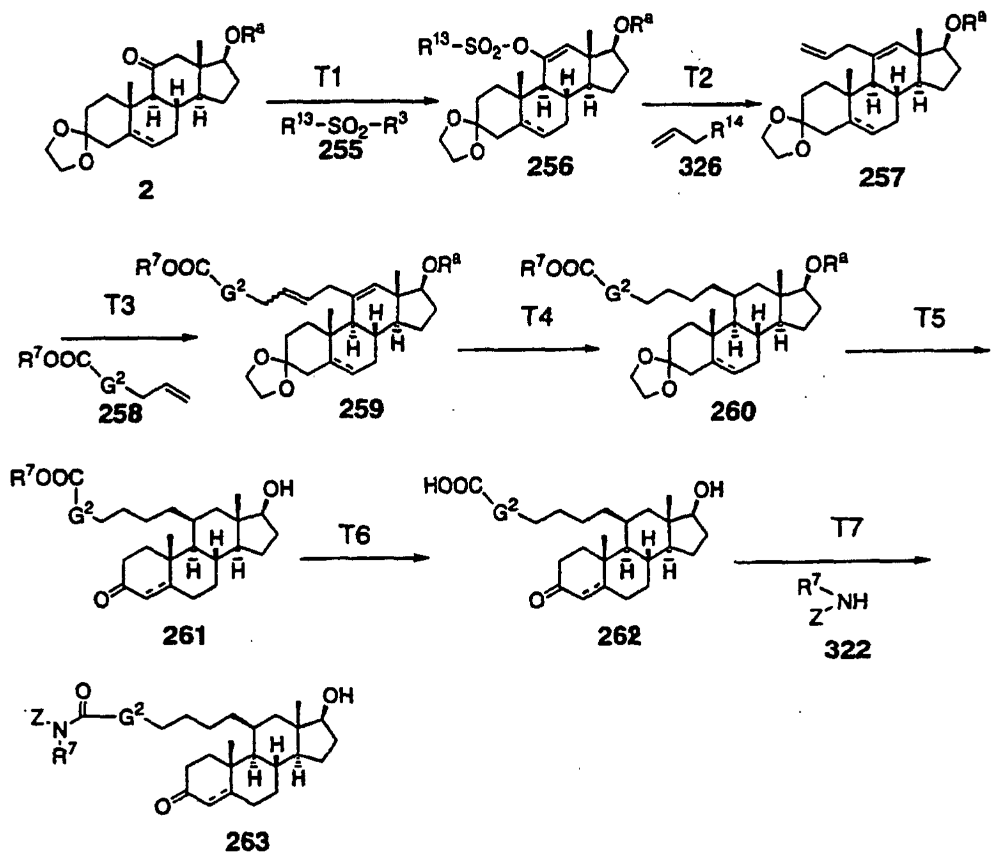


Step T1 is for producing compound (256) and implemented by reacting compound (2) with a base in an inert solvent to make a reactive derivative of compound (2) and reacting it with compound (255) in an inert solvent.

The inert solvent to be used is not limited in any particular way as long as it does not participate in the reaction; preferred examples are ether solvents such as ether, tetrahydrofuran, dioxane and dimethoxyethane, with tetrahydrofuran being more preferred. Preferred examples of the base to be used are n-butyllithium and lithium diisopropylamide. The reaction temperature which varies with the type of solvent and the like is typically in the range of -100°C - 50°C,

preferably -78°C - 30 dc. The reaction time which varies with the reaction temperature and the like is typically in the range of 10 minutes - 48 hours, preferably 30 minutes - 24 hours.

Step T2 is for producing compound (257) and implemented by reacting compound (256) with compound (326) in an inert solvent in the presence of a metal catalyst.

The inert solvent to be used is not limited in any particular way as long as it does not participate in the reaction; preferred examples are ether solvents such as ether, tetrahydrofuran, dioxane and dimethoxyethane, with tetrahydrofuran being more preferred. The metal catalyst to be used is not limited in any particular way and may be exemplified by tetrakis(triphenylphosphine)palladium, palladium(II) acetate-triphenylphosphine, bis(triphenylphosphine)palladium (II) chloride, etc, with tetrakis(triphenylphosphine)palladium being preferred. The reaction temperature which varies with the type of solvent and the like is typically in the range of 0°C - 100°C, preferably 10°C - 80°C. The reaction time which varies with the reaction temperature and the like is typically in the range of 10 minutes - 48 hours, preferably 30 minutes - 24 hours.

Step T3 is for producing compound (259) and implemented by reacting compound (257) with compound (258) in an inert solvent in the presence of an organometallic catalyst. The reaction is performed as in the aforementioned step A4 in process A.

Step T4 is for producing compound (260) and implemented by performing catalytic reduction in an alcoholic solvent or an inert solvent.

The solvent to be used may be exemplified by alcoholic solvents such as methanol, ethanol, n-propanol, i-propanol, n-butanol, s-butanol, t-butanol, pentanol, hexanol, cyclopropanol, cyclobutanol, cyclopentanol, cyclohexanol, ethylene glycol, 1,3-propanediol, 1,4-butanediol and 1,5-pentanediol, ether solvents such as ether, tetrahydrofuran, dioxane and dimethoxyethane, aromatic solvents such as benzene, toluene, xylene, quinoline and chlorobenzene, halogen-containing solvents such as dichloromethane, chloroform and carbon tetrachloride, as well as cyclohexane, dimethyl sulfoxide, dimethylacetamide, dimethylimidazolidinone, dimethylformamide, N-methylpyrrolidone, ethyl acetate, acetonitrile and nitromethane; preferred examples are ethanol, dioxane, benzene and ethyl acetate.

The condition to be used in catalytic reduction is a homogeneous system such as hydrogen-chlorotris(triphenylphosphine)rhodium(I), hydrogen-chlorotris(triparatolylphosphine)rhodium(I), hydrogen-chlorotris(triparamethoxyphenylphosphine)rhodium(I), hydrogen-hydridecarbonyltris(triphenylphosphine)rhodium(I), hydrogen-rhodium(II) acetate, hydrogen-ruthenium(II) acetate, hydrogen-chlorohydridetris(triphenylphosphine)ruthenium(II), hydrogen-carboxylatohydridetris(triphenylphosphine)ruthenium(II), hydrogen-hydridecarbonyltris(triphenylphosphine)iridium(I), hydrogen-platinum(II)-tin chloride complex, hydrogen-pentacyanocobalt(II) complex, hydrogen-tricyanobipyridine cobalt(II) complex, hydrogen-bis(dimethylglyoximato)cobalt(II) complex, hydrogen-methyl benzoate-tricarbonylchromium complex, hydrogen-bis(tricarbonylcyclopentadienylchromium), hydrogen-pentacarbonyliron, hydrogen-bis(cyclopentadienyl)dicarbonyltitanium, hydrogen-hydridecarbonylcobalt complex, hydrogen-octacarbonyldicobalt, hydrogen-hydridecarbonylrhodium, hydrogen-chromium(III) acetylacetonato-triisobutylaluminum, hydrogen-cobalt(II) acetylacetonato-triisobutylaluminum, or hydrogen-nickel(II)-2-hexanoato-triethylaluminum, or an inhomogeneous system condition such as hydrogen-platinum dioxide, hydrogen-platinum/carbon, hydrogen-palladium/carbon, hydrogen-palladium hydroxide/carbon, hydrogen-palladium/barium sulfate, hydrogen-palladium/calcium carbonate, hydrogen-Raney nickel, hydrogen-copper chromite, hydrogen-rhodium/carbon, hydrogen-rhodium/alumina, hydrogen-ruthenium dioxide, hydrogen-ruthenium/carbon, or hydrogen-iridium black; a preferred example is hydrogen-iridium black.

The reaction temperature is typically in the range of 0°C - 100°C, preferably 0°C - 60°C. The reaction time which varies with the reaction temperature and the like is typically in the range of 10 minutes - 100 hours, preferably 10 hours - 96 hours.

Step T5 is for producing compound (261) and implemented by reacting compound (260) with an acid in an aqueous solvent. The reaction is performed as in the aforementioned step A5 in process A.

In step T5, ester hydrolysis may occur and in that case, subsequent step T6 may be omitted.

Step T6 is for producing compound (262) and implemented by hydrolyzing compound (261) in water or a water-soluble solvent in the presence of a base or an acid (preferably a base). The reaction is performed as in the aforementioned step O6 in process O.

Step T7 is for producing compound (263) and implemented by reacting compound (262) or reactive derivatives thereof (acid halides, mixed acid anhydrides or active esters) with compound (322) or acid addition salts thereof in an inert solvent. The reaction is performed as in the aforementioned step C3 in process C.

Process T' is for producing compound (268) represented by the general formula (I) in which $X^1$ is a group of β configuration that is represented by the general formula (II) in which Ar is a single bond, A is a methylene group and $R^1$ is $-CH_2-CH_2-CH_2-G^2-CH_2-S-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (269) represented by the general formula (I) in which $X^1$ is a group of β configuration that is represented by the general formula (II) in which Ar is a single bond, A is a methylene group and $R^1$ is $-CH_2-CH_2-CH_2-G^2-CH_2-SO-Z$, $X^2$ is a hydrogen atom, $R^a$ is a hydrogen atom, $R^b$ and $R^c$, when taken together

with the carbon atom in 3-position to which they are bound, are -(C=O)-, and the dashed line together with the solid line is a single bond or a double bond; and compound (270) represented by the general formula (I) in which $X^1$ is a group of β configuration that is represented by the general formula (II) in which Ar is a single bond, A is a methylene group and $R^1$ is $-CH_2-CH_2-CH_2-G^2-CH_2-SO_2-Z$, $X^2$ is a hydrogen atom, $R^a$ is a hydrogen atom, $R^b$ and $R^c$, when taken together with the carbon atom in 3-position to which they are bound, are -(C=O), and the dashed line together with the solid line is a single bond or a double bond.

## Process T'

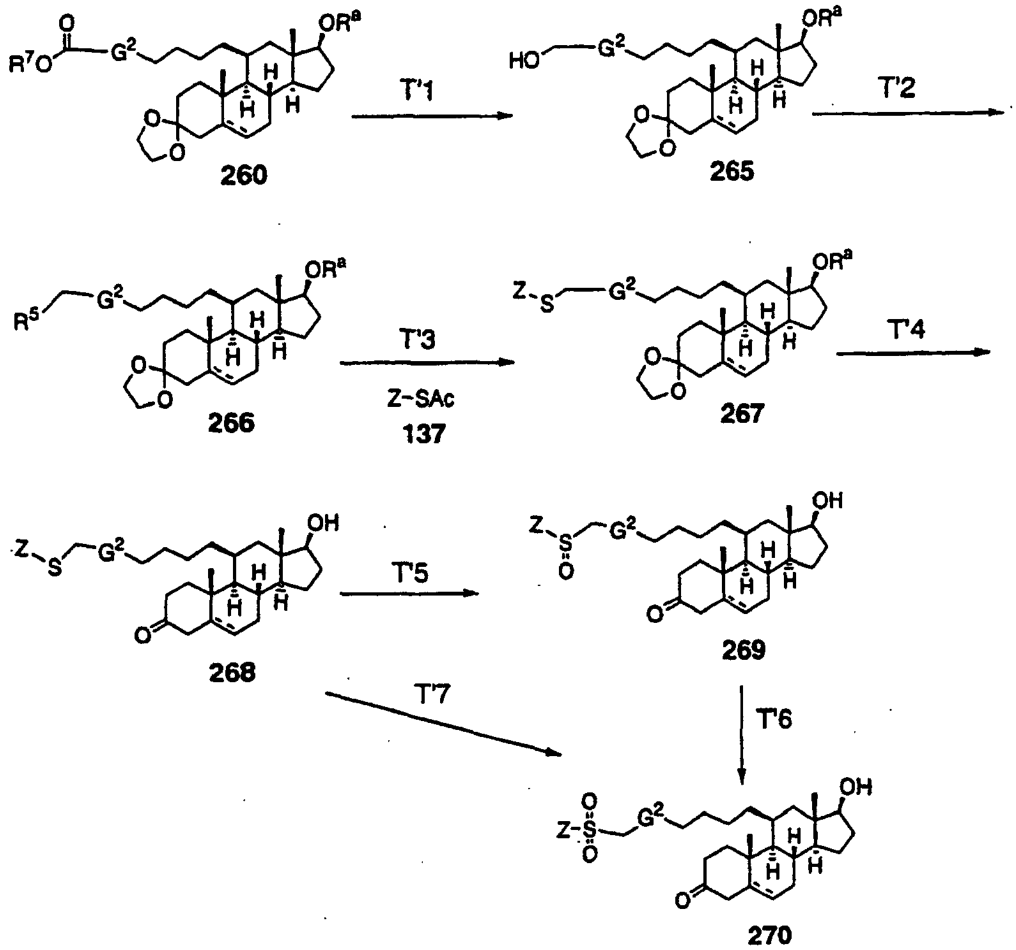


Step T'1 is for producing compound (265) and implemented by reacting compound (260) with a reducing agent in an inert solvent and the reaction is performed as in the aforementioned step A2 in process A.

Step T'2 is for producing compound (266) and implemented by reacting compound (265) with a sulfonyl chloride compound in an amine-containing solvent or reacting compound (265) with a halogenating agent in an inert solvent. The reaction is performed as in the aforementioned step B3 in process B.

Step T'3 is for producing compound (267) and implemented by reacting compound (137) with a metal alkoxide in an alcoholic solvent to make a reactive derivative of compound (137) and reacting it with compound (266) in an alcoholic solvent. The reaction is performed as in the aforementioned step B4 in process B.

Step T'4 is for producing compound (268) and implemented by reacting compound (267) with an acid in an aqueous solvent. The reaction is performed as in the aforementioned step A5 in process A.

Step T'5 is for producing compound (269) and implemented by reacting compound (268) with an oxidizing agent in an inert solvent. The reaction is performed as in the aforementioned step A8 in process A.

Step T'6 is for producing compound (270) and implemented by reacting compound (269) with an oxidizing agent in an inert solvent. The reaction is performed as in the aforementioned step A9 in process A.

Step T'7 is an alternative method for producing compound (270) and implemented by reacting compound

(268) with an oxidizing agent in an inert solvent. The reaction is performed as in the aforementioned step A9 in process A.

In process T', step T'3 and step T'4 may be interchanged in their sequence. If desired, step T'4 and step T'5 may also be interchanged in their sequence. Compound (270) can also be obtained from compound (267) if the sequence of reaction steps is T'6 → T'4.

Process U is for producing compound (276) represented by the general formula (I) in which $X^1$ is a group of β configuration that is represented by the general formula (II) in which Ar is an aromatic hydrocarbon group (preferably a p-phenylene group), A is -O- and $R^1$ is a methyl group, $X^2$ is a hydrogen atom, $R^b$ and $R^c$, when taken together with the carbon atom in 3-position to which they are bound, are -(C=O)-, and the dashed line together with the solid line is a single bond or a double bond; compound (278) represented by the general formula (I) in which $X^1$ is a group of β configuration that is represented by the general formula (II) in which Ar is an aromatic hydrocarbon group (preferably a p-phenylene group), A is -O- and $R^1$ is $-CH_2-CH=CH_2$, $X^2$ is a hydrogen atom, $R^b$ and $R^c$, when taken together with the carbon atom in 3-position to which they are bound, are -(C=O), and the dashed line together with the solid line is a single bond or a double bond; compound (279) represented by the general formula (I) in which $X^1$ is a group of β configuration that is represented by the general formula (II) in which Ar is an aromatic hydrocarbon group (preferably a p-phenylene group), A is -O- and $R^1$ is $-CH_2-CH=CH-CH_2-R^2$, $X^2$ is a hydrogen atom, $R^b$ and $R^c$, when taken together with the carbon atom in 3-position to which they are bound, are -(C=O)-, and the dashed line together with the solid line is a single bond or a double bond; compound (280) represented by the general formula (I) in which $X^1$ is a group of β configuration that is represented by the general formula (II) in which Ar is an aromatic hydrocarbon group (preferably a p-phenylene group), A is -O- and $R^1$ is $-CH_2-CH_2-CH_2-CH_2-R^2$, $X^2$ is a hydrogen atom, $R^b$ and $R^c$, when taken together with the carbon atom in 3-position to which they are bound, are -(C=O), and the dashed line together with the solid line is a single bond or a double bond; compound (281) represented by the general formula (I) in which $X^1$ is a group of β configuration that is represented by the general formula (II) in which Ar is an aromatic hydrocarbon group (preferably a p-phenylene group), A is -O- and $R^1$ is $-CH_2-CH_2-CH_2-CH_2-G^2-COOH$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; and compound (282) represented by the general formula (I) in which $X^1$ is a group of β configuration that is represented by the general formula (II) in which Ar is an aromatic hydrocarbon group (preferably a p-phenylene group), A is -O- and $R^1$ is $-CH_2-CH_2-CH_2-CH_2-G^2-CON(R^7)Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond

## Process U

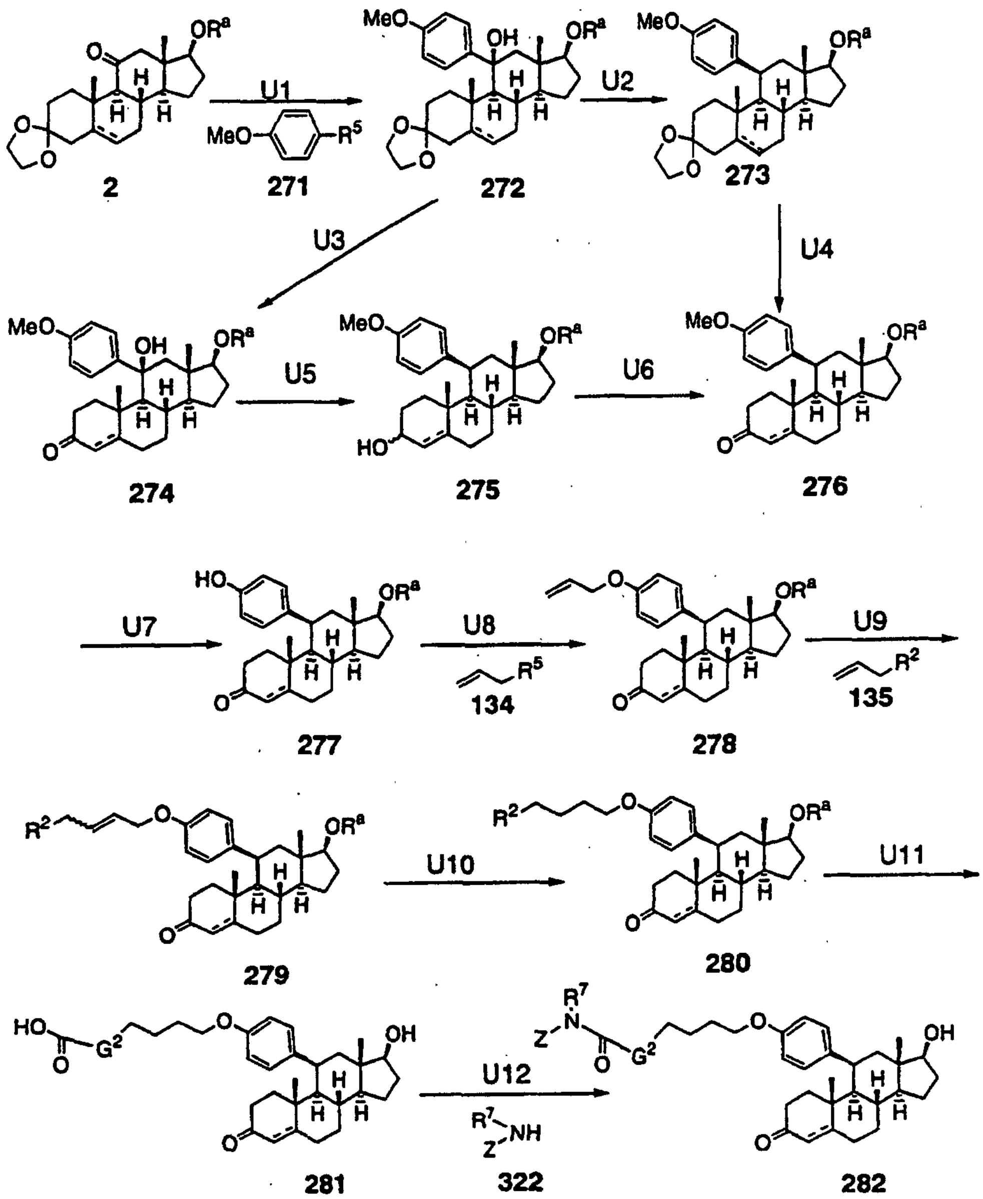


2, 271, 272, 273, 274, 275, 276, 277, 134, 278, 135, 279, 280, 281, 322, 282

Step U1 is for producing compound (272) and implemented by reacting compound (271) with a metal (preferably magnesium) or an alkyllithium (preferably n-butyllithium) in an inert solvent to make a reactive derivative of compound (271) and reacting it with compound (2) in an inert solvent. The reaction is performed as in the aforementioned step I1 in process I.

Step U2 is for producing compound (273) and implemented by reacting compound (272) with a reducing agent in an inert solvent in the presence of an additive. The reaction is performed as in the aforementioned step E2 in process E.

Step U3 is for producing compound (274) and implemented by reacting compound (272) with an acid in an aqueous solvent.

The solvent to be used is not limited in any particular way as long as it does not interfere with the reaction; examples are mixtures of water and ether solvents such as ether, tetrahydrofuran and dioxane, alcoholic solvents such as methanol and ethanol, or ketonic solvents such as acetone, and hydrous acetone is preferred.

The acid to be used may be exemplified by inorganic acids such as hydrochloric acid, hydrobromic acid, hydroiodic acid, sulfuric acid and phosphoric acid, and organic acids such as acetic acid, p-toluenesulfonic acid and pyridinium-p-toluenesulfonate, with hydrochloric acid being preferred. The reaction temperature which varies with the

type of solvent and the like is typically in the range of 0°C - 50°C (preferably 10°C - 30°C). The reaction time which varies with the reaction temperature and the like is typically in the range of 15 minutes - 24 hours (preferably 30 minutes - 5 hours).

Step U4 is for producing compound (276) and implemented by reacting compound (273) with an acid in an aqueous solvent. The reaction is performed as in the aforementioned step U3 in process U.

Step U5 is for producing compound (275) and implemented by reacting compound (274) with a reducing agent in an inert solvent in the presence of an additive. The reaction is performed as in the aforementioned step E2 in process E.

Step U6 is an alternative method for producing compound (276) and implemented by reacting compound (275) with an oxidizing agent in an inert solvent.

The inert solvent to be used is not limited in any particular way as long as it does not interfere with the reaction; examples are halogen-containing solvents such as dichloromethane, chloroform and carbon tetrachloride, ethers such as tetrahydrofuran, dioxane and dimethoxyethane, and hydrocarbon solvents such as benzene, toluene, xylene, quinoline and chlorobenzene, with dichloromethane and tetrahydrofuran being preferred. Water may optionally be added to these solvents. The oxidizing agent to be used is not limited in any particular way and examples can be manganese compounds such as potassium permanganate, manganese dioxide, manganese(III) acetate, tris(acetonylacetonite) manganese(III) (MTA), manganese sulfate and manganese(III) pyrophosphate, chromates such as chromium(IV) oxide, Jones reagent, Sarett reagent, Collins reagent, chromic acid t-butyl ester, potassium bichromate, Beckmann's mixture, sodium bichromate, Kiliani reagent, chromyl chloride, chromyl acetate, pyridinium chlorochromate (PCC), and pyridinium dichromate (PDC); ruthenium compounds such as ruthenium tetroxide, tris(triphenylphosphine)dichlororuthenium/iodosylbenzene, tris(triphenylphosphine)dichlororuthenium/N-methylmorpholin-N-oxide, tris(triphenylphosphine)dichlororuthenium/t-butyl hydroperoxide, tetrapropylammonium perruthenate (TPAP), tetrapropylammonium perruthenate (TPAP)/N-methylmorpholin-N-oxide, tetrabutylammonium perruthenate (TBAP), and tetrabutylammonium perruthenate (TBAP)/N-methylmorpholin-N-oxide; halogens such as hypochlorous acid, sodium hypochlorite, potassium hypobromite, potassium hypoiodite, sodium chlorate, potassium chlorate, sodium bromate, potassium bromate, sodium iodate, potassium iodate, perchloryl fluoride, orthoperiodic acid, sodium metaperiodate, potassium metaperiodate, N-bromoacetamide, N-bromosuccinimide and N-bromophthalimide; as well as dimethyl sulfoxide/oxalyl chloride; preferred examples are chromates such as pyridinium chlorochromate (PCC) and pyridinium dichromate (PDC), and ruthenium compounds such tetrapropylammonium perruthenate (TPAP)/N-methylmorpholin-N-oxide. The reaction temperature which varies with the type of solvent and the like is typically in the range of -30°C ~ 100°C, preferably 0°C - 30°C. The reaction time which varies with the reaction temperature and the like is typically in the range of 10 minutes - 48 hours, preferably 30 minutes - 24 hours.

Step U7 is an alternative method of producing compound (277) and implemented by reacting compound (276) with a deprotecting agent in an inert solvent.

The inert solvent to be used is not limited in any particular way as long as it does not interfere with the reaction; examples are dimethyl sulfoxide, dimethylacetamide, dimethylimidazolidinone, dimethylformamide, N-methylpyrrolidone, etc., with dimethylformamide being preferred. The deprotecting agent to be used is not limited in any particular way and may be exemplified by sodium thiomethoxide, sodium cyanide, trimethylsilane iodide, boron tribromide, boron trichloride and lithium chloride, with sodium thiomethoxide being preferred. The reaction temperature which varies with the type of solvent and the like is typically in the range of -80°C - 200°C, preferably 0°C ~ 180°C. The reaction time which varies with the reaction temperature and the like is typically in the range of 10 minutes - 96 hours, preferably 30 minutes - 48 hours.

Step U8 is for producing compound (278) and implemented by reacting compound (277) with a base in an inert solvent to make a salt of compound (277) and then reacting it with compound (134) in an inert solvent. The reaction is performed as in the aforementioned step A3 in process A.

Step U9 is for producing compound (279) and implemented by reacting compound (135) with compound (278) in an inert solvent in the presence of an organometallic catalyst. The reaction is performed as in the aforementioned step A4 in process A.

Step U10 is for producing compound (280) and implemented by performing catalytic reduction in an alcoholic solvent or an inert solvent. The reaction is performed as in the aforementioned step A6 in process A.

Step U11 is for producing compound (281) in the case where $R^2$ in compound (280) is $G^2$-$COOR^7$ and this is implemented by reacting compound (280) with an acid in an aqueous solvent. The reaction is performed as in the aforementined step A5 in process A.

Step U12 is for producing compound (282) and implemented by reacting compound (281) or reactive derivatives thereof (acid halides, mixed acid anhydrides or active esters) with compound (322) or acid addition salts thereof in an inert solvent. The reaction is performed as in the aforementioned step C3 in process C.

Process U' is for producing compound (283) represented by the general formula (I) in which $X^1$ is a group of β configuration that is represented by the general formula (II) in which Ar is an aromatic hydrocarbon group (preferably

a p-phenylene group), A is -O- and $R^1$ is an allyl group, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (284) represented by the general formula (I) in which $X^1$ is a group of β configuration that is represented by the general formula (II) in which Ar is an aromatic hydrocarbon group (preferably a p-phenylene group), A is -O- and $R^1$ is $-CH_2-CH=CH-G^3-R^5$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (285) represented by the general formula (I) in which $X^1$ is a group of β configuration that is represented by the general formula (II) in which Ar is an aromatic hydrocarbon group (preferably a p-phenylene group), A is -O- and $R^1$ is $-CH_2-CH=CH-G^3-S-Z$, $X^2$ is a hydrogen atom, $R^a$ is a hydrogen atom, $R^b$ and $R^c$, when taken together with the carbon atom in 3-position to which they are bound, are -(C=O), and the dashed line together with the solid line is a single bond or a double bond; compound (286) represented by the general formula (I) in which $X^1$ is a group of β configuration that is represented by the general formula (II) in which Ar is an aromatic hydrocarbon group (preferably a p-phenylene group), A is -O- and $R^1$ is $-CH_2-CH=CH-G^3-SO-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (287) represented by the general formula (I) in which $X^1$ is a group of β configuration that is represented by the general formula (II) in which Ar is an aromatic hydrocarbon group (peferably a p-phenylene group), A is -O- and $R^1$ is $-CH_2-CH=CH-G^3-SO_2-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; compound (288) represented by the general formula (I) in which $X^1$ is a group of β configuration that is represented by the general formula (II) in which Ar is an aromatic hydrocarbon group (peferably a p-phenylene group), A is -O- and $R^1$ is $-CH_2-CH_2-CH_2-G^3-SO-Z$, $X^2$ is a hydrogen atom, $R^a$ is a hydrogen atom, $R^b$ and $R^c$, when taken together with the carbon atom in 3-position to which they are bound, are -(C=O)-, and the dashed line together with the solid line is a single bond or a double bond; and compound (289) represented by the general formula (I) in which $X^1$ is a group of β configuration that is represented by the general formula (II) in which Ar is an aromatic hydrocarbon group (peferably a p-phenylene group), A is -O- and $R^1$ is $-CH_2-CH_2-CH_2-G^3-SO_2-Z$, $X^2$ is a hydrogen atom, $R^a$ is a hydrogen atom, $R^b$ and $R^c$, when taken together with the carbon atom in 3-position to which they are bound, are -(C=O), and the dashed line together with the solid line is a single bond or a double bond.

## Process U'

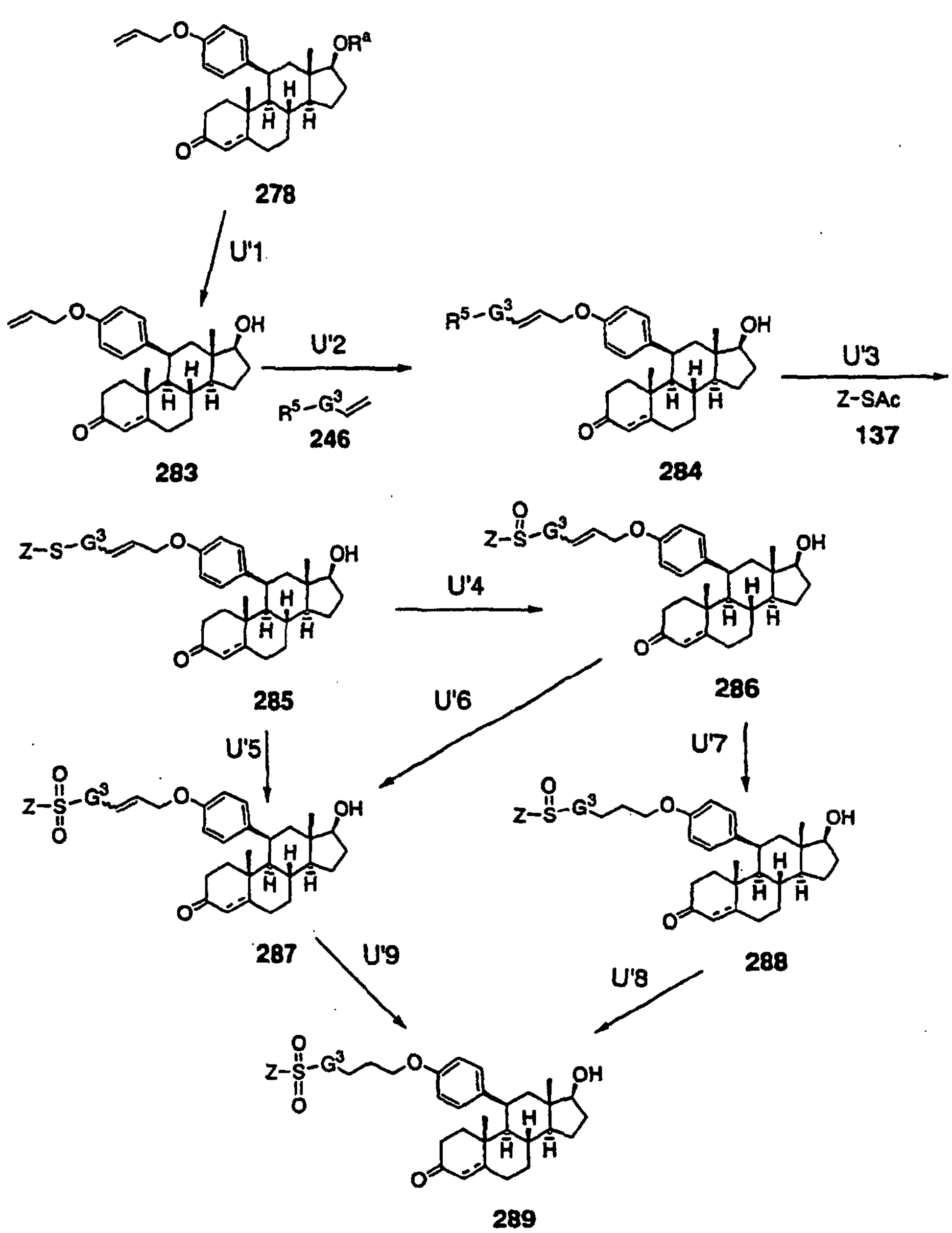


Step U'1 is for producing compound (283) and implemented by reacting compound (278) with an acid in an aqueous solvent. The reaction is performed as in the aforementioned step A5 in process A.

Step U'2 is for producing compound (284) and implemented by reacting compound (246) with compound (283) in an inert solvent in the presence of an organometallic catalyst. The reaction is performed as in the aforementioned step A4 in process A.

Step U'3 is for producing compound (285) and implemented by reacting compound (137) with a metal alkoxide in an alcoholic solvent to make a reactive derivative of compound (137) and then reacting it with compound (284) in an alcoholic solvent. The reaction is performed as in the aforementined step B4 in process B.

Step U'4 is for producing compound (286) and implemented by reacting compound (285) with an oxidizing agent in an inert solvent. The reaction is performed as in the aforementioned step A8 in process A.

Step U'5 is for producing compound (287) and implemented by reacting compound (285) with an oxidizing agent in an inert solvent. The reaction is performed as in the aforementioned step A9 in process A.

Step U'6 is an alternative method for producing compound (287) and implemented by reacting compound

(286) with an oxidizing agent in an inert solvent. The reaction is performed as in the aforementioned step A9 in process A.

Step U'7 is for producing compound (288) and implemented by performing catalytic reduction in an alcoholic solvent or an inert solvent. The reaction is performed as in the aforementioned step A6 in process A.

Step U'8 is an alternative method for producing compound (289) and implemented by reacting compound (288) with an oxidizing agent in an inert solvent. The reaction is performed as in the aforementioned step A9 in process A.

Step U'9 is an alternative method for producing compound (289) and implemented by performing catalytic reduction in an alcoholic solvent or an inert solvent. The reaction is performed as in the aforementioned step A6 in process A.

In process U', compound (288) can be obtained from compound (284) if the sequence of reaction steps is U'7 → U'3 → U'4. Compound (289) can also be obtained from compound (284) if the sequence of reaction steps is U'7 → U'3 → U'5.

Process U" is an alternative method to process U' for producing compound (273) and compound (275).

Process U"

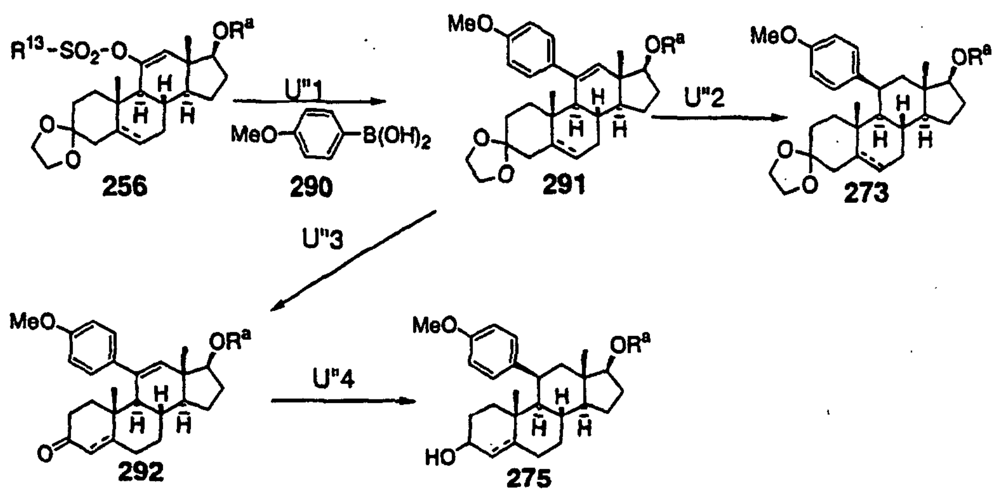


256 290 291 273

292 275

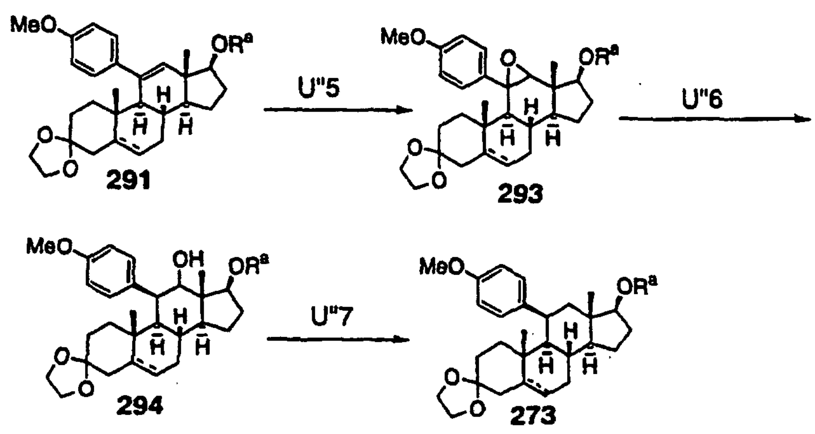


291 293

294 273

Step U"1 is for producing compound (291) and implemented by reacting compound (256) with compound (290) in an optionally miscible inert solvent in the presence of a metal catalyst and a base.

The inert solvent to be used is not limited in any particular way as long as it does not interfere with the reaction; examples are ether solvents such as dioxane and tetrahydrofuran, aromatic hydrocarbon solvents such as toluene, alcoholic solvents such as ethanol, as well as dimethylformamide, dimethylacetamide and acetonitrile, with dioxane

and ethanol-toluene being preferred. The metal catalyst to be used is not limited in any particular way and may be exemplified by etrakis(triphephenylphosphine)palladium, palladium(II) acetate-triphenylphosphine, bis(triphenylphosphine)palladium(II) chloride, etc., with tetrakis(triphephenylphosphine)palladium being preferred. The base to be used is not limited in any particular way and may be exemplified by potassium phosphate, sodium carbonate, etc., with sodium carbonate being preferred. The reaction temperature which varies with the type of solvent and the like is typically in the range of 0°C - 180°C, preferably 10°C - 120°C. The reaction time which varies with the reaction temperature and the like is typically in the range of 10 minutes - 48 hours, preferably 30 minutes - 24 hours.

Step U"2 is for producing compound (273) and implemented by performing catalytic reduction in an alcoholic solvent or an inert solvent. The solvent to be used may be exemplified by alcoholic solvents such as methanol, ethanol, n-propanol, i-propanol, n-butanol, s-butanol, t-butanol, pentanol, hexanol, cyclopropanol, cyclobutanol, cyclopentanol, cyclohexanol, ethylene glycol, 1,3-propanediol, 1,4-butanediol and 1,5-pentanediol, ether solvents such as ether, tetrahydrofuran, dioxane and dimethoxyethane, aromatic solvents such as benzene, toluene, xylene, quinoline and chlorobenzene, halogen-containing solvents such as dichloromethane, chloroform and carbon tetrachloride, as well as cyclohexane, dimethyl sulfoxide, dimethylacetamide, dimethylimidazolidinone, dimethylformamide, N-methylpyrrolidone, ethyl acetate, acetonitrile and nitromethane; preferred examples are ethanol, dioxane, benzene, ethyl acetate and acetonitrile.

The condition to be used in catalytic reduction is a homogeneous system such as hydrogen-chlorotris(triphenylphosphine)rhodium(I), hydrogen-chlorotris(triparatolylphosphine)rhodium(I), hydrogen-chlorotris(triparamethoxyphenylphosphine)rhodium(I), hydrogen-hydridecarbonyltris(triphenylphosphine)rhodium(I), hydrogen-rhodium(II) acetate, hydrogen-ruthenium(II) acetate, hydrogen-chlorohydridetris(triphenylphosphine)ruthenium(II), hydrogen-carboxylatohydridetris(triphenylphosphine)ruthenium(II), hydrogen-hydridecarbonyltris(triphenylphosphine)iridium(I), hydrogen-platinum(II)-tin chloride complex, hydrogen-pentacyanocobalt(II) complex, hydrogen-tricyanobipyridine cobalt(II) complex, hydrogen-bis(dimethylglyoximato)cobalt(II) complex, hydrogen-methyl benzoate-tricarbonylchromium complex, hydrogen-bis(tricarbonylcyclopentadienylchromium), hydrogen-pentacarbonyliron, hydrogen-bis(cyclopentadienyl)dicarbonyltitanium, hydrogen-hydridecarbonylcobalt complex, hydrogen-octacarbonyldicobalt, hydrogen-hydridecarbonylrhodium, hydrogen-chromium(III) acetylacetonato-triisobutylaluminum, hydrogen-cobalt(II) acetylacetonato-triisobutylaluminum, or hydrogen-nickel (II)-2-hexanoato-triethylaluminum, or an inhomogeneous system condition such as hydrogen-platinum dioxide, hydrogen-platinum/carbon, hydrogen-palladium/carbon, hydrogen-palladium hydroxide/carbon, hydrogen-palladium/barium sulfate, hydrogen-palladium/calcium carbonate, hydrogen-Raney nickel, hydrogen-copper chromite, hydrogen-rhodium/carbon, hydrogen-rhodium/alumina, hydrogen-ruthenium dioxide, hydrogen-ruthenium/carbon, or hydrogen-iridium black; preferred examples are hydrogen-palladium hydroxide/carbon, hydrogen-iridium black, etc.

The reaction temperature is typically in the range of 0°C - 100°C, preferably 0°C - 60°C. The reaction time which varies with the reaction temperature and the like is typically in the range of 10 minutes - 100 hours, preferably 10 minutes - 96 hours.

Step U"3 is for producing compound (292) and implemented by reacting compound (291) with an acid in an aqueous solvent. The reaction is performed as in the aforementioned step U3 in process U.

Step U"4 is for producing compound (275) and implemented by performing catalytic reduction in an alcoholic solvent or an inert solvent. The reaction is performed as in the aforementioned step U"2 in process U".

Step U"5 is for producing compound (293) and implemented by reacting compound (291) with an oxidizing agent in an inert solvent.

The inert solvent to be used is not limited in any particular way as long as it does not interfere with the reaction and examples include ether solvents such as dioxane and tetrahydrofuran, aromatic hydrocarbon solvents such as toluene, halogen-containing solvents such as dichloromethane, as well as dimethylformamide, dimethyl acetamide and acetonitrile; a preferred example is dichloromethane. The oxidizing agent to be used is not limited in any particular way and can be perbenzoic acid, metachloroperbenzoic acid, p-nitroperbenzoic acid, monoperoxyphthalic acid, performic acid, peracetic acid, trifluoroperacetic acid, etc.; a preferred example is metachloroperbenzoic acid. The reaction temperature which varies with the type of solvent and the like is typically in the range of -10°C - 50°C, preferably 0°C - 30°C. The reaction time which varies with the reaction temperature and the like is typically in the range of 10 minutes - 48 hours, preferably 30 minutes - 24 hours.

Step U"6 is for producing compound (294) and implemented by reacting compound (293) with a reducing agent in an inert solvent.

The inert solvent to be used is not limited in any particular way as long as it does not interfere with the reaction; examples are ether solvents such as tetrahydrofuran.

The reducing agent to be used can be sodium/liquid ammonia, lithium/liquid ammonia, lithium/methylamine, lithium/ethylamine, lithium/ethylenediamine, sodium/hexamethylphosphamide-t-butanol, sodiuim/ethanol, sodium/t-butanol-tetrahydrofuran, sodium/toluene-t-amyl alcohol, etc.; sodium/liquid ammonia is preferred. The reaction temperature which varies with the type of solvent and the like is typically in the range of -100°C ~ 20°C, preferably -80°C

- 0°C. The reaction time which varies with the reaction temperature and the like is typically in the range of 10 minutes - 24 hours, preferably 30 minutes - 5 hours.

Step U"7 is an alternative method for producing compound (273) and implemented by reacting compound (294) with a reducing agent in an inert solvent in the presence of an additive. The reaction is performed as in the aforementioned step E2 in process E.

Process V is for producing compound (296) represented by the general formula (I) in which $X^1$ is a hydrogen atom, $X^2$ is a group of α configuration that is represented by the general formula (II) in which Ar is a single bond, A is a methylene group and $R^1$ is -CH-CH=$G^3$-$R^5$, $R^a$ is a hydrogen atom, $R^b$ and $R^c$, when taken together with the carbon atom in 3-position to which they are bound, are -(C=O)-, and the dashed line together with the solid line is a single bond; compound (297) represented by the general formula (I) in which $X^1$ is a hydrogen atom, $X^2$ is a group of α configuration that is represented by the general formula (II) in which Ar is a single bond, A is a methylene group and $R^1$ is -CH-CH=$G^3$-S-Z, $R^a$ is a hydrogen atom, $R^b$ and $R^c$, when taken together with the carbon atom in 3-position to which they are bound, are -(C=O), and the dashed line together with the solid line is a single bond; compound (298) represented by the general formula (I) in which $X^1$ is a hydrogen atom, $X^2$ is a group of α configuration that is represented by the general formula (II) in which Ar is a single bond, A is a methylene group and $R^1$ is -CH=CH-$G^3$-SO-Z, $R^a$ is a hydrogen atom, $R^b$ and $R^c$, when taken together with the carbon atom in 3-position to which they are bound, are -(C=O), and the dashed line together with the solid line is a single bond; compound (299) represented by the general formula (I) in which $X^1$ is a hydrogen atom, $X^2$ is a group of α configuration represented by the general formula (II) in which Ar is a single bond, A is a methylene group and $R^1$ is -CH=CH-$G^3$-$SO_2$-Z,
$R^a$ is a hydrogen atom, $R^b$ and $R^c$, when taken together with the carbon atom in 3-position to which they are bound, are -(C=O), and the dashed line together with the solid line is a single bond; compound (300) represented by the general formula (I) in which $X^1$ is a hydrogen atom and $X^2$ is a group of α configuration that is represented by the general formula (II) in which Ar is a single bond, A is a methylene group and $R^1$ is -$CH_2$-$CH_2$-$G^3$-SO-Z, $R^a$ is a hydrogen atom, $R^b$ and $R^c$, when taken together with the carbon atom in 3-position to which they are bound, are -(C=O)-, and the dashed line together with the solid line is a single bond; and compound (301) represented by the general formula (I) in which $X^1$ is a hydrogen atom and $X^2$ is a group of α configuration that is represented by the general formula (II) in which Ar is a single bond, A is a methylene group and $R^1$ is -$CH_2$-$CH_2$-$G^3$-$SO_2$-Z, $R^a$ is a hydrogen atom, $R^b$ and $R^c$, when taken together with the carbon atom in 3-position to which they are bound, are -(C=O)-, and the dashed line together with the solid line is a single bond.

Process V

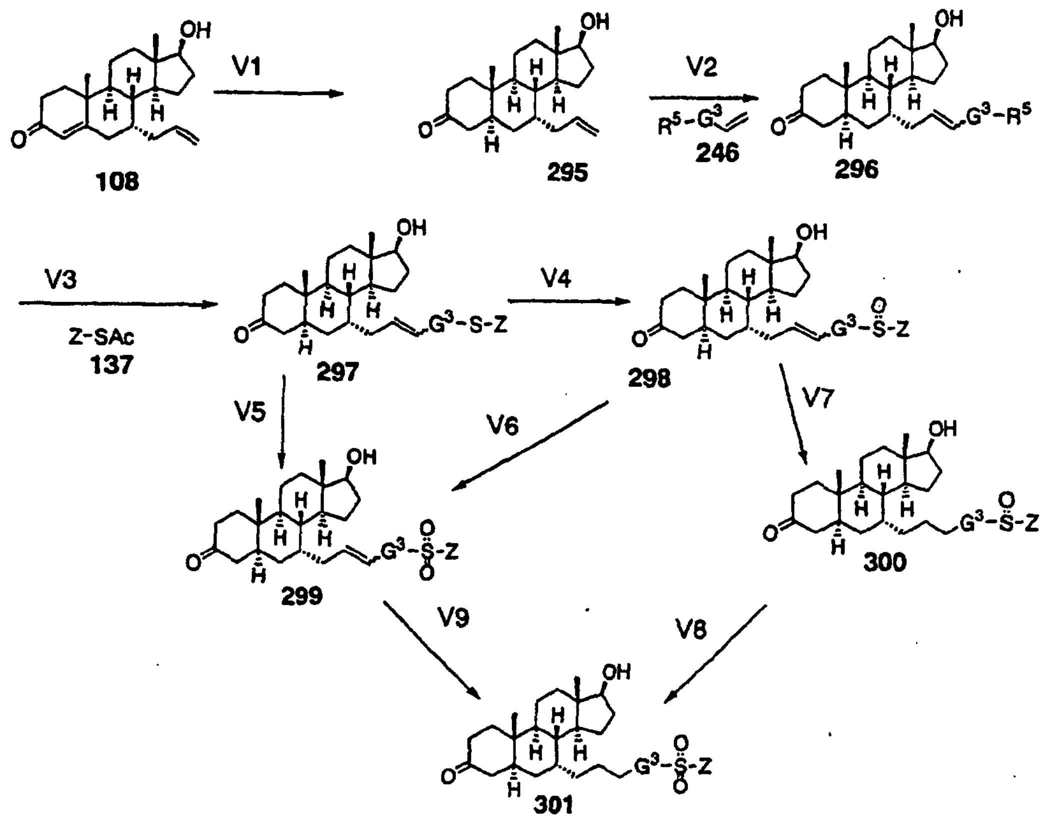


Step V1 is for producing compound (295) and implemented by reacting compound (108) with a reducing agent in an inert solvent. The reaction is performed as in the aforementioned step U"6 in process U".

Step V2 is for producing compound (296) and implemented by reacting compound (246) with compound (295) in an inert solvent in the presence of an organometallic catalyst. The reaction is performed as in the aforementined step A4 in process A.

Step V3 is for producing compound (297) and implemented by reacting compound (137) with a metal alkoxide in an alcoholic solvent to make a reactive derivative of compound (137) and reacting it with compound (296) in an alcoholic solvent. The reaction is performed as in the aforementioned step B4 in process B.

Step V4 is for producing compound (298) and implemented by reacting compound (297) with an oxidizing agent in an inert solvent. The reaction is performed as in the aforementioned step A8 in process A.

Step V5 is for producing compound (299) and implemented by reacting compound (297) with an oxidizing agent in an inert solvent. The reaction is performed as in the aforementioned step A9 in process A.

Step V6 is an alternative method for producing compound (299) and implemented by reacting compound (298) with an oxidizing agent in an inert solvent. The reaction is performed as in the aforementioned step A9 in process A.

Step V7 is for producing compound (300) and implemented by performing catalytic reduction in an alcoholic solvent or an inert solvent. The reaction is performed as in the aforementioned step A6 in process A.

Step V8 is an alternative method for producing compound (301) and implemented by reacting compound (300) with an oxidizing agent in an inert solvent. The reaction is performed as in the aforementioned step A9 in process A.

Step V9 is an alternative method of forming compound (301) and implemented by performing catalytic reduction in an alcoholic solvent or an inert solvent. The reaction is performed as in the aforementioned step A6 in process A.

In process V, compound (300) can be obtained from compound (296) if the sequence of rection steps is V7 → V3 → V4. Compound (301) can also be obtained from compound (300) if the sequence of reaction steps is V7 → V3 → V5.

Process V' is for producing compound (302) represented by the general formula (I) in which $X^1$ is a hydrogen

atom, $X^2$ is a group of α configuration that is represented by the general formula (II) in which Ar is a single bond, A is a methylene group and $R^1$ is $-CH=CH-CH_2-R^2$, $R^a$ is a hydrogen atom, $R^b$ and $R^c$, when taken together with the carbon atom in 3-position to which they are bound, are -(C=O)-, and the dashed line together with the solid line is a single bond; compound (303) represented by the general formula (I) in which $X^1$ is a hydrogen atom, $X^2$ is a group of α configuration that is represented by the general formula (II) in which Ar is a single bond, A is a methylene group and $R^1$ is $-CH_2-CH_2-CH_2-R^2$, $R^a$ is a hydrogen atom, $R^b$ and $R^c$, when taken together with the carbon atom in 3-position to which they are bound, are -(C=O), and the dashed line together with the solid line is a single bond; compound (304) represented by the general formula (I) in which $X^1$ is a hydrogen atom, $X^2$ is a group of α configuration that is represented by the general formula (II) in which Ar is a single bond, A is a methylene group and $R^1$ is $-CH_2-CH_2-CH_2-G^2-COOH$, $R^a$ is a hydrogen atom, $R^b$ and $R^c$, when taken together with the carbon atom in 3-position to which they are bound, are -(C=O), and the dashed line together with the solid line is a single bond; and compound (305) represented by the general formula (I) in which $X^1$ is a hydrogen atom and $X^2$ is a group of α configuration that is represented by the general formula (II) in which Ar is a single bond, A is a methylene group and $R^1$ is $-CH_2-CH_2-CH_2-G^2-CON(R^7)Z$, $R^a$ is a hydrogen atom, $R^b$ and $R^c$, when taken together with the carbon atom in 3-position to which they are bound, are -(C=O)-, and the dashed line together with the solid line is a single bond.

## Process V'

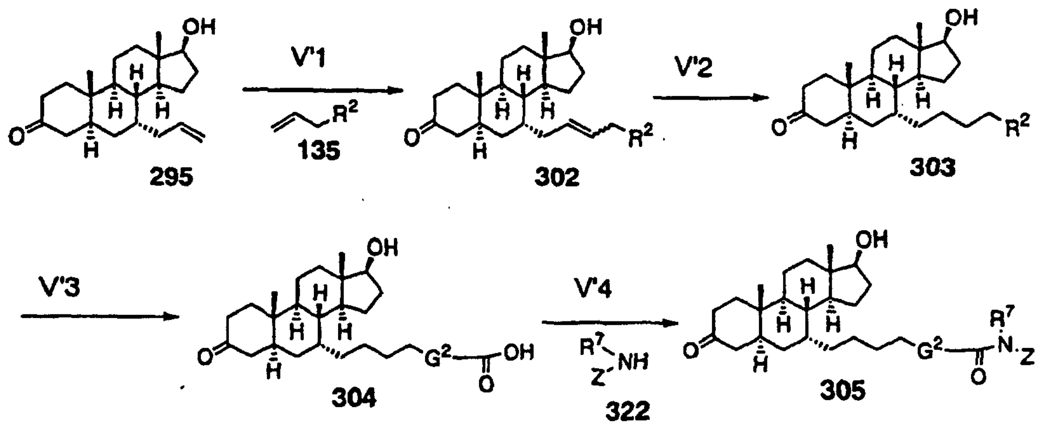

295, 135, 302, 303, 304, 322, 305

Step V'1 is for producing compound (302) and implemented by reacting compound (135) with compound (295) in an inert solvent in the presence of an organometallic catalyst. The reaction is performed as in the aforementioned step A4 in process A.

Step V'2 is for producing compound (303) and implemented by performing catalytic reduction in an alcoholic solvent or an inert solvent. The reaction is performed as in the aforementioned step A6 in process A.

Step V'3 is for producing compound (304) in the case where $R^2$ in compound (303) is $G^2-COOR^7$ and this is implemented by hydrolyzing compound (303) in water or a water-soluble solvent in the presence of a base or an acid (preferably a base). The reaction is performed as in the aforementioned step O6 in process O.

Step V'4 is for producing compound (305) and implemented by reacting compound (304) or reactive derivatives thereof (acid halides, mixed acid anhydrides or active esters) with compound (322) or acid addition salts thereof in an inert solvent. The reaction is performed as in the aforementioned step C3 in process C.

Process W is for producing compound (308) represented by the general formula (I) in which $X^1$ is a hydrogen atom, $X^2$ is a group of α configuration that is represented by the general formula (II) in which Ar is an aromatic hydrocarbon group (preferably a p-phenylene group), A is -O- and $R^1$ is a methyl group, $R^a$ is a hydrogen atom, $R^b$ and $R^c$, when taken together with the carbon atom in 3-position to which they are bound, are -(C=O)-, and the dashed line together with the solid line is a double bond; compound (309) represented by the general formula (I) in which $X^1$ is a hydrogen atom, $X^2$ is a group of α configuration that is represented by the general formula (II) in which Ar is an aromatic hydrocarbon group (preferably a p-phenylene group), A is -O- and $R^1$ is a methyl group, $R^a$ is a hydrogen atom, $R^b$ and $R^c$, when taken together with the carbon atom in 3-position to which they are bound, are -(C=O), and the dashed line together with the solid line is a single bond; compound (311) represented by the general formula (I) in which $X^1$ is a hydrogen atom, $X^2$ is a group of α configuration that is represented by the general formula (II) in which Ar is an aromatic hydrocarbon grouop (preferably a p-phenylene group), A is -O- and $R^1$ is an allyl group, $R^a$ is a hydrogen

atom, $R^b$ and $R^c$, when taken together with the carbon atom in 3-position to which they are bound, are -(C=O), and the dashed line together with the solid line is a single bond; compound (312) represented by the general formula (I) in which $X^1$ is a hydrogen atom, $X^2$ is a group of $\alpha$ configuration represented by the general formula (II) in which Ar is an aromatic hydrocarbon group (preferably a p-phenylene group), A is -O-and $R^1$ is $-CH_2-CH=CH-CH_2-R^2$, $R^a$ is a hydrogen atom, $R^b$ and $R^c$, when taken together with the carbon atom in 3-position to which they are bound, are -(C=O), and the dashed line together with the solid line is a single bond; compound (313) represented by the general formula (I) in which $X^1$ is a hydrogen atom and $X^2$ is a group of $\alpha$ configuration that is represented by the general formula (II) in which Ar is an aromatic hydrocarbon group, A is -O- and $R^1$ is $-CH_2-CH_2-CH_2-CH_2-R^2$, $R^a$ is a hydrogen atom, $R^b$ and $R^c$, when taken together with the carbon atom in 3-position to which they are bound, are -(C=O)-, and the dashed line together with the solid line is a single bond; compound (314) represented by the general formula (I) in which $X^1$ is a hydrogen atom, $X^2$ is a group of $\alpha$ configuration represented by the general formula (II) in which Ar is an aromatic hydrocarbon group (preferably a p-phenylene group), A is -O- and $R^1$ is $-CH_2-CH_2-CH_2-CH_2-G^2-SO-Z$, $R^a$ is a hydrogen atom, $R^b$ and $R^c$, when taken together with the carbon atom in 3-position to which they are bound, are -(C=O), and the dashed line together with the solid line is a single bond; compound (315) represented by the general formula (I) in which $X^1$ is a hydrogen atom, $X^2$ is a group of $\alpha$ configuration represented by the general formula (II) in which Ar is an aromatic hydrocarbon group (preferably a p-phenylene group), A is -O- and $R^1$ is $-CH_2-CH_2-CH_2-CH_2-G^2-SO_2-Z$, $R^a$ is a hydrogen atom, $R^b$ and $R^c$, when taken together with the carbon atom in 3-position to which they are bound, are -(C=O), and the dashed line together with the solid line is a single bond; compound (316) represented by the general formula (I) in which $X^1$ is a hydrogen atom, $X^2$ is a group of $\alpha$ configuration represented by the general formula (II) in which Ar is an aromatic hydrocarbon group (preferably a p-phenylene group), A is -O- and $R^1$ is $-CH_2-CH_2-CH_2-CH_2-G^2-COOH$, $R^a$ is a hydrogen atom, $R^b$ and $R^c$, when taken together with the carbon atom in 3-position to which they are bound, are -(C=O), and the dashed line together with the solid line is a single bond; and compound (317) represented by the general formula (I) in which $X^1$ is a hydrogen atom and $X^2$ is a group of $\alpha$ configuration that is represented by the general formula (II) in which Ar is an aromatic hydrocarbon group (preferably a p-phenylene group), A is -O- and $R^1$ is $-CH_2-CH_2-CH_2-CH_2-G^2-CON(R^7)Z$, $R^a$ is a hydrogen atom, $R^b$ and $R^c$, when taken together with the carbon atom in 3-position to which they are bound, are -(C=O)-, and the dashed line together with the solid line is a single bond.

Process W

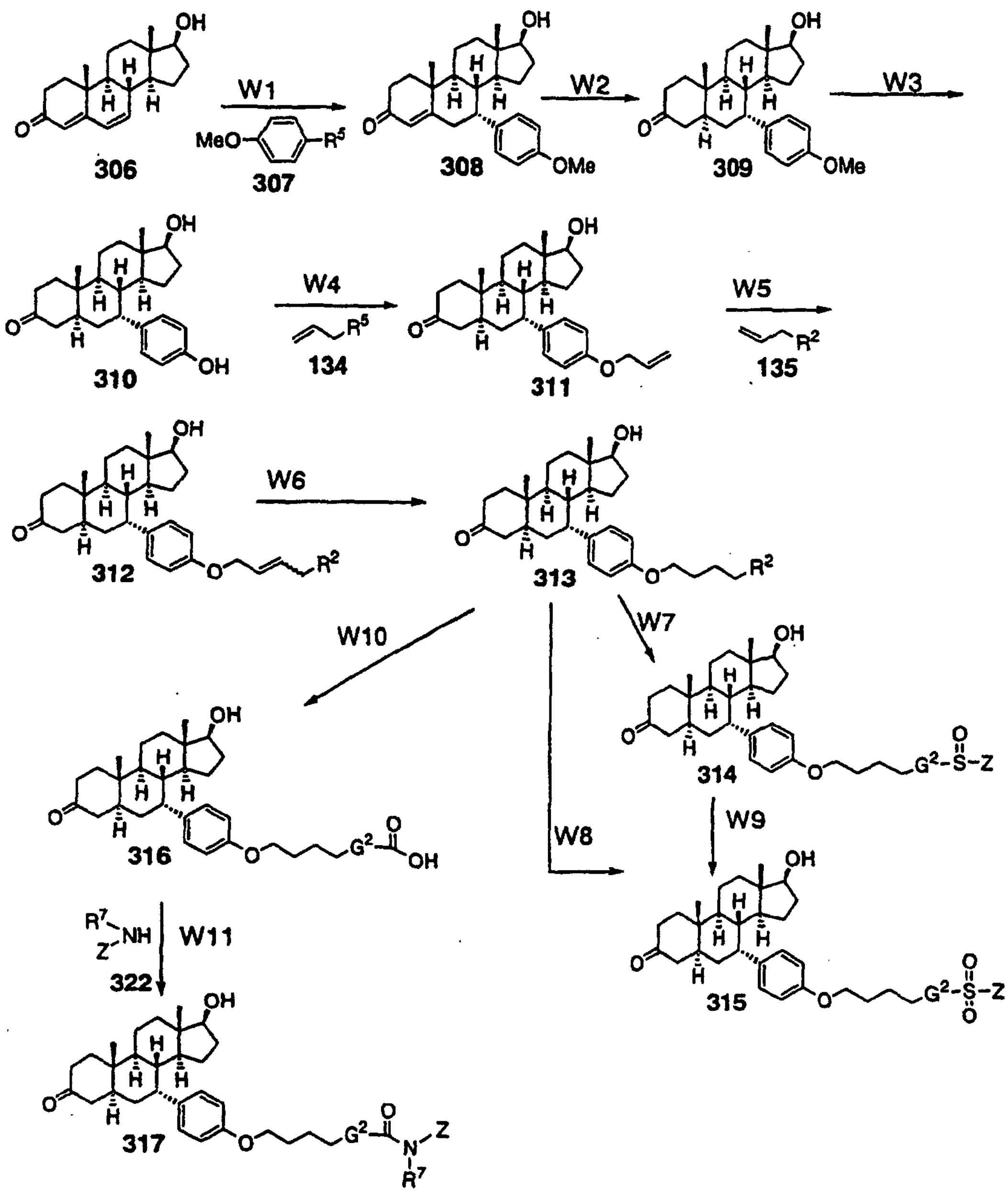


Step W1 is for producing compound (308) and implemented by reacting compound (307) with a metal (preferably magnesium) or an alkyllithium (preferably t-butyllithium) in an inert solvent to make a reactive derivative of compound (307) and reacting it with compound (306) in an inert solvent in the presence of an additive (preferably tetrakis[(tri-n-butylphosphine)copper(I) iodide]). The reaction is performed as in the aforementioned step O1 in process 0.

Step W2 is for producing compound (309) and implemented by reacting compound (308) with a reducing agent in an inert solvent. The reaction is performed as in the aforementioned step U"6 in process U".

Step W3 is for producing compound (310) and implemented by reacting compound (309) with a deprotecting agent in an inert solvent. The reaction is performed as in the aforementioned step U7 in process U.

Step W4 is for producing compound (311) and implemented by reacting compound (310) with a base in an inert solvent to make a salt of compound (310) and then reacting it with compound (134) in an inert solvent. The reaction is performed as in the aforementined step A3 in process A.

Step W5 is for producing compound (312) and implemented by reacting compound (135) with compound

(311) in an inert solvent in the presence of an organometallic catalyst. The reaction is performed as in the aforementioned step A4 in process A.

Step W6 is for producing compound (313) and implemented by performing catalytic reduction in an alcoholic solvent or an inert solvent. The reaction is performed as in the aforementioned step A6 in process A.

Step W7 is for producing compound (314) in the case where $R^2$ in compound (313) is $G^2$-S-Z and this is implemented by reacting compound (313) with an oxidizing agent in an inert solvent. The reaction is performed as in the aforementioned step A8 in process A.

Step W8 is for producing compound (315) in the case where $R^2$ in compound (313) is $G^2$-S-Z and this is implemented by reacting compound (313) with an oxidizing agent in an inert solvent. The reaction is performed as in the aforementioned step A9 in process A.

Step W10 is for producing compound (316) in the case where $R^2$ in compound (313) is $G^2$-$COOR^7$ and this is implemented by hydrolyzing compound (313) with a base or an acid (preferably a base) in water or a water-soluble solvent. The reaction is performed as in the aforementioned step O6 in process O.

Step W11 is for producing compound (317) and implemented by reacting compound (316) or reactive derivatives thereof (acid halides, mixed acid anhydrides or active esters) with compound (322) or acid addition salts thereof in an inert solvent. The reaction is performed as in the aforementioned step C3 in process C.

Process W' is for producing compound (319) represented by the general formula (I) in which $X^1$ is a hydrogen atom, $X^2$ is a group of $\alpha$ configuration that is represented by the general formula (II) in which Ar is an aromatic hydrocarbon group (preferably a p-phenylene group), A is -O- and $R^1$ is -$G^4$-$COOR^7$, $R^a$ is a hydrogen atom, $R^b$ and $R^c$, when taken together with the carbon atom in 3-position to which they are bound, are -(C=O), and the dashed line together with the solid line is a single bond; compound (320) represented by the general formula (I) in which $X^1$ is a hydrogen atom, $X^2$ is a group of $\alpha$ configuration that is represented by the general formula (II) in which Ar is an aromatic hydrocarbon group (preferably a p-phenylene group), A is -O- and $R^1$ is -$G^4$-COOH, $R^a$ is a hydrogen atom, $R^b$ and $R^c$, when taken together with the carbon atom in 3-position to which they are bound, are -(C=O), and the dashed line together with the solid line is a single bond; and compound (321) represented by the general formula (I) in which $X^1$ is a hydrogen atom and $X^2$ is a group of $\alpha$ configuration that is represented by the general formula (II) in which Ar is an aromatic hydrocarbon group (preferably a p-phenylene group), A is -O- and $R^1$ is -$G^4$-CON($R^7$)Z, $R^a$ is a hydrogen atom, $R^b$ and $R^c$, when taken together with the carbon atom in 3-position to which they are bound, are -(C=O)-, and the dashed line together with the solid line is a single bond.

Process W'

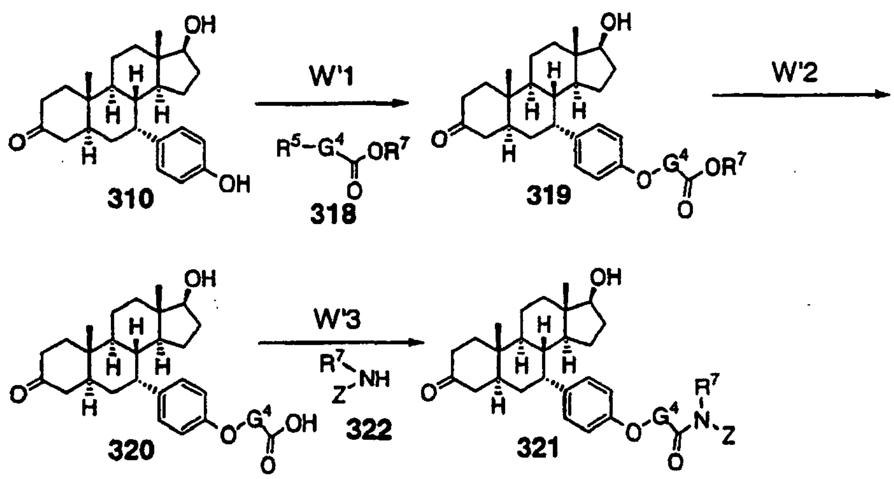


Step W'1 is for producing compound (319) and implemented by reacting compound (310) with a base in an inert solvent to make a salt of compound (310) and then reacting it with compound (318) in an inert solvent. The reaction is performed as in the aforementined step A3 in process A.

Step W'2 is for producing compound (320) and implemented by hydrolyzing compound (319) with a base or an acid (preferably a base) in water or a water-soluble solvent. The reaction is performed as in the aforementioned step O6 in process O.

Step W'3 is for producing compound (321) and implemented by reacting compound (320) or reactive derivatives thereof (acid halides, mixed acid anhydrides or active esters) with compound (322) or acid addition salts thereof

in an inert solvent. The reaction is performed as in the aforementioned step C3 in process C.

Process W" is for producing compound (323) represented by the general formula (I) in which $X^2$ is a hydrogen atom, $X^1$ is a group of β configuration that is represented by the general formula (II) in which Ar is an aromatic hydrocarbon group (preferably a p-phenylene group), A is -O- and $R^1$ is -$G^4$-$COOR^7$, $R^b$ and $R^c$, when taken together with the carbon atom in 3-position to which they are bound, are -(C=O)-, and the dashed line together with the solid line is a single bond or a double bond; compound (324) represented by the general formula (I) in which $X^2$ is a hydrogen atom, $X^1$ is a group of β configuration that is represented by the general formula (II) in which Ar is an aromatic hydrocarbon group (preferably a p-phenylene group), A is -O- and $R^1$ is -$G^4$-$COOR^7$, $R^a$ is a hydrogen atom, $R^b$ and $R^c$, when taken together with the carbon atom in 3-position to which they are bound, are -(C=O), and the dashed line together with the solid line is a single bond or a double bond; and compound (325) represented by the general formula (I) in which $X^2$ is a hydrogen atom and $X^1$ is a group of β configuration that is represented by the general formula (II) in which Ar is an aromatic hydrocarbon group (preferably a p-phenylene group), A is -O- and $R^1$ is -$G^4$-CON($R^7$)Z, $R^a$ is a hydrogen atom, $R^b$ and $R^c$, when taken together with the carbon atom in 3-position to which they are bound, are -(C=O)-, and the dashed line together with the solid line is a single bond or a double bond.

## Process W"

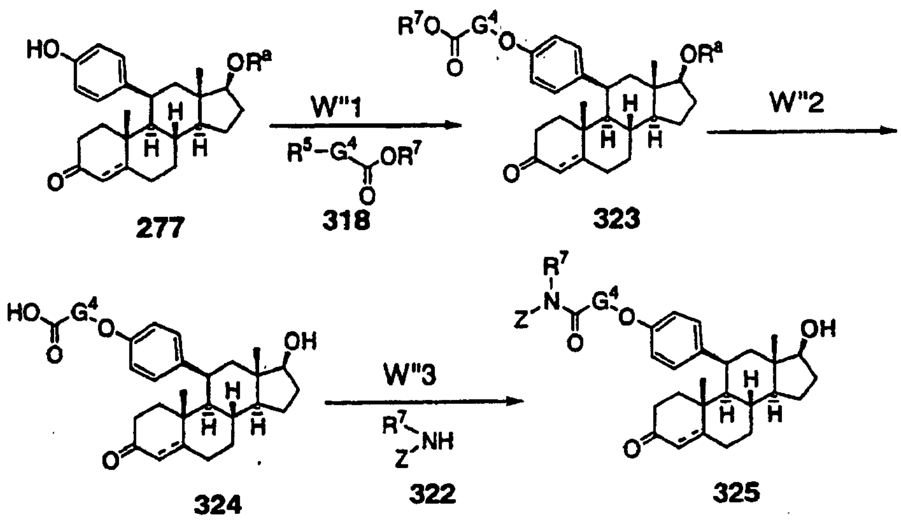


277 318 323

324 322 325

Step W"1 is for producing compound (323) and implemented by reacting compound (277) with a base in an inert solvent to make a salt of compound (277) and then reacting it with compound (318) in an inert solvent. The reaction is performed as in the aforementined step A3 in process A.

Step W"2 is for producing compound (324) and implemented by reacting compound (323) with an acid in an aqueous solvent. The reaction is performed as in the aforementioned step A5 in process A.

Step W"3 is for producing compound (325) and implemented by reacting compound (324) or reactive derivatives thereof (acid halides, mixed acid anhydrides or active esters) with compound (322) or acid addition salts thereof in an inert solvent. The reaction is performed as in the aforementioned step C3 in process C.

Process W'" is for producing compound (327) represented by the general formula (I) in which $X^1$ is a hydrogen atom, $X^2$ is a group of a configuration represented by the general formula (II) in which Ar is an aromatic hydrocarbon group (preferably a p-phenylene group), A is -O-and $R^1$ is -$CH_2$-CH=CH-$G^3$-$R^5$, $R^a$ is a hydrogen atom, $R^b$ and $R^c$, when taken together with the carbon atom in 3-position to which they are bound, are -(C=O), and the dashed line together with the solid line is a single bond; compound (328) represented by the general formula (I) in which $X^1$ is a hydrogen atom and $X^2$ is a group of α configuration that is represented by the general formula (II) in which Ar is an aromatic hydrocarbon group, A is -O- and $R^1$ is -$CH_2$-CH=CH-$G^3$-S-Z, $R^a$ is a hydrogen atom, $R^b$ and $R^c$, when taken together with the carbon atom in 3-position to which they are bound, are -(C=O)-, and the dashed line together with the solid line is a single bond; compound (329) represented by the general formula (I) in which $X^1$ is a hydrogen atom, $X^2$ is a group of α configuration represented by the general formula (II) in which Ar is an aromatic hydrocarbon group (preferably a p-phenylene group), A is -O- and $R^1$ is -$CH_2$-CH=CH-$G^3$-SO-Z, $R^a$ is a hydrogen atom, $R^b$ and $R^c$, when taken together with the carbon atom in 3-position to which they are bound, are -(C=O), and the dashed line together with the solid line is a single bond; compound (330) represented by the general formula (I) in which $X^1$ is a hydrogen

atom, $X^2$ is a group of α configuration represented by the general formula (II) in which Ar is an aromatic hydrocarbon group (preferably a p-phenylene group), A is -O- and $R^1$ is $-CH_2-CH_2-CH_2-G^3-SO-Z$, $R^a$ is a hydrogen atom, $R^b$ and $R^c$, when taken together with the carbon atom in 3-position to which they are bound, are -(C=O), and the dashed line together with the solid line is a single bond; and compound (331) represented by the general formula (I) in which $X^1$ is a hydrogen atom and $X^2$ is a group of α configuration that is represented by the general formula (II) in which Ar is an aromatic hydrocarbon group (preferably a p-phenylene group), A is -O- and $R^1$ is $-CH_2-CH_2-CH_2-G^3-SO_2-Z$, $R^a$ is a hydrogen atom, $R^b$ and $R^c$, when taken together with the carbon atom in 3-position to which they are bound, are -(C=O)-, and the dashed line together with the solid line is a single bond.

## Process W'"

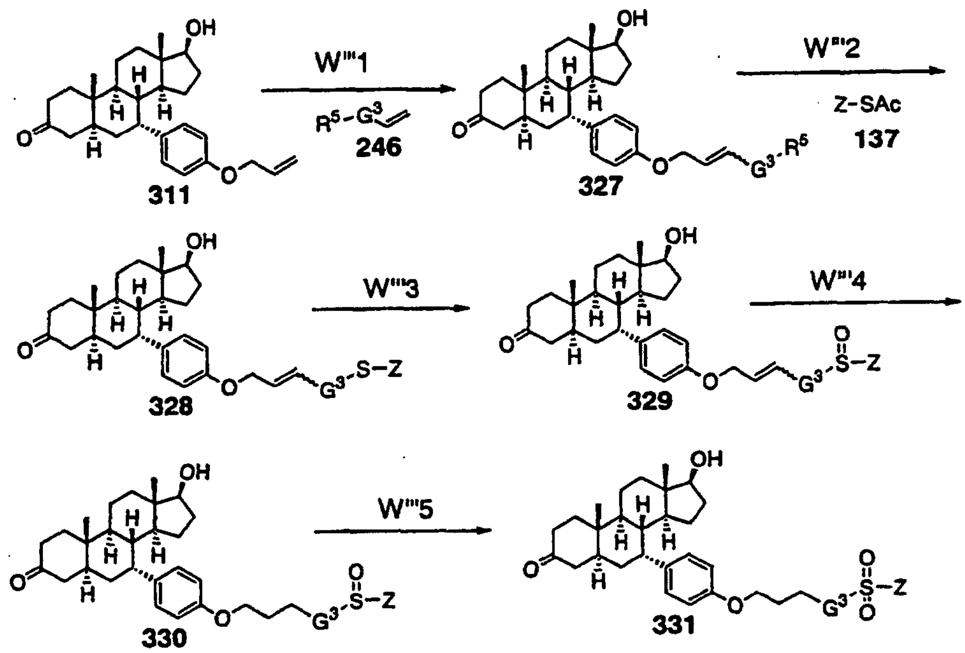

311
327
328
329
330
331

Step W'''1 is for producing compound (327) and implemented by reacting compound (246) with compound (311) in an inert solvent in the presence of an organometallic catalyst. The reaction is performed as in the aforementioned step A4 in process A.

Step W'''2 is for producing compound (328) and implemented by reacting compound (137) with a metal alkoxide in an alcoholic solvent to make a reactive derivative of compound (137) and then reacting it with compound (327) in an alcoholic solvent. The reaction is performed as in the aforementioned step B4 in process B.

Step W'''3 is for producing compound (329) and implemented by reacting compound (328) with an oxidizing agent in an inert solvent. The reaction is performed as in the aforementioned step A8 in process A.

Step W'''4 is for producing compound (330) and implemented by performing catalytic reduction in an alcoholic solvent or an inert solvent. The reaction is performed as in the aforementioned step A6 in process A.

Step W'''5 is an alternative method of producing compound (331) and implemented by reacting compound (330) with an oxidizing agent in an inert solvent. The reaction is performed as in the aforementioned step A9 in process A.

In process W''', compound (330) can also be obtained from compound if the sequence of reaction steps is W'''4 → W'''2 → W'''3. Compound (331) can also be obtained from compound (327) if the sequence of reaction steps is W'''4 → W'''2 → W'''5. Compound (331) can also be obtained from compound (329) if the sequence of reaction steps is W'''5 → W'''4.

In the above-described processes A - W, B'- L', S' - W', U", W" and W''', if G and/or J and/or $Q^2$ is a group containing a carboxyl group protected by a straight-chained or branched lower alkyl group having 1 - 6 carbon atoms, deprotection can easily be achieved by any known methods of hydrolysis to effect conversion to a carboxyl-containing group.

If any of the steps in the above-described processes A - W, B' - L', S' - W', U", W" and W''' involves groups that need be protected and deprotecetd, each of them can be protected and deprotected by methods well known to

the skilled artisan. For the purposes of protecting and deprotecting, reference can be had, for example, to "Protective Groups in Organic Synthesis", 2nd edition, Theodora W. Green, John Wiley & Sons, Inc., 1991.

Starting material compound (1) is either known or can be easily prepared by known methods or similar methods. [See, for example, J. Med. Chem. 35(11), 2113-2129 (1992); Synth. Commun. 24(16), 2325-2340 (1994); Steroids, 60(5), 414-422 (1995).]

Starting material compound (108) is either known or can be easily prepared by known methods or similar methods. [See, for example, Tetrahedron Letters, 29(13), 1533-1536 (1988).]

Starting material compound (96) is either readily available as a commercial product or can be easily prepared by known methods or similar methods. [See, for example, J. Chem. Res. Miniprint, 2, 0650-0669 (1986).]

Starting material compounds (119) and (144) are readily available as commercial products.

Starting material compounds (133) - (143), compound (183), compound (204), compounds (218) - (220), compound (224), compound (236) and compound (257) are either readily available as commercial products or can be easily prepared by known methods or similar methods.

Starting material compounds (148) and (164) are either known or can be easily prepared by known methods or similar methods. [See, for example, Steroids, 59, 190-195 (1994).]

Starting material compound (223) is either known or can be easily prepared by known methods or similar methods. [See, for example, Synth. Commun. 27(23), 4035-4040 (1997).]

The compounds of the invention which are represented by the general formula (I) and the substances which act as antagonist against but not as agonist for the androgen receptor (which are hereunder also referred to as the test substance) have antiandrogenic activity and other effects and these effects can be measured by the androgen receptor reporter gene assay which has been used in defining the expression "acting as antagonist" and/or the expression "not acting as agonist" for the purposes of the invention as it is optionally combined where appropriate with the following methods of measurement A - F.

Method A: Measurement by in vivo experiment with rats Method A-1: Measuring the antagonist action

If a castrated rat is administered testosterone or dihydrotestosterone, its prostate gland and seminal vesicles increase in weight. By checking to see if the test substance suppresses the action of testosterone or dihydrotestosterone for increasing the weights of prostate gland and seminal vesicles, one can evaluate the antagonist action of the test substance. For this measurement, reference can be had, for example, to J. Med. Chem., 41:623-639, 1998, and Kiso to Rinsho, 29(4):877-885, 1995.

Method A-2: Measuring the agonist action

A castrated rat is continuously administered the test substance. By checking to see if the weights of the prostate gland and seminal vesicles which are androgen-responsive organs increase after the administration, one can evaluate the agonist action of the test substance. For this measurement, reference can be had, for example, Folia endocrinol., 66:597-606, 1990.

Method B: Measurement based on dimer formation of the androgen receptor

Method B-1: Measuring the action for inhibiting dimer formation

Dihydrotestosterone helps the androgen receptor form a dimer. By applying a gel shift assay to determine if the test substance inhibits the dimer formation of the androgen receptor, one can evaluate the antagonist action of the test substance. For this measurement, reference can be had, for example, to J. Biol. Chem., 268:19004-19012, 1993 and J. Biol. Chem., 270:19998-20003, 1995.

Method B-2: Measuring the action for promoting dimer formation of the androgen receptor

By applying a gel shift assay to determine if the test substance promotes the dimer formation of the androgen receptor, one can evaluate the agonist action of the test substance. For this measurement, reference can be had, for example, to J. Biol. Chem., 268:19004-19012, 1993 and J. Biol. Chem., 270:19998-20003, 1995.

Method C: Measurement based on ornithine decarboxylase (ODC) activity

By determining whether the test substance elevates or lowers the ODC activity which is believed to reflect androgen-dependent activity, one can evaluate the agonist and antagonist actions of the test substance. For this measurement, reference can be had, for example, to Anal. Biochem., 113-352-355, 1981 and Folia endocrinol., 66:597-606, 1990.

Method D: Measurement based on androgen receptor binding activity

By applying a binding assay to determine whether the test substance inhibits the binding of the androgen receptor to androgen, one can evaluate the antagonist action of the test substance. For this measurement, reference can be had, for example, to Urology, 48:157-163, 1996, J. Biol. Chem., 270:19998-20003, 1995 and Kiso to Rinsho, 29(4):877-885, 1995.

Method E: Measurement based on the increase or decrease in the amount of the androgen receptor

Cells exprerssing the androgen receptor are treated with the test substance in both the presence and the absence of androgen. By measuring the change in the amount of the androgen receptor in the cells, one can evaluate the action of the test substance in working as agonist for or antagonist against the androgen receptor. For this measurement, reference can be had, for example, to Endrocrinology, 129:2000-2010, 1991.

Method F: Measurement based on nuclear migration of the androgen receptor

Cells exprerssing the androgen receptor are treated with the test substance in the presence or absence of androgen. By applying immunohistostaining to determine the localization of the androgen receptor in the cells, one can check for the nuclear migration of the androgen receptor and determine the action of the test substance for inhibiting the nuclear migration of the androgen receptor, thereby evaluating the action of the test substance as agonist and/or antagonist. For these measurements, reference can be had, for example, to J. Biol. Chem., 267:968-974, 1992.

The compounds of the invention which are represented by the general formula (I) and the substances of the invention which act as antagonist against but not as agonist for the androgen receptor are potential antiandrogenic agents that do exhibit any side effects such as the development of androgen tolerance due to long-term administration and/or hepatoxicity and, hence, are expected to be useful as pharmaceutical compositions, say, therapeutics for diseases such as prostate cancer, prostatomegaly, male pattern alopecia, sexual prematurity, acne vulgaris, seborrhea and hursutism. If the compounds of the invention which are represented by the general formula (I) and the substances of the invention which act as antagonist against but not as agonist for the androgen receptor are preliminarily administered, the onset of diseases such as prostate cancer, prostatomegaly, male pattern alopecia, sexual prematurity, acne vulgaris, seborrhea and hursutism can hopefully be prevented or retarded, so they are also potential preventives of these diseases.

Pharmaceutical compositions containing as an active ingredient the compounds of the invention which are represented by the general formula (I) and pharmaceutical compositions containing as an active ingredient the substances of the invention which act as antagonist against but not as agonist for the androgen receptor can be administered either orally or parenterally and oral administration is desirable. Prior to administration, such pharmaceutical compositions can be formulated as preparations suitable for the specific method of administration.

Pharmaceutical compositions containing as an active ingredient the compounds of the invention which are represented by the general formula (I) and pharmaceutical compositions containing as an active ingredient the substances of the invention which act as antagonist against but not as agonist for the androgen receptor can be formulated by customary pharmaceutical formulation techniques and, depending on their use, can be applied as solid and liquid preparations including tablets, capsules, granules, powder, syrup, injection and ointment. Carriers and excipients for such preparations include solid or liquid substances. These may be exemplified by lactose, magnesium stearate, starch, talc, gelatin, agar, pectin, gum arabic, olive oil, sesame oil, ethylene glycol and others in common use.

In these preparations, the pharmaceutical compositions containing as an active ingredient the compounds of the invention which are represented by the general formula (I) and the pharmaceutical compositions containing as an active ingredient the substances of the invention which act as antagonist against but not as agonist for the androgen receptor are incorporated in amounts that vary with their dosage form but it is generally desirable that they be contained at concentrations of 5 - 100 wt%. The pharmaceutical compositions containing as an active ingredient the compounds of the invention which are represented by the general formula (I) and pharmaceutical compositions containing as an active ingredient the substances of the invention which act as antagonist against but not as agonist for the androgen receptor can be adjusted over a broad range depending on the kind of warm-blooded animals including human to be treated, the severity of the disease, doctor's diagnosis, etc. In terms of the active ingredient, the range is from 1 μg to 500 mg/kg per day, preferably from 20 μg to 100 mg/kg per day. This dose can be administered once or several times in one or divided portions per day to month and is variable as appropriate according to the severity of the disease and at doctor's discretion.

Examples

Example 1: Evaluating the Agonist Action of Flutamide and Bicaltamide

Twenty-four hours before transfection, 1.0 x $10^5$ HeLa cells were cultured in phenol red free DMEM/5% DCC-FBS on 12-well microplates. Five hundred nanograms/well of MMTV-Luc vector, 100 ng/well of pSG5-hAR and 5 ng/well of Renilla Luc vector were transfected into the HeLa cells. The transfection was performed in a liquid culture of the phenol red free DMEM using 3 mL/well of lipofectoamine. Nine hours after the transfection, the liquid culture was replaced by phenol red free DMEM/3% DCC-FBS containing 10 mmol/L of hydroxyflutamide or bicaltamide. The transcriptional activity value was measured 48 hours after the replacement of the liquid culture. Transcriptional activity was measured with a dual-luciferase reporter assay system. The transcriptional activity value was calculated as the value for firefly luciferase divided by the value for sea pansy luciferase. Hydroxyflutamide and bicaltamide exhibited more than five times the value for the case of non-addition and, hence, the agonist action of hydroxyflutamide and bicaltamide was verified (Table 1).

<Table 1>

| Luciferase Activity (fold induction)[1)] | |
|---|---|
| Not added | 1.00 |
| 10 μmol/L of hydroxyflutamide | 7.84 (> 5.0) |
| 10 μmol/L of bicaltamide | 7.62 (> 5.0) |

1) The value with the luciferase activity value for "not added" being taken as 1.00.

Example 2: Evaluating the Antagonist Action of Flutamide and Bicaltamide

Twenty-four hours before transfection, 1.0 x $10^5$ HeLa cells were cultured in phenol red free DMEM/5% DCC-FBS on 12-well microplates. Five hundred nanograms/well of MMTV-Luc vector, 100 ng/well of pSG5/hAR and 5 ng/well of Renilla Luc vector were transfected into the HeLa cells. The transfection was performed in a liquid culture of the phenol red free DMEM using 3 mL/well of lipofectoamine. Nine hours after the transfection, the liquid culture was replaced by phenol red free DMEM/3% DCC-FBS containing 0.1 nmol/L of DHT and 1.0 mmol/L of hydroxyflutamide or bicaltamide. The transcriptional activity value was measured 48 hours after the replacement of the liquid culture. Transcriptional activity was measured with a dual-luciferase reporter assay system. The transcriptional activity value was calculated as the value for firefly luciferase divided by the value for sea pansy luciferase. Hydroxyflutamide and bicaltamide lowered the transcriptional activity value of DHT to less than 50% and, hence, the antagonist action of hydroxyflutamide and bicaltamide was verified (Table 2).

<Table 2>

| Luciferase Activity (relative activity)[2)] | |
|---|---|
| 0.1 nmol/L of DHT | 100 |
| 1.0 μmol/L of hydroxyflutamide | 29.0 (< 50.0) |
| 1.0 μmol/L of bicaltamide | 32.0 (< 50.0) |

2) The value with the luciferase activity value of 0.1 nmol/L of DHT being taken as 100.

Example 3: Synthesis of 17β-hydroxy-7α-(7-carboxyheptyl)-5α-androstan-3-one

(Step 1)

17β-t-butyldimethylsilyloxy-7α-(2-propen-1-yl)-5α-androstan-3-one

Metallic lithium (220 mg) was added to liquid ammonia (150 ml) at -78 °C. After 5-minute stirring, 17β-t-butyldimethylsilyloxy-7α-(2-propen-1-yl)-4-androsten-3-one (1.261 g) and a tetrahydrofuran solution (20 ml) of t-butanol (0.41 ml) were added and the mixture was stirred for 20 minutes. After adding 1,2-dibromoethane (3 ml) and ammonium chloride (30 g), the mixture was stirred at 25°C for 30 minutes. After adding water, extraction with ethyl acetate was conducted. The organic layer was dried with magnesium sulfate and, after filtering, the solvent was distilled off at reduced pressure. Purification by silica gel column chromatography (developing solvents: ethyl acetate/n-hexane = 1/10) gave the end compound in 810.7 mg (yield, 64%).

1H-NMR(270MHz, $CDCl_3$)δ: 0.01(6H, s), 0.73(3H, s), 0.88(9H, s), 1.04(3H, s), 0.92-2.45(23H, m), 3.55(1H, t, J=8.3Hz), 4.93(1H, d, J=3.8Hz), 4.99(1H, s), 5.58-5.72(1H, m).
Rf value (on silica gel plate, developing solvents: ethyl acetate/hexane = 1/10): 0.54

(Step 2)

17β-t-butyldimethylsilyloxy-7α-(7-methoxycarbonyl-2-hepten-1-yl)-5α-androstan-3-one

17β-t-Butyldimethylsilyloxy-7α-(2-propen-1-yl)-5α-androstan-3-one (596.1 mg) was dissolved in dichloromethane (5 ml) and, after adding methyl 6-heptenoate (384.4 mg) and benzylidenebis(tricyclohexylphosphine)-dichlororuthenium (57.0 mg), the mixture was heated under reflux for 5 hours in an argon atmosphere. After standing to cool, purification was effected by silica gel column chromatography (developing solvents: ethyl acetate/n-hexane = 1/10) to give the end compound in 527.6 mg (yield, 70%).
1H-NMR(270MHz, $CDCl_3$)δ: 0.01(6H, s), 0.71(3H, s), 0.88(9H, s), 1.03(3H, s), 0.90-2.10(26H, m), 2.18-2.43(5H, m), 3.51(1H, t, J=8.4Hz), 3.67(3H, s), 5.18-5.40(2H, m).
Rf value (on silica gel plate, developing solvents: ethyl acetate/n-hexane = 1/4): 0.43

(Step 3)

17β-hydroxy-7α-(7-carboxyheptyl)-5α-androstan-3-one

17β-t-Butyldimethylsilyloxy-7α-(7-methoxycarbonyl-2-hepten-1-yl)-5α-androstan-3-one (505.5 mg) was dissolved in ethyl acetate (30 ml) and, after adding 10%-palladium/carbon (148 mg), the mixture was stirred for 4 hours at 25°C in a hydrogen atmosphere. The reaction mixture was filtered and the solvent was distilled off at reduced pressure; the resulting residue was dissolved in acetone (10 ml) and, after adding 1 N-HCl (1 ml), the mixture was heated under reflux for 26 hours. After standing to cool, water was added and extraction with ethyl acetate was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 1/2 ~ 1/1) gave the end compound in 362.6 mg (yield, 93%).
1H-NMR(270MHz, $CDCl_3$)δ: 0.76(3H, s), 1.04(3H, s), 1.00-1.82(27H, m), 1.98-2.15(3H, m), 2.23-2.48(5H, m), 3.65 (1H, t, J=8.7Hz).
Mass(FAB): 433(M+1).
Rf value (on silica gel plate, developing solvents: ethyl acetate/n-hexane = 1/1): 0.27
The following compounds were synthesized by similar methods to Example 3.

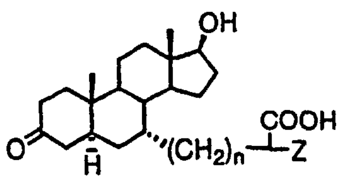

| Example | n | Z | MW (Molecular weight) | Mass |
|---|---|---|---|---|
| 4 | 4 | H | 404 | 405(FAB) |
| 5 | 8 | H | 460 | 461(FAB) |
| 6 | 10 | H | 488 | 489(FAB) |
| 7 | 12 | H | 516 | 517(FAB) |
| 8 | 8 | $-(CH_2)_3CF_2CF_3$ | 620 | 621(ESI) |

[Example 9]

Synthesis of 17β-hydroxy-7α-{7-(N,N-dimethylaminocarbonyl)-heptyl}-5α-androstan-3-one

The 17β-hydroxy-7α-(7-carboxyheptyl)-5α-androstan-3-one (9.9 mg) obtained in Example 3 was dissolved in tetrahydrofuran (0.5 ml) and, after adding 1-(N,N-dimethylaminopropyl)-3-ethylcarbodiimide hydrochloride (13.0 mg), 1-hydroxybenzotriazole monohydrate (10.5 mg) and a solution (68.7 μl) of 2.0 M-dimethylamine in tetrahydrofuran, the mixture was stirrerd for 15 hours at 25 °C. After adding ethyl acetate (2.0 ml), the mixture was washed with 1 N-hydrochloric acid, a saturated aqueous solution of sodium hydrogencarbonate and a saturated aqueous solution of sodium chloride. After drying with magnesium sulfate, the mixture was filtered through NH silica gel (Pro. No. DM1020; product of Fuji Silicia Chemical Co., Ltd.) and the solvent was distilled off at reduced pressure to give the end compound in 10.5 mg (99.7%).

1H-NMR(270MHz, $CDCl_3$)δ: 0.76(3H, s), 1.04(3H, s), 1.00-1.83(27H, m), 1.95-2.16(3H, m), 2.23-2.47(5H, m), 2.94 (3H, s), 3.01(3H, s), 3.65(1H, t, J=8.7Hz).

Mass(ESI): 460(M+1).

Rf value (on silica gel plate, developing solvents: methanol/chloroform = 1/1): 0.28

The following compounds were synthesized by similar methods to Example 9.

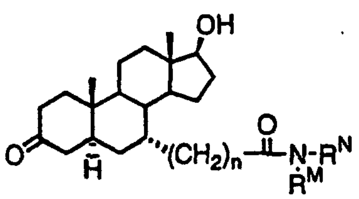

| Example | n | RM | RN | MW | Mass |
|---|---|---|---|---|---|
| 10 | 7 | H | Et | 459 | 460(FAB) |
| 11 | 7 | H | cyclohexylmethyl | 527 | 528(FAB) |
| 12 | 7 | H | cyclopropylmethyl | 485 | 486(FAB) |
| 13 | 7 | H | n-Bu | 487 | 488(ESI) |
| 14 | 7 | H | i-Pr | 473 | 474(FAB) |
| 15 | 7 | H | t-Bu | 487 | 488(FAB) |
| 16 | 7 | H | c-hexyl | 513 | 514(ESI) |
| 17 | 7 | H | $-(CH_2)_3OH$ | 489 | 490(ESI) |
| 18 | 7 | Me | n-Bu | 501 | 502(ESI) |
| 19 | 7 | Et | Et | 487 | 488(ESI) |
| 20 | 7 | $-(CH_2)_5-$ | | 499 | 500(ESI) |
| 21 | 7 | H | 4-t-butylbenzyl | 577 | 578(ESI) |
| 22 | 7 | H | $-CH_2CHPh_2$ | 611 | 612(ESI) |
| 23 | 7 | H | 2-furylmethyl | 511 | 512(ESI) |
| 24 | 7 | H | Me | 445 | 446(ESI) |
| 25 | 7 | Me | Et | 473 | 474(ESI) |
| 26 | 7 | Me | n-Pr | 487 | 488(ESI) |
| 27 | 7 | Me | i-Pr | 487 | 488(FAB) |
| 28 | 7 | Me | Bn | 535 | 536(ESI) |

(continued)

| Example | n | RM | RN | MW | Mass |
|---|---|---|---|---|---|
| 29 | 7 | $-(CH_2)_4-$ | | 485 | 486(ESI) |
| 30 | 7 | $-CH_2CH_2OCH_2CH_2-$ | | 501 | 502(ESI) |
| 31 | 7 | Me | t-Bu | 501 | 502(ESI) |
| 32 | 7 | H | cyclopropyl | 471 | 472(ESI) |
| 33 | 6 | Me | Me | 445 | 446(FAB) |
| 34 | 6 | Et | Et | 473 | 474(FAB) |
| 35 | 6 | $-(CH_2)_5-$ | | 485 | 486(FAB) |
| 36 | 8 | Me | Me | 473 | 474(ESI) |
| 37 | 8 | Et | Et | 501 | 524(ESI) |
| 38 | 8 | Me | n-Bu | 515 | 538(ESI) |
| 39 | 8 | H | Bn | 535 | 558(ESI) |
| 40 | 8 | H | $-(CH_2)_2OH$ | 489 | 512(ESI) |
| 41 | 8 | $-(CH_2)_5-$ | | 513 | 514(ESI) |
| 42 | 9 | Me | Me | 487 | 488(ESI) |
| 43 | 9 | Et | Et | 515 | 516(ESI) |
| 44 | 9 | $-(CH_2)_4-$ | | 513 | 514(ESI) |
| 45 | 9 | Me | Et | 501 | 502(ESI) |
| 46 | 9 | Me | n-Bu | 529 | 530(ESI) |
| 47 | 9 | H | Bn | 549 | 550(ESI) |
| 48 | 9 | $-(CH_2)_5-$ | | 527 | 528(ESI) |
| 49 | 9 | H | $-(CH_2)_2OH$ | 503 | 504(ESI) |
| 50 | 9 | Me | n-Pr | 515 | 516(ESI) |
| 51 | 9 | $-CH_2CH_2OCH_2CH_2-$ | | 529 | 530(ESI) |
| 52 | 10 | Me | Me | 501 | 502(FAB) |
| 53 | 10 | Et | Et | 529 | 530(FAB) |
| 54 | 10 | Me | Et | 515 | 516(FAB) |
| 55 | 10 | Me | n-Pr | 529 | 530(FAB) |
| 56 | 10 | Me | n-Bu | 543 | 544(FAB) |
| 57 | 10 | $-CH_2CH_2OCH_2CH_2-$ | | 543 | 544(FAB) |
| 58 | 11 | Me | Me | 515 | 516(FAB) |
| 59 | 11 | Et | Et | 543 | 544(FAB) |
| 60 | 11 | $-(CH_2)_5-$ | | 555 | 556(FAB) |
| 61 | 11 | H | Bn | 577 | 578(FAB) |
| 62 | 11 | Me | n-Bu | 557 | 558(FAB) |
| 63 | 11 | H | $-(CH_2)_2OH$ | 531 | 532(FAB) |

[Example 64]

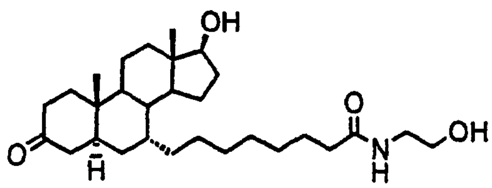

Synthesis of 17β-hydroxy-7α-[7-{N-(2-hydroxyethyl]-aminocarbonyl}heptyl]-5α-androstan-3-one

The 17β-hydroxy-7α-(7-carboxyheptyl)-5α-androstan-3-one (10.3 mg) obtained in Example 3 and O-(7-azabenzotriazol-1-yl)-1,1,3,3-tetramethyluromium hexafluorophosphate (30 mg) were dissolved in tetrahydrofuran (1 ml) and, after adding N,N-diisopropylethylamine (25 µl) and 2-aminoethanol (4.4 µl), the mixture was stirred for 2 hours at 25°C. After adding ethyl acetate, the reaction mixture was washed with a saturated aqueous solution of sodium hydrogencarbonate, 1 N-hydrochloric acid and a saturated aqueous solution of sodium chloride. To the organic layer, NH silica gel (Pro. No. DM1020; product of Fuji Silicia Chemical Co., Ltd.) was added and the mixture was stirred for 5 minutes; after filtering, the solvent was distilled off at reduced pressure. The resulting residue was purified by silica gel column chromatography (developing solvents: methanol/chloroform = 1/10) to give the end compound in 7.5 mg (yield, 66%).

1H-NMR(270MHz. $CDCl_3$)δ: 0.76(3H, s), 1.04(3H, s), 0.95-1.82(28H, m), 1.95-2.10(3H, m), 2.20(2H, t, J=7.4Hz), 2.28-2.45(2H, m), 3.43(2H, q, J=5.2Hz), 3.60-3.78(3H, m), 5.98(1H, brs).

Mass(ESI): 476(M+1).

Rf value (on silica gel plate, developing solvents: methanol/chloroform = 1/10): 0.12

The following compounds were synthesized by similar methods to Example 64.

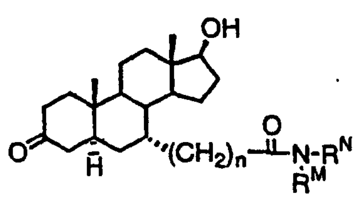

| Example | n | $R^M$ | $R^N$ | MW | Mass |
|---|---|---|---|---|---|
| 65 | 7 | H | n-Pr | 473 | 474(FAB) |
| 66 | 7 | H | n-hexyl | 515 | 516(ESI) |
| 67 | 7 | H | i-pentyl | 501 | 502(FAB) |
| 68 | 7 | H | i-Bu | 487 | 488(FAB) |
| 69 | 7 | H | neopentyl | 501 | 502(ESI) |
| 70 | 7 | H | 3-pentyl | 501 | 502(ESI) |
| 71 | 7 | n-hexyl | n-hexyl | 599 | 600(ESI) |
| 72 | 7 | H | Ph | 507 | 508(ESI) |
| 73 | 7 | H | Bn | 521 | 522(ESI) |
| 74 | 7 | H | $-CH_2CH_2Ph$ | 535 | 536(ESI) |

[Example 75]

Synthesis of 17β-hydroxy-11β-(9-carboxynonyl)-5α-androstan-3-one

(Step 1)

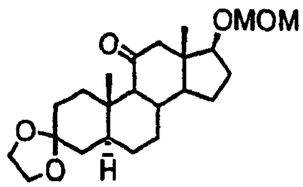

3,3-ethylenedioxy-17β-(methoxymethoxy)-5α-androstan-11-one

3,3-Ethylenedioxy-17β-hydroxy-5α-androstan-11-one (1.84 g) was dissolved in dichloromethane (30 ml) and, after adding N,N-diisopropylethylamine (2.7 ml) and chloromethylmethyl ether (1.2 ml) dropwise, the mixture was stirred for 8 hours at 25°C. The reaction mixture was poured into a saturated aqueous solution of ammonium chloride and subjected to extraction with dichloromethane. The organic layer was washed with a saturated aqueous solution of sodium chloride and dried with magnesium sulfate; after filtering, the solvent was distilled off at reduced pressure. The resulting residue was purified by silica gel column chromatography (developing solvents: ethyl acetate/n-hexane = 1/1) to give the end compound in 1.98 g (yield, 95%).

1H-NMR(270MHz, $CDCl_3$)δ: 0.72(3H, s), 1.03(3H, s), 1.03-1.38(7H, m), 1.52-1.80(9H, m), 2.05-2.23(2H, m) 2.36-2.48(2H, m), 3.33(3H, s), 3.70(1H, t, J=8.4Hz). 3.92(4H, s), 4.57(1H, d, J=14.2Hz), 4.60(1H, d, J=14.2Hz).
Rf value (on silica gel plate, developing solvents: ethyl acetate/n-hexane = 1/1): 0.61

(Step 2)

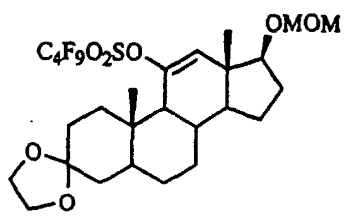

3,3-ethylenedioxy-17β-methoxymethoxy-11-[{(1,1,2,2,3,3,4,4,4-nonafluorobutyl)sulfonyl}oxy]-5α-androst-11-ene

To a solution of lithium diisopropylamide (as prepared from diisopropylamine (0.15 ml) and n-butyllithium (1.5 M hexane solution) (0.69 ml)) in tetrahydrofuran (1.3 ml), a solution of 3,3-ethylenedioxy-17β-methoxymethoxy-5α-androstan-11-one (100 mg) in tetrahydrofuran (1.3 ml) was added dropwise over 5 minutes. After stirring for 30 minutes at -78°C, perfluorobutanesulfonyl fluoride (0.13 ml) was added dropwise over 5 minutes. After stirring for 5 minutes at -78°C, the mixture was stirred for 2 hours at room temperature. A saturated aqueous solution of ammonium chloride was added to the reaction mixture and extraction with ethyl acetate was effected. The organic layer was washed with a saturated aqueous solution of sodium chloride and dried with magnesium sulfate; after filtering, the solvent was distilled off at reduced pressure.
Purification by silica gel column chromatography (developing solvents: ethyl acetate/n-hexane = 1/6) gave the end compound in 98.8 mg (yield, 57%).
1H-NMR(270MHz, $CDCl_3$)δ: 0.93(3H, s), 0.96(3H, s), 0.83-2.23(18H, m), 3.34(3H, s), 3.70(1H, t, J=8.2Hz), 3.93(4H, s), 4.58(1H, d, J=6.6Hz), 4.63(1H, d, J=6.6Hz), 6.20(1H, s). Rf value (on silica gel plate, developing solvents: ethyl acetate/hexane = 1/2): 0.67

(Step 3)

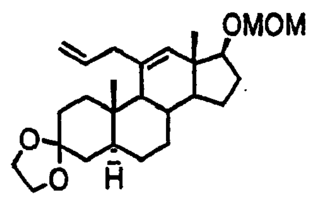

3.3-ethylenedioxy-17β-(methoxymethoxy)-11-(2-propen-1-yl)-5α-androst-11-ene

3,3-Ethylenedioxy-17β-methoxymethoxy-11-[{(1,1,2,2,3,3,4,4,4-nonafluorobutyl)sulfonyl)oxy]-5α-androst-11-ene) (454.5 mg) was dissolved in tetrahydrofuran (6 ml) and, after adding allyltributyltin (299.1 mg), lithium chloride (90.0 mg) and tetrakis(triphenylphosphine)palladium (47.7 mg), the mixture was heated under reflux for 22 hours in an argon atmosphere. After standing to cool, an aqueous solution of potassium fluoride was added and extraction with ethyl acetate was effected. The organic layer was washed with water and a saturated aqueous solution of sodium chloride and dried with magnesium sulfate; after filtering, the solvent was distilled off at reduced pressure. The resulting residue was purified by silica gel column chromatography (developing solvents: ethyl acetate/n-hexane = 1/9) to give the end compound in 275.8 mg (yield, 98%).

1H-NMR(270MHz, $CDCl_3$)δ: 0.83(3H, s), 0.89(3H, s), 0.93-1.88(16H, m), 1.98-2.12(2H, m), 2.72-2.92(2H, m), 3.37 (3H, s), 3.59(1H, t, J=8.7Hz), 3.94(4H, s), 4.62(1H, d, J=10.1Hz), 4.65(1H, d, J=10.1Hz), 4.91-5.02(2H, m), 5.65-5.82 (1H, m), 5.88(1H, s).

Rf value (on silica gel plate, developing solvents: ethyl acetate/n-hexane = 1/4): 0.53

(Step 4)

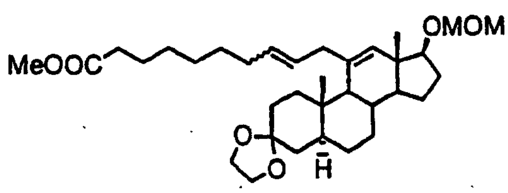

3,3-ethylenedioxy-17β-(methoxymethoxy)- 11-(9-methoxycarbonyl-2-nonen-1-yl)-5α-androst-11-ene

3,3-Ethylenedioxy-17β-(methoxymethoxy)-11-(2-propen-1-yl)-5α-androst-11-ene (248.5 mg) was dissolved in dichloromethane (3 ml) and, after adding methyl 8-nonenoate (202.9 mg) and benzylidenebis(tricyclohexylphosphine)-dichlororuthenium (27.3 mg), the mixture was heated under reflux for 5 hours in an argon atmosphere. After standing to cool, purification was effected by silica gel column chromatography (developing solvents: ethyl acetate/n-hexane = 1/6) to give the end compound in 227.8 mg (yield, 68%).

1H-NMR(270MHz, $CDCl_3$)δ: 0.83(3H, s), 0.88(3H, s), 0.90-1.40(13H, m), 1.40-1.83(11H, m), 1.90-2.12(4H, m), 2.30 (2H, t, J=7.6Hz), 2.65-2.84(2H, m), 3.36(3H, s), 3.60(1H, t, J=8.4Hz), 3.66(3H, s), 3.93(4H, s), 4.63(1H, d, J=11.2Hz), 4.65(1H, d, J=11.2Hz), 5.23-5.40(2H, m), 5.87(1H, s).

Rf value (on silica gel plate, developing solvents: ethyl acetate/n-hexane = 1/6): 0.23

(Step 5)

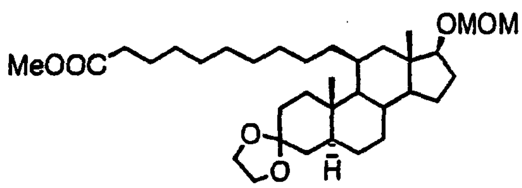

3,3-ethylenedioxy-17β-(methoxymethoxy)-11β-(9-methoxycarbonylnonyl)-5α-androstane

3,3-Ethylenedioxy-17β-(methoxymethoxy)-11-(9-methoxycarbonyl-2-nonen-1-yl)-5α-androst-11-ene (226.3 mg) was dissolved in ethyl acetate (5 ml) and, after adding iridium black (55.7 mg), the mixture was stirred for 5 days at 25°C in a hydrogen atmosphere. The reaction mixture was filtered through Celite and concentrated at reduced pressure; the resulting residue was purified by silica gel column chromatography (developing solvents: ethyl acetate/n-hexane = 1/10 - 1/6) to give the end compound in 165.9 mg (yield, 73%).
1H-NMR(270MHz, $CDCl_3$)δ: 0.83(3H, s), 0.93(3H, s), 0.95-1.05(3H, m), 1.10-1.41(20H, m), 1.45-1.80(11H, m), 1.90-2.07(2H, m), 2.16(1H, d, J=12.5Hz), 2.30(2H, t, J=7.6Hz), 3.35(3H, s), 3.43(1H, t, J=8.7Hz), 3.66(3H, s), 3.93 (4H, s), 4.62(2H, s).
Rf value (on silica gel plate, developing solvents: ethyl acetate/n-hexane = 1/4): 0.47

(Step 6) 32-54

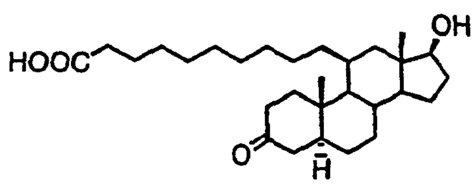

17β-hydroxy-11β-(9-carboxynonyl)-5α-androstan-3-one

3,3-Ethylenedioxy-17β-(methoxymethoxy)-11β-(9-methoxycarbonylnonyl)-5α-androstane (91.0 mg) was dissolved in acetone (3 ml) and, after adding 1 N-hydrochloric acid (0.5 ml), the mixture was heated under reflux for 24 hours. After standing to cool, water was added and extraction with ethyl acetate was effected. The organic layer was washed with a saturated aqueous solution of sodium chloride and dried with magnesium sulfate; after filtering, the solvent was distilled off at reduced pressure. The resulting residue was purified by silica gel column chromatography (developing solvents: ethyl acetate/chloroform = 1/6 - 1/2) to give the end compound in 70.0 mg (yield, 94%).
1H-NMR(270MHz, $CDCl_3$)δ: 0.85(3H, s), 0.90-1.03(4H, m), 1.15(3H, s), 1.16-1.88(25H, m), 1.98-2.48(10H, m), 3.58 (1H, t, J=8.7Hz).
Mass(FAB): 461(M+1).
Rf value (on silica gel plate, developing solvents: ethyl acetate/n-hexane = 1/2): 0.086

The following compounds were synthesized by similar methods to Example 75.

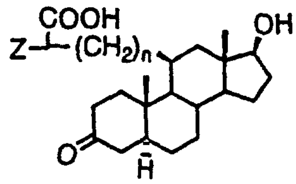

| Example | n | Z | MW | Mass |
|---|---|---|---|---|
| 76 | 6 | H | 432 | 433(FAB) |
| 77 | 7 | H | 446 | 447(FAB) |
| 78 | 10 | H | 488 | 489(FAB) |
| 79 | 8 | $-(CH_2)_3CF_2CF_3$ | 620 | 621(ESI) |

[Example 80]

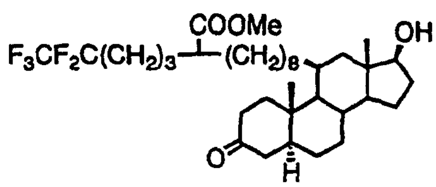

MW 634, Mass(ESI): 635(M+1).

[Example 81]

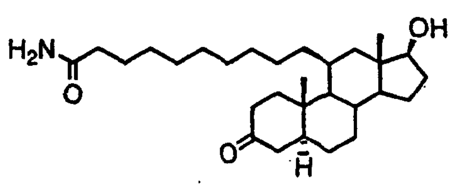

Synthesis of 17β-hydroxy-11β-(9-aminocarbonylnonyl)-5α-androstan-3-one

The 17β-hydroxy-11β-(9-carboxynonyl)-5α-androstan-3-one (16.6 mg) obtained in Example 75 was dissolved in tetrahydrofuran (1 ml) and, after adding triethylamine (6.0 µl) and ethyl chlorocarbonate (4.0 µl) at -10°C, the mixture was stirred for 10 minutes. Ammonia gas was blown into the reaction mixture for 5 minutes and the mixture was stirred for 20 minutes at -10°C. A saturated aqueous solution of ammonium chloride was added to the reaction mixture, which was then reverted to room temperature. After extraction with ethyl acetate, the organic layer was washed with a saturated aqueous solution of sodium chloride and dried with magnesium sulfate; after filtering, the solvnt was distilled off at reduced pressure. The resulting residue was purified by silica gel column chromatography (developing solvents: methanol/chloroform = 1/20) to give the end compound in 15.5 mg (yield, 94%).
1H-NMR(270MHz, $CDCl_3$)δ: 0.85(3H, s), 0.88-1.05(4H, m), 1.15(3H, s), 1.10-1.88(25H, m), 1.97-2.50(10H, m), 3.58 (1H, t, J=8.7Hz), 5.34(2H, brs).

Mass(FAB):460(M+1)
Rf value (on silica gel plate, developing solvents: methanol/chloroform = 1/10): 0.33

The following compounds were synthesized by similar methods to Example 81.

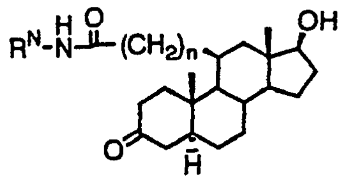

| Example | n | Z | MW | Mass |
|---|---|---|---|---|
| 82 | 9 | n-phenyl | 529 | 530(FAB) |
| 83 | 11 | H | 487 | 488(FAB) |
| 84 | 11 | n-phenyl | 557 | 558(FAB) |

The following compounds were synthesized by similar methods to Example 9.

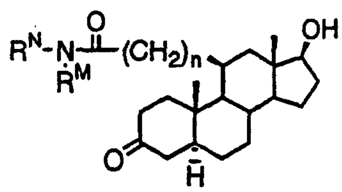

| Example | n | $R^M$ | $R^N$ | MW | Mass |
|---|---|---|---|---|---|
| 85 | 7 | Me | Me | 459 | 460(FAB) |
| 86 | 7 | H | Me | 445 | 446(FAB) |
| 87 | 7 | Me | Et | 473 | 474(FAB) |
| 88 | 7 | Me | n-Pr | 487 | 488(FAB) |
| 89 | 7 | $-CH_2CH_2OCH_2CH_2-$ | | 501 | 502(FAB) |
| 90 | 8 | Me | Me | 473 | 474(FAB) |
| 91 | 8 | H | Me | 459 | 460(FAB) |
| 92 | 8 | Me | Et | 487 | 488(ESI) |
| 93 | 8 | Me | n-Pr | 501 | 502(FAB) |
| 94 | 8 | $-CH_2CH_2OCH_2CH_2-$ | | 515 | 516(FAB) |
| 95 | 9 | Me | Me | 487 | 488(ESI) |
| 96 | 9 | Et | Et | 515 | 516(ESI) |
| 97 | 9 | $-(CH_2)_5-$ | | 527 | 528(ESI) |
| 98 | 9 | H | Bn | 549 | 550(ESI) |
| 99 | 9 | Me | n-Bu | 529 | 530(ESI) |
| 100 | 9 | H | $-CH_2CH_2OH$ | 503 | 504(ESI) |

[Example 101]

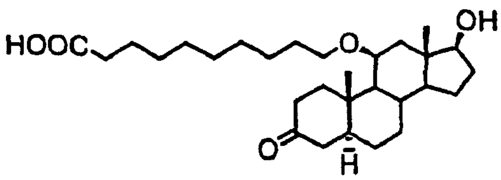

Synthesis of 17β-hydroxy-11β-(9-carboxynonyloxy)-5α-androstan-3-one

(Step 1)

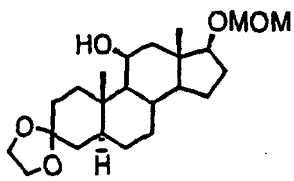

3,3-ethylenedioxy-11β-hydroxy-17β-(methoxymethoxy)-5α-androstane

3,3-Ethylenedioxy-17β-(methoxymethoxy)-5α-androstan-11-one (2.84 g) was dissolved in diethyl ether (500 ml) and, after adding lithium aluminum hydride (548 mg), the mixture was heated under reflux for 2 hours in an argon atmosphere. The reaction mixture was cooled to 0°C and, after adding water, filtered through Celite. After extraction with ethyl acetate, the organic layer was washed with a saturated aqueous solution of sodium chloride and dried with magnesium sulfate; after filtering, the solvent was distilled off at reduced pressure. The resulting residue was purified by silica gel column chromatography (developing solvents: ethyl acetate/n-hexane = 1/2) to give the end compound in 2.68 g (yield, 94%).

1H-NMR(270MHz, $CDCl_3$)δ: 0.75-0.99(3H, m), 1.01(3H, s), 1.06(3H, s), 1.20-1.92(16H, m), 1.94-2.07(2H, m), 3.35 (3H, s), 3.49(1H, t, J=8.3Hz), 3.94(4H, s), 4.29-4.36(1H, m), 4.61(2H, s).
Rf value (on silica gel plate, developing solvents: methanol/chloroform = 1/50): 0.31

(Step 2)

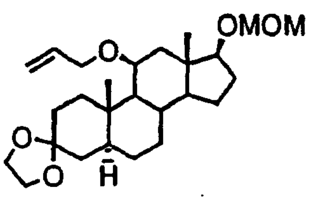

3,3-ethylenedioxy-17β-(methoxymethoxy)-11β-(2-propen-1-yloxy)-5α-androstane

In an argon atmoshere, 3,3-ethylenedioxy-11β-hydroxy-17β-(methoxymethoxy)-5α-androstane (953.9 mg) was dissolved in N,N-dimethylformamide (10 ml) and, after adding sodium hydride (60% in oil) (486.7 mg), the mixture was stirred for 3 hours at 50°C. After reversion to 25°C, allyl bromide (2.20 ml) and tetra-n-butylammonium iodide (208.5 mg) were added and the mixture was stirred for 3 hours at 50°C. The reaction mixture was cooled to 0°C and

water was added. After extraction with ethyl acetate, the organic layer was washed with a saturated aqueous solution of sodium chloride and dried with magnesium sulfate; after filtering, the solvent was distilled off at reduced pressure. The resulting residue was purified by silica gel column chromatography (developing solvents: ethyl acetate/n-hexane = 1/5) to give the end compound in 684.5 mg (yield, 65%).
1H-NMR(270MHz, $CDCl_3$)δ: 0.74-0.93(3H, m), 0.95(3H, s), 1.03(3H, s), 1.18-2.09(16H, m), 2.32(1H, dd, J=2.9, 14.4Hz), 3.36(3H, s), 3.47(1H, t, J=8.3Hz), 3.70(1H, dd, J=7.2, 16.2Hz), 3.77-3.83(1H, m), 3.93(4H, s), 4.10(1H, dd, J=7.2, 16.2Hz), 4.63(2H, AB-q), 5.08(1H, split-d, J=10.6Hz), 5.25(1H, dd, J=1.7, 17.2Hz), 5.83-6.00(1H, m).
Rf value (on silica gel plate, developing solvents: ethyl acetate/n-hexane = 1/2): 0.59

(Step 3)

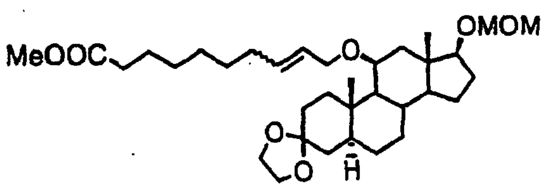

3,3-ethylenedioxy-17β-(methoxymethoxy)-11β-(9-methoxycarbonyl-2-nonen-1-yloxy)-5α-androstane

3,3-Ethylenedioxy-17β-(methoxymethoxy)-11β-(2-propen-1-yloxy)-5α-androstane (18.9 mg) was dissolved in dichloromethane (0.5 ml) and, after adding methyl 8-nonenoate (14.8 mg) and benzylidenebis(tricyclohexylphosphine)-dichlororuthenium (2.0 mg), the mixture was heated under reflux for 4 hours in an argon atmosphere. After standing to cool and concentrating at reduced pressure, purification was effected by silica gel column chromatography (developing solvents: ethyl acetate/n-hexane = 1/5) to give the end compound in 15.5 mg (yield, 62%).
1H-NMR(270MHz, $CDCl_3$)δ: 0.70-0.95(3H, m), 0.95(3H, s), 1.02(3H, s), 1.15-1.86(23H, m), 1.93-2.08(3H, m), 2.30 (3H, t, J=7.6Hz), 3.36(3H, s), 3.46(1H, t, J=8.7Hz), 3.64(1H, dd, J=5.0, 11.3Hz), 3.67(3H, s), 3.74-3.80(1H, m), 3.93 (4H, s), 4.02(1H, dd, J=5.0, 11.3Hz), 4.63(2H, AB-q), 5.44-5.69(1H, m).
Rf value (on silica gel plate, developing solvents: ethyl acetate/n-hexane = 1/4): 0.26

(Step 4)

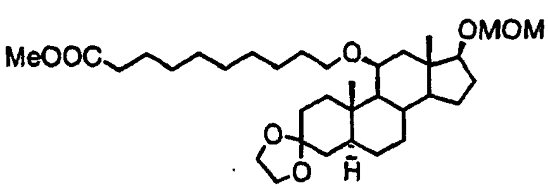

3,3-ethylenedioxy-17β-(methoxymethoxy)-11β-(9-methoxycarbonylnonyloxy)-5α-androstane

3,3-Ethylenedioxy-17β-(methoxymethoxy)-11β-(9-methoxycarbonyl-2-nonen-1-yloxy)-5α-androstane (17.2 mg) was dissolved in ethyl acetate (3 ml) and, after adding 10%-palladium/carbon (6.5 mg), the mixture was stirred for 1 hour at 25 °C in a hydrogen atmosphere. The reaction mixture was filtered and the solvent was distilled off at reduced pressure to give the residue in 16.7 mg. In a separate run, 3,3-ethylenedioxy-17β-(methoxymethoxy)-11β-(9-methoxycarbonyl-2-nonen-1-yloxy)-5α-androstane (32.1 mg) was dissolved in ethyl acetate (6 ml) and, after adding 10%-palladium/carbon (6.5 mg), the solution was stirred for 1 hour at 25°C in a hydrogen atmosphere. The reaction mixture was filtered and the solvent was distilled off at reduced pressure to give the residue in 30.1 mg. The two residues were combined and purified by silica gel column chromatography (developing solvents: ethyl acetate/n-hexane = 1/2) to give the end compound in 44.7 mg (yield, 90%).
1H-NMR(270MHz, $CDCl_3$)δ: 0.71-0.94(3H, m), 0.94(3H, s), 1.02(3H, s), 1.20-2.07(30H, m), 2.30(3H, t, J=7.6Hz), 3.07 (3H, dt, J=5.7, 8.4Hz), 3.36(3H, s), 3.43-3.58(2H, m), 3.67(3H, s), 3.68-3.74(1H, m), 3.93(4H, s), 4.63(2H, AB-q). Rf

value (on silica gel plate, developing solvents: ethyl acetate/n-hexane = 1/2): 0.55

(Step 5)

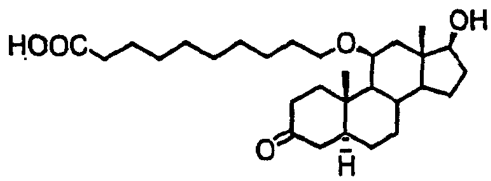

17β-hydroxy-11β-(9-carboxynonyloxy)-5α-androstan-3-one

3,3-Ethylenedioxy-17β-(methoxymethoxy)-11β-(9-methoxycarbonylnonyloxy)-5α-androstane (21.0 mg) was dissolved in acetone (2 ml) and, after adding 1 N-hydrochloric acid (0.5 ml), the mixture was heated under reflux for 10 hours. After adding water to the reaction mixture, extraction with dichloromethane was effected and the organic layer was washed with a saturated aqueous solution of sodium chloride and dried with magnesium sulfate; after filtering, the solvent was distilled off at reduced pressure to give the residue in 20.6 mg. In a separate run, 3,3-ethylenedioxy-17β-(methoxymethoxy)-11β-(9-methoxycarbonylnonyloxy)-5α-androstane (22.0 mg) was dissolved in acetone (2 ml) and, after adding 1 N-hydrochloric acid (0.5 ml), the mixture was heated under reflux for 10 hours. After adding water to the reaction mixture, extraction with dichloromethane was effected and the organic layer was washed with a saturated aqueous solution of sodium chloride and dried with magnesium sulfate: after filtering, the solvent was distilled off at reduced pressure to give the residue in 21.6 mg. The two residues were combined and purified by silica gel column chromatography (developing solvents: ethyl acetate/n-hexane = 1/1) to give the end compound in 29.3 mg (yield, 70%).
1H-NMR(270MHz, $CDCl_3$)δ: 0.74(1H, dd, J=3.3, 10.9Hz), 0.85-0.98(3H, m), 0.94(3H, s), 1.24(3H, s), 1.26-1.72(22H, m), 1.79-2.14(5H, m), 2.25-2.57(6H, m), 3.10(1H, dt, J=6.1, 8.8Hz), 3.53-3.63(2H, m), 3.71-3.78(1H, m).
Mass(FAB): 477(M+1).
Rf value (on silica gel plate, developing solvents: ethyl acetate/n-hexane = 1/1): 0.12

The following compounds were synthesized by similar methods to Example 101.

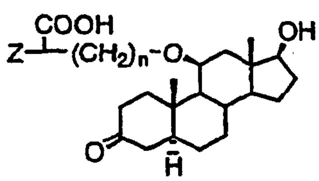

| Example | n | Z | MW | Mass(FAB) |
|---|---|---|---|---|
| 102 | 4 | H | 420 | 421 |
| 103 | 6 | H | 448 | 449 |
| 104 | 9 | H | 490 | 491 |
| 105 | 10 | H | 504 | 505 |
| 106 | 12 | H | 532 | 533 |
| 107 | 22 | H | 672 | 673 |
| 108 | 8 | $-(CH_2)_3CF_2CF_3$ | 636 | 637 |

[Example 109]

Synthesis of 17β-hydroxy-11β-(10-(4,4,5,5,5-pentafluoropentylsulfanyl)decyl)-5α-androstan-3-one

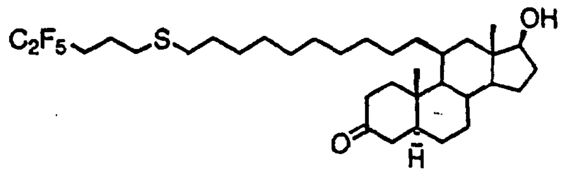

The 3,3-diethylenedioxy-17β-(methoxymethoxy)-11β-(9-methoxycarbonylnonyl)-5α-androstane (64.8 mg) obtained in step 5 of Example 75 was dissolved in tetrahydrofuran (3 ml) and, after adding lithium borohydride (11 mg), the mixture was stirred for 4 hours at 25°C. To the reaction mixture, lithium triethylborohydride (1.0 M-tetrahydrofuran solution, 100 µl) was added and the mixture was stirred for 4 hours at 25°C. To the reaction mixture, lithium borohydride (20 mg) was added and the mixture was stirred for 15 hours at 25°C. After adding water, extraction with ethyl acetate was conducted. The organic layer was washed with a saturated aqueous solution of sodium chloride and dried with magnesium sulfate; after filtering, the solvent was distilled off at reduced pressure. The resulting residue was purified by silica gel column chromatography (developing solvents: ethyl acetate/n-hexane = 1/4 - 1/2) to give an oil in 63.8 mg. This oil was dissolved in dichloromethane (2 ml) and, after adding triethylamine (30 µl) and methanesulfonyl chloride (15 µl) at 0°C, the mixture was stirred for 4 hours at 25°C. The reaction mixture was poured into a saturated aqueous solution of sodium chloride and extraction with dichloromethane was conducted. The organic layer was dried with magnesium sulfate and after filtering, the solvent was distilled off at reduced pressure. The resulting residue was dissolved in acetone (3 ml) and, after adding sodium iodide (300 mg), the mixture was stirred for 15 hours at 25°C. A saturated aqueous solution of sodium sulfite was added to the reaction mixture and extraction with ethyl acetate was conducted. The organic layer was washed with a saturated aqueous solution of sodium chloride and dried with magnesium sulfate; after filtering, the solvent was distilled off at reduced pressure. The residue was purified by silica gel column chromatography (developing solvents: ethyl acetate/n-hexane = 1/4) to give an oil in 53.9 mg. This oil and 1-(acetylsulfanyl)-4,4,5,5,5-pentafluoropentane (45 mg) were dissolved in methanol (1 ml) and tetrahydrofuran (0.5 ml) and, after adding a methanol solution (0.18 ml) of 1 N-sodium methoxide, the mixture was stirred for 15 hours at 25°C. After adding water to the reaction mixture, extraction with ethyl acetate was conducted. The organic layer was washed with a saturated aqueous solution of sodium chloride and dried with magnesium sulfate; after filtering, the solvent was distilled off at reduced pressure. The residue was purified by silica gel column chromatography (developing solvents: ethyl acetate/n-hexane = 1/4) to give an oil in 66.3 mg. This oil was dissolved in acetone (2 ml) and, after adding 1 N-hydrochloric acid (0.5 ml), the mixture was heated under reflux for 36 hours. After standing to cool, water was added and extraction with ethyl acetate was conducted. The organic layer was washed with a saturated aqueous solution of sodium chloride and dried with magnesium sulfate; after filtering, the solvent was distilled off at reduced pressure. The residue was purified by silica gel column chromatography (developing solvents: ethyl acetate/n-hexane = 1/4) to give the end compound in 52.9 mg (yield, 74%).
1H-NMR(270MHz, $CDCl_3$)δ: 0.85(3H, s), 0.89-1.09(3H, m), 1.15(3H, s), 1.18-1.74(28H, m), 1.80-2.45(13H, m), 2.51 (2H, t, J=7.4Hz), 2.59(2H, t, J=6.9Hz), 3.51-3.62(1H, m).
Mass(FAB): 623(M+1).
Rf value (on silica gel plate, developing solvents: ethyl acetate/n-hexane = 1/4): 0.19

[Example 110]

Synthesis of 17β-hydroxy-11β-(10-(4,4,5,5,5-pentafluoro-pentylsulfinyl)decyl)-5α-androstan-3-one

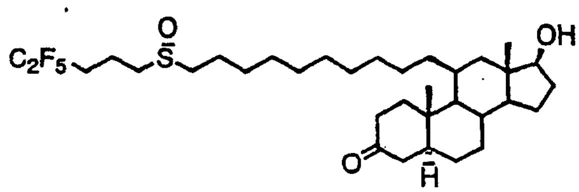

The 17β-hydroxy-11β-(10-(4,4,5,5,5-pentafluoro-pentylsulfanyl)decyl)-5α-androstan-3-one (20.5 mg) obtained in Example 109 was dissolved in tetrahydrofuran (0.8 ml) and, after adding OXONE (registered trademark, 12.4 mg) and water (0.4 ml), the mixture was stirred for 2 hours at 0°C. A saturated aqueous solution of sodium hydrogencarbonate was added to the reaction mixture and, after reverting it to room temperature, extraction with ethyl acetate was conducted. The organic layer was washed with a saturated aqueous solution of sodium chloride and dried with magnesium sulfate; after filtering, the solvent was distilled off at reduced pressure. The residue was purified by silica gel column chromatography (developing solvents: ethyl acetate/n-hexane = 1/2 ~ 1/1 ~ 2/1) to give the end compound in 19.5 mg (yield, 93%).
1H-NMR(270MHz, $CDCl_3$)δ: 0.85(3H, s), 0.86-1.05(3H, m), 1.15(3H, s), 1.10-1.90(27H, m), 1.95-2.50(13H, m), 2.60-2.82(4H, m), 3.57(1H, t, J=8.2Hz).
Mass(FAB): 639(M+1).
Rf value (on silica gel plate, developing solvents: ethyl acetate/n-hexane = 1/4): 0.056

[Example 111]

Synthesis of 17β-hydroxy-7α-(7-hydroxyheptyl)-5α-androstan-3-one

(Step 1)

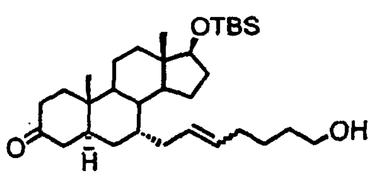

17β-t-butyldimethylsilyloxy-7α-(7-hydroxy-2-hepten-1-yl)-5α-androstan-3-one

17β-t-Butyldimethysilyloxy-7α-(2-propen-1-yl)-5α-androstan-3-one (33.4 mg) was dissolved in dichloromethane (0.5 ml) and, after adding 5-hexen-1-ol (20.0 mg) and benzylidenebis(tricyclohexylphosphine)-dichlororuthenium (5.2 mg), the mixture was heated under reflux for 2 hours in an argon atmosphere. After standing to cool, purification was effected by silica gel column chromatogrpahy (developing solvents: ethyl acetate/n-hexane = 1/4) to give the end compound in 19.7 mg (yield, 51%).
1H-NMR(270MHz, $CDCl_3$)δ: 0.01(6H, s), 0.72(3H, s), 0.88(9H, s), 1.04(3H, s), 0.90-2.10(27H, m), 2.20-2.47(3H, m), 3.54(1H, t, J=8.6Hz), 3.63(2H, t, J=6.8Hz), 5.19-5.42(2H, m).
Rf value (on silica gel plate, developing solvents: ethyl acetate/n-hexane = 1/4): 0.13

(Step 2)

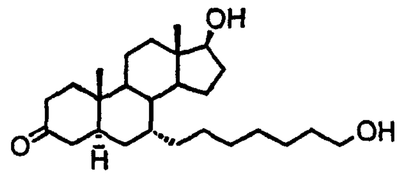

17β-hydroxy-7α-(7-hydroxyheptyl)-5α-androstan-3-one

17β-t-Butyldimethylsilyloxy-7α-(7-hydroxy-2-hepten-1-yl)-5α-androstan-3-one (19.6 mg) was dissolved in ethyl acetate (10 ml) and, after adding 10%-palladium/carbon (6.3 mg), the mixture was stirred for 1 hour at 25°C in a hydrogen atmosphere. The reaction mixture was filtered and the solvent was distilled off at reduced pressure; the resulting residue was dissolved in acetone (2 ml) and, after adding 2 N-hydrochloric acid (0.5 ml), the mixture was stirred for 2 hours at 25°C. After adding water to the reaction mixture, extraction with ethyl acetate was conducted. The organic layer was washed with a saturated aqueous solution of sodium chloride and dried with magnesium sulfate; after filtering, the solvent was distilled off at reduced pressure. The resulting residue was purified by silica gel column chromatography (developing solvents: ethyl acetate/n-hexane = 1/2 - 1/1) to give the end compound in 15.0 mg (yield, 98%).
1H-NMR(270MHz, $CDCl_3$)δ: 0.76(3H, s), 1.04(3H, s), 0.80-1.83(29H, m), 1.95-2.15(3H, m), 2.22-2.47(3H, m), 3.60-3.70(3H, m).
Mass(ESI): 405(M+1).
Rf value (on silica gel plate, developing solvents: ethyl acetate/n-hexane = 1/1): 0.24
The following compounds were synthesized by similar methods to Example 111.

| Example | n | MW | Mass (ESI) |
|---|---|---|---|
| 112 | 8 | 418 | 419 |
| 113 | 9 | 432 | 433 |

[Example 114]

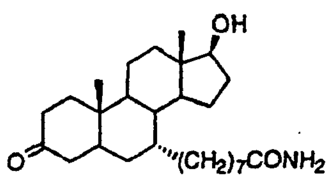

Synthesis of 17β-hydroxy-7α-(7-carbamoylheptyl)-5α-androstan-3-one

The 17β-hydroxy-7α-(7-carboxyheptyl)-5α-androstan-3-one (12.6 mg) obtained in Example 3 was dissolved in tetrahydrofuran (0.5 ml) and then triethylamine (8.1 μl) and ethyl chloroformate (4.2 μl) were added dropwise at 0°C. After stirring for 5 minutes, ammonia gas was bubbled for 30 seconds. After stirring for 30 minutes, water was added to the reaction mixture and extraction with dichloromethane was effected. The organic layer was washed with a saturated aqueous solution of sodium chloride and dried with magnesium sulfate; after filtering, the solvent was distilled off at reduced pressure. Purification by silica gel column chromatography (developing solvents: dichloromethane/methanol = 20/1) gave the end compound in 11.6 mg (92%).
1H-NMR(270MHz, $CDCl_3$)δ: 0.76(3H, s), 1.04(3H, s), 1.00-1.83(27H, m), 1.94-2.16(3H, m), 2.20-2.50(5H, m), 3.65(1H, t, J=8.4Hz), 5.34-5.54(2H, m).
Mass(FAB): 432(M+1).
Rf value (on silica gel plate, developing solvents: dichloromethane/methanol = 20/1): 0.48

The following compounds were synthesized by similar methods to Example 114.

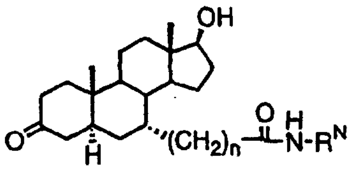

| Example | n | $R^N$ | MW | Mass (FAB) |
|---|---|---|---|---|
| 115 | 5 | H | 403 | 404 |
| 116 | 5 | n-pentyl | 473 | 474 |
| 117 | 7 | n-pentyl | 501 | 502 |
| 118 | 9 | H | 459 | 460 |
| 119 | 9 | n-pentyl | 529 | 529 |
| 120 | 11 | H | 487 | 487 |
| 121 | 11 | n-pentyl | 557 | 557 |
| 122 | 13 | H | 515 | 515 |
| 123 | 13 | n-pentyl | 585 | 585 |

[Example 124]

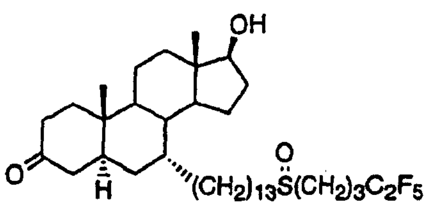

17β-hydroxy-7α-{13-(4,4,5,5,5-pentafluoropentylsulfinyl)-tridecyl}-5α-androstan-3-one

(Step 1)

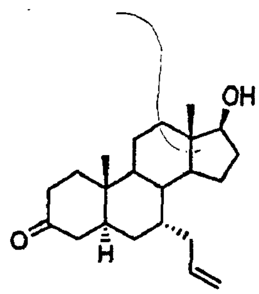

Synthesis of 17β-hydroxy-7α-(2-propen-1-yl)-5α-androstan-3-one

17β-t-Butyldimethylsilyloxy-7α-(2-propen-1-yl)-5α-androstan-3-one (170 mg) was dissolved in acetone (2 ml) and then 2 N-hydrochloric acid (0.5 ml) was added dropwise. After stirring for 4 hours at 25°C, water was added to the reaction mixture and extraction with ethyl acetate was conducted. The organic layer was washed with a saturated aqueous solution of sodium chloride and dried with magnesium sulfate; after filtering, the solvent was distilled off at reduced pressure to give the end compound in 126 mg (100%).
1H-NMR(270MHz, $CDCl_3$)δ: 0.77(3H, s), 1.04(3H, s), 0.96-2.44(23H, m), 3.60-3.70(1H, m), 4.94(1H, d, J=3.5Hz), 5.00 (1H, s), 5.58-5.72(1H, m).
Rf value (on silica gel plate, developing solvents: ethyl acetate/n-hexane = 1/1): 0.54

(Step 2)

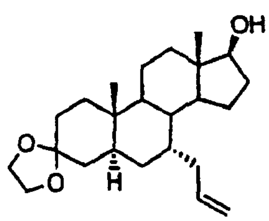

Synthesis of 3,3-ethylenedioxy-17β-hydroxy-7α-(2-propen-1-yl)-5α-androstane

To a benzene solution (5 ml) of 17β-hydroxy-7α-(2-propen-1-yl)-5α-androstan-3-one (126 mg), ethylene glycol (2 ml) and p-toluenesulfonic acid (13.2 mg) were added and the mixture was heated under reflux with water being continuously removed with a Dean-Stark trap. After two hours, a saturated aqueous solution of sodium hydrogencarbonate was added under cooling with ice and extraction with ethyl acetate was conducted. The organic layer was washed with a saturated aqueous solution of sodium chloride and dried with magnesium sulfate; after filtering, the solvent was distilled off at reduced pressure to give the end compound in 141 mg (yield, 99%).
1H-NMR(270MHz, $CDCl_3$)δ: 0.74(3H, s), 0.85(3H, s), 0.92-2.20(23H, m), 3.56-3.70(1H, m), 3.93(4H, s), 4.92-5.04(2H, m), 5.62-5.80(1H, m).
Rf value (on silica gel plate, developing solvents: ethyl acetate/n-hexane = 1/1): 0.60

(Step 3)

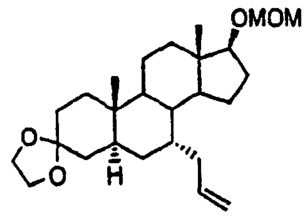

Synthesis of 3,3-ethylenedioxy-17β-methoxymethoxy-7α-(2-propen-1-yl)-5α-androstane

To a dichloromethane solution (4 ml) of 3,3-ethylenedioxy-17β-hydroxy-7α-(2-propen-2-yl)-5α-androstane (141 mg), diisopropylethylamine (0.227 ml) and chloromethyl methyl ether (0.087 ml) were added dropwise under cooling with ice. After stirring for 14 hours at 25°C, a saturated aqueous solution of sodium hydrogencarbonate was added to the reaction mixture and extraction with dichloromethane was effected. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/ n-hexane = 1/4) gave the end compound in 131 mg (yield, 83%).
1H-NMR(270MHz, $CDCl_3$)δ: 0.77(3H, s), 0.84(3H, s), 0.92-2.20(23H, m), 3.35(3H, s), 3.53(1H, t, J=8.3Hz), 3.92(4H, s), 4.62(2H, d, J=1.8Hz), 4.92-5.04(2H, m), 5.62-5.80(1H, m).
Rf value (on silica gel plate, developing solvents: ethyl acetate/n-hexane = 1/4): 0.50

(Step 4)

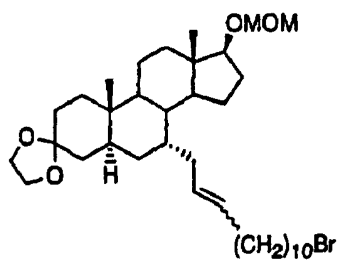

Synthesis of 3,3-ethylenedioxy-17β-methoxymethoxy-7α-(13-bromo-2-tridecen-1-yl)-5α-androstane

3,3-Ethylenedioxy-17β-methoxymethoxy-7α-(2-propen-1-yl)-5α-androstane (42.6 mg) was dissolved in dichloromethane (1.5 ml) and, after adding 12-bromododecene (50.4 mg) and benzylidenebis(tricyclohexylphosphine)-dichlororuthenium (8.4 mg), the mixture was heated under reflux for 5 hours in an argon atmosphere. After standing to cool, purification was performed by silica gel column chromatography (developing solvents: ethyl acetate/n-hexane = 1/10) to give the end compound in 56.0 mg (yield, 86%).
1H-NMR(270MHz, $CDCl_3$)δ: 0.76(3H, s), 0.83(3H, s), 0.94-2.14(41H, m), 3.34(3H, s), 3.41(2H, t, J=6.9Hz), 3.52(1H, t, J=8.3Hz), 3.92(4H, s), 4.62(2H, d, J=1.8Hz), 5.22-5.46(2H, m).
Rf value (on silica gel plate, developing solvents: ethyl acetate/n-hexane = 1/4): 0.56

(Step 5)

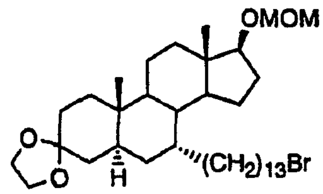

Synthesis of 3,3-ethylenedioxy-17β-methoxymethoxy-7α-(13-bromotridecyl)-5α-androstane

3,3-Ethylenedioxy-17β-methoxymethoxy-7α-(13-bromo-2-tridecen-1-yl)-5α-androstane (55.3 mg) was dissolved in ethyl acetate (2 ml) and, after adding 10%-palladium/carbon (10 mg), the mixture was stirred for 13 hours at 25 °C in a hydrogen atmosphere. After filtering the reaction mixture, the solvent was distilled off at reduced pressure to give the end compound in 47.4 mg (86%).
1H-NMR(270MHz, $CDCl_3$)δ: 0.76(3H, s), 0.84(3H, s), 0.94-2.10(45H, m), 3.34(3H, s), 3.41(2H, t, J=6.9Hz), 3.53(1H, t, J=8.3Hz), 3.93(4H, s), 4.62(2H, d, J=1.8Hz).
Rf value (on silica gel plate, developing solvents: ethyl acetate/n-hexane = 1/4): 0.56

(Step 6)

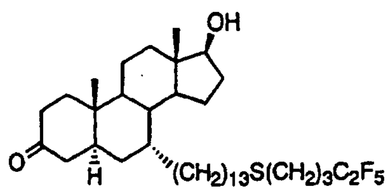

Synthesis of 17β-hydroxy-7α-{13-(4,4,5,5,5-pentafluoro-pentylsulfanyl)tridecyl}-5α-androstan-3-one

4,4,5,5,5-Pentafluoropentanethioacetate (35.0 mg) was dissolved in methanol (1 ml) and then 1 M sodium methylate/methanol solution (0.12 ml) was added dropwise. After stirring for 30 minutes, a solution of 3,3-ethylenedioxy-17β-methoxymethoxy-7α-(13-bromotridecyl)-5α-androstane (47.4 mg) in tetrahydrofuran (1 ml) was added to the reaction mixture. After stirring for 18 hours, water was added to the reaction mixture and extraction with ethyl acetate was conducted. The organic layer was washed with a saturated aqueous solution of sodium chloride and dried with magnesium sulfate; after filtering, the solvent was distilled off at reduced pressure. Crude purification by silica gel column chromatography (developing solvents: ethyl acetate/n-hexane = 1/10) gave 3,3-ethylenedioxy-17β-methoxymethoxy-7α-{13-(4,4,5,5,5-pentafluoropentylsulfanyl)-tridecyl}androstane (42. 6 mg), which was then dissolved in acetone (2 ml); after adding 2 N-hydrochloric acid (0.5 ml), the mixture was heated under reflux for 3 hours at 60°C. After standing to cool down to 0°C, water was added and extraction with chloroform was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 1/4) gave the end compound in 40.2 mg (82%).
1H-NMR(270MHz, $CDCl_3$)δ: 0.76(3H, s), 1.04(3H, s), 0.88-2.40(49H, m), 2.50(2H, t, J=7.3Hz), 2.59(2H, t, J=7.9Hz), 3.58-3.70(1H, m).
Rf value (on silica gel plate, developing solvents: ethyl acetate/n-hexane = 1/4): 0.32

(Step 7)

Synthesis of 17β-hydroxy-7α-{13-(4,4,5,5,5-pentafluoro-pentylsulfinyl)tridecyl}-5α-androstan-3-one

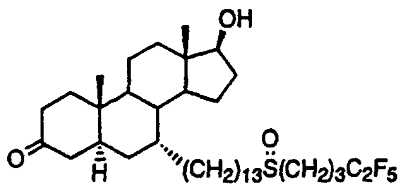

17β-Hydroxy-7α-{13-(4,4,5,5,5-pentafluoropentylsulfanyl)tridecyl)-5α-androstan-3-one (26.0 mg) was dissolved in tetrahydrofuran (1 ml) and then OXONE (14.4 mg) and water (0.2 ml) were added at 0°C. After stirring for 30 minutes, a saturated aqueous solution of sodium hydrogencarbonate was added to the reaction mixture and extraction with ethyl acetate was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 2/1) gave the end compound in 19.5 mg (73%).
1H-NMR(270MHz, $CDCl_3$)δ: 0.76(3H, s), 1.04(3H, s), 0.98-2.40(49H, m), 2.58-2.82(4H, m), 3.60-3.70(1H, m).
Mass(FAB): 681(M+1).
Rf value (on silica gel plate, developing solvents: ethyl acetate/n-hexane = 1/4): 0.10

The following compounds were synthesized by similar methods to Example 124.

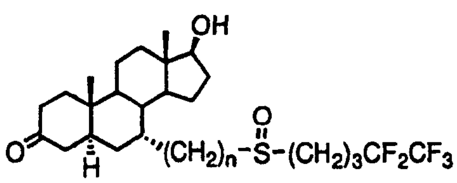

| Example | n | MW | Mass (FAB) |
|---|---|---|---|
| 125 | 5 | 568 | 569 |
| 126 | 7 | 596 | 597 |
| 127 | 9 | 624 | 625 |
| 128 | 11 | 652 | 653 |

[Example 129]

Synthesis of 17β-hydroxy-7α-{13-(4,4,5,5,5-pentafluoropentylsulfonyl)tridecyl}-5α-androstan-3-one

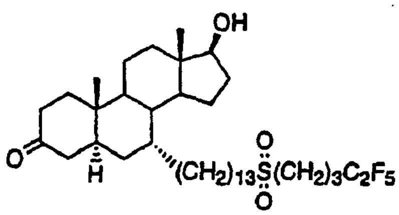

17β-Hydroxy-7α-{13-(4,4,5,5,5-pentafluoropentylsulfanyl)tridecyl}-5α-androstan-3-one (15.0 mg) was dissolved in tetrahydrofuran (1 ml) and then OXONE (27.8 mg) and water (0.2 ml) were added at 25°C. After stirring for 1 hour, a saturated aqueous solution of sodium hydrogencarbonate was added to the reaction mixture and extraction with ethyl acetate was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 1/2) gave the end compound in 15.0 mg (95%).
1H-NMR(270MHz, $CDCl_3$)δ: 0.76(3H, s), 1.04(3H, s), 0.98-2.40(49H, m), 2.92-3.08(4H, m), 3.60-3.70(1H, m).
Mass (FAB): 697(M+1).
Rf value (on silica gel plate, developing solvents: ethyl acetate/n-hexane = 1/4): 0.32

The following compounds were synthesized by similar methods to Example 129.

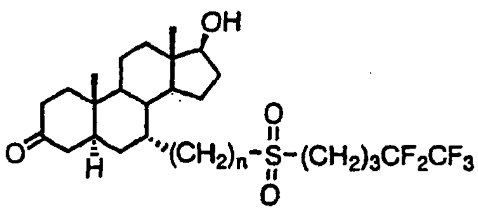

| Example | N | MW | Mass (FAB) |
|---|---|---|---|
| 130 | 7 | 612 | 613 |
| 131 | 9 | 640 | 641 |
| 132 | 11 | 668 | 669 |

[Example 133]

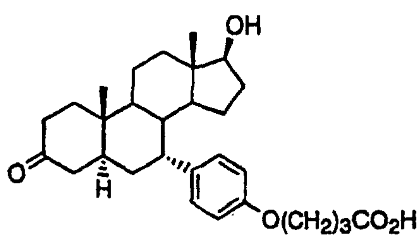

Synthesis of 17β-hydroxy-7α-(4-(3-carboxypropoxy)phenyl)-5α-androstan-3-one

(Step 1)

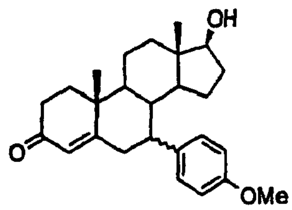

Synthesis of 17β-hydroxy-7-(4-methoxyphenyl)-5α-androst-4-en-3-one

In an argon atmosphere, copper(I) iodide (1.14 g) was dissolved in anhydrous tetrahydrofuran (5 ml) and then 0.5 M 4-methoxyphenylmagnesium bromide/tetrahydrofuran solution (11. 9 ml) was added dropwise at -50 °C. After stirring for 10 minutes, 17β-t-butyldimethylsilyloxyandrosta-4,6-dien-3-one (600 mg), chlorotrimethylsilane (0.376 ml) and a tetrahydrofuran solution (6 ml) of hexamethylphosphoric triamide (0.518 ml) were added dropwise at -78°C. The temperature of the mixture was elevated to -40°C over 1 hour. To the reaction mixture, 2 N-hydrochloric acid was added and, after stirring for 1 hour at 25°C, extraction with ethyl acetate was conducted. The organic layer was dried with magnesium sulfate and, after filtering, the solvent was distilled off at reduced pressure. Purification by silica gel column chromatography (developing solvents: ethyl acetate/n-hexane = 1/2) gave the end compound in 67.9 mg (yield, 12%) as a diastereomeric mixture.
1H-NMR(270MHz, $CDCl_3$)δ: 0.55-0.99(4/3H. m), 0.76(2H, s), 0.81(1H, s), 1.00-2.54(47/3H, m), 1.32(2H, s), 1.34(1H, s), 2.82-3.00(2/3H, m), 3.00-3.08(1/3H, m), 3.40-3.56(1H, m), 3.78(1H, s), 3.80(2H, s), 5.71(1H, s), 6.74-6.86(2H, m), 7.04-7.18(2H, m).
Rf value (on silica gel plate, developing solvents: ethyl acetate/n-hexane = 1/1): 0.38

(Step 2)

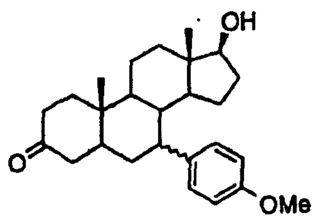

Synthesis of 17β-hydroxy-7-(4-methoxyphenyl)-5α-androstan-3-one

Metallic lithium (11.9 mg) was added to liquid ammonia (15 ml) at -78°C. After stirring for 5 minutes, 17β-hydroxy-7-(4-methoxyophenyl)-5α-androst-4-en-3-one (67.8 mg) and a solution of t-butanol (25.3 μl) in tetrahydrofuran (3 ml) were added and the mixture was stirred for 5 minutes. After adding 1,2-dibromoethane (0.1 ml) and ammonium chloride (1 g), the mixture was stirred for 30 minutes at 25°C. After adding water, extraction with ethyl acetate was conducted. The organic layer was dried with magnesium sulfate and, after filtering, the solvent was distilled off at reduced pressure. Purification by silica gel column chromatography (developing solvents: ethyl acetate/n-hexane = 1/2) gave the end compound in 49.8 mg (yield, 73%) as a diastereomeric mixture.
1H-NMR(270MHz, $CDCl_3$)δ: 0.50-0.62(2/3H, m), 0.73(2H, s), 0.78(1H, s), 0.84-1.00(2/3H, m), 1.11(1H, m), 1.15(2H, m), 1.04-2.44(58/3H, m), 2.90-3.00(1/3H, m), 3.42-3.58(1H, m), 3.78(2H, s), 3.79(1H, s), 6.70-7.30(4H, m).
Rf value (on silica gel plate, developing solvents: ethyl acetate/n-hexane = 1/1): 0.48

(Step 3)

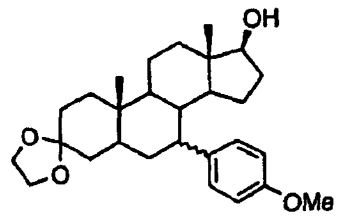

Synthesis of 3,3-ethylenedioxy-17β-hydroxy-7-(4-methoxyphenyl)-5α-androstane

17β-Hydroxy-7-(4-methoxyphenyl)-5α-androstan-3-one (49.7 mg) was dissolved in benzene (2 ml) and, after adding ethylene glycol (0.5 ml) and p-toluenesulfonic acid (2.2 mg), the mixture was heated under reflux as water was continuously removed by means of a Dean-Stark trap. After one hour, the reaction mixture was cooled to 0°C and, after adding a saturated aqueous solution of sodium hydrogencarbonate, extraction with ethyl acetate was conducted. The organic layer was washed with a saturated aqueous solution of sodium chloride and dried with magnesium sulfate; after filtering, the solvent was distilled off at reduced pressure to give the end compound in 55.0 mg (yield, 100%) as a diastereomeric mixture.
1H-NMR(270MHz, $CDCl_3$)δ: 0.54-0.60(2/3H, m), 0.70(2H, s), 0.75(1H, s), 0.91(1H, s), 0.95(2H, s), 0.90-1.98(58/3H, m), 2.22-2.38(2/3H, m), 2.86-2.94(1/3H, m), 3.44-3.58(1H, m), 3.72-3.98(4H, m), 3.78(2H, s), 3.80(1H, s), 6.70-7.30 (4H, m).
Rf value (on silica gel plate, developing solvents: ethyl acetate/n-hexane = 1/1): 0.52

(Step 4)

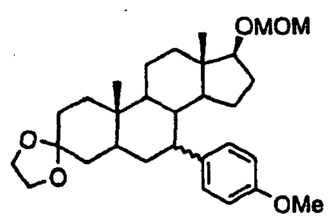

Synthesis of 3,3-ethylenedioxy-17β-methoxymethoxy-7-(4-methoxyphenyl)-5α-androstane

To a solution of 3,3-ethylenedioxy-17β-hydroxy-7-(4-methoxyphenyl)-5α-androstane (55.0 mg) in dichloromethane (2 ml), diisopropylethylamine (0.128 ml) and chloromethyl methyl ether (0.047 ml) were added dropwise at 0°C. After stirring for 12 hours at 25°C, a saturated aqueous solution of sodium hydrogencarbonate was added to the reaction mixture and extraction with dichloromethane was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 1/4) gave the end compound in 56.2 mg (yield, 93%) as a diastereomeric mixture.
1H-NMR(270MHz, $CDCl_3$)δ: 0.42-0.60(2/3H, m), 0.74(2H, s), 0.78(1H, s), 0.80-0.90(2/3H, m), 0.91(1H, s), 0.94(2H, s), 1.00-1.96(56/3H, m), 2.22-2.36(2/3H, m), 2.84-2.92(1/3H, m), 3.30(3H, s), 3.34-3.44(1H, m), 3.78(2H, s), 3.80(1H, s), 3.82-3.98(4H, m), 4.56(2H, s), 6.70-7.30(4H, m).
Rf value (on silica gel plate, developing solvents: ethyl acetate/n-hexane = 1/1): 0.68

(Step 5)

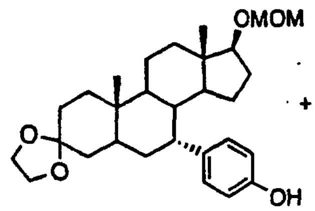

+

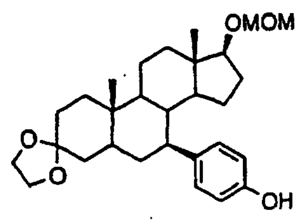

Synthesis of 3,3-ethylenedioxy-17β-methoxymethoxy-7α-(4-hydroxyphenyl)-5α-androstane and 3,3-ethylenedioxy-17β-methoxymethoxy-7β-(4-hydroxyphenyl)-5α-androstane

To a solution of 3,3-ethylenedioxy-17β-methoxymethoxy-7-(4-methoxyphenyl)-5α-androstane (151 mg) in N,N-dimethylacetamide (3 ml), sodium thiomethylate (109 mg) was added and the mixture was heated under reflux. After 3 hours, the reaction mixture was cooled to 0°C and, after adding water, extraction with ethyl acetate was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 1/4) gave the end compound as both 7α form in 42.3 mg (yield, 29%) and 7β form in 88.0 mg (yield, 60%). 3,3-ethylenedioxy-17β-methoxymethoxy-7α-(4-hydroxyphenyl)-5α-androstane;
1H-NMR(270MHz, $CDCl_3$)δ: 0.78(3H, s), 0.90(3H, s), 1.00-2.08(20H, m), 2.84-2.92(1H, m), 3.30(3H, s), 3.38(1H, t, J=8.6Hz), 3.80-3.94(4H, m), 4.56(2H, s), 4.64(1H, s), 6.72(2H, d, J=8.4Hz), 7.23(2H, d, J=8.4Hz).
Rf value (on silica gel plate, developing solvents: ethyl acetate/n-hexane = 1/4): 0.20
3,3-ethylenedioxy-17β-methoxymethoxy-7β-(4-hydroxyphenyl)-5α-androstane;
1H-NMR(270MHz, $CDCl_3$)δ: 0.46-0.60(1H, m), 0.73(3H, s), 0.94(3H, s), 0.82-1.96(19H, m), 2.22-2.34(1H, m), 3.30 (3H, s), 3.38(1H, t, J=8.6Hz), 3.93(4H, brs), 4.56(2H, s), 4.68(1H, s), 6.66(1H, dd, J=2.3, 8.2Hz), 6.74(1H, dd, J=2.3, 8.2Hz), 6.92(1H, dd, J=1.8, 8.2Hz), 7.07(1H, dd, J=1.8, 8.2Hz).
Rf value (on silica gel plate, developing solvents: ethyl acetate/n-hexane = 1/4): 0.28

(Step 6)

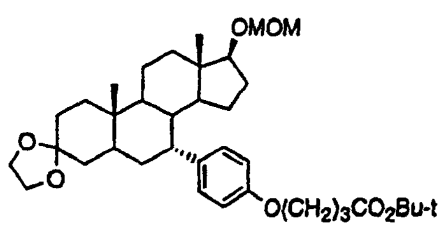

Synthesis of 3,3-ethylenedioxy-17β-methoxymethoxy-7α-{4-(3-t-butoxycarboxypropoxy)phenyl}-5α-androstane

To an N,N-dimethylacetamide solution (0.5 ml) of 3,3-ethylenedioxy-17β-methoxymethoxy-7α-(4-hydroxyphenyl)-5α-androstane (22.0 mg), t-butyl 4-bromobutyrate (31.3 mg), potassium carbonate (64.5 mg) and 18-crown-6 (123 mg) were added. After one hour, water was added to the reaction mixture and extraction with ethyl acetate was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 1/8) gave the end compound in 27.8 mg (yield, 99%).
1H-NMR(270MHz, $CDCl_3$)δ: 0.78(3H, s), 0.90(3H, s), 1.04-2.12(22H, m), 1.46(9H, s), 2.43(2H, t, J=7.3Hz), 2.82-2.92 (1H, m), 3.30(3H, s), 3.38(1H, t, J=8.4Hz), 3.80-3.94(4H, m), 3.97(2H, t, J=6.1Hz), 4.56(2H, s), 6.78(2H, d, J=8.6Hz), 7.26(2H, d, J=8.6Hz).

Rf value (on silica gel plate, developing solvents: ethyl acetate/n-hexane = 1/4): 0.40

(Step 7)

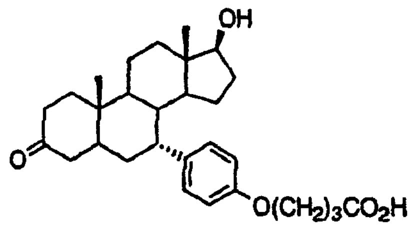

Synthesis of 17β-hydroxy-7α-{4-(3-carboxygropoxy)phenyl}-5α-androstan-3-one

3,3-Ethylenedioxy-17β-methoxymethoxy-7α-(4-(3-methoxycarbonylpropoxy)phenyl)-5α-androstane (27.6 mg) was dissolved in acetone (2 ml) and, after adding 2 N-hydrochloric acid (0.5 ml), the mixture was heated at 60°C. After two hours, water was added to the reaction mixture and extraction with dichloromethane was conducted. The organic layer was washed with a saturated aqueous solution of sodium chloride and dried with magnesium sulfate; after filtering, the solvent was distilled off at reduced pressure. Purification by silica gel column chromatography (developing solvents: dichloromethane/methanol = 10/1) gave the end compound in 19.2 mg (yield, 89%).
1H-NMR(270MHz, $CDCl_3$)δ: 0.78(3H, s), 1.10(3H, s), 1.00-2.44(22H, m), 2.60(2H, t, J=7.3Hz), 2.88-2.96(1H, m), 3.50 (1H, t, J=8.2Hz), 4.00(2H, t, J=5.9Hz), 6.77(2H, d, J=8.6Hz), 7.20(2H, d, J=8.6Hz).
Mass (FAB): 469(M+1).
Rf value (on silica gel plate, developing solvent: ethyl acetate): 0.54
The following compounds were synthesized by similar methods to Example 133.

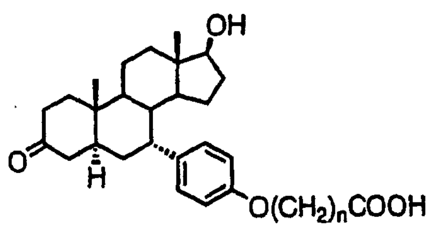

| Example | n | MW | Mass (FAB) |
|---|---|---|---|
| 134 | 1 | 440 | 441 |
| 135 | 7 | 524 | 525 |

[Example 136]

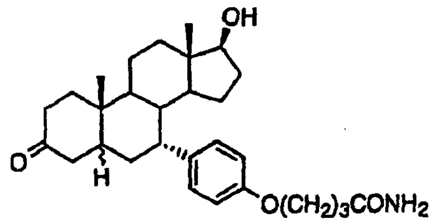

Synthesis of 17β-hydroxy-7α-{4-(3-carbamoylpropoxy)phenyl}-5α-androstan-3-one

The 17β-hydroxy-7α-{4-(3-carboxypropoxy)phenyl}-5α-androstan-3-one obtained in Example 133 was dissolved in tetrahydrofuran (0.5 ml) and then triethylamine (3.2 μl) and ethyl chloroformate (1.8 μl) were added dropwise under cooling with ice. After stirring for 5 minutes, ammonia gas was bubbled for 1 minute. After stirring for 15 minutes, water was added to the reaction mixture and extraction with dichloromethane was conducted. The organic layer was washed with a saturated aqueous solution of sodium chloride and dried with magnesium sulfate; after filtering, the solvent was distilled off at reduced pressure. Purification by preparative chromatography (developing solvents: dichloromethane/methanol = 20/1) gave the end compound in 6.6 mg (yield, 90%).
1H-NMR(270MHz, $CDCl_3$)δ: 0.78(3H, s), 1.11(3H, s), 1.00-2.44(22H, m), 2.45(2H, t, J=7.1Hz), 2.88-2.98(1H, m), 3.50 (1H, t, J=8.2Hz), 4.01(2H, t, J=5.7Hz), 5.30-5.60(2H, m), 6.77(2H, d, J=8.6Hz), 7.20(2H, d, J=8.6Hz).
Mass (FAB): 468(M+1).
Rf value (on silica gel plate, developing solvents: dichloromethane/methanol = 20/1): 0.14

The following compounds were synthesized by similar methods to Example 136.

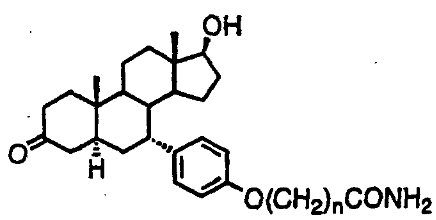

| Example | n | MW | Mass (FAB) |
|---|---|---|---|
| 137 | 1 | 439 | 440 |
| 138 | 7 | 523 | 524 |

[Example 139]

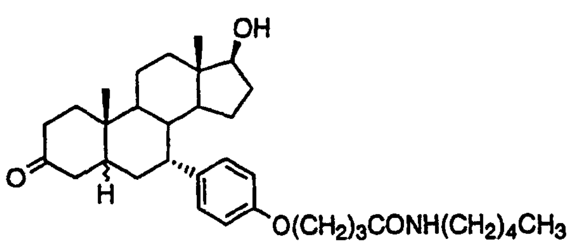

Synthesis of 17β-hydroxy-7α-4-(3-(N-pentyl carbamoyl)propoxy}phenyl)-5α-androstan-3-one

The 17β-hydroxy-7α-{4-(3-carboxypropoxy)phenyl}-5α-androstan-3-one (7.0 mg) obtained in Example 133 was dissolved in tetrahydrofuran (0.5 ml) and then 1-ethyl-3-(3-dimethylaminopropyl)carbodiimide hydrochloride (8.6 mg), 1-hydroxybenzotriazole monohydrate (6.8 mg) and pentylamine (10.4 ml) were added at 25°C. After stirring for 4 hours, a saturated aqueous solution of sodium hydrogencarbonate was added to the reaction mixture and extraction with ethyl acetate was conducted. The organic layer was washed with a saturated aqueous solution of sodium chloride and dried with magnesium sulfate; after filtering, the solvent was distilled off at reduced pressure. Purification by preparative chromatography (developing solvent: ethyl acetate) gave the end compound in 5.8 mg (yield, 72%).
1H-NMR(270MHz, $CDCl_3$)δ: 0.78(3H, s), 0.80-0.94(3H, m), 1.11(3H, s), 1.00-2.44(30H, m), 2.88-2.98(1H, m), 3.24 (2H, dt, J=6.1, 7.1Hz), 3.50(1H, t, J=8.3Hz), 3.99(2H, t, J=5.8Hz), 5.50(1H, brs), 6.77(2H, d, J=8.6Hz), 7.20(2H, d, J=8.6Hz).
Mass (FAB): 538(M+1).
Rf value (on silica gel plate, developing solvent: ethyl acetate): 0.62
The following compound was synthesized by a similar method to Example 139.

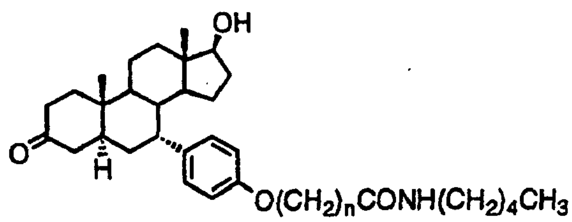

| Example | n | MW | Mass (FAB) |
|---|---|---|---|
| 140 | 7 | 593 | 594 |

[Example 141]

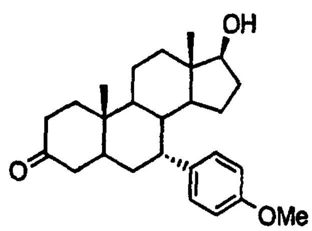

Synthesis of 17β-hydroxy-7α-(4-methoxyphenyl)-5α-androstan-3-one

(Step 1)

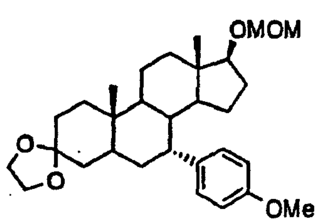

Synthesis of 3,3-ethylenedioxy-17β-methoxymethoxy-7α-(4-methoxyphenyl)-5α-androptane

The 3,3-ethylenedioxy-17β-methoxymethoxy-7α-(4-hydroxyphenyl)-5α-androstane (10.0 mg) obtained in step 5 of Example 136 was dissolved in N,N-dimethylformamide (1 ml) and then 60% sodium hydride (2.5 mg) and iodomethane (13.2 μl) were added at 0°C. After stirring for 13 hours at 25°C, a saturated aqueous solution of $NH_4Cl$ was added to the reaction mixture and extraction with ethyl acetate was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 1/4) gave the end compound in 10.2 mg (yield, 99%).
1H-NMR(270MHz, $CDCl_3$)δ: 0.78(3H, s), 0.91(3H, s), 1.00-1.96(20H, m), 2.84-2.92(1H, m), 3.30(3H, s), 3.38(1H, t, J=8.6Hz), 3.80(3H, s), 3.82-3.92(4H, m), 4.56(2H, s), 6.80(2H, d, J=8.6Hz), 7.27(2H, d, J=8.6Hz).
Rf value (on silica gel plate, developing solvents: ethyl acetate/n-hexane = 1:1): 0.68

(Step 2)

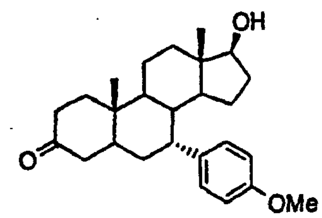

Synthesis of 17β-hydroxy-7α-(4-methoxyphenyl)androstan-3-one

3,3-Ethylenedioxy-17β-methoxymethoxy-7α-(4-methoxyphenyl)androstane (10.2 mg) was dissolved in acetone (2 ml) and, after adding 2 N-hydrochloric acid (0.5 ml), the mixture was heated under reflux. After 2 hours, water was added to the reaction mixture and extraction with dichloromethane was conducted. The organic layer was washed with a saturated aqueous solution of sodium chloride and dried with magnesium sulfate; after filtering, the solvent was distilled off at reduced pressure.
Purification by silica gel column chromatography (developing solvents: ethyl acetate/n-hexane = 1/1) gave the end compound in 7.2 mg (yield, 85%).
1H-NMR(270MHz, $CDCl_3$)δ: 0.78(3H, s), 1.11(3H, s), 1.00-2.50(20H, m), 2.88-2.96(1H, m), 3.50(1H, t, J=8.6Hz), 3.79 (3H, s), 6.79(2H, d, J=8.7Hz), 7.22(2H, d, J=8.7Hz). Mass (FAB): 397(M+1).
Rf value (on silica gel plate, developing solvents: ethyl acetate/n-hexane = 1/1): 0.48

[Example 142]

Synthesis of 17β-hydroxy-11β-{13-(4,4,5,5,5-pentafluoropentylsulfinyl)tridecyloxy}-5α-androstan-3-one

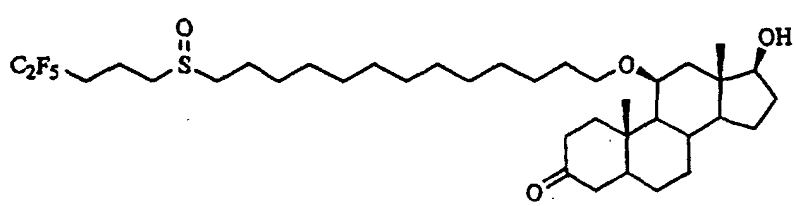

(Step 1)

3.3-ethylenedioxy-17β-methoxymethoxy-11β-(13-bromo-2-tridecenyloxy)-5α-androstane

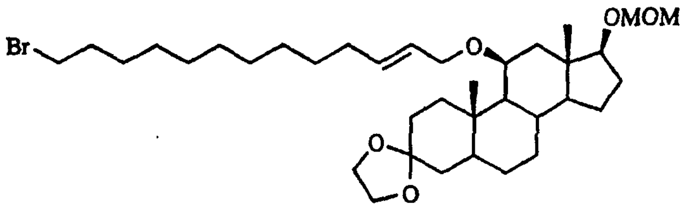

In an argon atmosphere, the 3,3-ethylenedioxy-17β-(methoxymethoxy)-11β-(2-propen-1-yloxy)-5α-androstane (100.8 mg) obtained in step 2 of Example 104, 12-bromododecan-1-ene (114.7 mg) and benzylidenebistricyclohexylphosphine dichlororuthenium (19.1 mg) were dissolved in toluene (2.3 ml) and the mixture was stirred for 26 hours at 110 °C. The solvent was distilled off at reduced pressure and purification by silica gel column chromatography (developing solvents: ethyl acetate/n-hexane = 1/5) gave the end compound in 98.6 mg (yield, 65%).
1H-NMR(270MHz, $CDCl_3$)δ: 0.95(3H, s), 1.02(3H, s), 0.70-2.38(38H, m), 3.36(3H, s), 3.93(4H, s), 3.31-4.12(6H, m), 4.56-4.67(2H, m), 5.38-5.69(2H, m).
Rf value (on silica gel plate, developing solvents: ethyl acetate/hexane = 1/4): 0.49

(Step 2)

3,3-ethylenedioxy-17β-methoxymethoxy-11β-{13-(4,4,5,5,5-pentafluoropentylthio)-2-tridecenyloxy}-5α-androstane

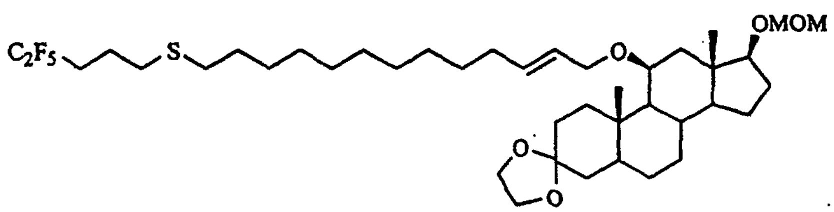

In a nitrogen atmosphere, 3,3-ethylenedioxy-17β-methoxymethoxy-11β-(13-bromo-2-tridecenyloxy)-5α-androstane (98.6 mg), 4,4,5,5,5-pentafluoropentane-1-thiol acetate (71.2 mg) and sodium methylate (1.0 M methanol solution) (0.30 ml) were dissolved in methanol (1.5 ml) and tetrahydrofuran (0.8 ml) and the mixture was stirred for 11 hours at room temperature. After adding water to the reaction mixture, extraction with ethyl acetate was conducted. The organic layer was washed with a saturated aqueous solution of sodium chloride and dried with magnesium sulfate; then, the solvent was distilled off at reduced pressure. Purification by silica gel column chromatography (developing solvents: ethyl acetate/n-hexane = 1/4) gave the end compound in 97.3 mg (yield, 84%).
1H-NMR(270MHz, $CDCl_3$)δ: 0.95(3H, s), 1.02(3H, s), 0.69-2.38(42H, m), 2.50(2H, t, J=7.3Hz), 2.59(2H, t, J=6.9Hz), 3.36(3H, s), 3.47(1H, t, J=8.2Hz), 3.59-4.17(3H, m), 3.93(4H, s), 4.57-4.68(2H, m), 5.39-5.70(2H, m).
Rf value (on silica gel plate, developing solvents: ethyl acetate/hexane = 1/4): 0.42

(Step 3)

3,3-ethylenedioxy-17β-methoxymethoxy-11β-{13-(4,4,5,5,5-pentafluoropentylsulfinyl)-2-tridecenyloxy}-5α-androstane

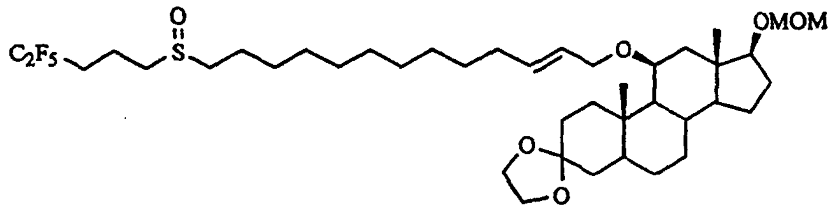

3,3-Ethylenedioxy-17β-methoxymethoxy-11β-{13-(4,4,5,5,5-pentafluoropentylthio)-2-tridecenyloxy}-5α-androstane (50.1 mg) was dissolved in tetrahydrofuran (0.6 ml) and, after adding oxone (20.1 mg) and water (0.3 ml) under cooling with ice, the mixture was stirred for 1 hour. After adding a saturated aqueous solution of sodium hydrogencarbonate to the reaction mixture, extraction with ethyl acetate was conducted. The organic layer was washed with a saturated aqueous solution of sodium chloride and dried with magnesium sulfate; then, the solvent was distilled off at reduced pressure. Purification by silica gel column chromatography (developing solvents: ethyl acetate/n-hexane = 3/2) gave the end compound in 29.4 mg (yield, 57%).
1H-NMR(270MHz, $CDCl_3$)δ: 0.95(3H, s), 1.02(3H, s), 0.70-2.37(42H, m), 2.58-2.82(4H, m), 3.36(3H, s), 3.46(1H, t, J=8.2Hz), 3.93(4H, s), 3.59-4.17(3H, m), 4.57-4.68(2H, m), 5.35-5.70(2H, m).
Rf value (on silica gel plate, developing solvents: ethyl acetate/hexane = 1/2): 0.12

(Step 4)

17β-hydroxy-11β-{13-(4,4,5,5,5-pentafluoropentylsulfinyl)tridecyloxy}-5α-androstan-3-one

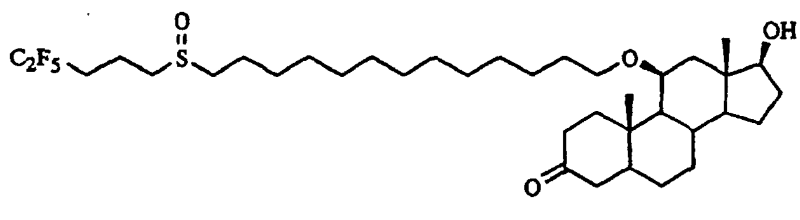

3,3-Ethylenedioxy-17β-methoxymethoxy-11β-{13-(4,4,5,5,5-pentafluoropentylsulfinyl)-2-tridecenyloxy}-5α-androstane (29.4 mg) was dissolved in ethyl acetate (1 ml) and, after adding 10% palladium/carbon (10 mg), the mixture was stirred for 3 hours at room temperature in a hydrogen atmosphere. After filtering the reaction mixture, the solvent was distilled off at reduced pressure and the resulting residue was dissolved in acetone (2 ml); after adding 1 N-hydrochloric acid (1 ml), the mixture was heated under reflux for 3 hours. After standing to cool, a saturated aqueous solution of sodium hydrogencarbonate was added to the reaction mixture and extraction with ethyl acetate was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 2/1) gave the end compound in 20.5 mg (yield, 78%).
1H-NMR(270MHz, $CDCl_3$)δ: 0.94(3H, s), 1.24(3H, s), 0.68-2.54(47H, m), 2.58-2.83(4H, m), 3.04-3.16(1H, m), 3.51-3.63(2H, m), 3.72-3.79(1H, m).
Mass (FAB): 697(M+1).

The following compounds were synthesized by similar methods to Example 142.

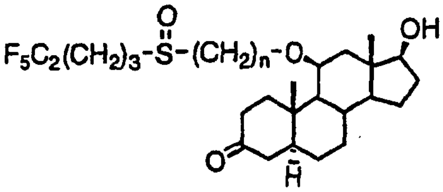

| Example | n | MW | Mass (FAB) |
|---|---|---|---|
| 143 | 5 | 584 | 585 |
| 144 | 7 | 612 | 613 |
| 145 | 9 | 640 | 641 |
| 146 | 11 | 668 | 669 |

[Example 147]

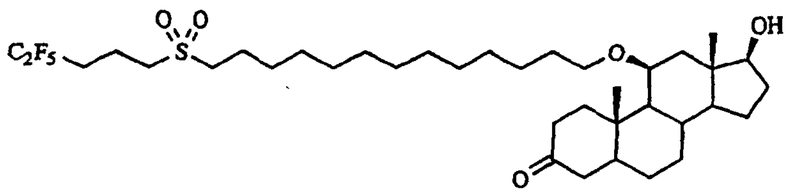

Synthesis of 17β-hydroxy-11β-{13-(4,4,5,5,5-pentafluoropentylsulfonyl)tridecyloxy}-5α-androstan-3-one

(Step 1)

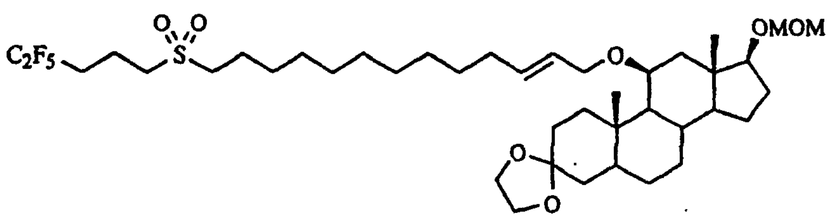

3,3-ethylenedioxy-17β-methoxymethoxy-11β-{13-(4,4,5,5,5-pentafluoropentylsulfonyl)-2-tridecenyloxy}-5α-androstane

3,3-Ethylenedioxy-17β-methoxymethoxy-11β-{13-(4,4,5,5,5-pentafluoropentylthio)-2-tridecenyloxy}-5α-androstane (47.2 mg) was dissolved in tetrahydrofuran (0.6 ml) and, after adding OXONE (75.7 mg) and water (0.3 ml) at room temperature, the mixture was stirred for 1 hour. After adding a saturated aqueous solution of sodium hydrogencarbonate to the reaction mixture, extraction with ethyl acetate was conducted. The organic layer was washed with a saturated aqueous solution of sodium chloride and dried with magnesium sulfate; then, the solvent was distilled off at reduced pressure. Purification by silica gel column chromatography (developing solvents: ethyl acetate/n-hexane =

1/3) gave the end compound in 33.6 mg (yield, 68%).
1H-NMR(270MHz, $CDCl_3$)δ: 0.95(3H, s), 1.02(3H, s), 0.70-2.39(42H, m), 2.92-3.12(4H, m), 3.36(3H, s), 3.46(1H, t, J=8.1Hz), 3.93(4H, s), 3.59-4.18(3H, m), 4.57-4.68(2H, m), 5.37-5.70(2H, m).
Rf value (on silica gel plate, developing solvents: ethyl acetate/hexane = 1/2): 0.47

(Step 2)

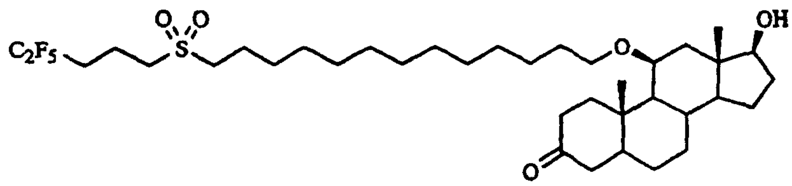

17β-hydroxy-11β-{13-(4,4,5,5,5-pentafluoropentylsulfonyl)tridecyloxy}-5α-androstan-3-one

3,3-Ethylenedioxy-17β-methoxymethoxy-11β-{13-(4,4,5,5,5-pentafluoropentylsulfonyl)-2-tridecenyloxy}-5α-androstane (33.6 mg) was dissolved in ethyl acetate (1 ml) and, after adding 10% palladium/carbon (10 mg), the mixture was stirred for 4 hours at room temperature in a hydrogen atmosphere. After filtering the reaction mixture, the solvent was distilled off at reduced pressure and the resulting residue was dissolved in acetone (2 ml); after adding 1 N-hydrochloric acid (1 ml), the mixture was heated under reflux for 4 hours. After standing to cool, a saturated aqueous solution of sodium hydrogencarbonate was added and extraction with ethyl acetate was effected. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 2/3) gave the end compound in 22.9 mg (yield, 76%).
1H-NMR(270MHz, $CDCl_3$)δ: 0.94(3H, s), 1.24(3H, s), 0.68-2.54(47H, m), 2.92-3.15(5H, m), 3.51-3.64(2H, m), 3.72-3.78(1H, m).
Mass (FAB): 713(M+1).
The following compounds were synthesized by similar methods to Example 147.

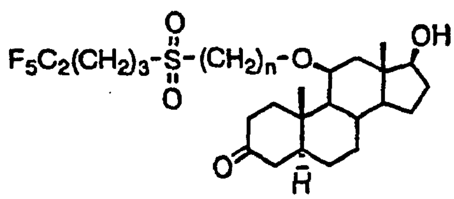

| Example | n | MW | Mass (FAB) |
|---|---|---|---|
| 148 | 7 | 628 | 629 |
| 149 | 9 | 656 | 657 |
| 150 | 11 | 684 | 685 |

[Example 151]

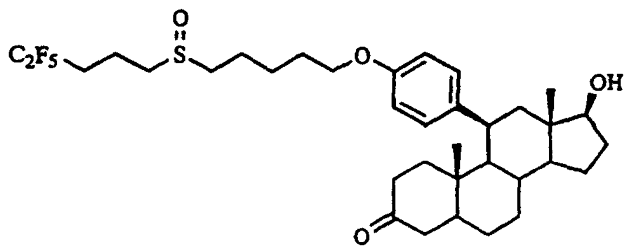

17β-hydroxy-11β-[4-{5-(4,4,5,5,5-pentafluoropentylsulfinyl)pentyloxy}phenyl]-5α-androstan-3-one

(Step 1)

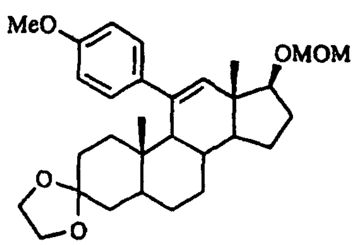

3,3-ethylenedioxy-17β-methoxymethoxy-11-(4-methoxyphenyl)-5α-androst-11-ene

A mixture of 3,3-ethylenedioxy-17β-methoxymethoxy-11-[{1,1,2,2,3,3,4,4,4-nonafluorobutyl)sulfonyl}oxy]-5α-androst-11-ene (98.8 mg), 4-methoxyphenylboronic acid (223 mg), tetrakistriphenylphosphine palladium (6.8 mg), lithium chloride (12.4 mg), 2 M aqueous solution of sodium carbonate (0.5 ml), toluene (2 ml) and ethanol (1 ml) was heated under reflux for 13 hours in an argon atmosphere. After adding a saturated aqueous solution of sodium hydrogencarbonate to the reaction mixture, extraction with ethyl acetate was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 1/6) gave the end compound in 65.4 mg (yield, 93%).
1H-NMR(270MHz, $CDCl_3$)δ: 0.59-2.28(18H, m), 0.85(3H, s), 0.94(3H, s), 3.31(3H, s), 3.63(1H, t, J=8.0Hz), 3.78(3H, s), 3.80-3.96(4H, m), 4.57(1H, d, J=6.6Hz), 4.61(1H, d, J=6.6Hz), 5.86(1H, d, J=1.7Hz), 6.68-6.83(2H, m), 6.95-7.08 (2H, m).
Rf value (on silica gel plate, developing solvents: ethyl acetate/hexane = 1/4): 0.40

(Step 2)

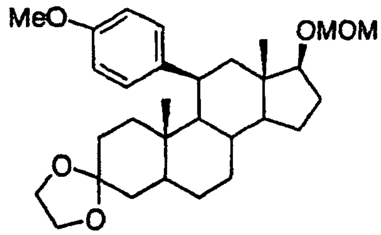

3,3-ethylenedioxy-17β-methoxymethoxy-11β-(4-methoxyphenyl)-5α-androstane

3,3-Ethylenedioxy-17β-methoxymethoxy-11-(4-methoxyphenyl)-5α-androst-11-ene (29.9 mg) was dissolved in ethyl acetate (2 ml) and, after adding acetic acid (0.2 ml) and 10%-palladium/carbon (30 mg), the mixture was stirred for 3 hours at 25°C under hydrogen pressure (25 atm). After filtering the reaction mixture, the solvent was distilled off at reduced pressure and the reaction mixture was purified by silica gel column chromatography (developing solvents: ethyl acetate/dichloromethane = 1/20) to give the end compound in 20.1 mg (yield, 67%).
1H-NMR(300MHz, $CDCl_3$)δ: 7.40-7.25(2H, m), 6.75(2H, d, J=8.2Hz), 4.55(3H, s), 3.92(4H, s), 3.78(3H, s), 3.42(1H, dd, J=6.8, 6.6Hz), 3.38-3.28(1H, m), 3.28(3H, s), 2.40-0.80(20H, m), 0.76(3H, s), 0.65(3H, s).
Mass (EI): 484(M+).

(Step 3)

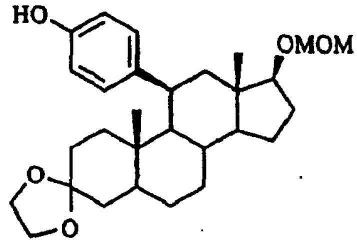

3,3-ethylenedioxy-17β-methoxymethoxy-11β-(4-hydroxyghenyl)-5α-androstane

3,3-Ethylenedioxy-17β-methoxymethoxy-11β-(4-methoxyphenyl)-5α-androstane (114.8 mg) and a solution of sodium methanethiolate (69.9 mg) in dimethylformamide (3 mml) were heated under reflux for 1 hour in a nitrogen atmosphere. After standing to cool, a saturated aqueous solution of ammonium chloride was added to the reaction mixture and extraction with ethyl acetate was effected. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 1/2) gave the end compound in 105.2 mg (yield, 94%).
1H-NMR(270MHz, $CDCl_3$)δ: 0.65(3H, s), 0.75(3H, s), 0.90-2.19(20H, m), 3.28(3H, s), 3.24-3.34(1H, m), 3.43(1H, t, J=8.1Hz), 3.91(4H, s), 4.54(2H, s), 4.64(1H, s), 6.64(2H, d, J=8.7Hz), 7.13-7.32(2H, m).
Rf value (on silica gel plate, developing solvents: ethyl acetate/hexane = 1/2): 0.29

(Step 4)

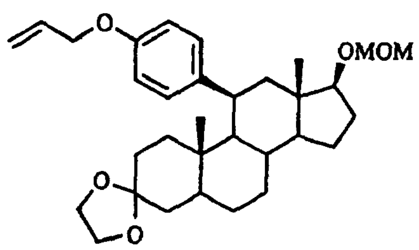

3,3-ethylenedioxy-17β-methoxymethoxy-11β-{4-(2-propen-1-yloxy)phenyl}-5α-androstane

To a solution of 3,3-ethylenedioxy-17β-methoxymethoxy-11β-(4-hydroxyphenyl)-5α-androstane (56.2 mg) in dimethylformamide (2 ml), sodium hydride (9.6 mg) was added under cooling with ice and the mixture was stirred for 5 minutes. After adding allyl bromide (28.9 mg), the mixture was stirred for 1 hour under cooling with ice. After adding water to the reaction mixture, extraction with ethyl acetate was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 1/2) gave the end compound in 51.0 mg (yield, 84%).
1H-NMR(270MHz, $CDCl_3$)δ: 0.65(3H, s), 0.76(3H, s), 0.82-2.18(20H, m), 3.28(3H, s), 3.24-3.36(1H, m), 3.43(1H, t, J=8.0Hz), 3.90(4H, s), 4.49(2H, d, J=5.3Hz), 4.53(2H, s), 5.26(1H, dd, J=10.5, 1.2Hz), 5.40(1H, dd, J=17.3, 1.5Hz), 5.98-6.13(1H, m), 6.72(2H, d, J=8.7Hz), 7.26(2H, brs).
Rf value (on silica gel plate, devloping solvents: ethyl acetate/hexane = 1/2): 0.59

(Step 5)

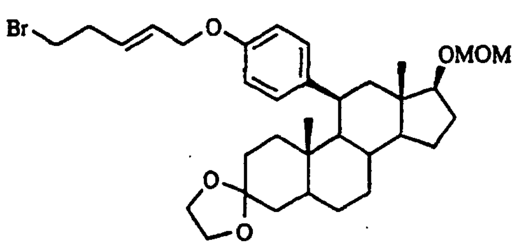

3,3-ethylenedioxy-17β-methoxymethoxy-11β-{4-(5-bromo-2-penten-1-yloxy)phenyl}-5α-androstane

In an argon atmosphere, 3,3-ethylenedioxy-17β-methoxymethoxy-11β-(4-(2-propen-1-yloxy)phenyl)-5α-androstane (16.0 mg), 4-bromo-1-butene (8.5 mg) and benzylidenebistricyclohexylphosphine dichlororuthenium (2.6 mg) were dissolved in dichloromethane (0.3 ml) and the mixture was stirred for 16.5 hours at room temperature. The solvent was distilled off at reduced pressure and purification by silica gel column chromatography (developing solvents: ethyl acetate/n-hexane = 1/4) gave the end compound in 14.4 mg (yield, 74%).
1H-NMR(270MHz, $CDCl_3$)δ: 0.65(3H, s), 0.76(3H, s), 0.81-2.18(20H, m), 2.60-2.78(2H, m), 3.28(3H, s), 3.41(2H, t, J=6.9Hz), 3.25-3.34(1H, m), 3.37-3.48(1H, m), 3.90(4H, s), 4.43-4.51(2H, m), 4.54(2H, s), 5.77-5.88(2H, m), 6.71(2H, d, J=8.7Hz), 7.26(2H, brs).
Rf value (on silica gel plate, developing solvents: ethyl acetate/hexane = 1/4): 0.48

(Step 6)

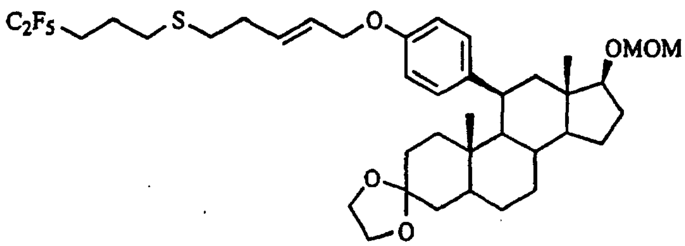

3,3-ethylenedioxy-17β-methoxymethoxy-11β-[4-{5-(4,4,5,5,5-pentafluoropentylsulfonyl)-2-pentenyloxy}phenyl]-5α-androstane

In a nitrogen atmosphere, 3-ethylenedioxy-17β-methoxymethoxy-11β-{4-(5-bromo-2-penten-1-yloxy)phenyl}-5α-androstane (14.4 mg), 4,4,5,5,5-pentafluoropentane-1-thiol acetate (11.0 mg) and sodium methylate (1.0 M methanol solution) (0.05 ml) were dissolved in methanol (0.2 ml) and tetrahydrofuran (0.2 ml) and the solution was stirred for 17 hours at room temperature. After adding water to the reaction mixture, extraction with ethyl acetate was conducted. The organic layer was washed with a saturated aqueous solution of sodium chloride and dried with magnesium sulfate; then, the solvent was distilled off at reduced pressure. Purification by silica gel column chromatography (developing solvents: ethyl acetate/n-hexane = 1/4) gave the end compound in 14.8 mg (yield, 87%).
1H-NMR(270MHz, $CDCl_3$)δ: 0.65(3H, s), 0.75(3H, s), 0.81-2.49(26H, m), 2.54-2.67(4H, m), 3.28(3H, s), 3.43(1H, t, J=7.9Hz), 3.24-3.37(1H, m), 3.90(4H, s), 4.45(2H, d, J=4.8Hz), 4.53(2H, s), 5.66-5.94(2H, m), 6.71(2H, d, J=8.6Hz), 7.26(2H, brs).
Rf value (on silica gel plate, developing solvents: ethyl acetate/hexane = 1/4): 0.48

(Step 7)

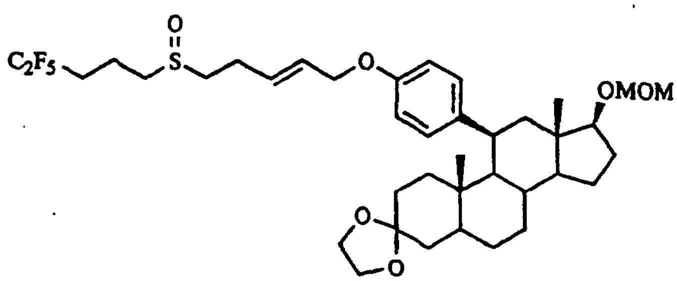

3,3-ethylenedioxy-17β-methoxymethoxy-11β-[4-{5-(4,4,5,5,5-pentafluoropentylsulfinyl)-2-pentenyloxy}phenyl]-5α-androstane

3,3-Ethylenedioxy-17β-methoxymethoxy-11β-[4-{5-(4,4,5,5,5-pentafluoropentylsulfonyl)-2-pentenyloxy}phenyl]-5α-androstane (14.8 mg) was dissolved in tetrahydrofuran (0.5 ml) and, after adding OXONE (6.2 mg) and water (0.3 ml) under cooling with ice, the mixture was stirred for 50 minutes. After adding a saturated aqueous solution of sodium hydrogencarbonate to the reaction mixture, extraction with ethyl acetate was effected. The organic layer was washed with a saturated aqueous solution of sodium chloride and dried with magnesium sulfate; then, the solvent was distilled off at reduced pressure. Purification by silica gel column chromatography (developing solvents: ethyl acetate/n-hexane = 4/1) gave the end compound in 14.2 mg (yield, 94%).
1H-NMR(270MHz, $CDCl_3$)δ: 0.65(3H, s), 0.75(3H, s), 0.82-2.37(24H, m), 2.55-2.90(6H, m), 3.28(3H, s), 3.43(1H, t, J=7.8Hz), 3.24-3.37(1H, m), 3.90(4H, s), 4.38-4.51(2H, m), 4.53(2H, s), 5.68-5.95(2H, m), 6.71(2H, d, J=8.6Hz), 7.26(2H, brs).
Rf value (on silica gel plate, developing solvents: ethyl acetate/hexane = 1/2): 0.07

(Step 8)

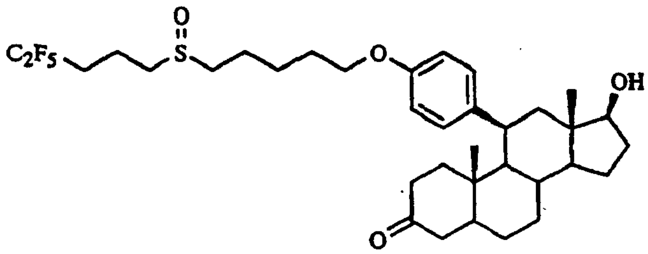

17β-hydroxy-11β-[4-{5-(4,4,5,5,5-pentafluoropentylsulfinyl)pentyloxy}phenyl]-5α-androstan-3-one

3,3-Ethylenedioxy-17β-methoxymethoxy-11β-[4-{5-(4,4,5,5,5-pentafluoropentylsulfinyl)-2-pentenyloxy}phenyl]-5α-androstane (14.2 mg) was dissolved in ethyl acetate (1 ml) and, after adding 10% palladium/carbon (10 mg), the mixture was stirred for 2 hours at room temperature in a hydrogen atmosphere. After filtering the reaction mixture, the solvent was distilled off at reduced pressure and the resulting residue was dissolved in acetone (2 ml); after adding 1 N-hydrochloric acid (1 ml), the reaction mixture was heated under reflux for 1.5 hours. After standing to cool, a saturated aqueous solution of sodium hydrogencarbonate was added and extraction with ethyl acetate was effected. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 4/1) gave the end compound in 11.5 mg (yield, 92%).
1H-NMR(270MHz, $CDCl_3$)δ: 0.75(3H, s), 0.86(3H, s), 0.82-2.37(31H, m), 2.62-2.87(4H, m), 3.28-3.40(1H, m), 3.48-3.58(1H, m), 3.96(2H, t, J=6.0Hz), 6.72(2H, d, J=8.7Hz), 7.26(2H, brs).
Mass (FAB): 661(M+1).
The following compound was synthesized by a similar method to Example 151.

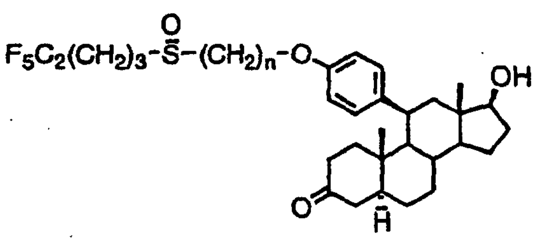

| Example | n | MW | Mass(ESI) |
|---|---|---|---|
| 152 | 7 | 688 | 689 |

[Example 153]

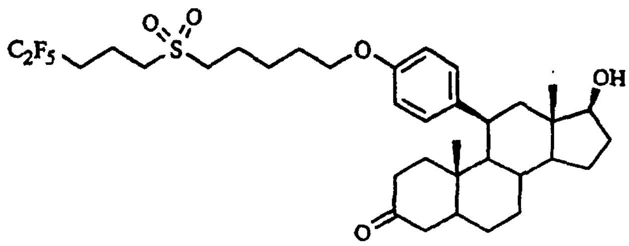

Synthesis of 17β-hydroxy-11β-[4-{5-(4,4,5,5,5-pentafluoronentylsulfonyl)pentyloxy}phenyl]-5α-androstan-3-one

The 17β-hydroxy-11β-[4-{7-(4,4,5,5,5-pentafluoropentylsulfinyl)pentyloxy}phenyl]-5α-androstan-3-one (3.0 mg) obtained in Example 151 was dissolved in tetrahydrofuran (1.0 ml) and, after adding OXONE (2.8 mg) and water (0.5 ml) at room temperature, the mixture was stirred for 1.5 hours. After adding a saturated aqueous solution of sodium hydrogencarbonate to the reaction mixture, extraction with ethyl acetate was conducted. The organic layer was washed with a saturated aqueous solution of sodium chloride and dried with magnesium sulfate; then, the solvent was distilled off at reduced pressure. Purification by silica gel column chromatography (developing solvents: ethyl acetate/n-hexane = 2/1) gave the end compound in 2.6 mg (yield, 85%).
1H-NMR(270MHz, $CDCl_3$)δ: 0.75(3H, s), 0.86(3H, s), 0.78-2.39(31H, m), 2.98-3.09(4H, m), 3.29-3.38(1H, m), 3.48-3.59(1H, m), 3.96(2H, t, J=5.9Hz), 6.72(2H, d, J=8.4Hz), 7.26(2H, brs).
Mass (EI): 676(M+).
The following compound was synthesized by a similar method to Example 153.

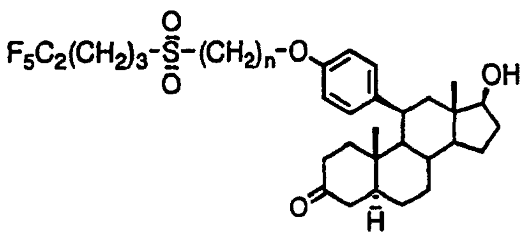

| Example | n | MW | Mass(FAB) |
|---|---|---|---|
| 154 | 7 | 704 | 705 |

[Example 155]

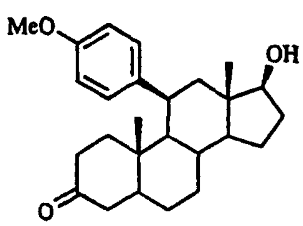

Synthesis of 17β-hydroxy-11β-(4-methoxyphenyl)-5α-androstan-3-one

To a solution of 3,3-ethylenedioxy-17β-methoxymethoxy-11β-(4-methoxyphenyl)-5α-androstane (5.8 mg) in acetone (2 ml), 1 N-hydrochloric acid (1 ml) was added and the mixture was heated under reflux for 1 hour. After standing to cool, a saturated aqueous solution of sodium hydrogencarbonate was added and extraction with ethyl acetate was conducted. The organic layer was washed with a saturated aqueous solution of sodium chloride and dried with magnesium sulfate; after filtering, the solvent was distilled off at reduced pressure. Purification by silica gel column chromatography (developing solvents: ethyl acetate/n-hexane = 1/1) gave the end compound in 4.6 mg (yield, 97%).
1H-NMR(270MHz, $CDCl_3$)δ: 0.75(3H, s), 0.86(3H, s), 0.82-2.30(21H, m), 3.31-3.39(1H, m), 3.48-3.59(1H, m), 3.79 (3H, s), 6.74(2H, d, J=8.7Hz), 7.28(2H, brs).
Mass (EI): 396(M+).

[Example 156]

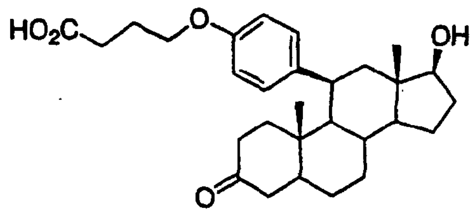

Synthesis of 17β-hydroxy-11β-[4-(3-carboxypropyloxy)phenyl]-5α-androstan-3-one

(Step 1)

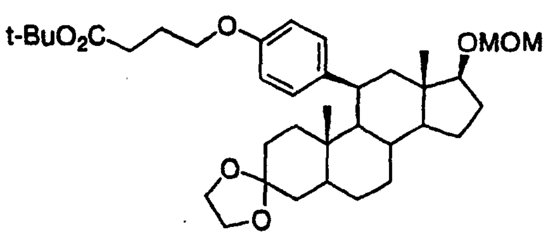

3,3-ethylenedioxy-17β-methoxymethoxy-11β-[4-(3-t-butoxycarbonylpropyloxy)phenyl]-5α-androstane

In a nitrogen atmosphere, 3,3-ethylenedioxy-17β-methoxymethoxy-11β-(4-hydroxyphenyl)-5α-androstane (30.2 mg), potassium carbonate (89 mg), 3-bromobutanoic acid t-butyl ester (0.029 ml), potassium iodide (21.3 mg) and 18-crown-6 (200 mg) were dissolved in N,N-dimethylacetamide (0.5 ml) and the mixture was stirred for 10 minutes at 60°C. After adding water to the reaction mixture, extraction was effected with a solvent system consisting of a mixture of ethyl acetate and hexane. The organic layer was dried with sodium sulfate and, after filtering, the solvents were distilled off at reduced pressure. The resulting residue was purified by silica gel column chromatography (developing solvents: ethyl acetate/n-hexane = 1/5) to give the end compound in 38.1 mg (yield, 97%).
1H-NMR(270MHz, $CDCl_3$)δ: 7.40-7.20(2H, m), 6.70(2H, d, J=8.4Hz), 4.54(2H, s), 3.96(2H, t, J=6.1Hz), 3.91(4H, s), 3.46(1H, dd, J=7.9, 8.1H2). 3.38-3.27(1H, m), 3.28(3H, s), 2.42(2H, t, J=7.3Hz), 2.20-0.82(22H, m), 1.45(9H, s), 0.76 (3H, s), 0.65(3H, s).
Mass (EI): 612(M+).

(Step 2)

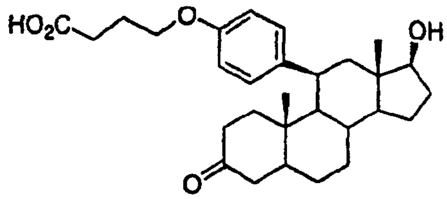

17β-hydroxy-11β-[4-(3-carboxypropyloxy)phenyl]-5α-androstan-3-one

3,3-Ethylenedioxy-17β-methoxymethoxy-11β-[4-(3-t-butoxycarbonylpropyloxy)phenyl]-5α-androstane (38.1 mg) was dissolved in acetone (1 ml) and, after adding 6 N-hydrochloric acid (0.5 ml), the mixture was heated under reflux for 20 minutes. After adding dichloromethane, the reaction mixture was dried and filtered and the solvent was distilled off at reduced pressure; purification by silica gel column chromatography (developing solvents: ethyl acetate/n-hexane: 1/1) gave the end compound in 29.0 mg (yield, 100%).
1H-NMR(300MHz, $CDCl_3$)δ: 7.40-7.10(2H, m), 6.72(2H, d, J=8.8Hz), 3.84(1H, brs), 3.99(2H, t, J=6.0Hz), 3.54(1H, dd, J=7.1, 8.5Hz), 3.33(1H, dd, J=5.8, 6.0Hz), 2.57(2H, t, J=7.1Hz), 2.28-1.86(11H, m), 1.74-1.16(9H, m), 1.08-0.90(2H, m), 0.85(3H, s), 0.74(3H, s).
Mass (ESI): 469(M+1).

The following compound was synthesized by a similar method to Example 156.

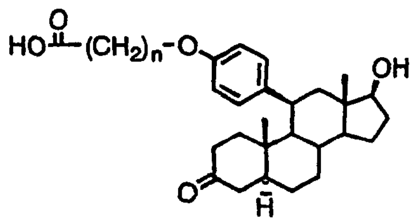

| Example | n | MW | Mass(FAB) |
|---|---|---|---|
| 157 | 7 | 524 | 525 |

[Example 158]

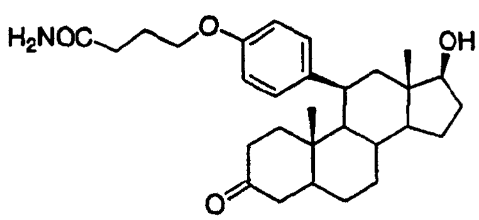

17β-hydroxy-11β-[4-(3-aminocarbonylpropyloxy)phenyl]-5α-androstan-3-one

The 17β-hydroxy-11β-[4-(3-carboxypropyloxy)phenyl]-5α-androstan-3-one (3.6 mg) obtained in Example 156 was dissolved in dichloromethane (0.2 ml) and, after adding triethylamine (5.4 µl) and ethyl chlorocarbonate (2.2 µl)

at -10°C, the mixture was stirred for 5 minutes. Ammonia gas was blown into the reaction mixture for 5 minutes and the mixture was stirred for 15 minutes at -10°C. After adding a saturated aqueous solution of sodium chloride to the reaction mixture, extraction with dichloromethane and drying with sodium sulfate were effected; after filtering, the solvent was distilled off at reduced pressure. The resulting residue was purified by silica gel column chromatography (developing solvent: ethyl acetate) to give the end compound in 3.6 mg (yield, 100%).

1H-NMR(300MHz, $CDCl_3$)δ: 7.40-7.18(2H, m), 6.73(2H, d, J=9.lHz), 5.48(2H, br), 4.01(2H, t, J=6.0Hz), 3.54(1H, dd, J=7.1, 8.5Hz), 3.34(1H, dd, J=6.0, 6.6Hz), 2.29-1.87(10H, m), 1.78-1.18(10H, m), 1.08-0.90(2H, m), 0.85(3H, s), 0.74 (3H, s).

Mass (ESI): 468(M+1).

The following compounds were synthesized by similar methods to Examle 158.

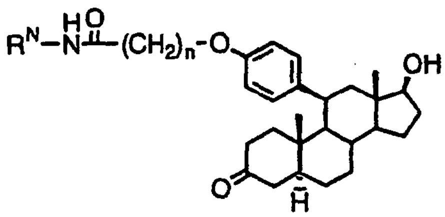

| Example | n | $R^N$ | MW | Mass |
|---|---|---|---|---|
| 159 | 3 | n-pentyl | 537 | 537(EI) |
| 160 | 7 | H | 523 | 524(ESI) |
| 161 | 7 | n-pentyl | 593 | 593(EI) |

[Example 162]

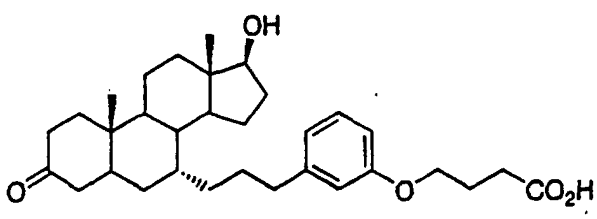

Synthesis of 17β-hydroxy-7α-[3-(3-carboxypropyloxyphenyl)propyl]-5α-androstan-3-one

(Step 1)

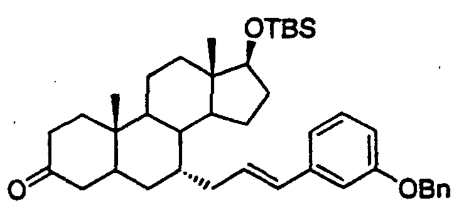

17β-(t-butyldimethylsilyloxy)-7α-[3-(3-benzyloxy)phenyl-2-propenyl]-5α-androstan-3-one

The 17β-(t-butyldimethylsilyloxy)-7α-(2-propen-1-yl)androstan-3-one (110 mg) obtained in step 1 of Example 3 was dissolved in dichloromethane (0.5 ml) and, after adding 3-benzyloxystyrene (156 mg) and benzylidenebis(tricyclohexylphosphine)-dichlororuthenium (10.0 mg), the mixture was heated under reflux for 24 hours in an argon atmosphere. After standing to cool, the reaction mixture was concentrated at reduced pressure and purified by silica gel column chromatography (developing solvents: ethyl acetate/n-hexane = 1/3) to give the end compound in 104.1 mg (yield, 67%).
1H-NMR(300MHz, $CDCl_3$)δ: 7.49-7.28(5H, m), 7.21(1H, dd, J=7.9, 8.0Hz), 6.98-6.90(2H, m), 6.82(1H, dd, J=1.4, 8.0Hz), 6.30(1H, d, J=5.7Hz), 6.14-6.00(1H, m), 5.07(2H, s), 3.57(1H, dd, J=8.0, 8.5Hz), 2.46-0.92(22H, m), 1.05(3H, s), 0.88(9H, s), 0.74(3H, s), 0.01(6H, s).
Mass (EI): 626(M+).

(Step 2)

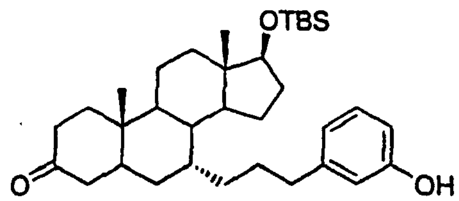

17β-(t-butyldimethylsilyloxy)-7α-[3-(3-hydroxyphenyl)propyl]-5α-androstan-3-one

17β-(t-Butyldimethylsilyloxy)-7α-[3-(3-benzyloxy)phenyl-2-propenyl]-5α-androstan-3-one (104,1 mg) was dissolved in ethyl acetate (20 ml) and, after adding acetic acid (0.2 ml) and 10%-palladium/carbon (20 mg), the mixture was stirred for 4 hours at 25 ° in a hydrogen atmosphere. After filtering the reaction mixture, the solvent was distilled off at reduced pressure to give the end compound in 79.8 mg (yield, 89%).
1H-NMR(300MHz, $CDCl_3$)δ: 7.18-7.09(1H, m), 6.73(1H, d, J=7.7Hz), 6.69-6.62(2H, m), 5.07(1H, s), 3.54(1H, dd, J=8.0, 8.8Hz), 2.66-2.20(5H, m), 2.07-0.85(21H, m), 1.02(3H, s), 0.88(9H, s), 0.70(3H, s), 0.010(3H, s), 0.008(3H, s).
Mass (EI): 538(M+).

(Step 3)

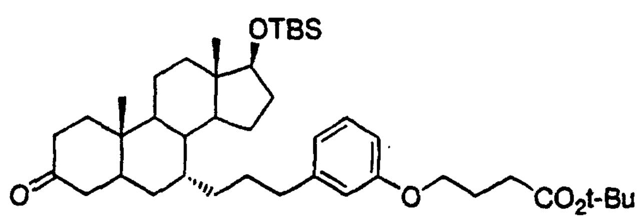

17β-(t-butyldimethylsilyloxy)-7α-[3-{3-(3-t-butoxycarbonylpropoxy)phenyl}propyl]-5α-androstan-3-one

In a nitrogen atmosphere, 17β-(t-butyldimethylsilyloxy)-7α-[3-(3-hydroxyphenyl)propyl]-5α-androstan-3-one (30.2 mg), potassium carbonate (62 mg), 3-bromobutanoic acid t-butyl ester (0.020 ml) and 18-crown-6 (100 mg) were dissolved in N,N-dimethylacetamide (0.2 ml) and the solution was stirred for 10 minutes at 60°C. After adding water to the reaction mixture, extraction was effected using a solvent system consisting of a mixture of ethyl acetate and hexane. The organic layer was dried with sodium sulfate and, after filtering, the solvents were distilled off at reduced

pressure. The resulting residue was purified by silica gel column chromatography (developing solvents: ethyl acetate/n-hexane = 1/5) to give the end compound in 24.8 mg (yield, 80%).
1H-NMR(300MHz, $CDCl_3$)δ: 7.22-7.13(1H, m), 6.79-6.67(3H, m), 3.98(2H, t, J=6.1Hz), 3.54(1H, dd, J=8.0, 8.5Hz), 2.65-2.14(5H, m), 2.43(2H, t, J=7.4Hz), 2.12-0.90(23H, m), 1.45(9H, s), 1.02(3H, s), 0.88(9H, s), 0.71(3H, s), 0.009 (3H, s), 0.005(3H, s).
Mass (EI): 567(M+).

(Step 4)

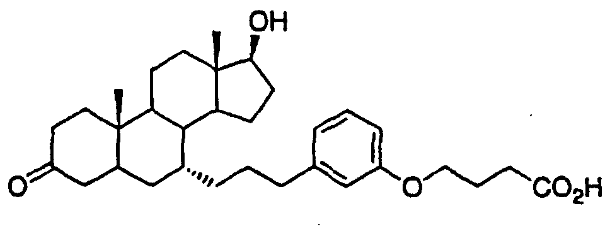

17β-hydroxy-7α-[3-{3-(3-carboxypropoxy)phenyl}propyl]-5α-androstan-3-one

17β-(t-Butyldimethylsilyloxy)-7α-[3-{3-(3-t-butoxycarbonylpropoxy)phenyl}propyl]-5α-androstan-3-one (24.8 mg) was dissolved in acetone (4 ml) and, after adding 6 N-hydrochloric acid (1 ml), the mixture was heated under reflux for 2 hours. After distilling off the solvent at reduced pressure, purification was effected by silica gel column chromatography (developing solvents: ethyl acetate/n-hexane = 2/1) to give the end compound in 19.0 mg (yield, 100%).
1H-NMR(300MHz, $CDCl_3$)δ: 7.16(1H, dd, J=7.1, 8.0Hz), 6.80-6.68(3H, m), 4.02(2H, dt, J=1.4, 6.3Hz), 3.63(1H, dd, J=8.2, 8.8Hz), 3.56(1H, br), 3.13-2.98(4H, m), 2.45-1.95(8H, m), 1.82-0.94(18, m), 1.03(3H, s), 0.74(3H, s).
Mass (ESI): 511(M+1).

The following compound was synthesized by a similar method to Example 162.

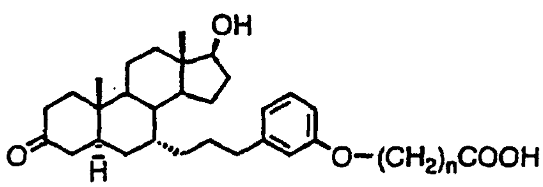

| Example | n | MW | Mass(FAB) |
|---|---|---|---|
| 163 | 4 | 524 | 525 |

[Example 164]

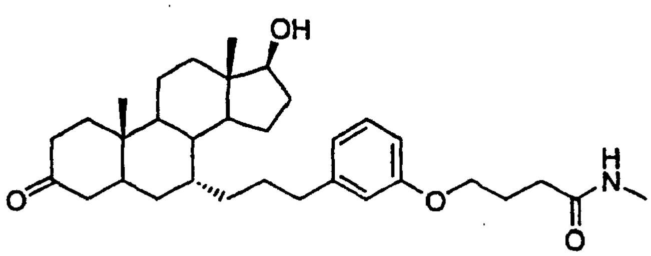

Synthesis of 17β-hydroxy-7α-[3-[3-{3-(N-methylaminocarbonyl)propoxy}phenyl]propyl]-5α-androstan-3-one

The 17β-hydroxy-7α-[3-{3-(3-carboxypropoxy)phenyl}propyl]-5α-androstan-3-one (6.6 mg) obtained in Example 162 was dissolved in tetrahydrofuran (0.5 ml) and, after adding 1-(N,N-dimethylaminopropyl)-3-ethylcarbodiimide hydrochloride (8.1 mg), 1-hydroxybenzotriazole monohydrate (6.4 mg) and methylamine 40% methanol solution (60 μl), the mixture was stirred for 18 hours at 25°C. After adding ethyl acetate (2.0 ml), the reaction mixture was washed with 1 N-hydrochloric acid, 1 N aqueous solution of sodium hydroxide, and a saturated aqueous solution of sodium chloride. The organic layer was dried with magnesium sulfate and, after filtering, the solvent was distilled off at reduced presure. The resulting residue was purified by silica gel column chromatography (developing solvents: ethyl acetate/n-hexane = 5/1) to give the end compound in 0.6 mg (yield, 8.8%).
1H-NMR(270MHz, $CDCl_3$)δ: 0.75(3H, s), 1.03(3H, s), 1.05-1.95(22H, m), 1.95-2.21(5H, m), 2.26-2.42(4H, m), 2.51-2.65(2H, m), 2.81(3H, d), 3.63(1H, t, J=8.1Hz), 3.99(2H, t, J=5.9Hz), 6.70(3H, m), 7.17(1H, dd).
Mass(ESI): 524(M+1).
Rf value (on silica gel plate, developing solvents: ethyl acetate/n-hexane = 4/1): 0.21.

The following compounds were synthesized by similar methods to Example 164.

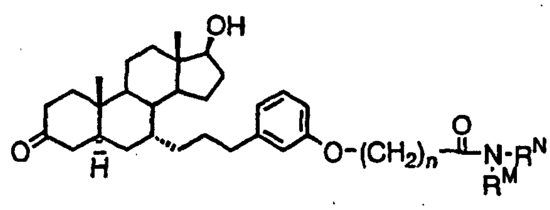

| Example | n | $R^M$ | $R^N$ | MW | Mass(ESI) |
|---|---|---|---|---|---|
| 165 | 4 | H | Me | 537 | 538 |
| 166 | 4 | Me | Me | 551 | 552 |
| 167 | 4 | $-(CH_2)_4-$ | | 577 | 578 |
| 168 | 3 | Me | Me | 537 | 538 |
| 169 | 3 | $-(CH_2)_4-$ | | 563 | 564 |

The following compounds were synthesized by similar methods to Example 114.

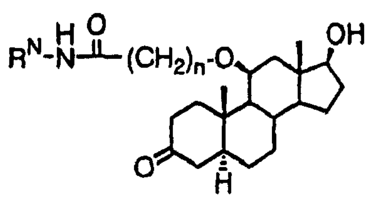

| Example | n | $R^N$ | MW | Mass(FAB) |
|---|---|---|---|---|
| 170 | 5 | H | 419 | 420 |
| 171 | 5 | n-pentyl | 489 | 490 |
| 172 | 7 | H | 447 | 548 |
| 173 | 7 | n-pentyl | 517 | 518 |
| 174 | 9 | H | 475 | 476 |
| 175 | 9 | n-pentyl | 545 | 546 |

(continued)

| Example | n | $R^N$ | MW | Mass(FAB) |
|---|---|---|---|---|
| 176 | 11 | H | 503 | 504 |
| 177 | 11 | n-pentyl | 573 | 574 |
| 178 | 13 | H | 531 | 532 |
| 179 | 13 | n-pentyl | 601 | 602 |

[Example 180] Evaluating the agonist and antagonist actions

The compound of Example 4 was evaluated for its agonist and antagonist actions in relation to the androgen receptor mediated transcriptional activity.

The agonist action was measured by the same method as described in Example 1; the agonist activity was computed by the following formula and the determined agonist activity was used to compute the $FI_5$ value (the concentration for a compound treated group at which it shows a transcriptional activity five times the transcriptional activity for the case where the compound is not added). The compound was added at concentrations of 1, 10, 100, 1000 and 10000 nmol/L.

Agonist activity = Transcriptional activity when the compound was added/Transcriptional activity when the compound was not added

The antagonist action was measured by the same method as described in Example 2; the antagonist activity was computed by the following formula and the determined antagonist activity was used to compute the $IC_{50}$ value (the concentration for a compound treated group at which it shows a 50% decrease in the transcriptional activity of DHT 0.1 nmol/L when the compound was not added). The compound was added at concentrations of 1, 10, 100, 1000 and 10000 nmol/L and at each of these concentrations, measurement was done in the presence of DHT (0.1 nmol/L).

Antagonist activity = Transcriptional activity when the compound was added/Transcriptional activity when the compound was not added x 100

| Compound | $IC_{50}$ value (nM) | $FI_5$ value (nM) |
|---|---|---|
| Compound of Example 9 | 451 | ND* |
| 10 | 937 | ND |
| 14 | 1984 | ND |
| 18 | 342 | ND |
| 19 | 295 | ND |
| 20 | 37 | ND |
| 23 | 1302 | ND |
| 24 | 477 | ND |
| 25 | 415 | ND |

* ND* in the table signifies that even when the compound was added at a concentration of 10000 nM, the transcriptional activity of the compound-treated group was less than 5 times the transcriptional activity of the control group, making it impossible to compute the $FI_5$ value.

(continued)

| Compound | $IC_{50}$ value (nM) | $FI_5$ value (nM) |
|---|---|---|
| 26 | 1128 | ND |
| 27 | 421 | ND |
| 28 | 1614 | ND |
| 29 | 304 | ND |
| 30 | 733 | ND |
| 42 | 342 | ND |
| 43 | 1299 | ND |
| 46 | 1751 | ND |
| 50 | 737 | ND |
| 51 | 474 | ND |
| 52 | 277 | ND |
| 64 | 809 | ND |
| 65 | 1831 | ND |
| 73 | 1099 | ND |
| 74 | 2036 | ND |
| 96 | 1601 | ND |
| 164 | 291 | ND |
| 167 | 475 | ND |
| 168 | 540 | ND |
| EM-101 | 2619 | ND |
| Hydroxyflutamide | 31 | 1000 |
| Bicaltamide | 136 | 767 |

The above test results verify that existing antiandrogenic agents, hydroxyflutamide and bicaltamide, also exhibit agonist action for the androgen receptor mediated transcriptional activity whereas the compounds of the invention are substantially free of such agonist action for the androgen receptor mediated transcriptional activity. Thus, it is suggested that the compounds of the invention can potentially reduce the development of androgen tolerance which has been a problem with the conventionally used antiandrogenic agents.
It has also been verified that the compounds of the invention have better agonist action than EM-101. Thus, it is suggested that the compounds of the invention have a sufficient antiandrogenic action to be used as pharmaceuticals and that they can advantageously be used as antiandrogenic agents.

## INDUSTRIAL APPLICABILITY

The compounds of the invention which are represented by the general formula (I) and the substances of the invention which act as antagonist against but not as agonist for the androgen receptor are potential antiandrogenic agents that do exhibit any side effects such as the development of androgen tolerance due to long-term administration and/or hepatoxicity and, hence, are expected to be useful as pharmaceutical compositions, say, therapeutics for diseases such as prostate cancer, prostatomegaly, male pattern alopecia, sexual prematurity, acne vulgaris, seborrhea and hursutism. If the compounds of the invention which are represented by the general formula (I) and the substances of the invention which act as antagonist against but not as agonist for the androgen receptor are preliminarily administered, the onset of diseases such as prostate cancer, prostatomegaly, male pattern alopecia, sexual prematurity, acne vulgaris, seborrhea and hursutism can hopefully be prevented or retarded, so they are also potential preventives of these diseases. Further, the compounds of the invention which are represented by the general formula (I) and the substances of the invention which act as antagonist against but not as agonist for the androgen receptor have toxicity

such as cytotoxicity sufficiently reduced that they are expected to find advantageous use as therapeutics and/or preventives of the diseases mentioned above.

## Claims

1. A compound represented by the general formula (I), pharmaceutically acceptable salts thereof, or prodrugs of the compound or its salts:

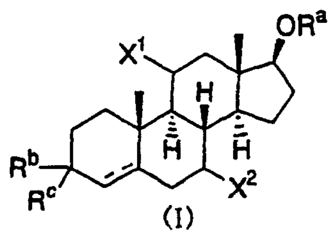

(I)

[wherein $X^1$ and $X^2$ represent independently a hydrogen atom or a group represented by the general formula (II)

$$-Ar-A-R^1 \quad (II)$$

$R^a$ represents a hydrogen atom or a protective group of a hydroxyl group, $R^b$ and $R^c$, when taken together with the carbon atom in 3-position to which they are bound, represent an optionally protected -(C=O)-, and the dashed line in combination with the solid line represents the formation of a single bond or a double bond;

in addition, Ar represents a single bond or an aromatic hydrocarbon group, A represents a methylene group or -O-, $R^1$ represents an optionally substituted alkyl group, an optionally substituted alkenyl group or an optionally substituted alkynyl group;

provided that $X^1$ and $X^2$ are not a hydrogen atom at the same time].

2. The compound according to claim 1, pharmaceutically acceptable salts thereof, or prodrugs of the compound or its salts, wherein $R^1$ is $R^{1a}$
[where $R^{1a}$ is the general formula (III)

$$-G-E-J-Y-L-Q-Z \quad (III)$$

{wherein G represents an optionally substituted straight-chained or branched alkylene group having 1 - 30 carbon atoms, an optionally substituted straight-chained or branched alkenylene groups having 2 - 30 carbon atoms or an optionally substituted straight-chained or branched alkynylene group having 2 - 30 carbon atoms, E represents a single bond or -O-, J represents a single bond, an optionally substituted aromatic hydrocarbon group or an optionally substituted heterocyclic group, Y represents a single bond or -O-, L represents a single bond, a straight-chained or branched alkylene group having 1 - 10 carbon atoms, a straight-chained or branched alkenylene group having 2 - 10 carbon atoms or a straight-chained or branched alkynylene group having 2 - 10 carbon atoms, Q represents a single bond or one group selected from among the following formulae:

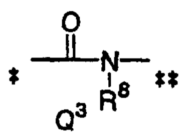
$Q^3$

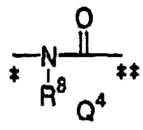
$Q^4$

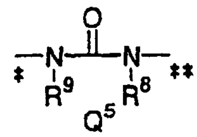
$Q^5$

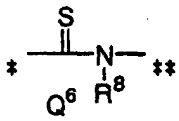
$Q^6$

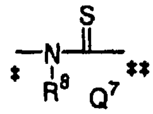
$Q^7$

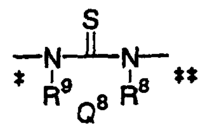
$Q^8$

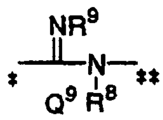
$Q^9$

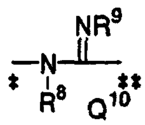
$Q^{10}$

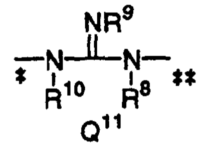
$Q^{11}$

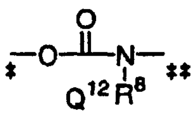
$Q^{12}$

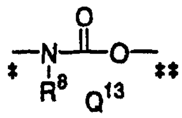
$Q^{13}$

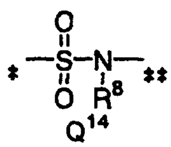
$Q^{14}$

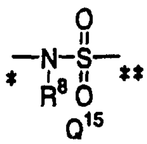
$Q^{15}$

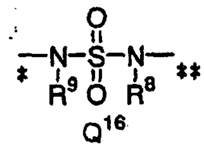
$Q^{16}$

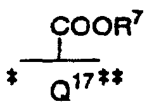
$Q^{17}$

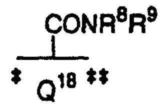
$Q^{18}$

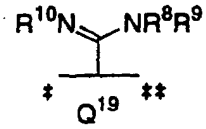
$Q^{19}$

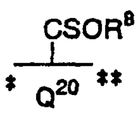
$Q^{20}$

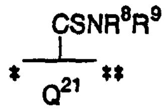
$Q^{21}$

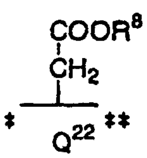
$Q^{22}$

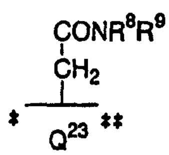
$Q^{23}$

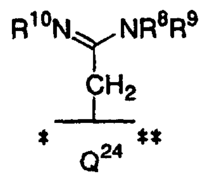
$Q^{24}$

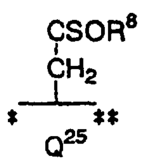
$Q^{25}$

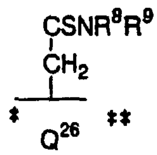
$Q^{26}$

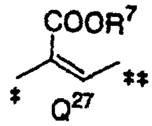
$Q^{27}$

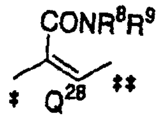
$Q^{28}$

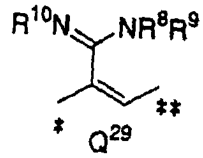
$Q^{29}$

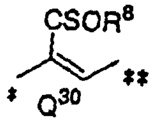
$Q^{30}$

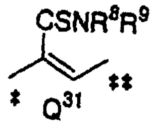
$Q^{31}$

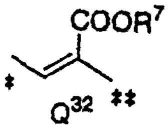
$Q^{32}$

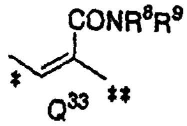
$Q^{33}$

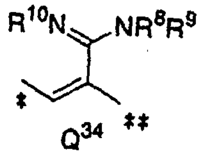
$Q^{34}$

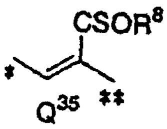
$Q^{35}$

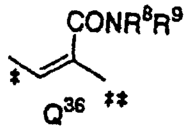
$Q^{36}$

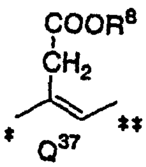
$Q^{37}$

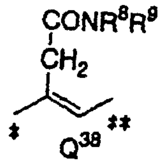
$Q^{38}$

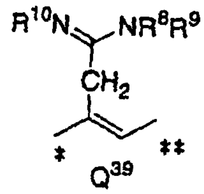
$Q^{39}$

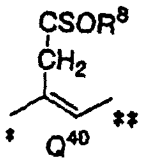
$Q^{40}$

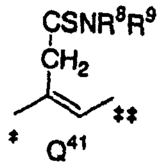
$Q^{41}$

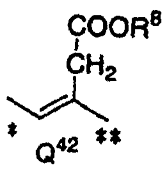
$Q^{42}$

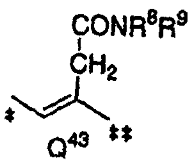
$Q^{43}$

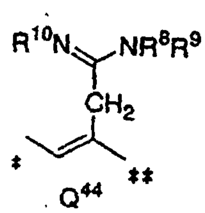
$Q^{44}$

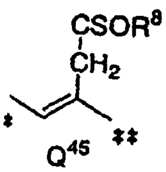
$Q^{45}$

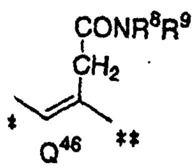
$Q^{46}$

and

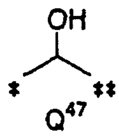
$Q^{47}$

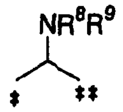
$Q^{48}$

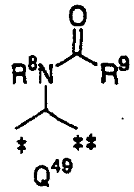
$Q^{49}$

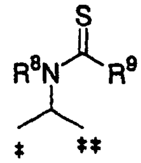
$Q^{50}$

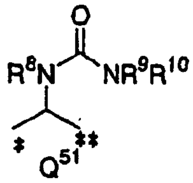
$Q^{51}$

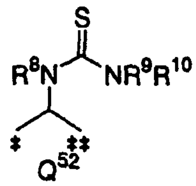
$Q^{52}$

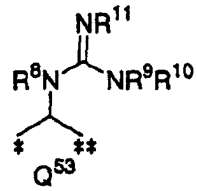
$Q^{53}$

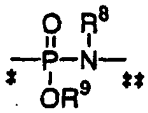
$Q^{54}$

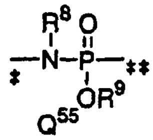
$Q^{55}$

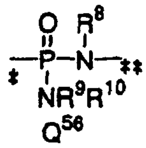
$Q^{56}$

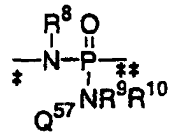
$Q^{57}$

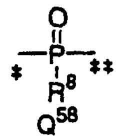
$Q^{58}$

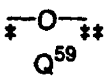
$Q^{59}$

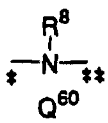
$Q^{60}$

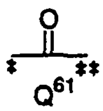
$Q^{61}$

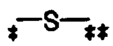
$Q^{62}$

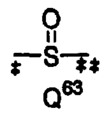
$Q^{63}$

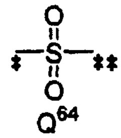
$Q^{64}$

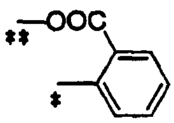
$Q^{65}$

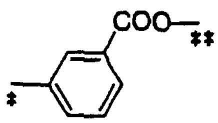
$Q^{66}$

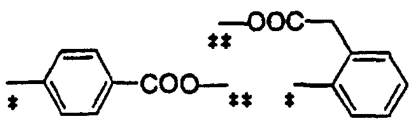
$Q^{67}$ $Q^{68}$

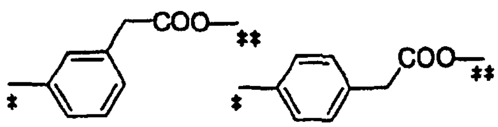
$Q^{69}$ $Q^{70}$

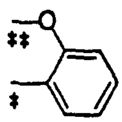
$Q^{71}$

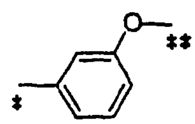
$Q^{72}$

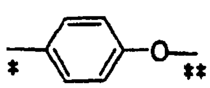
$Q^{73}$

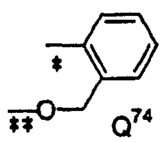
$Q^{74}$

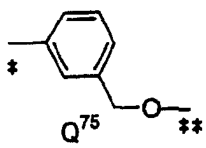
$Q^{75}$

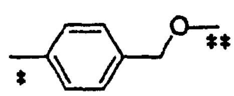
$Q^{76}$

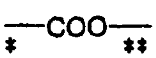
$Q^{77}$

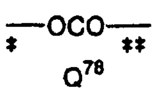
$Q^{78}$

(where $R^7$ and $R^8$ represent independently a hydrogen atom or a straight-chained or branched lower alkyl group having 1 - 6 carbon atoms, $R^9$, $R^{10}$ and $R^{11}$ each independently represent a hydrogen atom or a straight-chained or branched lower alkyl group having 1 - 3 carbon atoms), Z represents a hydrogen atom, a straight-chained or branched alkyl group having 1 - 10 carbon atoms that may optionally be substituted by a halogen atom, a straight-chained or branched alkenyl group having 2 - 10 carbon atoms that may optionally be substituted by a halogen atom, a straight-chained or branched alkynyl group having 2 - 10 carbon atoms that may optionally be substituted by a halogen atom, -O-$R^d$ (where $R^d$ represents a hydrogen atom or a protective group of a hydroxyl group), or -COOH), provided that when Q is $Q^3$, the nitrogen atom and $R^8$ in $Q^3$ may combine with Z to form a heterocyclic group}].

**3.** The compound according to claim 1 or 2, pharmaceutically acceptable salts thereof, or prodrugs of the compound or its salts, wherein Q is $Q^2$ [where $Q^2$ represents a single bond, $Q^{62}$, $Q^{63}$, $Q^{64}$, $Q^3$ (where $R^8$ has the same

meaning as defined above), $Q^4$ (where $R^8$ has the same meaning as defined above), $Q^{17}$ (where $R^7$ has the same meaning as defined above), $Q^{32}$ (where $R^7$ has the same meaning as defined above) or $Q^{27}$ (where $R^7$ has the same meaning as defined above).

**4.** The compound according to any one of claims 1 - 3, pharmaceutically acceptable salts thereof, or prodrugs of the compound or its salts, wherein $X^1$ is -Ar-A-$R^1$ (wherein Ar, A and $R^1$ have the same meanings as defined above) and $X^2$ is a hydrogen atom.

**5.** The compound according to any one of claims 1 - 3, pharmaceutically acceptable salts thereof, or prodrugs of the compound or its salts, wherein $X^1$ is a hydrogen atom and $X^2$ is -Ar-A-$R^1$ (wherein Ar, A and $R^1$ have the same meanings as defined above).

**6.** The compound according to any one of claims 1 - 5, pharmaceutically acceptable salts thereof, or prodrugs of the compound or its salts, wherein the dashed line forms a single bond together with the solid line.

**7.** The compound according to claim 1, 2, 3, 4 or 6, pharmaceutically acceptable salts thereof, or prodrugs of the compound or its salts, wherein the steric configuration of $X^1$ in 11-position is β-configuration.

**8.** The compound according to claim 1, 2, 3, 5 or 6, pharmaceutically acceptable salts thereof, or prodrugs of the compound or its salts, wherein the steric configuration of $X^2$ in 7-position is α-configuration.

**9.** The compound according to any one of claims 1 - 8, pharmaceutically acceptable salts thereof, or prodrugs of the compound or its salts, wherein Z is a straight-chained or branched alkyl group having 1 - 10 carbon atoms which may optionally be substituted by a halogen atom.

**10.** The compound according to claim 9, pharmaceutically acceptable salts thereof, or prodrugs of the compound or its salts, wherein Z is a 4,4,5,5,5-pentafluoropentyl group.

**11.** The compound according to any one of claims 1 - 10, pharmaceutically acceptable salts thereof, or prodrugs of the compound or its salts, wherein J is a single bond.

**12.** The compound according to any one of claims 1 - 11, pharmaceutically acceptable salts thereof, or prodrugs of the compound or its salts, wherein Ar is a single bond.

**13.** The compound according to any one of claims 1 - 12, pharmaceutically acceptable salts thereof, or prodrugs of the compound or its salts, wherein A is a methylene group.

**14.** The compound according to any one of claims 1 - 13, pharmaceutically acceptable salts thereof, or prodrugs of the compound or its salts, wherein Q is $Q^{62}$, $Q^{63}$ or $Q^{64}$.

**15.** The compound according to any one of claims 1 - 13, pharmaceutically acceptable salts thereof, or prodrugs of the compound or its salts, wherein Q is $Q^3$ where $R^8$ is a hydrogen atom or $Q^4$ where $R^8$ is a hydrogen atom.

**16.** The compound according to any one of claims 1 - 13, pharmaceutically acceptable salts thereof, or prodrugs of the compound or its salts, wherein Q is $Q^{17}$ where $R^7$ is a hydrogen atom, $Q^{32}$ where $R^7$ is a hydrogen atom or $Q^{27}$ where $R^7$ is a hydrogen atom.

**17.** The compound according to any one of claims 1 - 11, pharmaceutically acceptable salts thereof, or prodrugs of the compound or its salts, wherein Ar is an aromatic hydrocarbon group and A is -O-.

**18.** The compound according to any one of claims 1 - 17, pharmaceutically acceptable salts thereof, or prodrugs of the compound or its salts, wherein G is an optionally substituted straight-chained alkylene group having 2 - 15 carbon atoms.

**19.** The compound according to claim 18, pharmaceutically acceptable salts thereof, or prodrugs of the compound or its salts, wherein G is an optionally substituted straight-chained alkylene group having 2 - 13 carbon atoms.

**20.** The compound or substance according to claim 1, pharmaceutically acceptable salts thereof, or prodrugs of the

compound or substance or their salts, wherein $X^2$ is any one group selected from the group consisting of $-(CH_2)_pCO-NR^8Z^1$ (p represents an integer of at least 1, $R^8$ represents a hydrogen atom, a straight-chained or branched lower alkyl group having 1 - 6 carbon atoms, and $Z^1$ represents a hydrogen atom or a straight-chained or branched alkyl group having 1 - 10 carbon atoms that may optionally be substituted by a halogen atom), $-(CH_2)_p-SO_2-Z^1$ (p and $Z^1$ have the same meanings as defined above), $-(CH_2)_p-SO-Z^1$ (p and $Z^1$ have the same meanings as defined above), $-Ph-O-(CH_2)_p-CO-NR^8Z^1$ (Ph represents a phenylene group and p, $R^8$ and $Z^1$ have the same meanings as defined above), and $-Ph-O-(CH_2)_p-H$ (p has the same meaning as defined above).

**21.** The compound or substance according to claim 1, pharmaceutically acceptable salts thereof, or prodrugs of the compound or substance or their salts, wherein $X^2$ is any one group selected from the group consisting of $-(CH_2)_p-COOH$ (p is an integer of at least 1), $-(CH_2)_p-OH$ (p has the same meaning as defined above), $-Ph-O-(CH_2)_p-COOH$ (Ph represents a phenylene group and p has the same meaning as defined above), $-(CH_2)_p-CO-NR^8Z^2$ (p has the same meaning as defined above, $R^8$ represents a hydrogen atom or a straight-chained or branched lower alkyl group having 1 - 6 carbon atoms, $Z^2$ represents a straight-chained or branched alkyl group having 1 - 10 carbon atoms that is substituted by any one group selected from the group consisting of a cycloalkyl group, a hydroxyl group, a carboxyl group, a heterocyclic group and a phenyl group, or $-NR^3Z^2$ may be such that N, $R^8$ and $Z^2$ combine together to form a hetero ring), $-(CH_2)_p-Ph-O-(CH_2)_q-CO-NR^8Z^3$ (Ph, p and $R^8$ have the same meanings as defined above, q represents an integer of at least 1, and $Z^3$ represents a hydrogen atom or a straight-chained or branched alkyl group having 1 - 10 carbon atoms that may optionally be substituted by any one group selected from the group consisting of a cycloalkyl group, a hydroxyl group, a carboxyl group, a heterocyclic group and a phenyl group, or
$-NR^8Z^3$ may be such that N, $R^8$ and $Z^3$ combine together to form a hetero ring) and $-(CH_2)_p-CH(COOH)-(CH_2)_3-CF_2-CF_3$ (p has the same meaning as defined above).

**22.** The compound or substance according to claim 1, pharmaceutically acceptable salts thereof, or prodrugs of the compound or substance or their salts, wherein $X^1$ is any one group selected from the group consisting of $-(CH_2)_p-COOH$ (p is an integer of at least 1), $-(CH_2)_p-CH(COOH)-(CH_2)_3-CF_2-CF_3$ (p has the same meaning as defined above), $-(CH_2)_p-CH(COOMe)-(CH_2)_3-CF_2-CF_3$ (p has the same meaning as defined above), $-O-(CH_2)_p-COOH$ (p has the same meaning as defined above), $-O-(CH_2)_p-CH(COOH)-(CH_2)_3-CF_2-CF_3$ (p has the same meaning as defined above), $-(CH_2)_p-S-(CH_2)_3-CF_2-CF_3$ (p has the same meaning as defined above), $-(CH_2)_p-SO-(CH_2)_3-CF_2-CF_3$ (p has the same meaning as defined above), $-O-(CH_2)_p-SO-(CH_2)_3-CF_2-CF_3$ (p has the same meaning as defined above), $-O-(CH_2)_p-SO_2-(CH_2)_3-CF_2-CF_3$ (p has the same meaning as defined above), $-Ph-O-CH_3$ (Ph represents a phenylene group), $-Ph-O-(CH_2)_p-COOH$ (Ph and p have the same meanings as defined above), $-(CH_2)_p-CO-NR^8Z^3$ (p has the same meaning as defined above, $R^8$ represents a hydrogen atom or a straight-chained or branched lower alkyl group having 1 - 6 carbon atoms, $Z^3$ represents a hydrogen atom or a straight-chained or branched alkyl group having 1 - 10 carbon atoms that may optionally be substituted by any one group selected from the group consisting of a cycloalkyl group, a hydroxyl group, a carboxyl group, a heterocyclic group and a phenyl group, or $-NR^8Z^3$ may be such that N, $R^8$ and $Z^3$ combine together to form a hetero ring), $-Ph-O-(CH_2)_p-CO-NR^8Z^3$ (Ph, p, $R^8$, $Z^3$ and $-NR^8Z^3$ have the same meanings as defined above) and $-O-(CH_2)_p-CO-NR^8Z^3$ (p, $R^8$, $Z^3$ and $-NR^8Z^3$ have the same meanings as defined above).

**23.** The compound according to any one of claims 1 - 3, pharmaceutically acceptable salts thereof, or prodrugs of the compound or its salts, which is selected from among 17β-hydroxy-7α-{7-(N,N-dimethylaminocarbonyl)heptyl}-5α-androstan-3-one;
17β-hydroxy-7α-{7-(N-ethylaminocarbonyl)heptyl}-5α-androstan-3-one;
17β-hydroxy-7α-[7-(N-(isopropylaminocarbonyl)heptyl]-5α-androstan-3-one;
17β-hydroxy-7α-[7-(N-methyl-N-butylaminocarbonyl)heptyl]-5α-androstan-3-one;
17β-hydroxy-7α-[7-(N,N-diethylaminocarbonyl)heptyl]-5α-androstan-3-one;
17β-hydroxy-7α-[7-(piperidinocarbonyl)heptyl]-5α-androstan-3-one;
17β-hydroxy-7α-[7-{N-(2-furylmethyl)aminocarbonyl}heptyl]-5α-androstan-3-one;
17β-hydroxy-7α-[7-{7-(N-methylaminocarbonyl)heptyl]-5α-androstan-3-one;
17β-hydroxy-7α-[7-(N-methyl-N-ethylaminocarbonyl)heptyl]-5α-androstan-3-one;
17β-hydroxy-7α-[7-(N-methyl-N-propylaminocarbonyl)heptyl]-5α-androstan-3-one;
17β-hydroxy-7α-[7-(N-methyl-N-isopropylaminocarbonyl)heptyl]-5α-androstan-3-one;
17β-hydroxy-7α-[7-(N-methyl-N-benzylaminocarbonyl)heptyl]-5α-androstan-3-one;
17β-hydroxy-7α-[7-(1-pyrrolidinylcarbonyl)heptyl]-5α-androstan-3-one;
17β-hydroxy-7α-[7-(morpholinocarbonyl)heptyl]-5α-androstan-3-one;
17β-hydroxy-7α-[9-(N,N-dimethylaminocarbonyl)nonyl]-5α-androstan-3-one;

17β-hydroxy-7α-[9-(N,N-diethylaminocarbonyl)nonyl]-5α-androstan-3-one;
17β-hydroxy-7α-[9-(N-methyl-N-butylaminocarbonyl)nonyl]-5α-androstan-3-one;
17β-hydroxy-7α-[9-(N-methyl-N-propylaminocarbonyl)nonyl]-5α-androstan-3-one;
17β-hydroxy-7α-[9-(morpholinocarbonyl)nonyl]-5α-androstan-3-one;
17β-hydroxy-7α-[10-(N,N-dimethylaminocarbonyl)decyl]-5α-androstan-3-one;
17β-hydroxy-7α-[7-{N-(2-hydroxyethyl)aminocarbonyl}heptyl]-5α-androstan-3-one;
17β-hydroxy-7α-[7-(N-propylaminocarbonyl)heptyl]-5α-androstan-3-one;
17β-hydroxy-7α-[7-(N-benzylaminocarbonyl)heptyl]-5α-androstan-3-one:
17β-hydroxy-7α-(7-{N-(2-phenylethyl)aminocarbonyl)heptyl]-5α-androstan-3-one;
17β-hydroxy-11β-[9-(N,N-diethylaminocarbonyl)nonyl]-5α-androstan-3-one;
17β-hydroxy-7α-[3-[3-{3-(N-methylaminocarbonyl)propoxy}phenyl]propyl]-5α-androstan-3-one;
17β-hydroxy-7α-[3-[3-{3-(N,N-dimethylaminocarbonyl)propoxy}phenyl]propyl]-5α-androstan-3-one; and
17β-hydroxy-7α-[3-[3-{4-(1-pyrrolidinylcarbonyl)butoxy}phenyl]propyl]-5α-androstan-3-one.

**24.** A substance which acts as antagnoist against but not as agonist for the androgen receptor, or pharmaceutically acceptable salts thereof, or prodrugs of the substance or its salts.

**25.** A pharmaceutical composition containing as an active ingredient the compound or substance according to any one of claims 1 - 24, or pharmaceutically acceptable salts thereof, or prodrugs of the compound or substance or their salts.

**26.** An antiandrogenic agent containing as an active ingredient the compound or substance according to any one of claims 1 - 24, or pharmaceutically acceptable salts thereof, or prodrugs of the compound or substance or their salts.

**27.** An agent for preventing or treating a disease selected from prostate cancer, prostatomegaly, male pattern alopecia, sexual prematurity, acne vulgaris, seborrhea and hursutism, said agent containing as an active ingredient the compound or substance according to any one of claims 1 - 24, or pharmaceutically acceptable salts thereof, or prodrugs of the compound or substance or their salts.

## INTERNATIONAL SEARCH REPORT

International application No.
PCT/JP00/05636

A. CLASSIFICATION OF SUBJECT MATTER
Int.Cl[7] C07J1/00, 31/00, 41/00, A61K31/568

According to International Patent Classification (IPC) or to both national classification and IPC

B. FIELDS SEARCHED

Minimum documentation searched (classification system followed by classification symbols)
Int.Cl[7] C07J1/00, 31/00, 41/00, A61K31/568

Documentation searched other than minimum documentation to the extent that such documents are included in the fields searched

Electronic data base consulted during the international search (name of data base and, where practicable, search terms used)
CA(STN)

C. DOCUMENTS CONSIDERED TO BE RELEVANT

| Category* | Citation of document, with indication, where appropriate, of the relevant passages | Relevant to claim No. |
|---|---|---|
| A | WO, 9100732, A (ENDORECHERCHE INC.), 24 January, 1991 (24.01.91) & EP, 485392, A | 1-27 |
| A | US, 5227375, A (Endorecherche, Inc.), 13 July, 1993 (13.07.93) & WO, 9112206, A | 1-27 |
| A | US, 3413287, A (G.D.Searle & Co.), 26 November, 1968 (26.11.68) (Family: none) | 1-27 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 September, 2000 (12.09.00) | 26 September, 2000 (26.09.00) |
| Name and mailing address of the ISA/ Japanese Patent Office | Authorized officer |
| Facsimile No. | Telephone No. |

Form PCT/ISA/210 (second sheet) (July 1992)